# EUROPEAN PATENT APPLICATION

(11) **EP 4 501 930 A1**
(43) Date of publication of application: **05.02.2025**
(21) Application number: 23778490.5
(22) Date of filing: 31.03.2023
(51) Int. Cl.: C07D 471/04, C07D 213/74, C07D 401/04, C07C 237/40, C07D 277/42, C07D 233/60, C07D 401/06, C07D 239/42, C07D 213/75, A61P 35/00, A61P 35/02, C12Q 1/6886, C12Q 1/02

(54) **AROMATIC RING COMPOUND AND USE THEREOF**

(30) Priority: 31.03.2022 CN 202210329428
(71) Applicant: Shanghai Shijiang Biotechnology Co., Ltd, Shanghai 200333 (CN)
(72) Inventor: SHI, Yufeng, Nanjing, Jiangsu 210032 (CN); MA, Wenjiang, Nanjing, Jiangsu 210032 (CN); WANG, Yingcai, Nanjing Jiangsu 210032 (CN)
(74) Representative: reuteler & cie SA
(86) International application number: PCT/CN2023/085584
(87) International publication number: WO 2023/186126

(57) **Abstract**

The present invention relates to an aromatic ring compound and use thereof. Specifically proyided is a compound of formula I, or an optical isomer thereof, or a racemate thereof, or a solvate thereof, or a pharmaceutically acceptable salt thereof, or a deuterated compound thereof. The compound has a significantly excellent therapeutic effect on tumors with low expression, no expression, low activity or no activity of mitochondrial permeablity transition pores; low expression, no expression, low activity or no activity of peptidyl-prolyl isomerase F; low expression or no expression of the NNMT gene; high expression of DNA methylases; high expression of UHRFl; high levels of methylation of nucleotide sites in the NNM'T gene; and/or high levels of methylation of DNA CpG sites in the NNMT gene region.

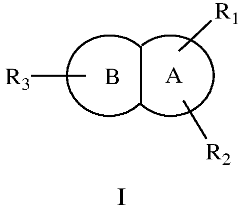

## Description

### FIELD OF THE INVENTION

The present invention relates to the field of medicine. Specifically, the present invention relates to aromatic ring compound and use thereof.

### BACKGROUND TECHNOLOGY

Tumor is a common disease that seriously endangers human health, and the mortality rate of malignant tumors is rising. Due to the heterogeneity of tumor, simply using the same treatment method or medication based on source or pathological characteristic of tumors can easily lead to improper treatment, which can delay valuable treatment time and opportunities for patient. Therefore, it is very necessary to take precision treatment according to the different features of tumor. With the development of biological technology, tumors are typed at the molecular level such as gene and protein level, etc, and more and more changes in tumor-related gene and the expression and activity of protein have been discovered, changes in tumor-related gene and the expression and activity of protein play an important role in the development of malignant tumors, the discovery and application of biomarkers can provide precise guidance for the application of related drug and make precision treatment of tumor possible, thereby achieving targeted administration of drug, significantly improving treatment effect, reducing the dose of drug and reducing toxic side effects.

Therefore, there is an urgent need in the art to develop a drug that can achieve precision treatment on tumor.

### SUMMARY OF THE INVENTION

The present invention provide a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In the first aspect of the present invention, it provides a compound of formula I, or an optical isomer thereof, or a racemate thereof, or a solvate thereof, or a pharmaceutically acceptable salt thereof, or a deuterated compound thereof 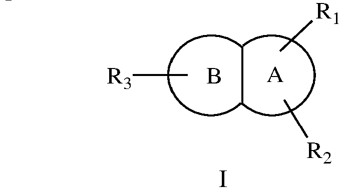 wherein,
ring A is substituted or unsubstituted C6-C14 aromatic ring, substituted or unsubstituted C3-C14 cycloalkane ring, substituted or unsubstituted 3-14 membered heterocycloalkane ring, or substituted or unsubstituted 5-14 membered heteroaromatic ring;
ring B is absent, substituted or unsubstituted C6-C14 aromatic ring, substituted or unsubstituted C3-C14 cycloalkane ring, substituted or unsubstituted 3-14 membered heterocycloalkane ring, or substituted or unsubstituted 5-14 membered heteroaromatic ring;
R₁ is 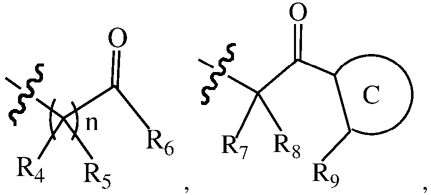 substituted or unsubstituted C6-C16 aryl-substituted or unsubstituted C1-C6 alkyl-;
ring C is substituted or unsubstituted C6-C16 aromatic ring, substituted or unsubstituted C3-C16 cycloalkane ring, substituted or unsubstituted 3-16 membered heterocycloalkane ring, or substituted or unsubstituted 5-16 membered heteroaromatic ring;
R₂ is hydrogen, 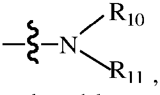 substituted or unsubstituted C6-C12 aryl, substituted or unsubstituted 5-12 membered heteroaryl, substituted or unsubstituted C6-C12 aryl-substituted or unsubstituted C1-C8 alkyl-, substituted or unsubstituted 5-12 membered heteroaryl-substituted or unsubstituted C1-C8 alkyl-;
R₃ is absent, hydrogen, halogen, substituted or unsubstituted C1-C10 alkyl, substituted or unsubstituted C3-C12 cycloalkyl, substituted or unsubstituted C3-C12 cycloalkyl-substituted or unsubstituted C1-C8 alkyl-, substituted or unsubstituted C1-C8 alkoxyl, substituted or unsubstituted C1-C8 alkylthio, substituted or unsubstituted C2-C8 acyl, substituted or unsubstituted C2-C8 acyl-substituted or unsubstituted C1-C8 alkyl-, substituted or unsubstituted C6-C14 aryl, substituted or unsubstituted 5-12 membered heteroaryl, substituted or unsubstituted C6-C14 aryl-sulfuryl-, substituted or unsubstituted C6-C14 aryl-C(O)-, substituted or unsubstituted C6-C14 aryl-C(O)-substituted or unsubstituted C1-C8 alkyl-, substituted or unsubstituted C6-C14 aryl-substituted or unsubstituted C1-C8 alkyl-, substituted or unsubstituted 5-12 membered heteroaryl-substituted or unsubstituted C1-C8 alkyl-;
R₄ and R₅ are each independently hydrogen, substituted or unsubstituted C1-C10 alkyl, substituted or unsubstituted C3-C12 cycloalkyl;
R₆ is substituted or unsubstituted C6-C14 aryl, substituted or unsubstituted 5-12 membered heteroaryl, substituted or unsubstituted C3-C14 cycloalkyl, substituted or unsubstituted 3-14 membered heterocycloalkyl;
R₇ is hydrogen, substituted or unsubstituted C1-C8 alkyl, substituted or unsubstituted C3-C10 cycloalkyl;
R₈ and R₉ are connected to form substituted or unsubstituted C3-C14 cycloalkane ring, substituted or unsubstituted 3-14 membered heterocycloalkane ring;
R₁₀ and R₁₁ are each independently hydrogen, substituted or unsubstituted C1-C10 alkyl, substituted or unsubstituted C2-C10 acyl, substituted or unsubstituted C6-C14 aryl, substituted or unsubstituted 5-12 membered heteroaryl, substituted or unsubstituted C6-C14 aryl-substituted or unsubstituted C1-C6 alkyl-, substituted or unsubstituted C6-C14 aryl-C(O)-substituted or unsubstituted C1-C6 alkyl-, substituted or unsubstituted 5-12 membered heteroaryl-substituted or unsubstituted C1-C6 alkyl-; or R₁₀ and R₁₁ are connected to form substituted or unsubstituted C3-C12 cycloalkane ring, substituted or unsubstituted 3-12 membered heterocycloalkane ring, substituted or unsubstituted 5-12 membered heteroaromatic ring;
n is 0, 1, 2, 3, 4 or 5.

In another preferred embodiment, each "substituted" means that one or more (preferably 1, 2, 3, 4, 5, 6, 7, or 8) hydrogen atoms on the ring or group are independently substituted by substituent selected from the group consisting of C1-C8 alkyl, C3-C8 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C6 alkyl, C3-C8 cycloalkyl, C1-C6 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C6 alkoxyl, C1-C6 alkylthio, C1-C6 haloalkoxyl, C1-C6 haloalkylthio, C6-C12 aryl, 5-10 membered heteroaryl, 5-10 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C5 alkyl, C3-C7 cycloalkyl, C1-C5 haloalkyl, C3-C7 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C5 carboxyl, C2-C5 ester group, C2-C5 amide group, C1-C5 alkoxyl, C1-C5 alkylthio, C1-C5 haloalkoxyl, C1-C5 haloalkylthio, C6-C8 aryl, 5-8 membered heteroaryl, 5-8 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C4 alkyl, C3-C8 cycloalkyl, C1-C4 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C4 alkoxyl, C1-C4 alkylthio, C1-C4 haloalkoxyl, C1-C4 haloalkylthio, C6-C12 aryl, 5-10 membered heteroaryl, 5-10 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C3 alkyl, C3-C7 cycloalkyl, C1-C3 haloalkyl, C3-C7 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C3 carboxyl, C2-C4 ester group, C2-C4 amide group, C1-C3 alkoxyl, C1-C3 alkylthio, C1-C3 haloalkoxyl, C1-C3 haloalkylthio, C6-C10 aryl, 5-8 membered heteroaryl, 5-8 membered heterocycloalkyl.

In another preferred embodiment, the heterocyclic ring of the heterocycloalkyl, heteroaryl, heterocycloalkane ring and heteroaromatic ring has 1-4 (preferably 1, 2, 3 or 4) heteroatoms independently selected from the group consisting of N, O and S.

In another preferred embodiment, the heterocyclic ring of the heterocycloalkyl has 1-4 (preferably 1, 2, 3 or 4) heteroatoms independently selected from the group consisting of N, O and S.

In another preferred embodiment, the heterocyclic ring of the heteroaryl has 1-4 (preferably 1, 2, 3 or 4) heteroatoms independently selected from the group consisting of N, O and S.

In another preferred embodiment, the heterocyclic ring of the heterocycloalkane ring has 1-4 (preferably 1, 2, 3 or 4) heteroatoms independently selected from the group consisting of N, O and S.

In another preferred embodiment, the heterocyclic ring of the heteroaromatic ring has 1-4 (preferably 1, 2, 3 or 4) heteroatoms independently selected from the group consisting of N, O and S.

In another preferred embodiment, each "substituted" means that one or more (preferably 1, 2, 3, 4, 5, 6, 7 or 8) hydrogen atoms on the ring or group are each independently substituted by a substituent.

In another preferred embodiment, the heterocycloalkyl has 0, 1 or 2 C=C ring double bonds.

In another preferred embodiment, the halogen is F, Cl, Br, or I.

In another preferred embodiment, the cycloalkane ring has 1, 2, or 3 C=C ring double bonds.

In another preferred embodiment, the heterocycloalkane ring has 1, 2, or 3 C=C ring double bonds.

In another preferred embodiment, ring A is substituted or unsubstituted C6-C12 aromatic ring, substituted or unsubstituted C3-C12 cycloalkane ring, substituted or unsubstituted 3-12 membered heterocycloalkane ring, or substituted or unsubstituted 5-12 membered heteroaromatic ring.

In another preferred embodiment, ring A is substituted or unsubstituted C6-C9 aromatic ring, substituted or unsubstituted C3-C9 cycloalkane ring, substituted or unsubstituted 3-9 membered heterocycloalkane ring, or substituted or unsubstituted 5-9 membered heteroaromatic ring.

In another preferred embodiment, ring A is substituted or unsubstituted C6-C10 aromatic ring, substituted or unsubstituted C3-C10 cycloalkane ring, substituted or unsubstituted 3-10 membered heterocycloalkane ring, or substituted or unsubstituted 5-12 membered heteroaromatic ring.

In another preferred embodiment, ring A is substituted or unsubstituted C6-C8 aromatic ring, substituted or unsubstituted C3-C8 cycloalkane ring, substituted or unsubstituted 3-8 membered heterocycloalkane ring, or substituted or unsubstituted 5-12 membered heteroaromatic ring.

In another preferred embodiment, ring A is substituted or unsubstituted C6-C10 aromatic ring, substituted or unsubstituted C3-C10 cycloalkane ring, substituted or unsubstituted 3-10 membered heterocycloalkane ring, or substituted or unsubstituted 5-10 membered heteroaromatic ring.

In another preferred embodiment, ring A is substituted or unsubstituted C6-C8 aromatic ring, substituted or unsubstituted C3-C8 cycloalkane ring, substituted or unsubstituted 3-8 membered heterocycloalkane ring, or substituted or unsubstituted 5-8 membered heteroaromatic ring.

In another preferred embodiment, ring A is substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring, substituted or unsubstituted 9 membered heteroaromatic ring, substituted or unsubstituted 10 membered heteroaromatic ring, substituted or unsubstituted 11 membered heteroaromatic ring, substituted or unsubstituted 12 membered heteroaromatic ring.

In another preferred embodiment, ring A is substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring, substituted or unsubstituted 9 membered heteroaromatic ring, substituted or unsubstituted 10 membered heteroaromatic ring, substituted or unsubstituted 11 membered heteroaromatic ring, substituted or unsubstituted 12 membered heteroaromatic ring.

In another preferred embodiment, ring A is substituted or unsubstituted pyridine ring, substituted or unsubstituted pyrimidine ring, substituted or unsubstituted quinoline ring, substituted or unsubstituted pyrazole ring, substituted or unsubstituted pyrrole ring, substituted or unsubstituted thiazol ring, substituted or unsubstituted imidazol ring.

In another preferred embodiment, R₁ is connected to the ring heteroatom in the ring A.

In another preferred embodiment, R₁ is connected to the ring N atom in the ring A.

In another preferred embodiment, R₁ is 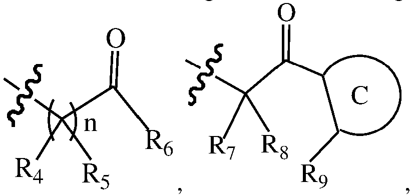 substituted or unsubstituted C6-C12 aryl-substituted or unsubstituted C1-C6 alkyl-.

In another preferred embodiment, R₁ is 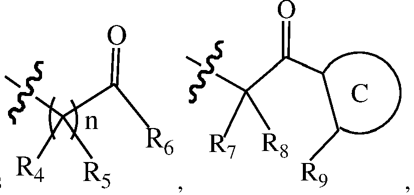 substituted or unsubstituted C6-C10 aryl-substituted or unsubstituted C1-C6 alkyl-.

In another preferred embodiment, R₁ is 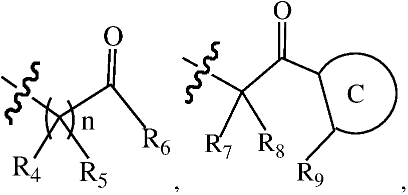 substituted or unsubstituted C6-C8 aryl-substituted or unsubstituted C1-C4 alkyl-.

In another preferred embodiment, R₁ is 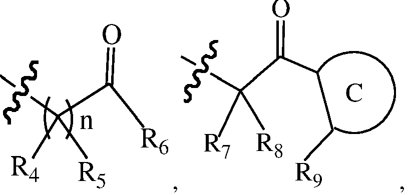 substituted or unsubstituted C6-C8 aryl-substituted or unsubstituted C1-C2 alkyl-.

In another preferred embodiment, R₁ is 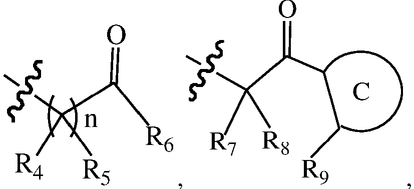 substituted or unsubstituted phenyl-substituted or unsubstituted methyl-.

In another preferred embodiment, R₁ is 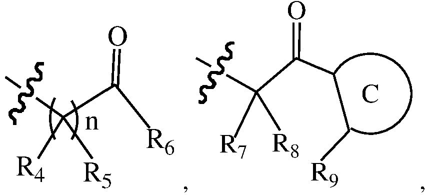 phenyl-methyl-.

In another preferred embodiment, R₂ is connected to the ring C atom in the ring A.

In another preferred embodiment, R₂ is connected to the ring heteroatom in the ring A.

In another preferred embodiment, R₂ is connected to the ring N atom in the ring A.

In another preferred embodiment, ring B is absent, substituted or unsubstituted C6-C12 aromatic ring, substituted or unsubstituted C3-C12 cycloalkane ring, substituted or unsubstituted 3-12 membered heterocycloalkane ring, or substituted or unsubstituted 5-12 membered heteroaromatic ring.

In another preferred embodiment, ring B is absent, substituted or unsubstituted C6-C9 aromatic ring, substituted or unsubstituted C3-C9 cycloalkane ring, substituted or unsubstituted 3-9 membered heterocycloalkane ring, or substituted or unsubstituted 5-9 membered heteroaromatic ring.

In another preferred embodiment, ring B is absent, substituted or unsubstituted C6-C10 aromatic ring, substituted or unsubstituted C3-C10 cycloalkane ring, substituted or unsubstituted 3-10 membered heterocycloalkane ring, or substituted or unsubstituted 5-10 membered heteroaromatic ring.

In another preferred embodiment, ring B is absent, substituted or unsubstituted C6-C8 aromatic ring, substituted or unsubstituted C5-C8 cycloalkane ring, substituted or unsubstituted 5-8 membered heterocycloalkane ring, or substituted or unsubstituted 5-8 membered heteroaromatic ring.

In another preferred embodiment, ring B is absent, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 5 membered heteroaromatic ring, substituted or unsubstituted 6 membered heteroaromatic ring, substituted or unsubstituted 7 membered heteroaromatic ring, substituted or unsubstituted 8 membered heteroaromatic ring.

In another preferred embodiment, the ring o the heterocycloalkane ring has 1, 2, or 3 C=C ring double bonds.

In another preferred embodiment, ring B is absent, substituted or unsubstituted pyridine ring, substituted or unsubstituted tetrahydropyridine ring, substituted or unsubstituted pyrrole ring, substituted or unsubstituted dihydropyrrole ring, substituted or unsubstituted imidazole ring, substituted or unsubstituted pyrazole ring.

In another preferred embodiment, R₂ is hydrogen 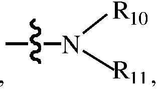 substituted or unsubstituted C6-C10 aryl, substituted or unsubstituted 5-10 membered heteroaryl, substituted or unsubstituted C6-C10 aryl-substituted or unsubstituted C1-C6 alkyl-, substituted or unsubstituted 5-10 membered heteroaryl-substituted or unsubstituted C1-C6 alkyl-.

In another preferred embodiment, R₂ is hydrogen 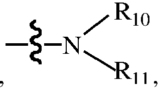 substituted or unsubstituted C6-C8 aryl, substituted or unsubstituted 5-8 membered heteroaryl, substituted or unsubstituted C6-C8 aryl-substituted or unsubstituted C1-C4 alkyl-, substituted or unsubstituted 5-8 membered heteroaryl-substituted or unsubstituted C1-C4 alkyl-.

In another preferred embodiment, R₂ is hydrogen 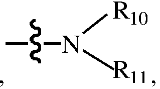 substituted or unsubstituted C6-C8 aryl, substituted or unsubstituted 5-8 membered heteroaryl, substituted or unsubstituted C6-C8 aryl-substituted or unsubstituted C1-C2 alkyl-, substituted or unsubstituted 5-8 membered heteroaryl-substituted or unsubstituted C1-C2 alkyl-.

In another preferred embodiment, R₂ is hydrogen 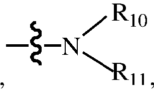 substituted or unsubstituted phenyl-substituted or unsubstituted methyl-.

In another preferred embodiment, R₃ is connected to the ring C atom in the ring B.

In another preferred embodiment, R₃ is connected to the ring heteroatom in the ring B.

In another preferred embodiment, R₃ is connected to the ring N atom in the ring B.

In another preferred embodiment, ring A fused-ring B is substituted or unsubstituted 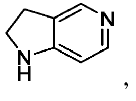 substituted or unsubstituted 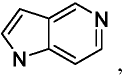 substituted or unsubstituted 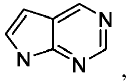 substituted or unsubstituted 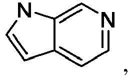 substituted or unsubstituted 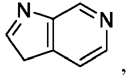 substituted or unsubstituted 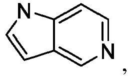 substituted or unsubstituted 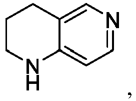 substituted or unsubstituted 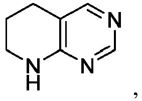 substituted or unsubstituted 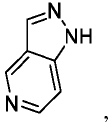 substituted or unsubstituted 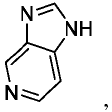 substituted or unsubstituted

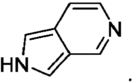

In another preferred embodiment, the tetrahydropyridine ring is 1,2,3,4-tetrahydropyridine ring.

In another preferred embodiment, the pyrrole ring is 1-hydropyrrole ring.

In another preferred embodiment, the dihydropyrrole ring is 2,3-dihydropyrrole ring.

In another preferred embodiment, ring B is absent.

In another preferred embodiment, ring B is absent, the compound of formula I has the following structure of formula I-1:

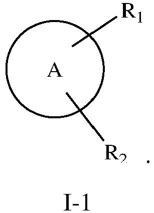

In another preferred embodiment, R₃ is absent, hydrogen, halogen, substituted or unsubstituted C1-C8 alkyl, substituted or unsubstituted C3-C10 cycloalkyl, substituted or unsubstituted C3-C10 cycloalkyl-substituted or unsubstituted C1-C6 alkyl-, substituted or unsubstituted C1-C6 alkoxyl, substituted or unsubstituted C1-C6 alkylthio, substituted or unsubstituted C2-C6 acyl, substituted or unsubstituted C2-C6 acyl-substituted or unsubstituted C1-C6 alkyl-, substituted or unsubstituted C6-C12 aryl, substituted or unsubstituted 5-10 membered heteroaryl, substituted or unsubstituted C6-C12 aryl-sulfuryl-, substituted or unsubstituted C6-C12 aryl-C(O)-, substituted or unsubstituted C6-C12 aryl-C(O)-substituted or unsubstituted C1-C6 alkyl-, substituted or unsubstituted C6-C12 aryl-substituted or unsubstituted C1-C6 alkyl-, substituted or unsubstituted 5-10 membered heteroaryl-substituted or unsubstituted C1-C6 alkyl-.

In another preferred embodiment, R₃ is absent, hydrogen, halogen, substituted or unsubstituted C1-C6 alkyl, substituted or unsubstituted C3-C8 cycloalkyl, substituted or unsubstituted C3-C8 cycloalkyl-substituted or unsubstituted C1-C4 alkyl-, substituted or unsubstituted C1-C4 alkoxyl, substituted or unsubstituted C1-C4 alkylthio, substituted or unsubstituted C2-C4 acyl, substituted or unsubstituted C2-C4 acyl-substituted or unsubstituted C1-C4 alkyl-, substituted or unsubstituted C6-C10 aryl, substituted or unsubstituted 5-10 membered heteroaryl, substituted or unsubstituted C6-C10 aryl-sulfuryl-, substituted or unsubstituted C6-C10 aryl-C(O)-, substituted or unsubstituted C6-C10 aryl-C(O)-substituted or unsubstituted C1-C4 alkyl-, substituted or unsubstituted C6-C10 aryl-substituted or unsubstituted C1-C4 alkyl-, substituted or unsubstituted 5-10 membered heteroaryl-substituted or unsubstituted C1-C4 alkyl-.

In another preferred embodiment,R₃ is absent, hydrogen, halogen, substituted or unsubstituted C3-C6 alkyl, substituted or unsubstituted C3-C7 cycloalkyl, substituted or unsubstituted C3-C7 cycloalkyl-substituted or unsubstituted C1-C2 alkyl-, substituted or unsubstituted C1-C4 alkoxyl, substituted or unsubstituted C1-C4 alkylthio, substituted or unsubstituted C2-C4 acyl, substituted or unsubstituted C2-C4 acyl-substituted or unsubstituted C1-C4 alkyl-, substituted or unsubstituted C6-C8 aryl, substituted or unsubstituted C12 aryl, substituted or unsubstituted 5 membered heteroaryl, substituted or unsubstituted 6 membered heteroaryl, substituted or unsubstituted 7 membered heteroaryl, substituted or unsubstituted 8 membered heteroaryl, substituted or unsubstituted C6-C10 aryl-sulfuryl-, substituted or unsubstituted C6-C10 aryl-C(O)-, substituted or unsubstituted C6-C10 aryl-C(O)-substituted or unsubstituted C1-C4 alkyl-, substituted or unsubstituted C6-C10 aryl-substituted or unsubstituted C1-C4 alkyl-, substituted or unsubstituted 5-10 membered heteroaryl-substituted or unsubstituted C1-C4 alkyl-.

In another preferred embodiment, R₃ is absent, hydrogen, halogen, substituted or unsubstituted C1-C6 alkyl, substituted or unsubstituted C3-C7 cycloalkyl, substituted or unsubstituted C3-C7 cycloalkyl-substituted or unsubstituted C1-C2 alkyl-, substituted or unsubstituted C1-C4 alkoxyl, substituted or unsubstituted C1-C4 alkylthio, substituted or unsubstituted C2 acyl, substituted or unsubstituted C2 acyl-substituted or unsubstituted C1-C2 alkyl-, substituted or unsubstituted C6-C8 aryl, substituted or unsubstituted 5-8 membered heteroaryl, substituted or unsubstituted C6-C8 aryl-sulfuryl-, substituted or unsubstituted C6-C8 aryl-C(O)-, substituted or unsubstituted C6-C8 aryl-C(O)-substituted or unsubstituted C1-C2 alkyl-, substituted or unsubstituted C6-C8 aryl-substituted or unsubstituted C1-C2 alkyl-, substituted or unsubstituted 5-8 membered heteroaryl-substituted or unsubstituted C1-C2 alkyl-.

In another preferred embodiment, R₃ is hydrogen, substituted or unsubstituted methyl, substituted or unsubstituted ethyl, substituted or unsubstituted propyl, substituted or unsubstituted butyl, substituted or unsubstituted pentyl, substituted or unsubstituted cyclopropyl, substituted or unsubstituted cyclopropyl-substituted or unsubstituted methyl-, substituted or unsubstituted cyclopentyl-substituted or unsubstituted methyl-, substituted or unsubstituted phenyl, substituted or unsubstituted phenyl-substituted or unsubstituted methyl-, substituted or unsubstituted phenyl-substituted or unsubstituted ethyl-, substituted or unsubstituted phenyl-substituted or unsubstituted propyl-, substituted or unsubstituted phenylsulfuryl, substituted or unsubstituted phenyl-C(O)-, substituted or unsubstituted phenyl-C(O)-substituted or unsubstituted methyl-, substituted or unsubstituted thiazole-substituted or unsubstituted methyl-, substituted or unsubstituted acetylsubstituted or unsubstituted methyl-, substituted or unsubstituted pyridyl, substituted or unsubstituted pyridyl-substituted or unsubstituted methyl-, halophenyl, halophenyl-substituted or unsubstituted methyl-.

In another preferred embodiment, R₃ is hydrogen, methyl, ethyl, propyl, butyl, pentyl, cyclopropyl, cyclopropyl-methyl-, cyclopentyl-methyl-, phenyl, phenyl-methyl-, methyl-phenyl-methyl-, methyl-phenyl-, phenyl-ethyl-, phenyl-propyl-, phenylsulfuryl, phenyl-C(O)-, phenyl-C(O)-methyl-, thiazole-methyl-, acetyl-methyl-, pyridyl, pyridyl-methyl-, halophenyl, halophenyl-methyl-.

In another preferred embodiment, in the substituted or unsubstituted phenyl, the substituted or unsubstituted phenyl is mono-substituted, di-substituted, tri-substituted, tetra-substituted, pentasubstituted or unsubstituted phenyl

In another preferred embodiment, in the substituted or unsubstituted phenyl, the substituted or unsubstituted phenyl is monosubstituted or unsubstituted phenyl, and the substitution is ortho, para and/or meta substitution on the phenyl.

In another preferred embodiment, in the substituted or unsubstituted phenyl, the substituted or unsubstituted phenyl is monosubstituted or unsubstituted phenyl, and the substitution is ortho, para and/or meta substitution on the phenyl, the substituent is halogen (e.g., Cl ), nitro, amino, C1-C4 alkyl (e.g., methyl), C1-C4 alkoxyl, C1-C4 alkylthio, F₃C -, FsC-O -.

In another preferred embodiment, in the substituted or unsubstituted phenyl-methyl-, the substituted or unsubstituted phenyl is monosubstituted or unsubstituted phenyl.

In another preferred embodiment, in the substituted or unsubstituted phenyl-methyl-, the substituted or unsubstituted phenyl is monosubstituted or unsubstituted phenyl, and the substitution is ortho, para or meta substitution on the phenyl.

In another preferred embodiment, in the substituted or unsubstituted phenyl-methyl-, the substituted or unsubstituted phenyl is monosubstituted or unsubstituted phenyl, and the substitution is ortho, para or meta substitution on the phenyl, the substituent is halogen (e.g., Cl), nitro, amino, C1-C4 alkyl (e.g., methyl), C1-C4 alkoxyl, C1-C4 alkylthio, F₃C-, FsC-O-.

In another preferred embodiment, the substituted phenyl is monosubstituted phenyl.

In another preferred embodiment, the substituted or unsubstituted propyl is substituted or unsubstituted n-propyl or substituted or unsubstituted isopropyl.

In another preferred embodiment, the substituted or unsubstituted butyl is substituted or unsubstituted n-butyl.

In another preferred embodiment, the substituted or unsubstituted pentyl is substituted or unsubstituted n-pentyl.

In another preferred embodiment, the substituted or unsubstituted phenyl-substituted or unsubstituted ethyl- is

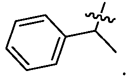

In another preferred embodiment, the substituted or unsubstituted phenyl-substituted or unsubstituted propyl is

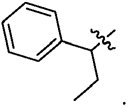

In another preferred embodiment, the substituted or unsubstituted thiazole-substituted or unsubstituted methyl- is

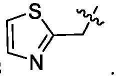

In another preferred embodiment, methyl-phenyl is

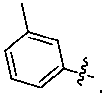

In another preferred embodiment, methyl-phenyl-methyl- is

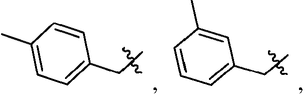

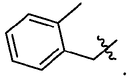

In another preferred embodiment, halophenyl-methyl- is

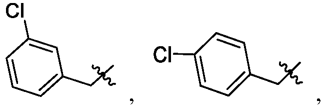

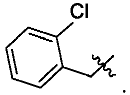

In another preferred embodiment, R₄ and R₅ are each independently hydrogen, substituted or unsubstituted C1-C8 alkyl, substituted or unsubstituted C3-C10 cycloalkyl.

In another preferred embodiment, R₄ and R₅ are each independently hydrogen, substituted or unsubstituted C1-C6 alkyl, substituted or unsubstituted C3-C10 cycloalkyl.

In another preferred embodiment, R₄ and R₅ are each independently hydrogen, substituted or unsubstituted C1-C5 alkyl, substituted or unsubstituted C3-C9 cycloalkyl.

In another preferred embodiment, R₄ and R₅ are each independently hydrogen, substituted or unsubstituted C1-C4 alkyl, substituted or unsubstituted C3-C8 cycloalkyl.

In another preferred embodiment, R₄ and R₅ are each independently hydrogen, substituted or unsubstituted C1-C3 alkyl, substituted or unsubstituted C3-C7 cycloalkyl.

In another preferred embodiment, R₄ and R₅ are each independently hydrogen, substituted or unsubstituted C1-C2 alkyl, substituted or unsubstituted C3-C6 cycloalkyl.

In another preferred embodiment, R₄ and R₅ are each independently hydrogen, methyl, ethyl, propyl or butyl.

In another preferred embodiment, R₄ and R₅ are each independently hydrogen.

In another preferred embodiment, n is 0, 1, 2, 3, 4 or 5.

In another preferred embodiment, n is 1 or 2.

In another preferred embodiment, n is 1.

In another preferred embodiment, R₆ is substituted or unsubstituted C6-C12 aryl, substituted or unsubstituted 5-12 membered heteroaryl, substituted or unsubstituted C3-C12 cycloalkyl, substituted or unsubstituted 3-12 membered heterocycloalkyl.

In another preferred embodiment, R₆ is substituted or unsubstituted C6-C10 aryl, substituted or unsubstituted 5-10 membered heteroaryl, substituted or unsubstituted C3-C8 cycloalkyl, substituted or unsubstituted 3-8 membered heterocycloalkyl.

In another preferred embodiment, R₆ is substituted or unsubstituted C6-C8 aryl, substituted or unsubstituted 5-8 membered heteroaryl, substituted or unsubstituted C3-C8 cycloalkyl, substituted or unsubstituted 3-8 membered heterocycloalkyl.

In another preferred embodiment, R₆ is substituted or unsubstituted C6-C7 aryl, substituted or unsubstituted 5-7 membered heteroaryl, substituted or unsubstituted C5-C7 cycloalkyl, substituted or unsubstituted 5-7 membered heterocycloalkyl.

In another preferred embodiment, R₆ is substituted or unsubstituted C6 aryl, substituted or unsubstituted C7 aryl, substituted or unsubstituted C8 aryl, substituted or unsubstituted C6 heteroaryl, substituted or unsubstituted C7 heteroaryl, substituted or unsubstituted C8 heteroaryl.

In another preferred embodiment, R₆ is 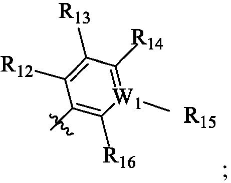
W₁ is C or N;
R₁₂, R₁₃, R₁₄ and R₁₆ are each independently hydrogen, halogen, nitro, amino, C1-C8 alkyl, C1-C8 alkoxyl, C1-C8 alkylthio, C1-C8 haloalkyl, C1-C8 haloalkoxyl, C1-C8 haloalkylthio;
R₁₅ is absent, hydrogen, halogen, nitro, amino, C1-C8 alkyl, C1-C8 alkoxyl, C1-C8 alkylthio, C1-C8 haloalkyl, C1-C8 haloalkoxyl, C1-C8 haloalkylthio.

In another preferred embodiment, W₁ is C or N.

In another preferred embodiment, W₁ is N, R₁₅ is absent.

In another preferred embodiment, W₁ is N, R₁₅ is absent, R₆ is

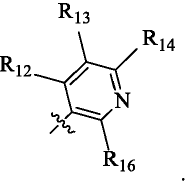

In another preferred embodiment, W₁ is C, R₁₅ is not absent.

In another preferred embodiment, W₁ is C, R₁₅ is hydrogen, halogen, nitro, amino, C1-C8 alkyl, C1-C8 alkoxyl, C1-C8 alkylthio, C1-C8 haloalkyl, C1-C8 haloalkoxyl, C1-C8 haloalkylthio.

In another preferred embodiment, R₁₂, R₁₃, R₁₄ and R₁₆ are each independently hydrogen, halogen, nitro, amino, C1-C6 alkyl, C1-C6 alkoxyl, C1-C6 alkylthio, C1-C6 haloalkyl, C1-C6 haloalkoxyl, C1-C6 haloalkylthio.

In another preferred embodiment, R₁₂, R₁₃, R₁₄ and R₁₆ are each independently hydrogen, halogen, nitro, amino, C1-C4 alkyl, C1-C4 alkoxyl, C1-C4 alkylthio, C1-C4 haloalkyl, C1-C4 haloalkoxyl, C1-C4 haloalkylthio.

In another preferred embodiment, R₁₂, R₁₃, R₁₄ and R₁₆ are each independently hydrogen, halogen, nitro, amino, C1-C2 alkyl, C1-C2 alkoxyl, C1-C2 alkylthio, C1-C2 haloalkyl, C1-C2 haloalkoxyl, C1-C2 haloalkylthio.

In another preferred embodiment, haloalkyl is F₃C-.

In another preferred embodiment, haloalkoxyl is F₃C-O-.

In another preferred embodiment, R₁₂, R₁₃, R₁₄ and R₁₆ are each independently hydrogen, halogen, methyl, ethyl, propyl, F₃C-, F₃C-O-.

In another preferred embodiment, R₁₅ is absent, hydrogen, halogen, nitro, amino, C1-C6 alkyl, C1-C6 alkoxyl, C1-C6 alkylthio, C1-C6 haloalkyl, C1-C6 haloalkoxyl, C1-C6 haloalkylthio.

In another preferred embodiment, R₁₅ is absent, hydrogen, halogen, nitro, amino, C1-C4 alkyl, C1-C4 alkoxyl, C1-C4 alkylthio, C1-C4 haloalkyl, C1-C4 haloalkoxyl, C1-C4 haloalkylthio.

In another preferred embodiment, R₁₅ is absent, hydrogen, halogen, nitro, amino, C1-C2 alkyl, C1-C2 alkoxyl, C1-C2 alkylthio, C1-C2 haloalkyl, C1-C2 haloalkoxyl, C1-C2 haloalkylthio.

In another preferred embodiment, R₁₅ is absent, hydrogen, halogen, methyl, ethyl, propyl, F₃C-, F₃C-O-.

In another preferred embodiment, R₇ is hydrogen, substituted or unsubstituted C1-C8 alkyl, substituted or unsubstituted C3-C8 cycloalkyl.

In another preferred embodiment, R₇ is hydrogen, substituted or unsubstituted C1-C6 alkyl, substituted or unsubstituted C3-C6 cycloalkyl.

In another preferred embodiment, R₇ is hydrogen, substituted or unsubstituted C1-C4 alkyl, substituted or unsubstituted C3-C6 cycloalkyl.

In another preferred embodiment, R₇ is hydrogen, substituted or unsubstituted C1-C3 alkyl, substituted or unsubstituted C3-C5 cycloalkyl.

In another preferred embodiment, R₇ is hydrogen.

In another preferred embodiment, R₈ and R₉ are connected to form substituted or unsubstituted C3-C12 cycloalkane ring, substituted or unsubstituted 3-12 membered heterocycloalkane ring.

In another preferred embodiment, R₈ and R₉ are connected to form substituted or unsubstituted C3-C10 cycloalkane ring, substituted or unsubstituted 3-10 membered heterocycloalkane ring.

In another preferred embodiment, R₈ and R₉ are connected to form substituted or unsubstituted C3-C8 cycloalkane ring, substituted or unsubstituted 3-8 membered heterocycloalkane ring.

In another preferred embodiment, R₈ and R₉ are connected to form substituted or unsubstituted C5-C8 cycloalkane ring, substituted or unsubstituted 5-8 membered heterocycloalkane ring.

In another preferred embodiment, R₈ and R₉ are connected to form substituted or unsubstituted C5-C6 cycloalkane ring, substituted or unsubstituted 5-6 membered heterocycloalkane ring.

In another preferred embodiment, R₈ and R₉ are connected to form substituted or unsubstituted C5 cycloalkane ring, substituted or unsubstituted C6 cycloalkane ring, substituted or unsubstituted 5 membered heterocycloalkane ring, substituted or unsubstituted 6 membered heterocycloalkane ring.

In another preferred embodiment, R₈ and R₉ are connected to form substituted or unsubstituted

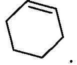

In another preferred embodiment, the ring of the cycloalkane ring has 1, 2, or 3 C=C double bonds.

In another preferred embodiment, R₈ and R₉ are connected to form substituted or unsubstituted cyclopentene ring or cyclohexene ring.

In another preferred embodiment, ring C is substituted or unsubstituted C6-C12 aromatic ring, substituted or unsubstituted C3-C12 cycloalkane ring, substituted or unsubstituted 3-12 membered heterocycloalkane ring, or substituted or unsubstituted 5-12 membered heteroaromatic ring.

In another preferred embodiment, ring C is substituted or unsubstituted C6-C10 aromatic ring, substituted or unsubstituted C3-C10 cycloalkane ring, substituted or unsubstituted 3-10 membered heterocycloalkane ring, or substituted or unsubstituted 5-10 membered heteroaromatic ring.

In another preferred embodiment, ring C is substituted or unsubstituted C6-C8 aromatic ring, substituted or unsubstituted C5-C10 cycloalkane ring, substituted or unsubstituted 5-10 membered heterocycloalkane ring, or substituted or unsubstituted 5-8 membered heteroaromatic ring.

In another preferred embodiment, ring C is substituted or unsubstituted benzene ring, substituted or unsubstituted pyridine ring, substituted or unsubstituted thiophene ring, substituted or unsubstituted furan ring, substituted or unsubstituted thiazole ring.

In another preferred embodiment, ring C is substituted or unsubstituted benzene ring, substituted or unsubstituted thiophene ring, substituted or unsubstituted furan ring, substituted or unsubstituted thiazole ring.

In another preferred embodiment, ring C is substituted or unsubstituted benzene ring, substituted or unsubstituted pyridine ring, substituted or unsubstituted thiophene ring, substituted or unsubstituted furan ring, substituted or unsubstituted thiazole ring, each "substituted" means that 1, 2 or 3 hydrogen atoms on the ring are independently substituted by substituent selected from the group consisting of halogen, methyl, ethyl, propyl, F₃C-, F₃C-O-.

In another preferred embodiment, ring C is substituted or unsubstituted benzene ring, substituted or unsubstituted thiophene ring, substituted or unsubstituted furan ring, substituted or unsubstituted thiazole ring, each "substituted" means that 1, 2 or 3 hydrogen atoms on the ring are independently substituted by substituent selected from the group consisting of halogen, methyl, ethyl, propyl, F₃C-, F₃C-O-.

In another preferred embodiment, ring C is

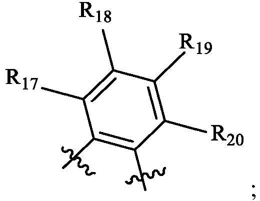

R₁₇, R₁₈, R₁₉ and R₂₀ are each independently hydrogen, halogen, C1-C6 alkyl, C1-C6 alkoxyl, C1-C6 alkylthio, C1-C6 haloalkyl, C1-C6 haloalkoxyl, C1-C6 haloalkylthio.

In another preferred embodiment, R₁₇, R₁₈, R₁₉ and R₂₀ are each independently hydrogen, halogen, C1-C6 alkyl, C1-C6 alkoxyl, C1-C6 alkylthio, C1-C6 haloalkyl, C1-C6 haloalkoxyl, C1-C6 haloalkylthio.

In another preferred embodiment, R₁₇, R₁₈, R₁₉ and R₂₀ are each independently hydrogen, halogen, C1-C4 alkyl, C1-C4 alkoxyl, C1-C4 alkylthio, C1-C4 haloalkyl, C1-C4 haloalkoxyl, C1-C4 haloalkylthio.

In another preferred embodiment, R₁₇, R₁₈, R₁₉ and R₂₀ are each independently hydrogen, halogen, C1-C2 alkyl, C1-C2 alkoxyl, C1-C2 alkylthio, C1-C2 haloalkyl, C1-C2 haloalkoxyl, C1-C2 haloalkylthio.

In another preferred embodiment, haloalkyl is FsC-.

In another preferred embodiment, haloalkoxyl is F₃C-O-.

In another preferred embodiment, R₁₇, R₁₈, R₁₉ and R₂₀ are each independently hydrogen, halogen, methyl, ethyl, propyl, F₃C-, F₃C-O-.

In another preferred embodiment, ring C is 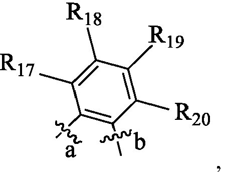 wherein, the connecting site " 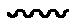 " indicated by a or b is connected to R₉.

In another preferred embodiment, the connecting site " - " indicated by a or b is connected to C linked to -C(O)-.

In another preferred embodiment, 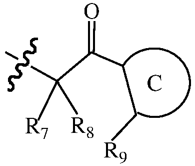 is

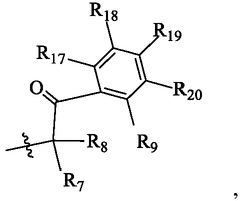

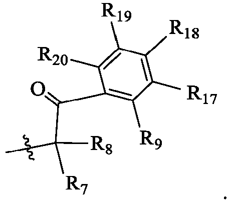

In another preferred embodiment, ring C is 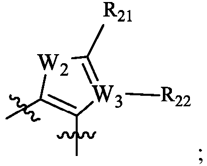
W₂ is CH₂, NH, O or S;
W₃ is C or N;
R₂₁ is hydrogen, halogen, C1-C8 alkyl, C3-C8 cycloalkyl;
R₂₂ is absent, hydrogen, halogen, C1-C8 alkyl, C3-C8 cycloalkyl.

In another preferred embodiment, W₂ is O or S.

In another preferred embodiment, W₃ is C or N.

In another preferred embodiment, W₃ is N, R₂₂ is absent.

In another preferred embodiment, W₃ is N, R₂₂ is absent, ring C is

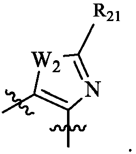

In another preferred embodiment, W₃ is C, R₂₂ is not absent.

In another preferred embodiment, W₃ is C, R₂₂ is hydrogen, halogen, C1-C8 alkyl, C3-C8 cycloalkyl.

In another preferred embodiment, R₂₁ is hydrogen, halogen, C1-C8 alkyl, C3-C8 cycloalkyl.

In another preferred embodiment, R₂₁ is hydrogen, halogen, C1-C6 alkyl, C3-C8 cycloalkyl.

In another preferred embodiment, R₂₁ is hydrogen, halogen, C1-C4 alkyl, C3-C6 cycloalkyl.

In another preferred embodiment, R₂₁ is hydrogen, halogen, C1-C2 alkyl, C3-C6 cycloalkyl.

In another preferred embodiment, R₂₁ is hydrogen, halogen (e.g., F, Cl), methyl.

In another preferred embodiment, R₂₁ is hydrogen, F, Cl, methyl.

In another preferred embodiment, R₂₂ is absent, hydrogen, halogen, C1-C8 alkyl, C3-C8 cycloalkyl.

In another preferred embodiment, R₂₂ is absent, hydrogen, halogen, C1-C6 alkyl, C3-C8 cycloalkyl.

In another preferred embodiment, R₂₂ is absent, hydrogen, halogen, C1-C4 alkyl, C3-C6 cycloalkyl.

In another preferred embodiment, R₂₂ is absent, hydrogen, halogen, C1-C2 alkyl, C3-C6 cycloalkyl.

In another preferred embodiment, R₂₂ is absent, hydrogen, halogen (e.g., F, Cl), methyl. In another preferred embodiment, R₂₂ is absent, hydrogen, F, Cl, methyl.

In another preferred embodiment, ring C is 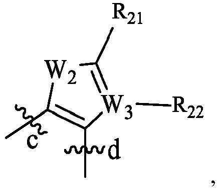 wherein, the connecting site " 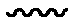 " indicated by c or d is connected to R₉.

In another preferred embodiment, the connecting site " - " indicated by c or d is connected to C linked to -C(O)-.

In another preferred embodiment, 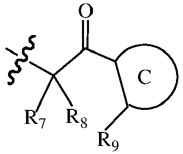 is

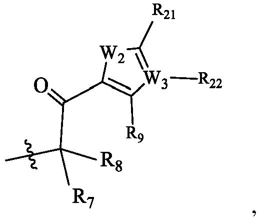

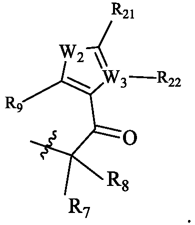

In another preferred embodiment, 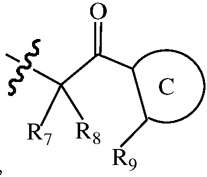 is 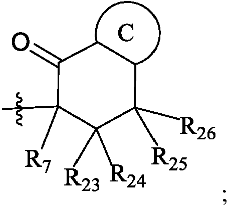
R₇ and ring C are each independently defined as described above;
R₂₃, R₂₄, R₂₅ and R₂₆ are each independently hydrogen, halogen, C1-C8 alkyl, C3-C8 cycloalkyl.

In another preferred embodiment, R₂₃, R₂₄, R₂₅ and R₂₆ are each independently hydrogen, halogen, C1-C6 alkyl, C3-C8 cycloalkyl.

In another preferred embodiment, R₂₃, R₂₄, R₂₅ and R₂₆ are each independently hydrogen, halogen, C1-C4 alkyl, C3-C6 cycloalkyl.

In another preferred embodiment, R₂₃, R₂₄, R₂₅ and R₂₆ are each independently hydrogen, halogen, C1-C2 alkyl, C3-C6 cycloalkyl.

In another preferred embodiment, R₂₃, R₂₄, R₂₅ and R₂₆ are each independently hydrogen.

In another preferred embodiment, 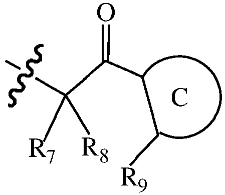 is

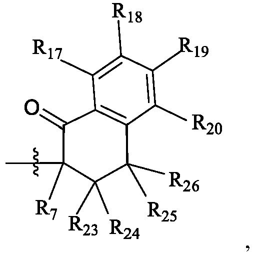

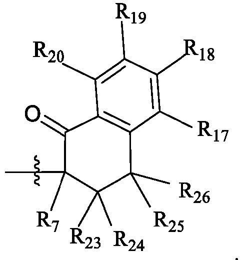

In another preferred embodiment, 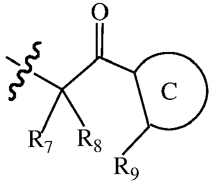 is

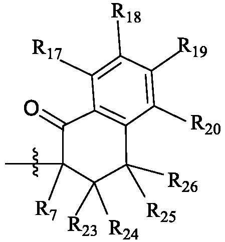

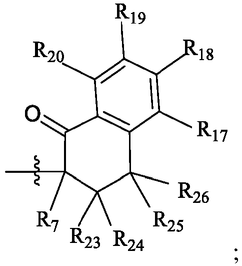

R₇, R₁₇, R₁₈, R₁₉, R₂₀, R₂₃, R₂₄, R₂₅ and R₂₆ are each independently defined as described above.

In another preferred embodiment, 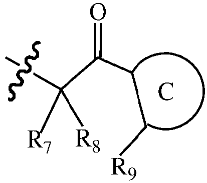 is

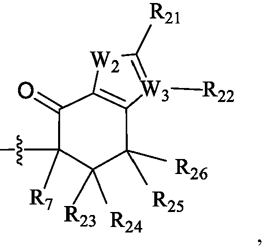

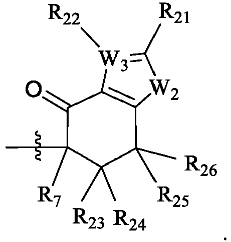

In another preferred embodiment, 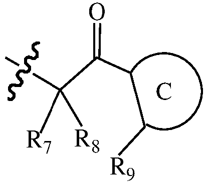 is

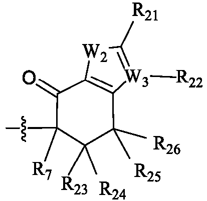

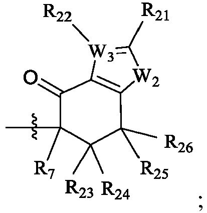

R₇, R₂₁, R₂₂, R₂₃, R₂₄, R₂₅, R₂₆, W₂ and W₃ are each independently defined as described above.

In another preferred embodiment, ring C is

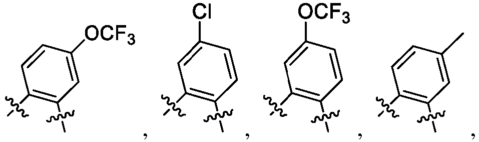

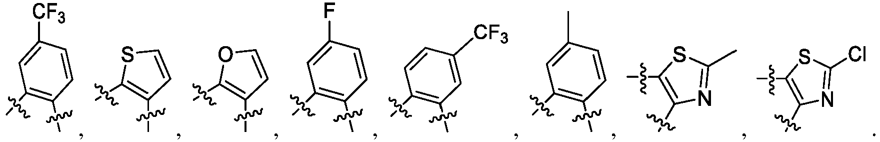

In another preferred embodiment, R₁₀ and R₁₁ are each independently hydrogen, substituted or unsubstituted C1-C8 alkyl, substituted or unsubstituted C2-C7 acyl, substituted or unsubstituted C6-C12 aryl, substituted or unsubstituted 5-10 membered heteroaryl, substituted or unsubstituted C6-C12 aryl-substituted or unsubstituted C1-C4 alkyl-, substituted or unsubstituted C6-C12 aryl-C(O)-substituted or unsubstituted C1-C4 alkyl-, substituted or unsubstituted 5-10 membered heteroaryl-substituted or unsubstituted C1-C4 alkyl-; or R₁₀ and R₁₁ are connected to form substituted or unsubstituted C3-C10 cycloalkane ring, substituted or unsubstituted 5-12 membered heterocycloalkane ring, substituted or unsubstituted 5-12 membered heteroaromatic ring.

In another preferred embodiment, R₁₀ and R₁₁ are each independently hydrogen, substituted or unsubstituted C1-C6 alkyl, substituted or unsubstituted C2-C5 acyl, substituted or unsubstituted C6-C10 aryl, substituted or unsubstituted 5-10 membered heteroaryl, substituted or unsubstituted C6-C10 aryl-substituted or unsubstituted C1-C6 alkyl-, substituted or unsubstituted C6-C10 aryl-C(O)-substituted or unsubstituted C1-C4 alkyl-, substituted or unsubstituted 5-10 membered heteroaryl-substituted or unsubstituted C1-C4 alkyl-; or R₁₀ and R₁₁ are connected to form substituted or unsubstituted C3-C10 cycloalkane ring, substituted or unsubstituted 5-12 membered heterocycloalkane ring, substituted or unsubstituted 5-12 membered heteroaromatic ring.

In another preferred embodiment, R₁₀ and R₁₁ are each independently hydrogen, substituted or unsubstituted C1-C4 alkyl, substituted or unsubstituted C2-C5 acyl, substituted or unsubstituted C6-C8 aryl, substituted or unsubstituted 5-8 membered heteroaryl, substituted or unsubstituted C6-C8 aryl-substituted or unsubstituted C1-C4 alkyl-, substituted or unsubstituted C6-C8 aryl-C(O)-substituted or unsubstituted C1-C4 alkyl-, substituted or unsubstituted 5-8 membered heteroaryl-substituted or unsubstituted C1-C4 alkyl-; or R₁₀ and R₁₁ are connected to form substituted or unsubstituted C3-C10 cycloalkane ring, substituted or unsubstituted 5-10 membered heterocycloalkane ring, substituted or unsubstituted 5-10 membered heteroaromatic ring.

In another preferred embodiment, R₁₀ and R₁₁ are each independently hydrogen, substituted or unsubstituted C1-C2 alkyl, substituted or unsubstituted C2-C5 acyl, substituted or unsubstituted C6-C8 aryl, substituted or unsubstituted 5-8 membered heteroaryl, substituted or unsubstituted C6-C8 aryl-substituted or unsubstituted C1-C2 alkyl-, substituted or unsubstituted C6-C8 aryl-C(O)-substituted or unsubstituted C1-C2 alkyl-, substituted or unsubstituted 5-8 membered heteroaryl-substituted or unsubstituted C1-C2 alkyl-; or R₁₀ and R₁₁ are connected to form substituted or unsubstituted C5-C8 cycloalkane ring, substituted or unsubstituted 5-10 membered heterocycloalkane ring, substituted or unsubstituted 5-10 membered heteroaromatic ring.

In another preferred embodiment, R₁₀ and R₁₁ are each independently hydrogen, substituted or unsubstituted methyl, substituted or unsubstituted acetyl, substituted or unsubstituted propionyl, substituted or unsubstituted butyryl, substituted or unsubstituted phenyl-substituted or unsubstituted methyl-, substituted or unsubstituted pyridyl-substituted or unsubstituted methyl-, substituted or unsubstituted phenyl-C(O)-substituted or unsubstituted methyl-; or R₁₀ and R₁₁ are connected to form substituted or unsubstituted indoline ring, substituted or unsubstituted indole ring, or substituted or unsubstituted piperidine ring.

In another preferred embodiment, R₁₀ and R₁₁ are each independently hydrogen, methyl, acetyl, propionyl, butyryl, phenyl-methyl-, pyridyl-methyl-, methyl-pyridyl-methyl-, phenyl-C(O)-methyl-, methyl-phenyl-C(O)-methyl-; or R₁₀ and R₁₁ are connected to form indoline ring, indole ring, or piperidine ring.

In another preferred embodiment, butyryl is

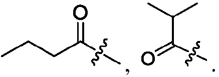

In another preferred embodiment, substituted or unsubstituted pyridyl-substituted or unsubstituted methyl- is monomethyl-substituted pyridyl-methyl-.

In another preferred embodiment, substituted or unsubstituted pyridyl-substituted or unsubstituted methyl- is

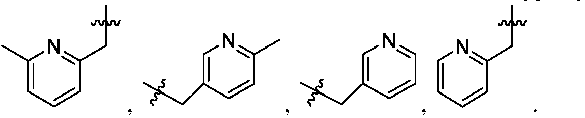

In another preferred embodiment, the methyl-phenyl-C(O)-methyl- is

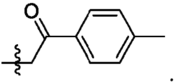

In another preferred embodiment, the butyl is n-butyl.

In another preferred embodiment, the butyl is

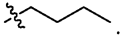

In another preferred embodiment, the pentyl is n-pentyl.

In another preferred embodiment, the pentyl is

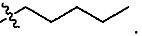

In another preferred embodiment, the phenyl-ethyl- is

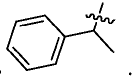

In another preferred embodiment, the phenyl-propyl- is

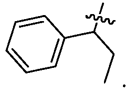

In another preferred embodiment, the halogen is F, Cl, Br or I.

In another preferred embodiment, the aryl is phenyl or naphthyl

In another preferred embodiment, the halo is mono-halo, di-halo, tri-halo or full-halo.

In another preferred embodiment, halo is fluoro, chloro, bromo, iodo.

In another preferred embodiment, the deuterated is mono-deuterated, di-deuterated, trideuterated or fully-deuterated.

In another preferred embodiment, the compound of formula I has the following structure of formula I-2: 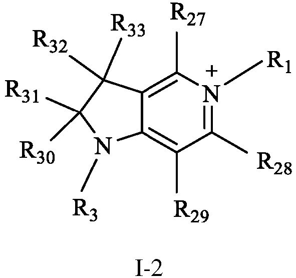 wherein,
R₁ and R₃ are each independently defined as described above;
R₂₇, R₂₈, R₂₉, R₃₀, R₃₁, R₃₂ and R₃₂ are each independently hydrogen, halogen, C1-C8 alkyl, C3-C8 cycloalkyl.

In another preferred embodiment, R₂₇, R₂₈, R₂₉, R₃₀, R₃₁, R₃₂ and R₃₃ are each independently hydrogen, halogen, C1-C6 alkyl, C3-C8 cycloalkyl.

In another preferred embodiment, R₂₇, R₂₈, R₂₉, R₃₀, R₃₁, R₃₂ and R₃₃ are each independently hydrogen, halogen, C1-C4 alkyl, C3-C6 cycloalkyl.

In another preferred embodiment, R₂₇, R₂₈, R₂₉, R₃₀, R₃₁, R₃₂ and R₃₃ are each independently hydrogenl.

In another preferred embodiment, the compound of formula I has the following structure of formula I-3: 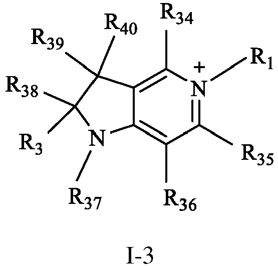 wherein,
R₁ and R₃ are each independently defined as described above;
R₃₄, R₃₅, R₃₆, R₃₇, R₃₈, R₃₉ and R₄₀ are each independently hydrogen, halogen, C1-C8 alkyl, C3-C8 cycloalkyl.

In another preferred embodiment, R₃₄, R₃₅, R₃₆, R₃₇, R₃₈, R₃₉ and R₄₀ are each independently hydrogen, halogen, C1-C6 alkyl, C3-C8 cycloalkyl.

In another preferred embodiment, R₃₄, R₃₅, R₃₆, R₃₇, R₃₈, R₃₉ and R₄₀ are each independently hydrogen, halogen, C1-C4 alkyl, C3-C8 cycloalkyl.

In another preferred embodiment, R₃₄, R₃₅, R₃₆, R₃₇, R₃₈, R₃₉ and R₄₀ are each independently hydrogen.

In another preferred embodiment, the compound of formula I has the following structure of formula I-4-1 or I-4-2: 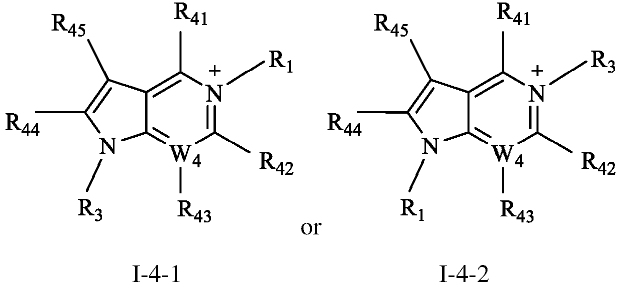 wherein,
R₁ and R₃ are each independently defined as described above;
W₄ is C or N;
R₄₁, R₄₂, R₄₄ and R₄₅ are each independently hydrogen, halogen, C1-C8 alkyl, C3-C8 cycloalkyl;
R₄₃ is absent, hydrogen, halogen, C1-C8 alkyl, C3-C8 cycloalkyl.

In another preferred embodiment, W₄ is C or N.

In another preferred embodiment, W₄ is N, R₄₃ is absent.

In another preferred embodiment, W₄ is C, R₄₃ is not absent.

In another preferred embodiment, W₄ is C, Ras is hydrogen, halogen, C1-C8 alkyl, C3-C8 cycloalkyl.

In another preferred embodiment, W₄ is N, Ras is absent, the structure of formula I-4-1 has the following structure of formula I-4-1-1:

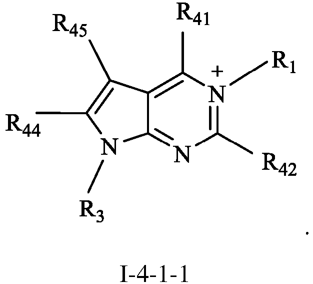

In another preferred embodiment, W₄ is N, Ras is absent, the structure of formula I-4-2 has the following structure of formula I-4-2-1:

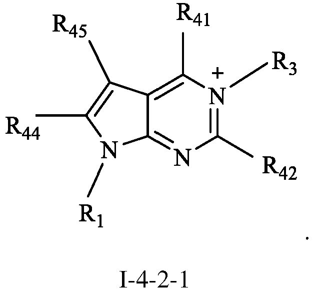

In another preferred embodiment, R₄₁, R₄₂, R₄₄ and R₄₅ are each independently hydrogen, halogen, C1-C8 alkyl, C3-C8 cycloalkyl.

In another preferred embodiment, R₄₁, R₄₂, R₄₄ and R₄₅ are each independently hydrogen, halogen, C1-C6 alkyl, C3-C8 cycloalkyl.

In another preferred embodiment, R₄₁, R₄₂, R₄₄ and R₄₅ are each independently hydrogen, halogen, C1-C4 alkyl, C3-C8 cycloalkyl.

In another preferred embodiment, R₄₁, R₄₂, R₄₄ and R₄₅ are each independently hydrogen, ethyl, halogen (e.g. F, Cl, Br), methyl.

In another preferred embodiment, R₄₃ is absent, hydrogen, halogen, C1-C8 alkyl, C3-C8 cycloalkyl.

In another preferred embodiment, R₄₃ is absent, hydrogen, halogen, C1-C6 alkyl, C3-C8 cycloalkyl.

In another preferred embodiment, R₄₃ is absent, hydrogen, halogen, C1-C4 alkyl, C3-C8 cycloalkyl.

In another preferred embodiment, R₄₃ is absent, hydrogen, ethyl, halogen (e.g. F, Cl, Br), methyl.

In another preferred embodiment, the compound of formula I has the following structure of formula I-5: 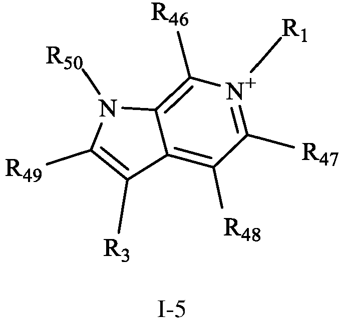 wherein,
R₁ and R₃ are each independently defined as described above;
R₄₆, R₄₇, R₄₈, R₄₉ and R₅₀ are each independently hydrogen, C1-C8 alkyl, C3-C8 cycloalkyl, halogen.

In another preferred embodiment, R₄₆, R₄₇, R₄₈, R₄₉ and R₅₀ are each independently hydrogen, C1-C6 alkyl, C3-C8 cycloalkyl, halogen.

In another preferred embodiment, R₄₆, R₄₇, R₄₈, R₄₉ and R₅₀ are each independently hydrogen, C1-C4 alkyl, C3-C8 cycloalkyl, halogen.

In another preferred embodiment, R₄₆, R₄₇, R₄₈, R₄₉ and R₅₀ are each independently hydrogen, C1-C2 alkyl, C3-C8 cycloalkyl, halogen.

In another preferred embodiment, R₄₆, R₄₇, R₄₈, R₄₉ and R₅₀ are each independently hydrogen, methyl, halogen (e.g. F, Cl).

In another preferred embodiment, the compound of formula I has the following structure of formula I-6: 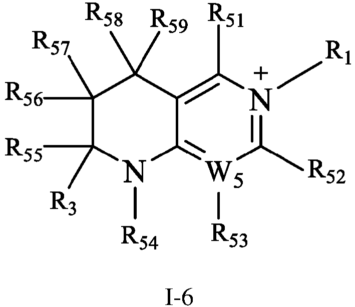 wherein,
R₁ and R₃ are each independently defined as described above;
W₅ is C or N;
R₅₁, R₅₂, R₅₄, R₅₅, R₅₆, R₅₇, R₅₈ and R₅₉ are each independently hydrogen, C1-C8 alkyl, C3-C8 cycloalkyl, halogen;
R₅₃ is absent, hydrogen, C1-C8 alkyl, C3-C8 cycloalkyl, halogen.

In another preferred embodiment, W₅ is C or N.

In another preferred embodiment, W₅ is N, R₅₃ is absent.

In another preferred embodiment, W₅ is C, R₅₃ is not absent.

In another preferred embodiment, W₅ is C, R₅₃ is hydrogen, C1-C8 alkyl, C3-C8 cycloalkyl, halogen.

In another preferred embodiment, W₅ is N, R₅₃ is absent, the structure of formula I-6 has the following structure of formula 1-6-1:

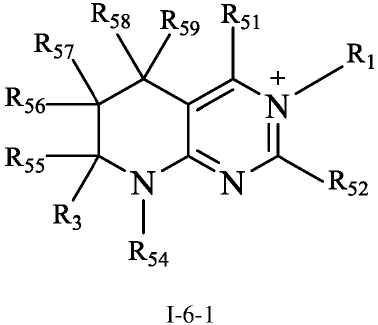

In another preferred embodiment, R₅₁, R₅₂, R₅₄, R₅₅, R₅₆, R₅₇, R₅₈ and R₅₉ are each independently hydrogen, C1-C6 alkyl, C3-C8 cycloalkyl, halogen.

In another preferred embodiment, R₅₁, R₅₂, R₅₄, R₅₅, R₅₆, R₅₇, R₅₈ and R₅₉ are each independently hydrogen, C1-C4 alkyl, C3-C8 cycloalkyl, halogen.

In another preferred embodiment, R₅₁, R₅₂, R₅₄, R₅₅, R₅₆, R₅₇, R₅₈ and R₅₉ are each independently hydrogen, C1-C2 alkyl, C3-C8 cycloalkyl, halogen.

In another preferred embodiment, R₅₁, R₅₂, R₅₄, R₅₅, R₅₆, R₅₇, R₅₈ and R₅₉ are each independently hydrogen, methyl.

In another preferred embodiment, R₅₃ is absent, hydrogen, C1-C6 alkyl, C3-C8 cycloalkyl, halogen.

In another preferred embodiment, R₅₃ is absent, hydrogen, C1-C4 alkyl, C3-C8 cycloalkyl, halogen.

In another preferred embodiment, R₅₃ is absent, hydrogen, C1-C2 alkyl, C3-C8 cycloalkyl, halogen.

In another preferred embodiment, R₅₃ is absent, hydrogen, methyl.

In another preferred embodiment, the compound of formula I has the following structure of formula I-7: 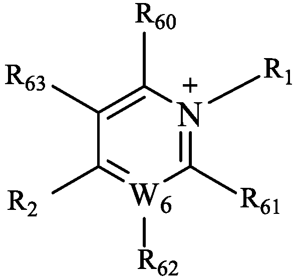 I-7
wherein,
R₁ and R₂ are each independently defined as described above;
W₆ is C or N;
R₆₀ and R₆₃ are each independently hydrogen, C1-C8 alkyl, C3-C8 cycloalkyl;
R₆₁ and R₆₂ are each independently absent, hydrogen, C1-C8 alkyl, C3-C8 cycloalkyl, C1-C8 haloalkyl, C1-C8 haloalkoxyl, C1-C8 haloalkylthio, halogen; or R₆₁ and R₆₂ are connected to form 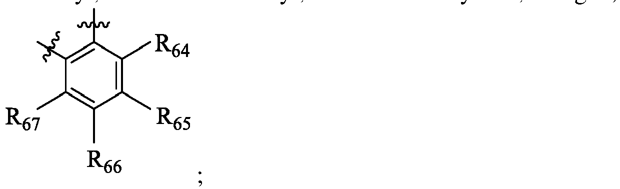
R₆₄, R₆₅, R₆₆ and R₆₇ are each independently hydrogen, C1-C8 alkyl, C3-C8 cycloalkyl.

In another preferred embodiment, W₆ is C or N.

In another preferred embodiment, W₆ is N, R₆₂ is absent.

In another preferred embodiment, W₆ is C, R₆₂ is not absent.

In another preferred embodiment, W₆ is C, R₆₂ is hydrogen, C1-C8 alkyl, C3-C8 cycloalkyl, C1-C8 haloalkyl, C1-C8 haloalkoxyl, C1-C8 haloalkylthio, halogen; or R₆₁ and R₆₂ are connected to form

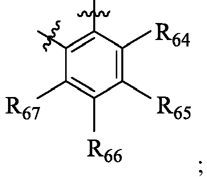

In another preferred embodiment, W₆ is N, R₆₂ is absent, the structure of formula I-7 has the following structure of formula 1-7-1:

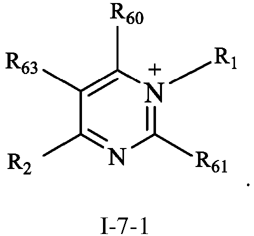

In another preferred embodiment, W₆ is C, R₆₂ is not absent, the structure of formula I-7 has the following structure of formula I-7-2:

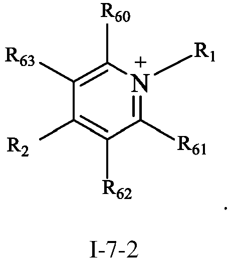

In another preferred embodiment, R₆₀ and R₆₃ are each independently hydrogen, C1-C6 alkyl, C3-C6 cycloalkyl.

In another preferred embodiment, R₆₀ and R₆₃ are each independently hydrogen, C1-C4 alkyl, C3-C6 cycloalkyl.

In another preferred embodiment, R₆₀ and R₆₃ are each independently hydrogen, C1-C2 alkyl, C3-C6 cycloalkyl.

In another preferred embodiment, R₆₀ and R₆₃ are each independently hydrogen, methyl.

In another preferred embodiment, R₆₁ and R₆₂ are each independently absent, hydrogen, C1-C6 alkyl, C3-C8 cycloalkyl, C1-C6 haloalkyl, C1-C6 haloalkoxyl, C1-C6 haloalkylthio, halogen; or R₆₁ and R₆₂ are connected to form

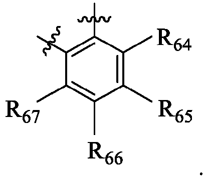

In another preferred embodiment, R₆₁ and R₆₂ are each independently absent, hydrogen, C1-C4 alkyl, C3-C6 cycloalkyl, C1-C4 haloalkyl, C1-C4 haloalkoxyl, C1-C4 haloalkylthio, halogen; or R₆₁ and R₆₂ are connected to form

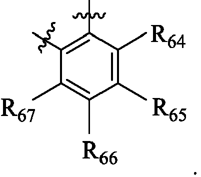

In another preferred embodiment, R₆₁ and R₆₂ are each independently absent, hydrogen, C1-C3 alkyl, C3-C6 cycloalkyl, C1-C3 haloalkyl, C1-C3 haloalkoxyl, C1-C3 haloalkylthio, halogen; or R₆₁ and R₆₂ are connected to form

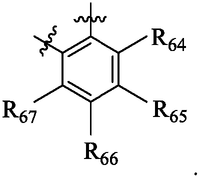

In another preferred embodiment, R₆₁ and R₆₂ are each independently absent, hydrogen, methyl, ethyl, propyl, cyclopropyl; or R₆₁ and R₆₂ are connected to form

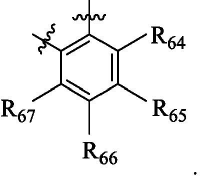

In another preferred embodiment, R₆₁ and R₆₂ are connected to form 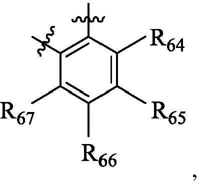 the structure of formula I-7 has the following structure of formula I-7-3:

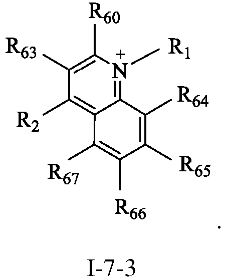

In another preferred embodiment, R₆₄, R₆₅, R₆₆ and R₆₇ are each independently hydrogen, C1-C6 alkyl, C3-C8 cycloalkyl.

In another preferred embodiment, R₆₄, R₆₅, R₆₆ and R₆₇ are each independently hydrogen, C1-C4 alkyl, C3-C8 cycloalkyl.

In another preferred embodiment, R₆₄, R₆₅, R₆₆ and R₆₇ are each independently hydrogen, C1-C2 alkyl, C3-C8 cycloalkyl.

In another preferred embodiment, the propyl is n-propyl, isopropyl.

In another preferred embodiment, the propyl is

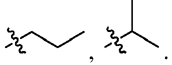

In another preferred embodiment, the compound of formula I has the following structure of formula I-8: 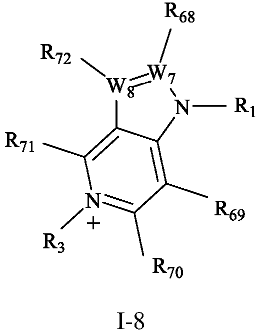 wherein,
R₁ and R₃ are each independently defined as described above;
W₇ is C or N;
W₈ is C or N;
R₆₉, R₇₀ and R₇₁ are each independently hydrogen, C1-C8 alkyl, C3-C8 cycloalkyl, C1-C8 haloalkyl, C1-C8 haloalkoxyl, C1-C8 haloalkylthio, halogen;
R₆₈ and R₇₂ are each independently absent, hydrogen, C1-C8 alkyl, C3-C8 cycloalkyl, C1-C8 haloalkyl, C1-C8 haloalkoxyl, C1-C8 haloalkylthio, halogen.

In another preferred embodiment, W₇ is C or N.

In another preferred embodiment, W₈ is C or N.

In another preferred embodiment, W₇ is N, R₆₈ is absent.

In another preferred embodiment, W₇ is C, R₆₈ is not absent.

In another preferred embodiment, W₇ is C, R₆₈ is hydrogen, C1-C8 alkyl, C3-C8 cycloalkyl, C1-C8 haloalkyl, C1-C8 haloalkoxyl, C1-C8 haloalkylthio, halogen.

In another preferred embodiment, W₈ is N, R₇₂ is absent.

In another preferred embodiment, W₈ is C, R₇₂ is not absent.

In another preferred embodiment, W₈ is C, R₇₂ is hydrogen, C1-C8 alkyl, C3-C8 cycloalkyl,

C1-C8 haloalkyl, C1-C8 haloalkoxyl, C1-C8 haloalkylthio, halogen.

In another preferred embodiment, W₇ is N, and W₈ is C.

In another preferred embodiment, W₇ is N, and W₈ is C, the compound of formula I-8 has the following structure of formula I-8-1:

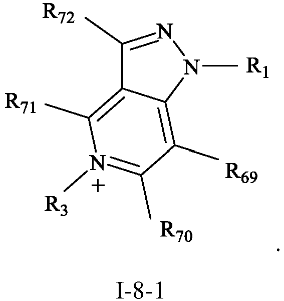

In another preferred embodiment, W₇ is C, and W₈ is N.

In another preferred embodiment, W₇ is C, and W₈ is N, the compound of formula I-8 has the following structure of formula I-8-2:

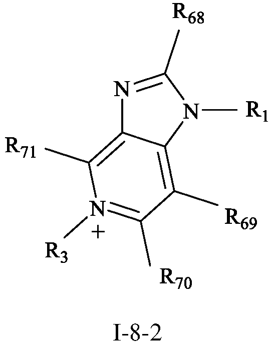

In another preferred embodiment, R₆₉, R₇₀ and R₇₁ are each independently hydrogen, C1-C6 alkyl, C3-C8 cycloalkyl, C1-C6 haloalkyl, C1-C6 haloalkoxyl, C1-C6 haloalkylthio, halogen.

In another preferred embodiment, R₆₉, R₇₀ and R₇₁ are each independently hydrogen, C1-C4 alkyl, C3-C8 cycloalkyl, C1-C4 haloalkyl, C1-C4 haloalkoxyl, C1-C4 haloalkylthio, halogen.

In another preferred embodiment, R₆₉, R₇₀ and R₇₁ are each independently hydrogen, C1-C2 alkyl, C3-C8 cycloalkyl, C1-C2 haloalkyl, C1-C2 haloalkoxyl, C1-C2 haloalkylthio, halogen.

In another preferred embodiment, R₆₉, R₇₀ and R₇₁ are each independently hydrogen, methyl, halogen (e.g.Cl, Br).

In another preferred embodiment, R₆₈ and R₇₂ are each independently absent, hydrogen, C1-C6 alkyl, C3-C8 cycloalkyl, C1-C6 haloalkyl, C1-C6 haloalkoxyl, C1-C6 haloalkylthio, halogen.

In another preferred embodiment, R₆₈ and R₇₂ are each independently absent, hydrogen, C1-C4 alkyl, C3-C8 cycloalkyl, C1-C4 haloalkyl, C1-C4 haloalkoxyl, C1-C4 haloalkylthio, halogen.

In another preferred embodiment, R₆₈ and R₇₂ are each independently absent, hydrogen, C1-C2 alkyl, C3-C8 cycloalkyl, C1-C2 haloalkyl, C1-C2 haloalkoxyl, C1-C2 haloalkylthio, halogen.

In another preferred embodiment, R₆₈ and R₇₂ are each independently absent, hydrogen, methyl, halogen (e.g.Cl, Br).

In another preferred embodiment, the compound of formula I has the following structure of formula I-9: 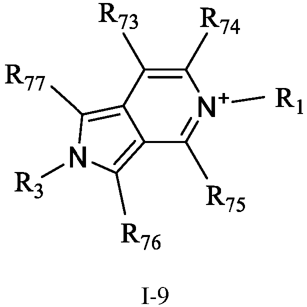 wherein,
R₁ and R₃ are each independently defined as described above;
R₇₃, R₇₄, R₇₅, R₇₆ and R₇₇ are each independently hydrogen, C1-C8 alkyl, C3-C8 cycloalkyl, C1-C8 haloalkyl, C1-C8 haloalkoxyl, C1-C8 haloalkylthio, halogen.

In another preferred embodiment, R₇₃, R₇₄, R₇₅, R₇₆ and R₇₇ are each independently hydrogen, C1-C6 alkyl, C3-C8 cycloalkyl, C1-C6 haloalkyl, C1-C6 haloalkoxyl, C1-C6 haloalkylthio, halogen.

In another preferred embodiment, R₇₃, R₇₄, R₇₅, R₇₆ and R₇₇ are each independently hydrogen, C1-C4 alkyl, C3-C8 cycloalkyl, C1-C4 haloalkyl, C1-C4 haloalkoxyl, C1-C4 haloalkylthio, halogen.

In another preferred embodiment, R₇₃, R₇₄, R₇₅, R₇₆ and R₇₇ are each independently hydrogen, C1-C4 alkyl, C3-C8 cycloalkyl, C1-C2 haloalkyl, C1-C2 haloalkoxyl, C1-C2 haloalkylthio, halogen.

In another preferred embodiment, the pharmaceutically acceptable salt of the compound of formula I comprises the salt formed by the compound of formula I and an acid.

In another preferred embodiment, the acid comprises one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methane sulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises the salt root formed by the loss of a H⁺ in the acid.

In another preferred embodiment, the salt root of the pharmaceutically acceptable salt of the compound of formula I comprises the salt root formed by the loss of a H⁺ in hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises F-, Cl-, Br-, I-, HCOO-, CH3COO-, CF3COO-, SO42- or NO3-.

In another preferred embodiment, the compound of formula I, or an optical isomer thereof, or a racemate thereof, or a solvate thereof, or a pharmaceutically acceptable salt thereof, or a deuterated compound thereof is selected from the following group:

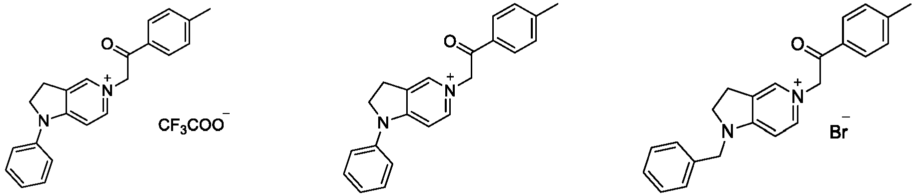

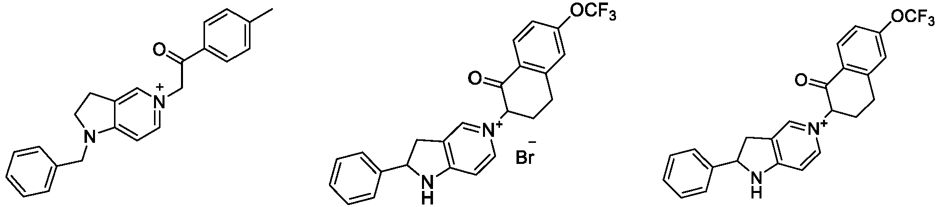

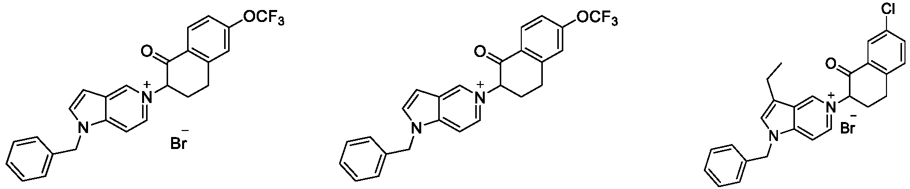

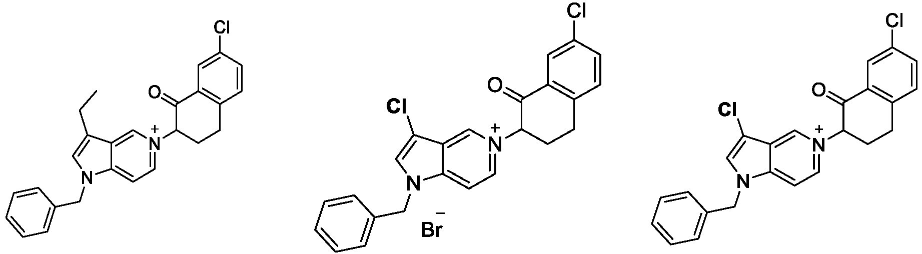

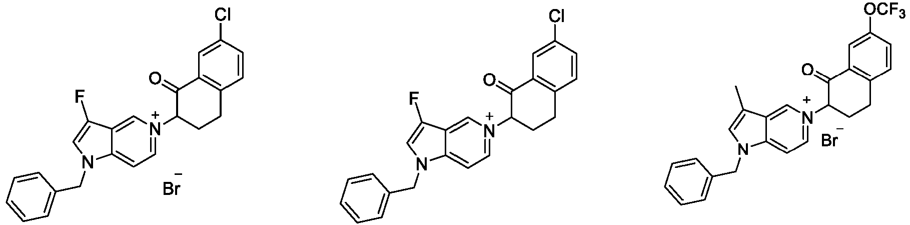

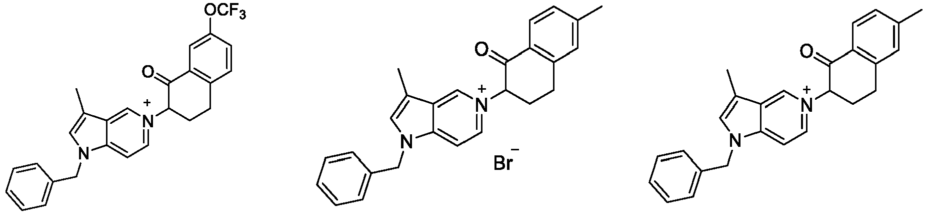

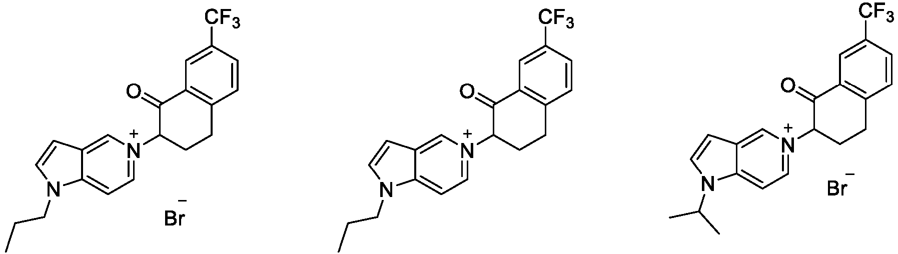

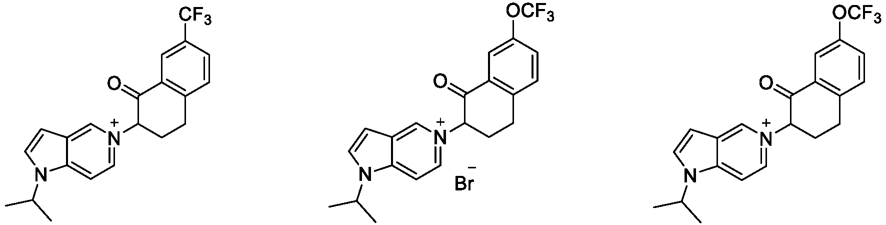

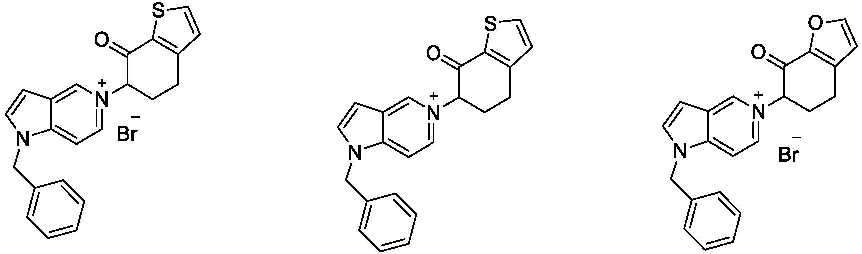

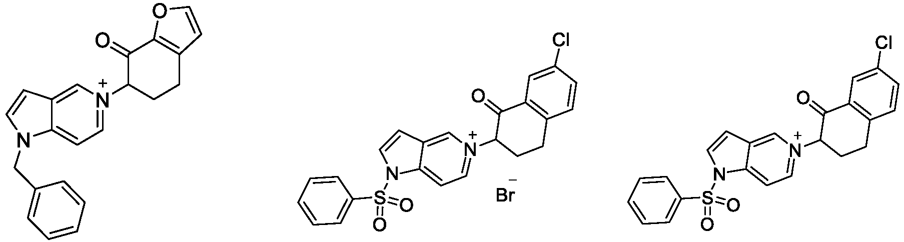

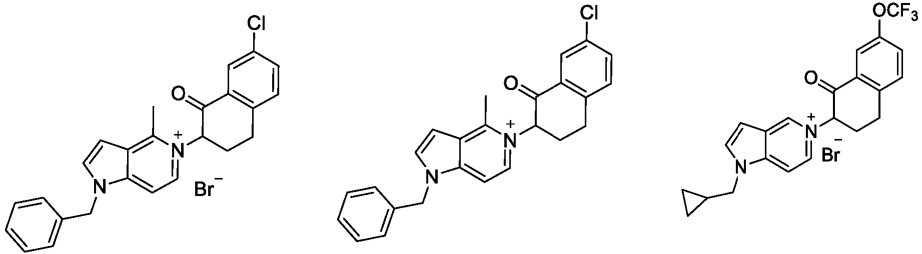

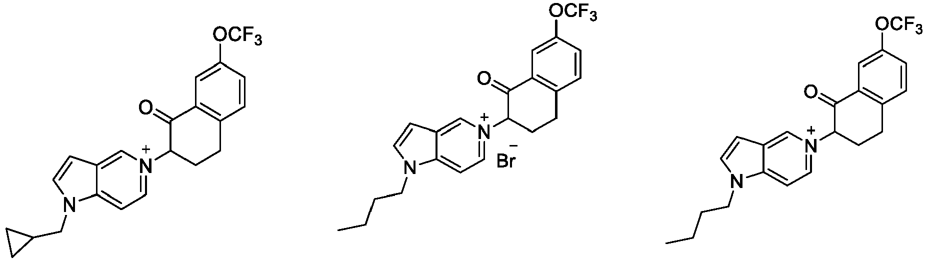

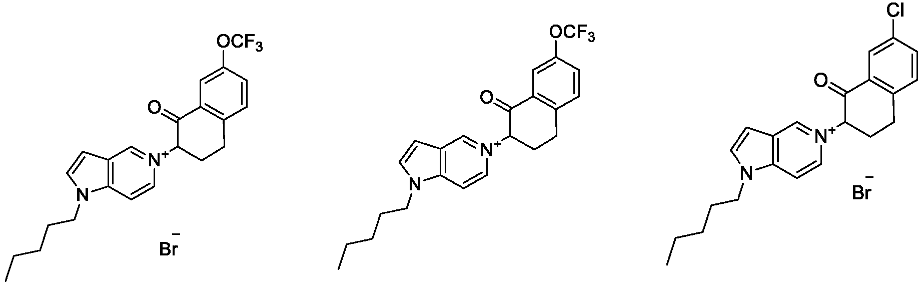

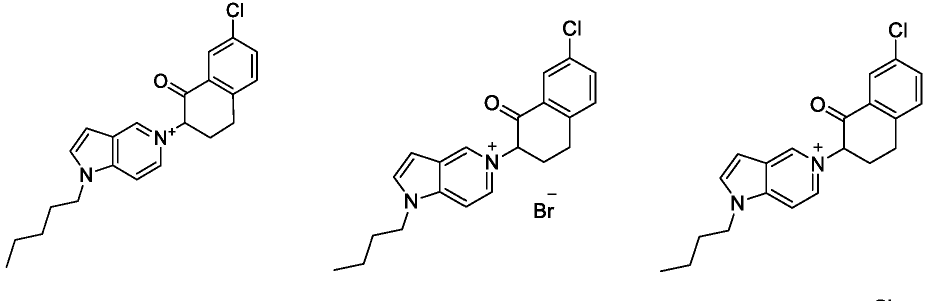

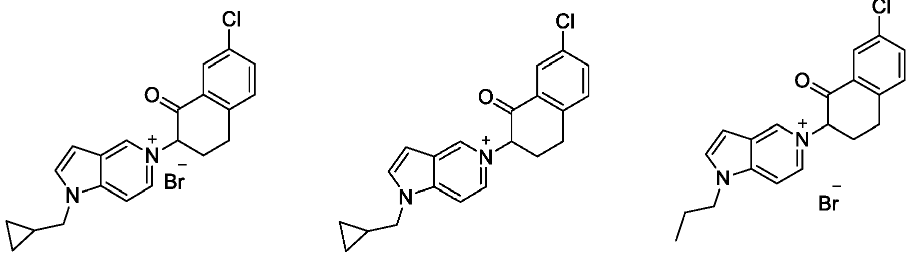

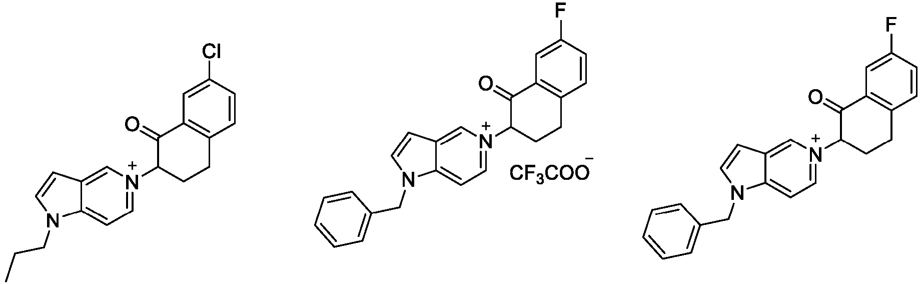

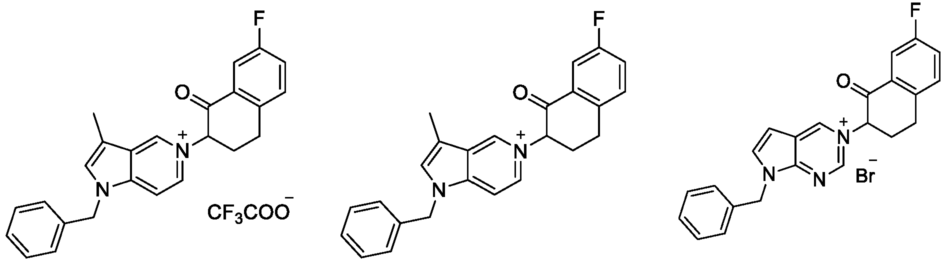

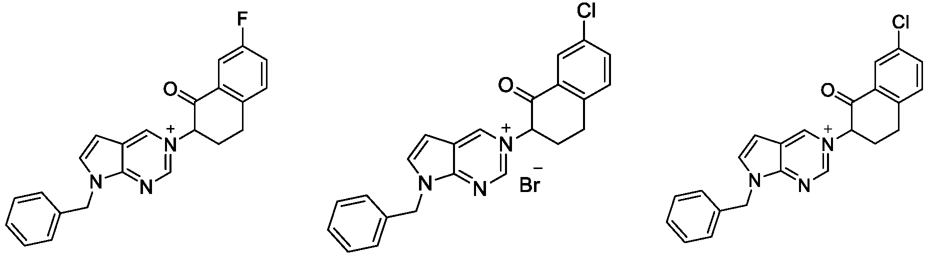

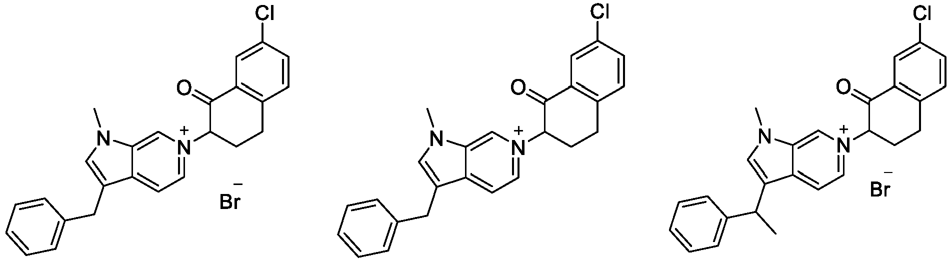

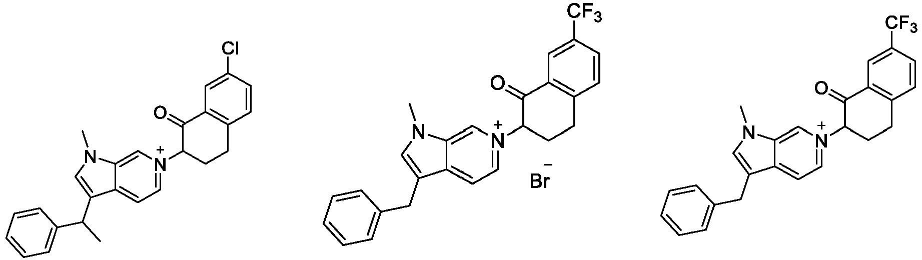

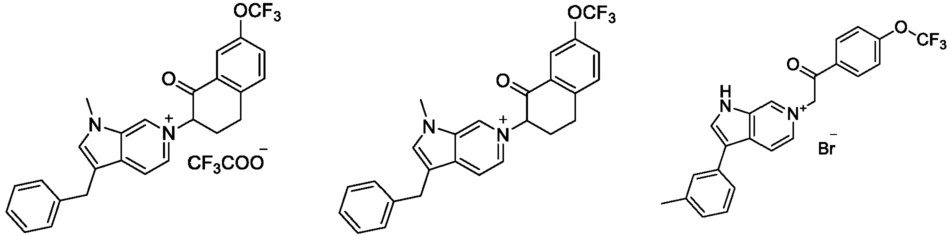

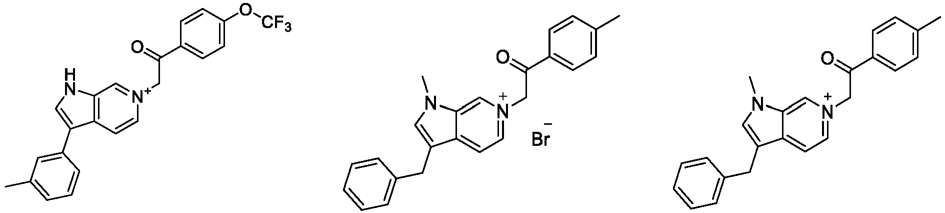

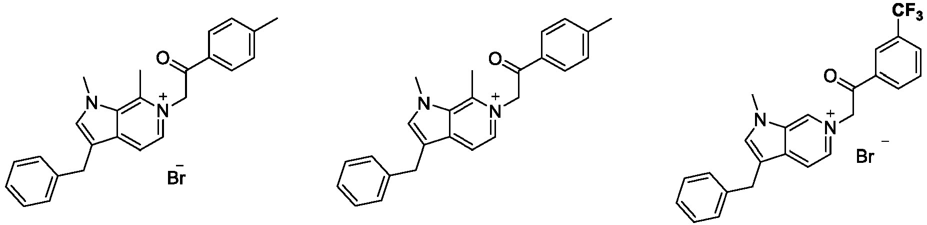

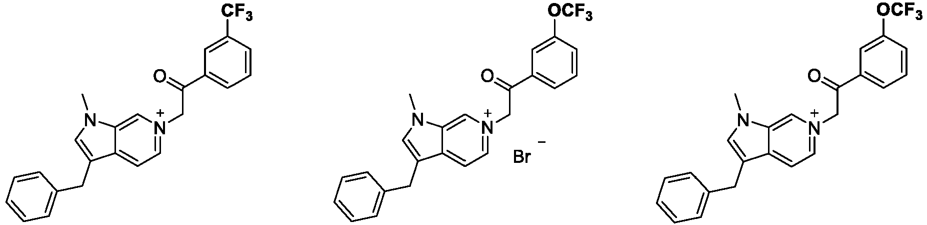

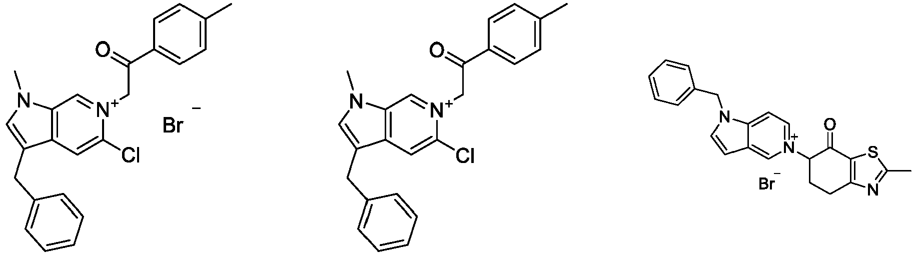

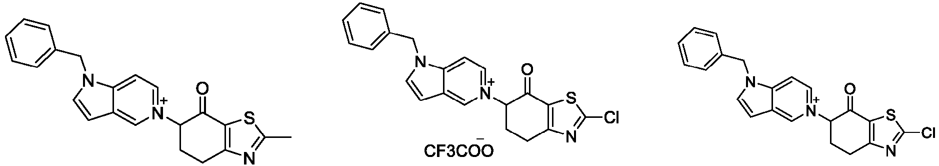

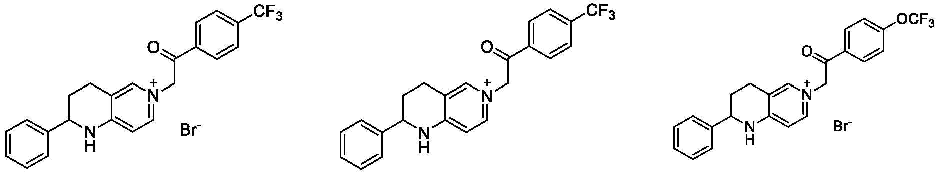

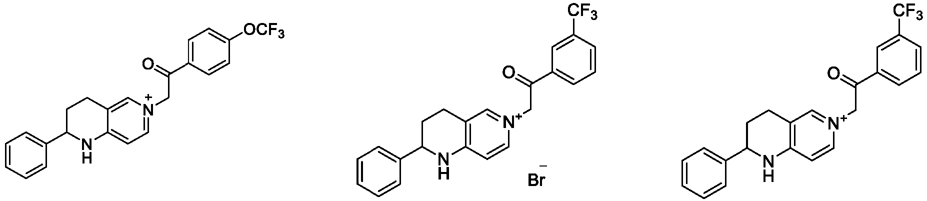

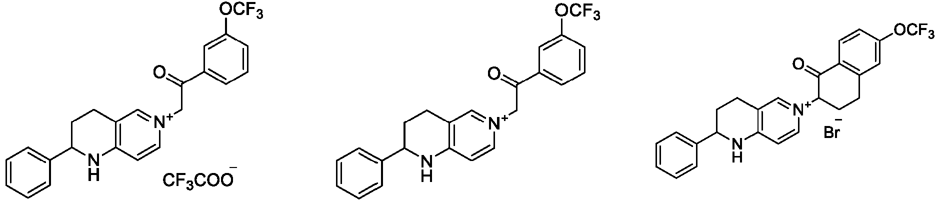

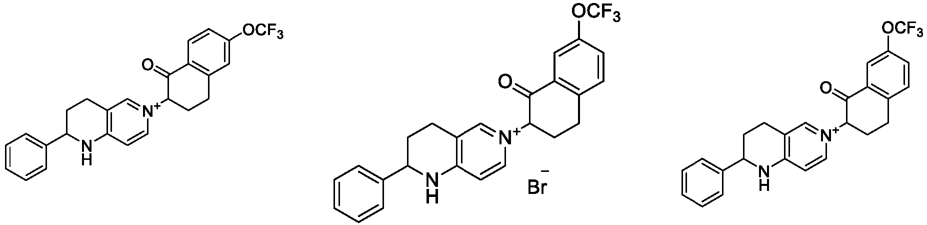

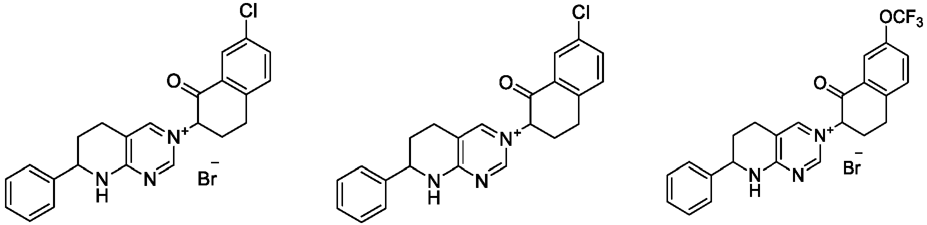

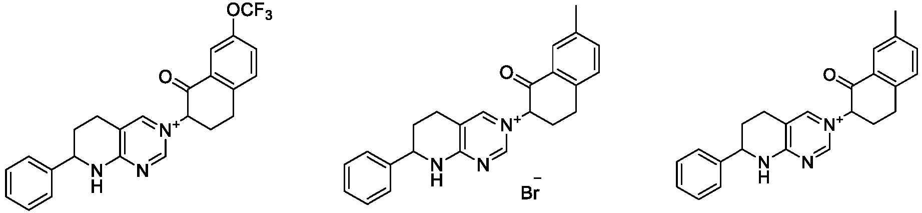

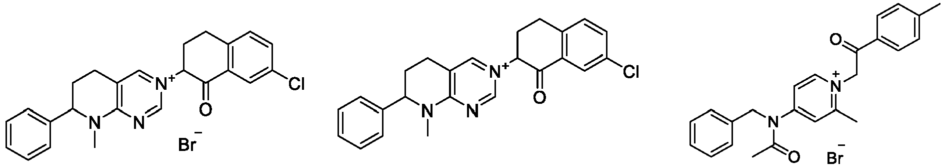

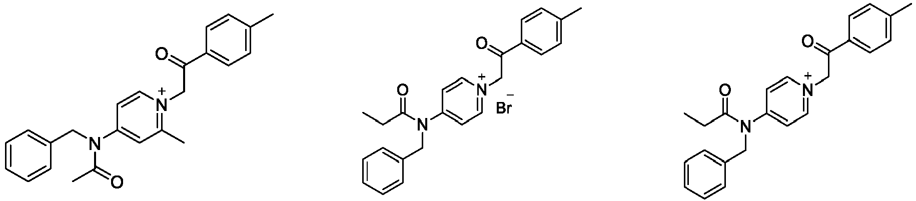

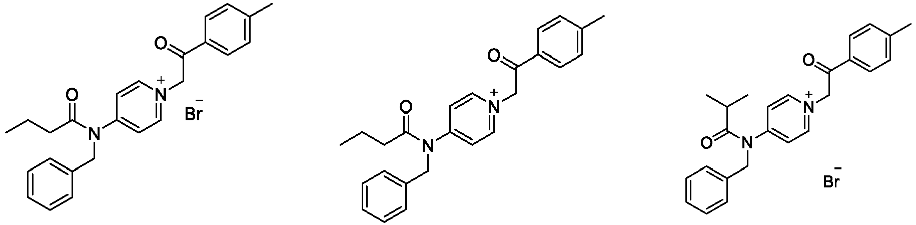

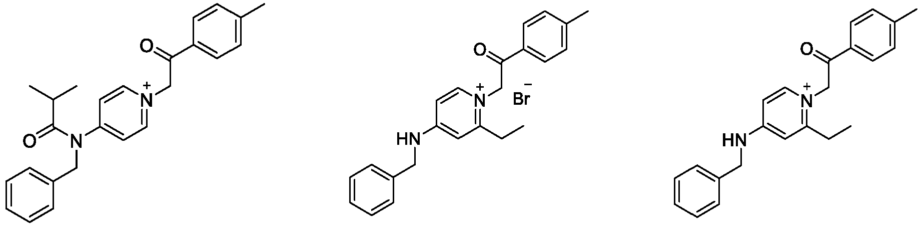

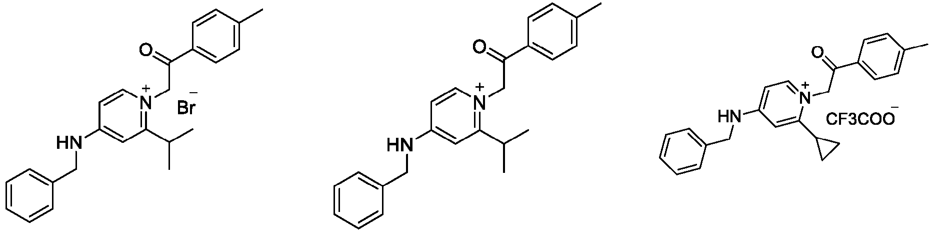

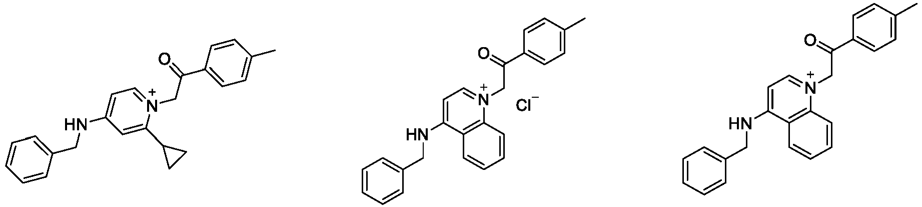

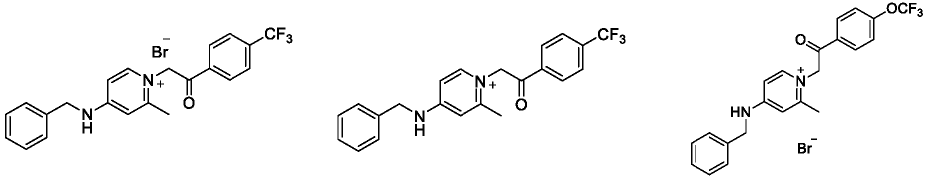

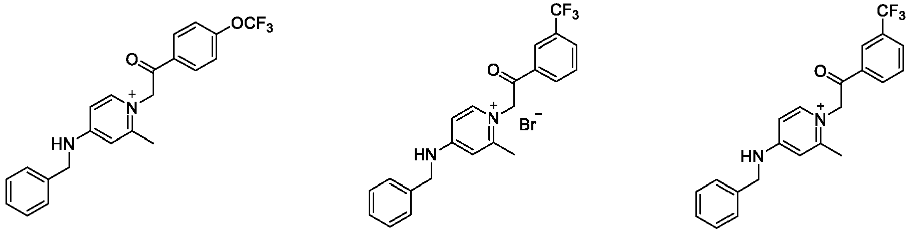

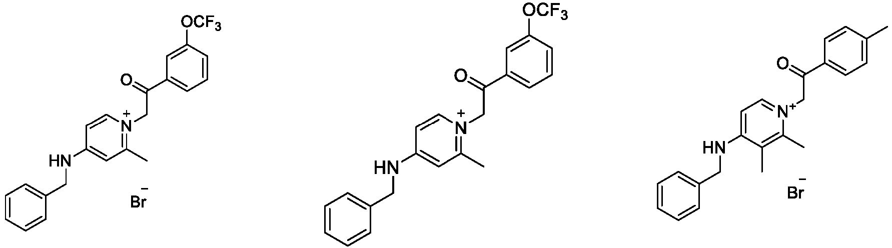

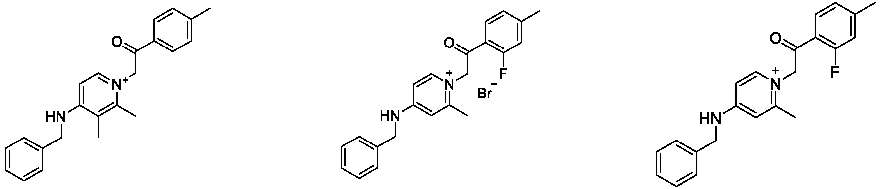

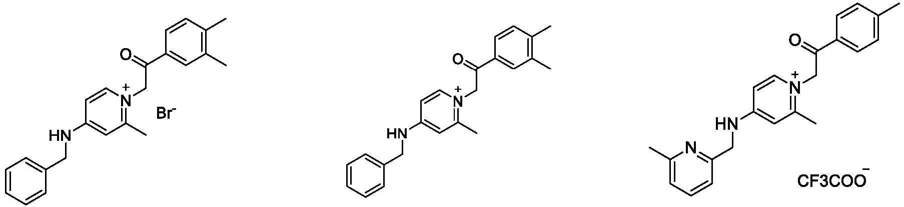

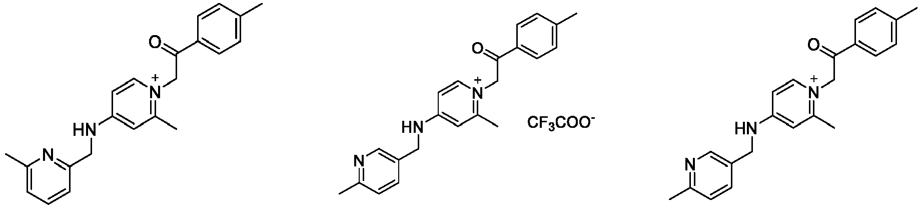

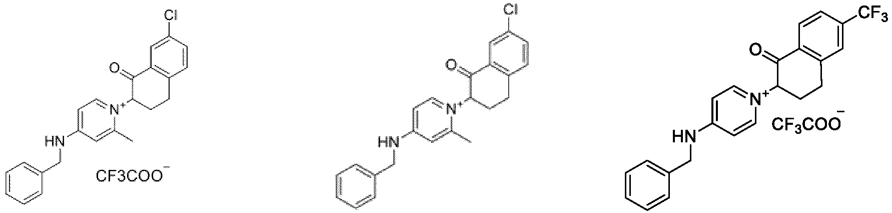

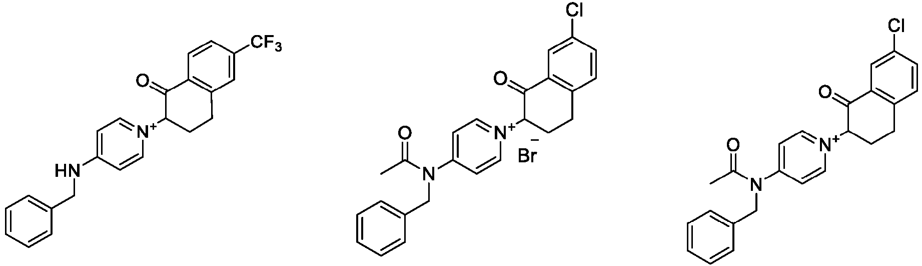

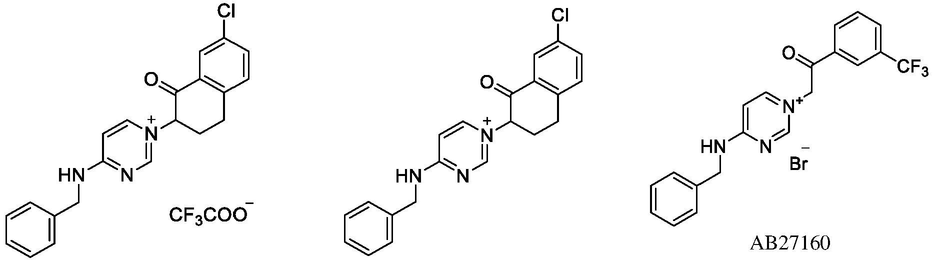

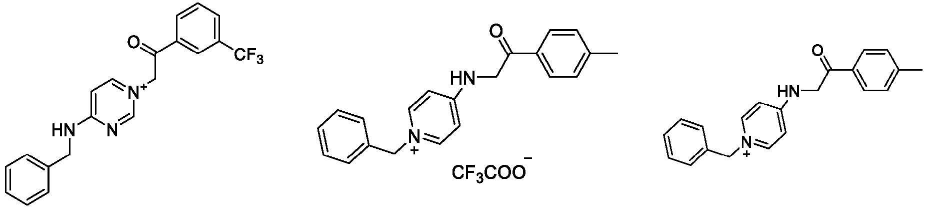

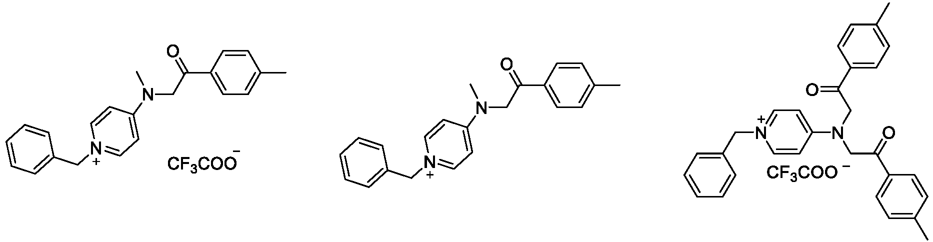

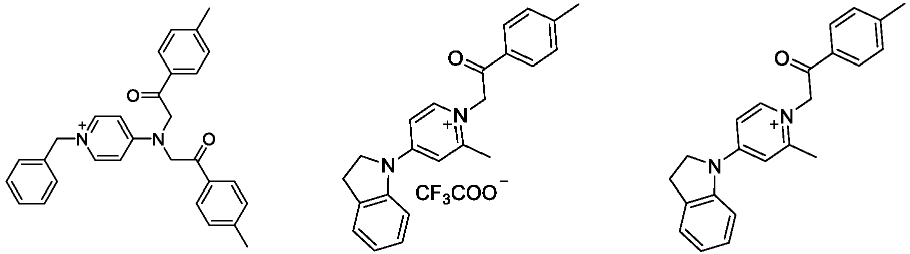

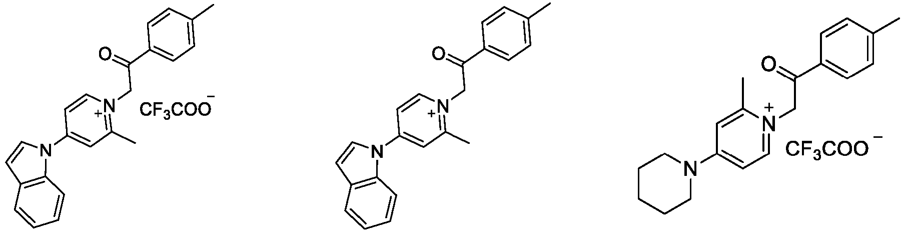

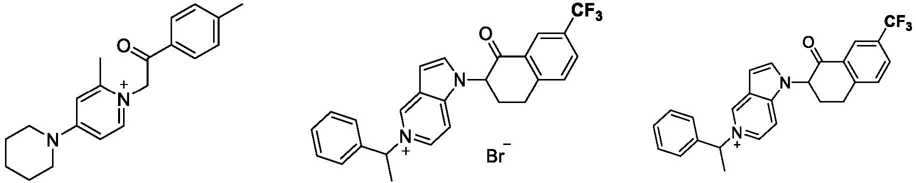

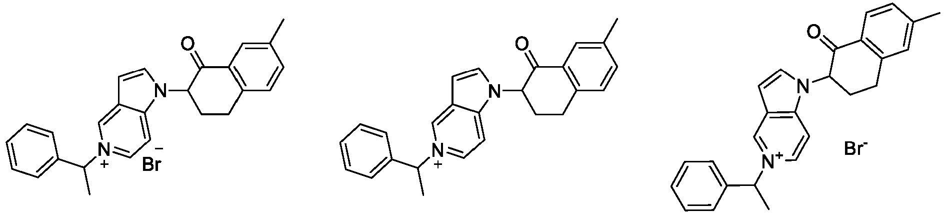

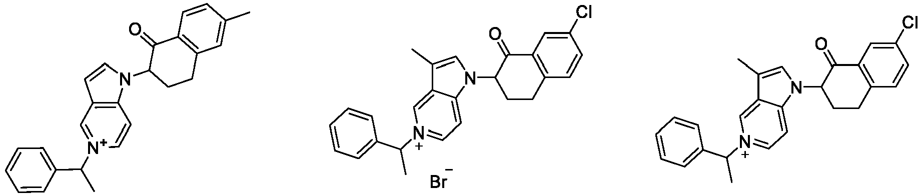

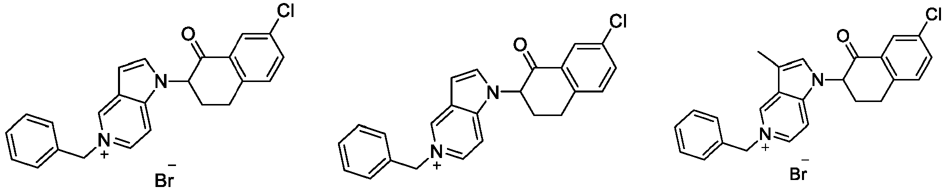

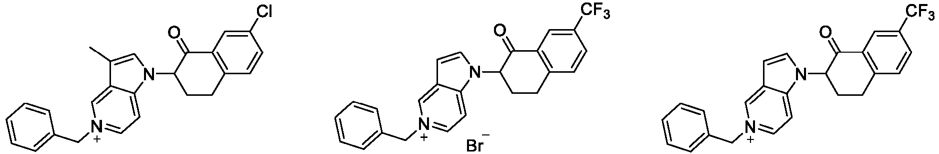

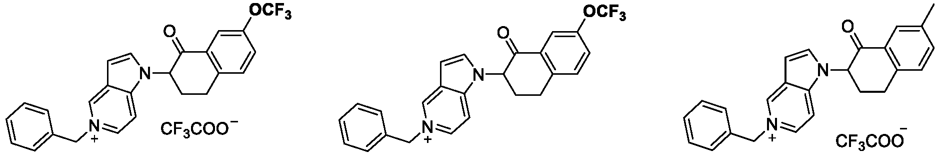

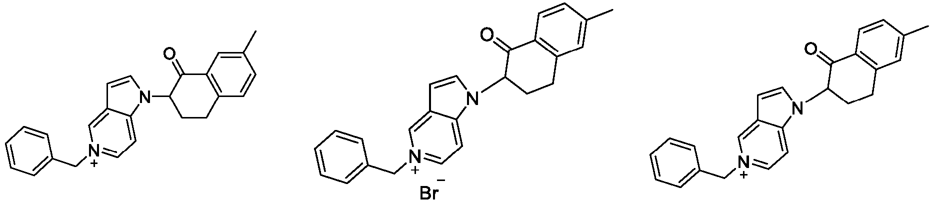

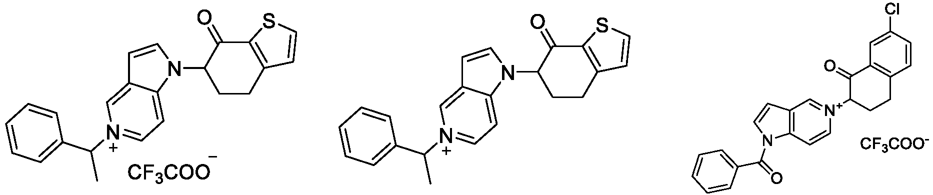

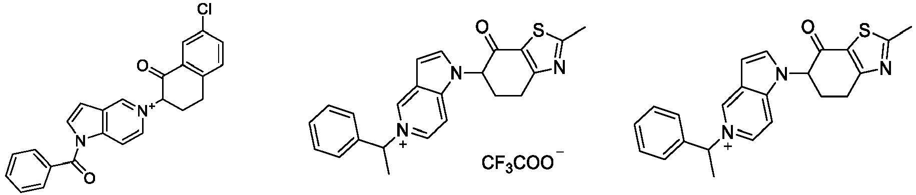

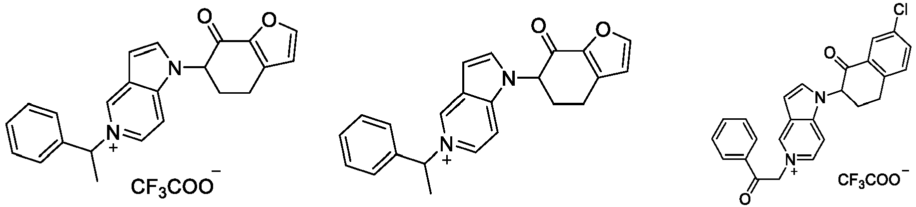

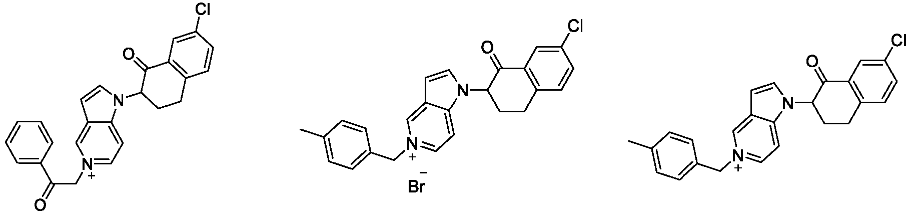

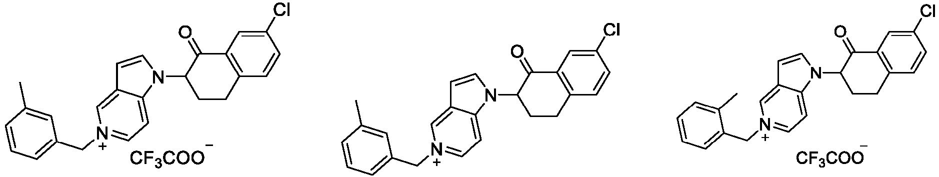

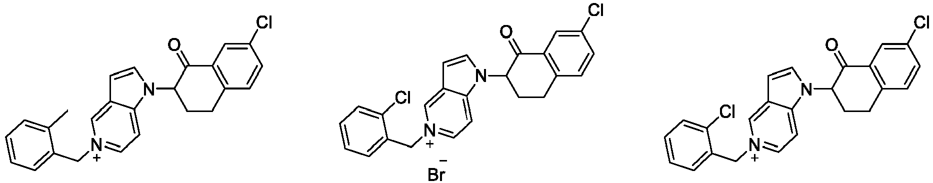

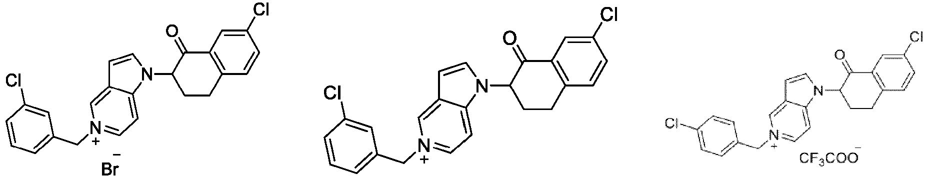

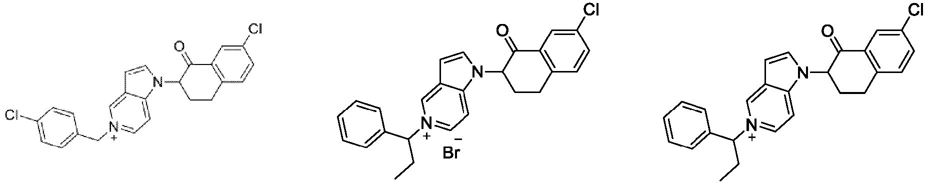

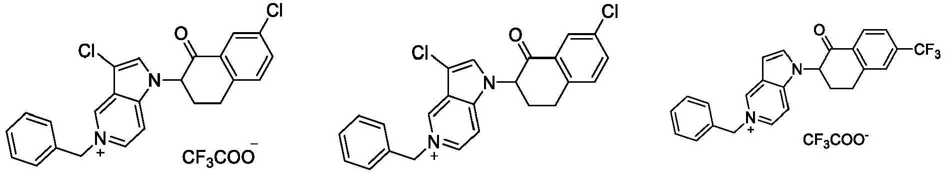

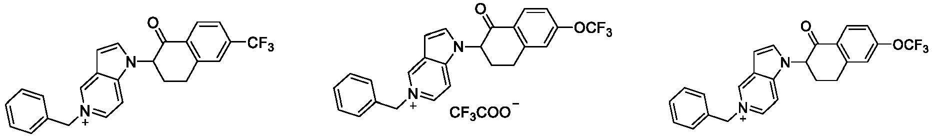

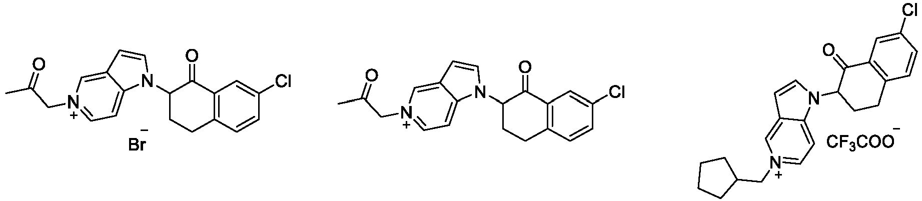

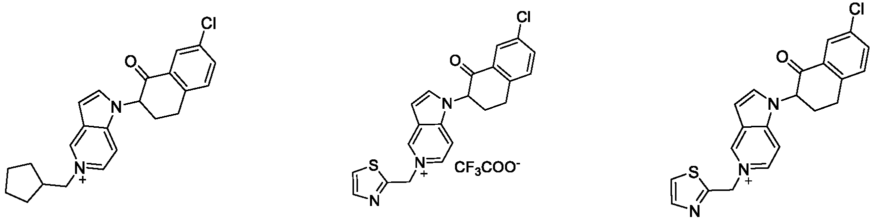

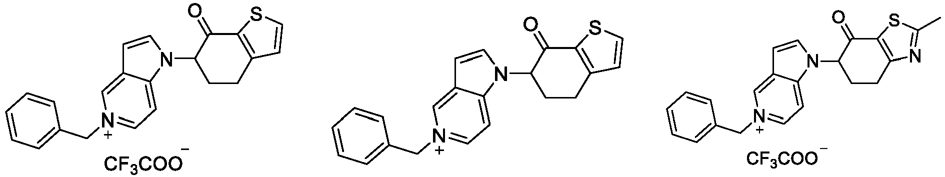

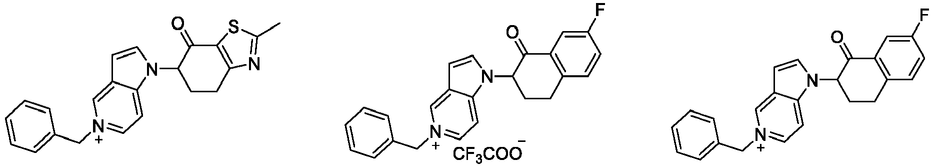

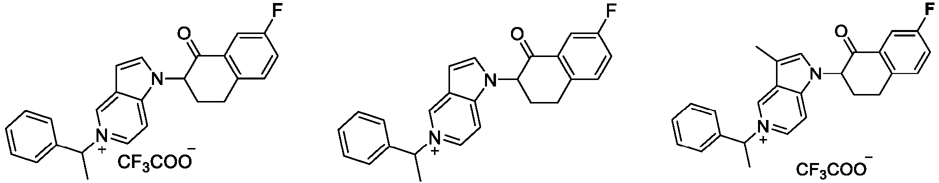

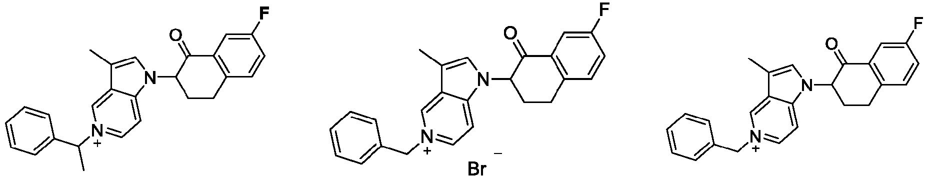

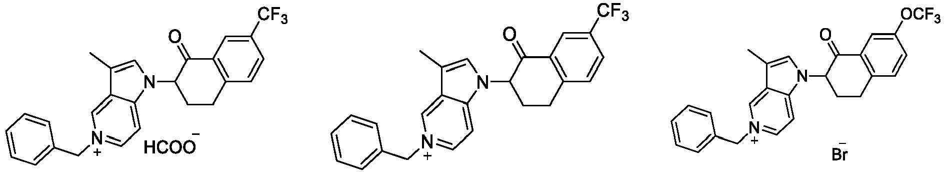

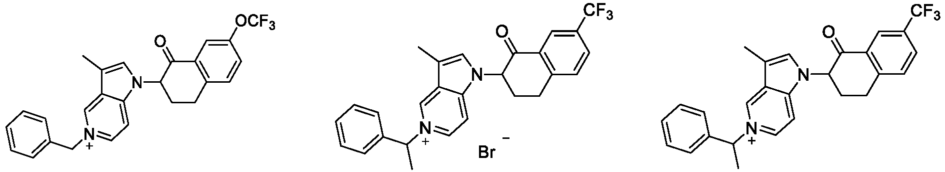

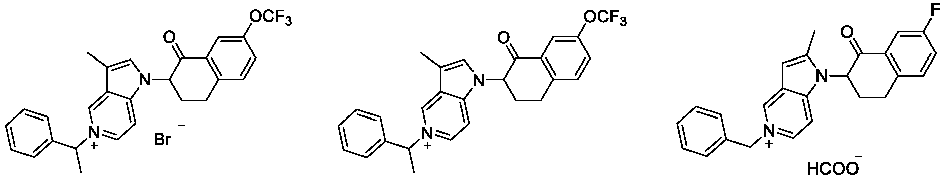

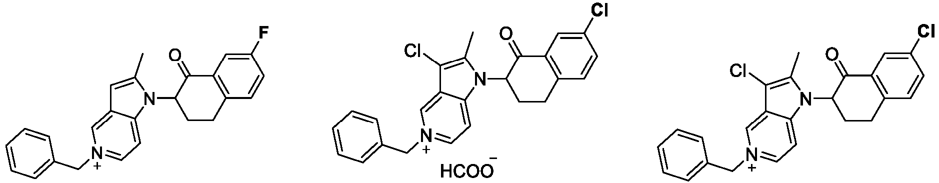

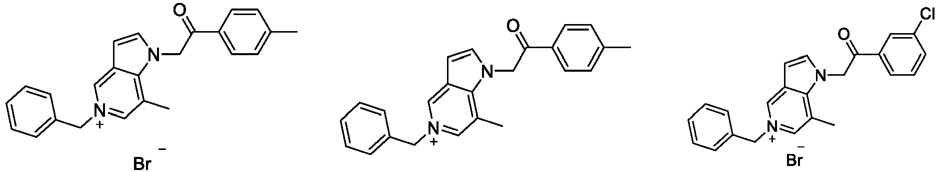

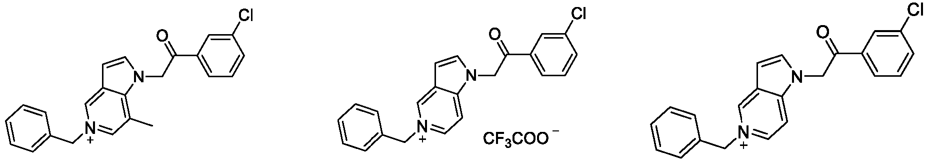

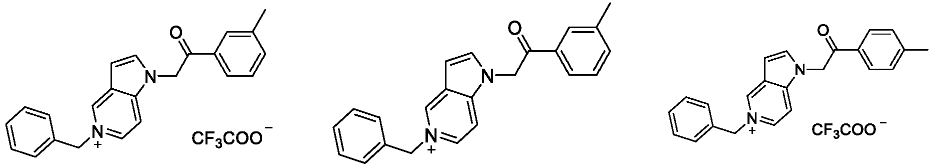

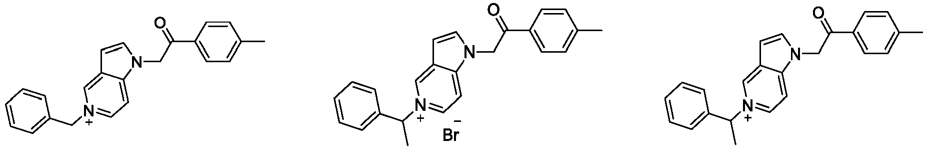

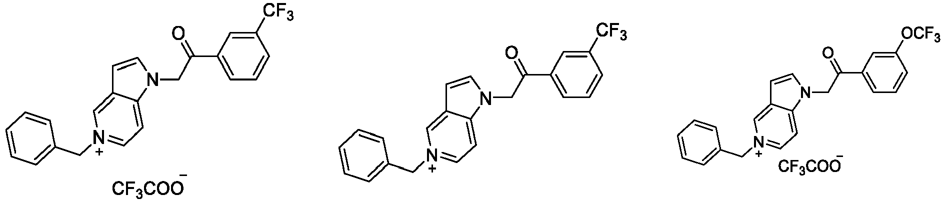

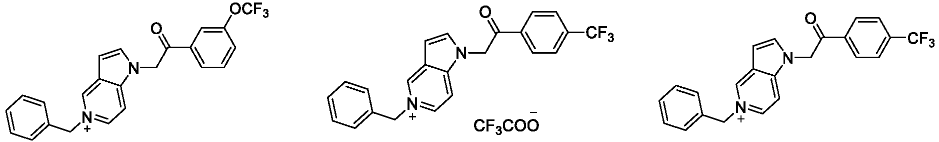

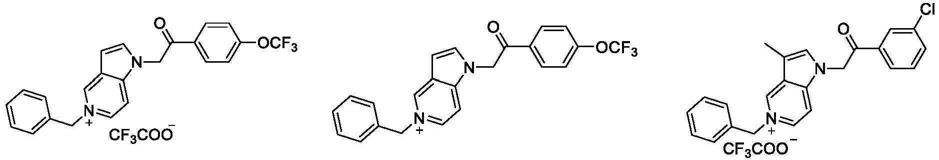

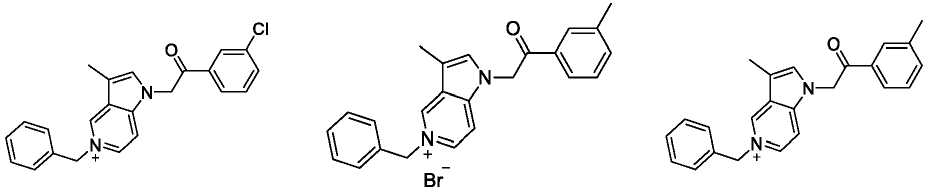

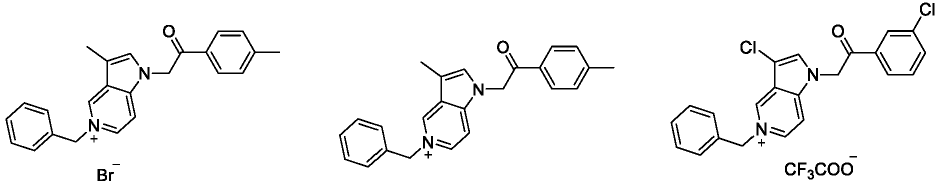

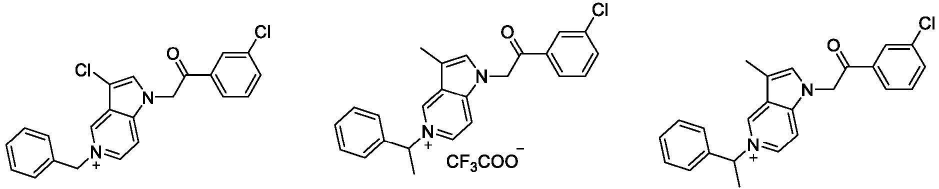

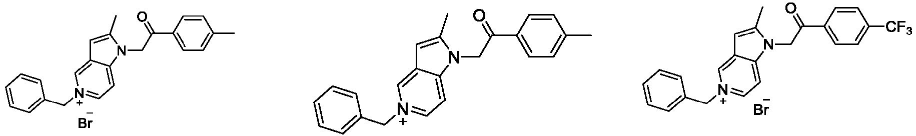

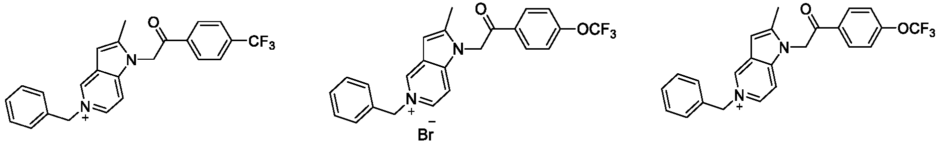

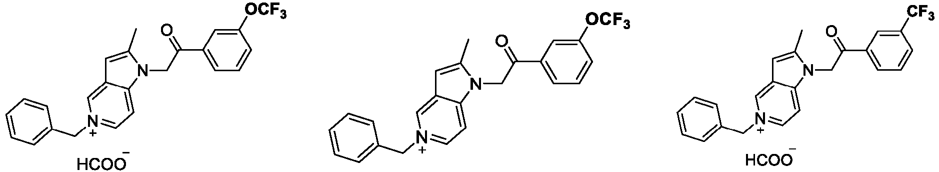

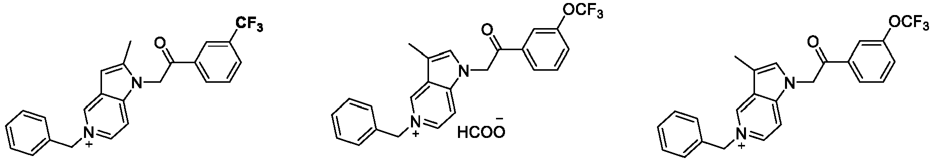

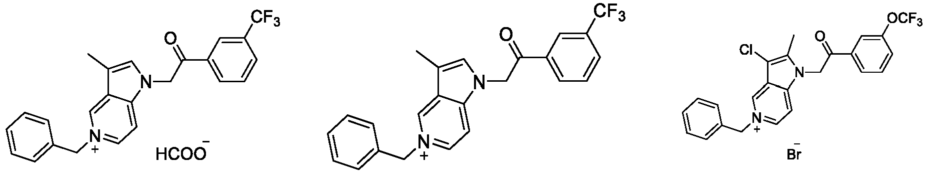

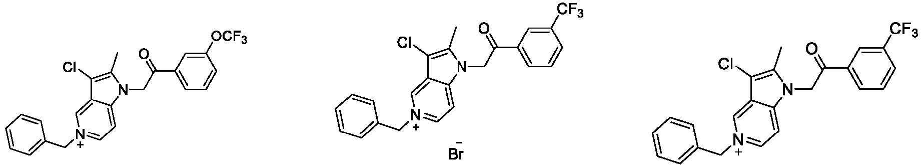

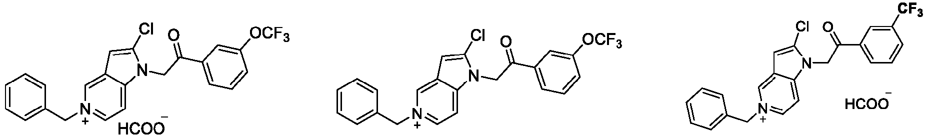

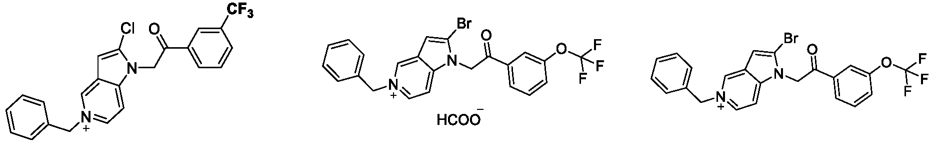

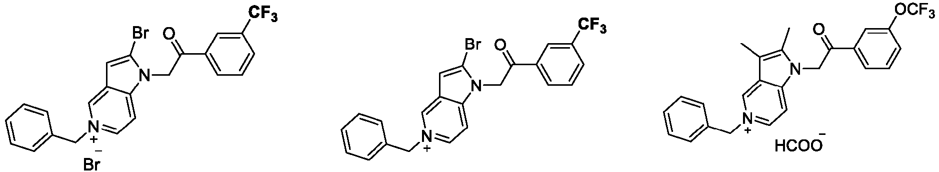

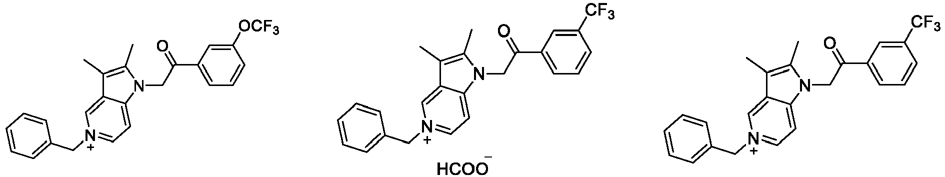

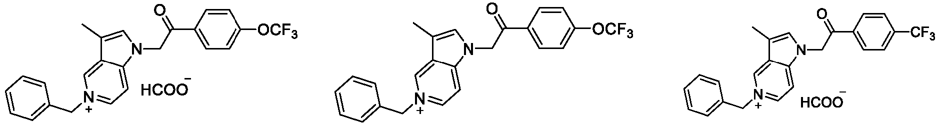

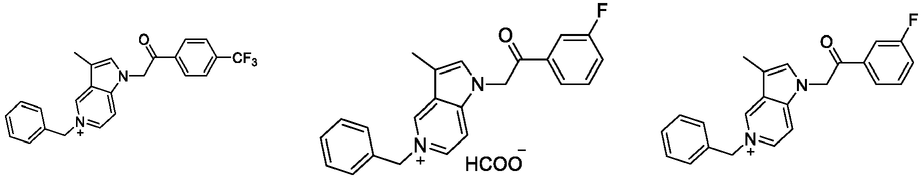

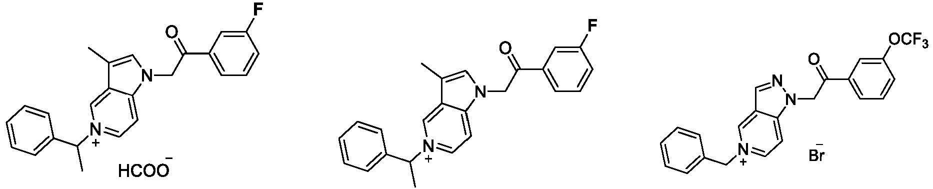

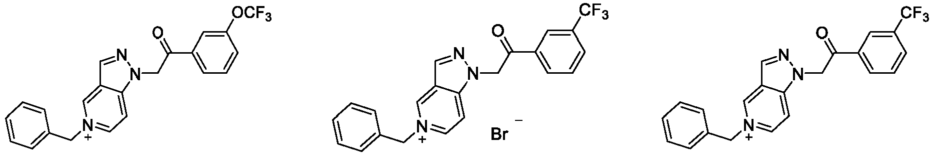

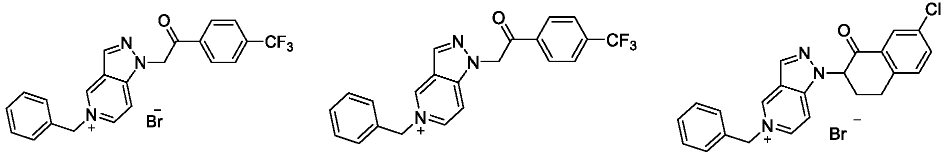

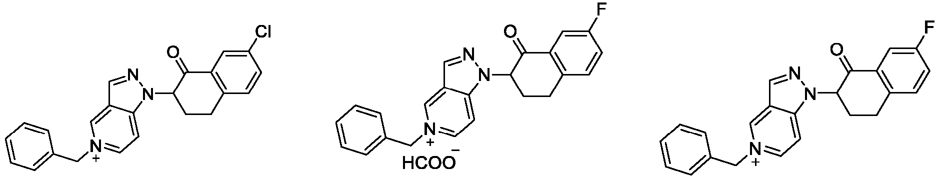

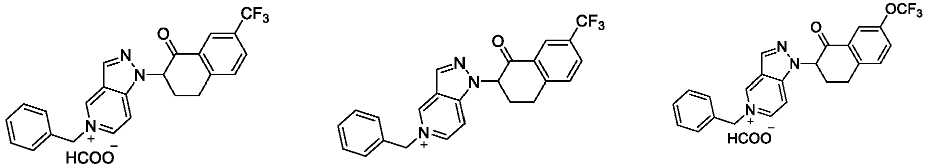

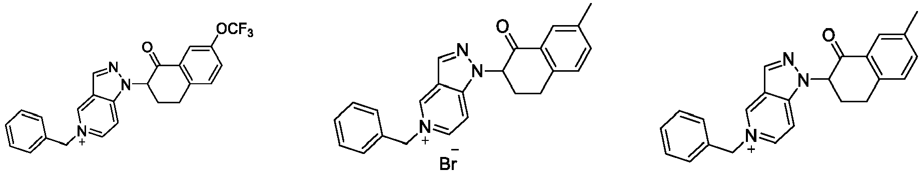

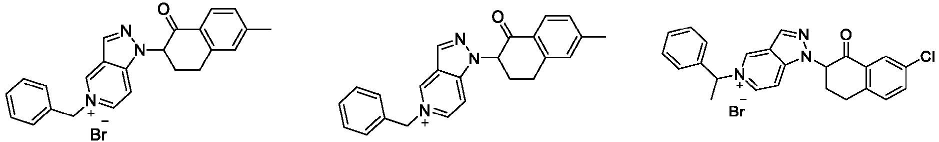

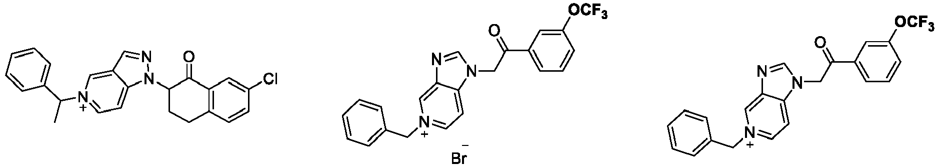

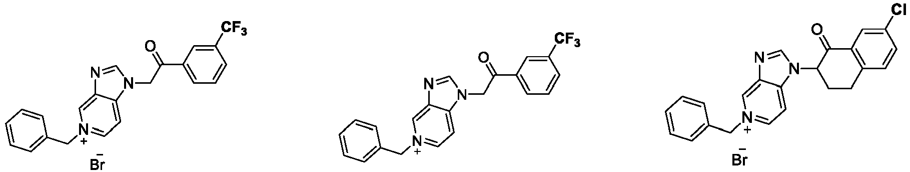

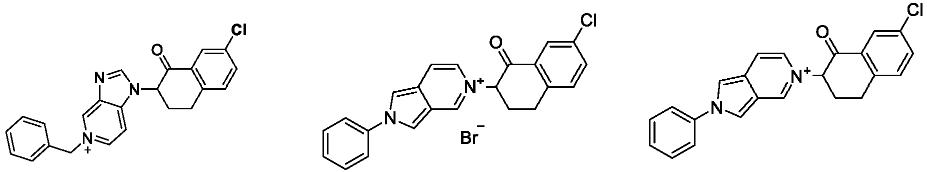

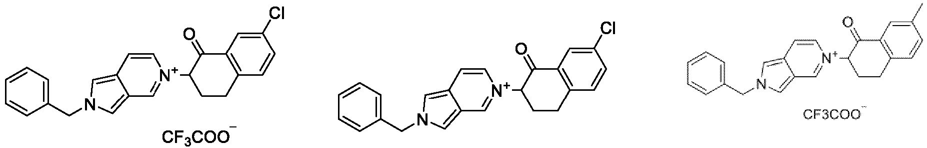

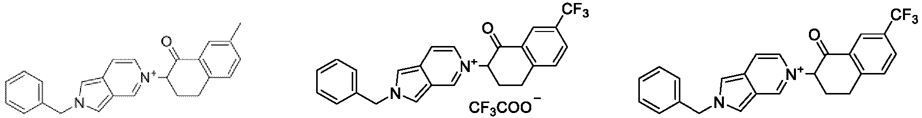

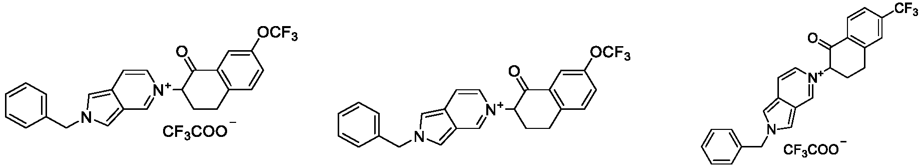

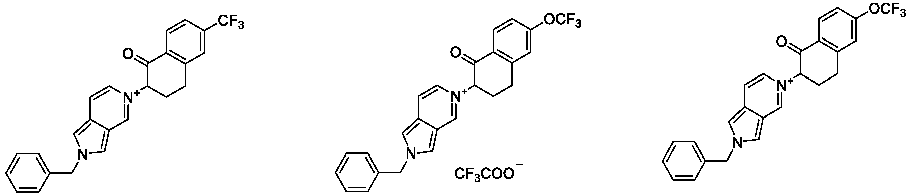

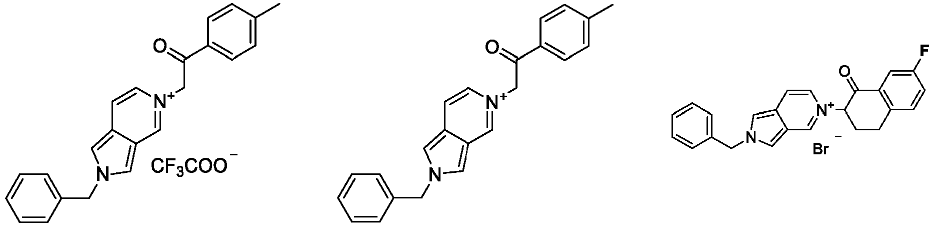

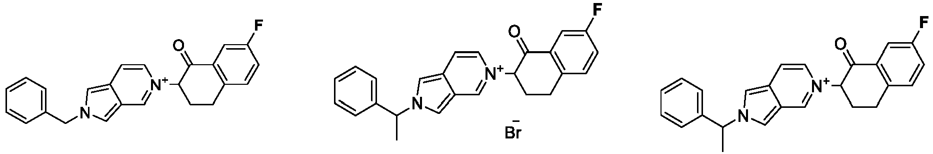

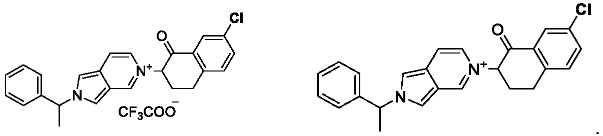

In the second aspect of the present invention, it provides a composition, the composition comprises (a) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the content of the (a) the compound of formula I, or an optical isomer thereof, or a racemate thereof, or a solvate thereof, or a pharmaceutically acceptable salt thereof, or a deuterated compound thereof according to the first aspect of the present invention is 0.001-99.9 wt%, based on the weight of the composition.

In another preferred embodiment, the composition is pharmaceutical composition.

In another preferred embodiment, the composition further comprises (b) a medically acceptable carrier

In another preferred embodiment, the composition further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the third aspect of the present invention, it provides a use of the compound of formula I, or an optical isomer thereof, or a racemate thereof, or a solvate thereof, or a pharmaceutically acceptable salt thereof, or a deuterated compound thereof according to the first aspect of the present invention in the preparation of a composition or a preparation for preventing and/or treating tumor.

In another preferred embodiment, the tumor is human-derived tumor.

In another preferred embodiment, the tumor is human tumor.

In another preferred embodiment, the tumor comprises tumor with low expression, no expression, low activity or no activity of mitochondria permeability transition pore.

In another preferred embodiment, the tumor comprises tumor with low expression, no expression, low activity or no activity of peptidyl-prolyl cis-trans isomerase F.

In another preferred embodiment, the protein identification number of peptidyl-prolyl cis-trans isomerase F is UniProtKB/Swiss Prot: P30405, and the gene identification number of peptidyl-prolyl cis-trans isomerase F is NCBI Entrez Gene: 10105.

In another preferred embodiment, the tumor comprises tumor with low or no expression of NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1.

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRFl.

In another preferred embodiment, the tumor comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the methylation of nucleotide site of NNMT gene comprises the methylation of cytosine nucleotide site of NNMT gene.

In another preferred embodiment, the methylation of nucleotide site of NNMT gene comprises the methylation of cytosine of NNMT gene.

In another preferred embodiment, the methylation of nucleotide site of NNMT gene comprises the methylation of the 5'carbon atom on the cytosine of the NNMT gene.

In another preferred embodiment, the tumor comprises tumor with high methylation level of DNA CpG site of NNMT gene.

In another preferred embodiment, the methylation of DNA CpG site of NNMT gene comprises the methylation of cytosine nucleotide site of DNA CpG site of NNMT gene.

In another preferred embodiment, the methylation of DNA CpG site of NNMT gene comprises the methylation of cytosine of DNA CpG site of NNMT gene.

In another preferred embodiment, the methylation of DNA CpG site of NNMT gene comprises the methylation of the 5'carbon atom on the cytosine of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low expression or low activity of mitochondria permeability transition pore means the expression level or activity level of mitochondria permeability transition pore in tumor cell is lower than the expression level or activity level of mitochondria permeability transition pore in the same type of cell or a normal cell (e.g., para-tumor tissue cell).

In another preferred embodiment, the low expression or low activity of mitochondria permeability transition pore means the ratio (H1/H0) of the expression level or activity level Hlof mitochondria permeability transition pore in a cell ( e.g., tumor cell ) to the expression level or activity level H0 of mitochondria permeability transition pore in the same type of cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the same type of cell comprises the same type of tumor cell.

In another preferred embodiment, the same type of cell comprises the cell (e.g., the same type of tumor cell) with normal expression, high expression, normal activity or high activity of mitochondria permeability transition pore.

In another preferred embodiment, the same type of cell comprises the same type of cell with normal expression, high expression, normal activity or high activity of mitochondria permeability transition pore

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression or normal activity of mitochondria permeability transition pore.

In another preferred embodiment, H0 refers to the expression level or activity level H0 of mitochondria permeability transition pore in the cell with normal expression, high expression, normal activity or high activity of mitochondria permeability transition pore.

In another preferred embodiment, the cell with normal expression, high expression, normal activity or high activity of mitochondria permeability transition pore comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with low expression or low activity of peptidyl-prolyl cis-trans isomerase F means the expression level or activity level of peptidyl-prolyl cis-trans isomerase F in tumor cell is lower than the expression level or activity level of peptidyl-prolyl cis-trans isomerase F in the same type of cell or a normal cell (e.g., para-tumor tissue cell).

In another preferred embodiment, the low expression or low activity of peptidyl-prolyl cis-trans isomerase F means the ratio (G1/G0) of the expression level or activity level Glof peptidyl-prolyl cis-trans isomerase F in a cell ( e.g., tumor cell ) to the expression level or activity level G0 of peptidyl-prolyl cis-trans isomerase F in the same type of cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises the same type of cell.

In another preferred embodiment, the same type of cell comprises the same type of tumor cell.

In another preferred embodiment, the same type of cell comprises the cell (e.g., the same type of tumor cell) with normal expression, high expression, normal activity or high activity of peptidyl-prolyl cis-trans isomerase F.

In another preferred embodiment, the same type of cell comprises the same type of cell with normal expression, high expression, normal activity or high activity of peptidyl-prolyl cis-trans isomerase F.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression or normal activity of peptidyl-prolyl cis-trans isomerase F.

In another preferred embodiment, G0 refers to the expression level or activity level H0 of peptidyl-prolyl cis-trans isomerase F in the cell with normal expression, high expression, normal activity or high activity of peptidyl-prolyl cis-trans isomerase F.

In another preferred embodiment, the cell with normal expression, high expression, normal activity or high activity of peptidyl-prolyl cis-trans isomerase F comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell (e.g., para-tumor tissue cell).

In another preferred embodiment, the tumor with low or no expression of NNMT gene means the ratio (E1/E0) of the expression level E1 of NNMT gene in the tumor cell to the expression level E0 of NNMT gene in the same type of cell or a normal cell (e.g., para-tumor tissue cell) is <1.0.

In another preferred embodiment, the low or no expression of NNMT gene means the ratio (E1/E0) of the expression E1 of NNMT gene in a cell ( e.g., tumor cell ) to the expression E0 of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the same type of cell comprises the same type of tumor cell.

In another preferred embodiment, the same type of cell comprises the cell (e.g., the same type of tumor cell) with normal or high expression of NNMT gene.

In another preferred embodiment, the same type of cell comprises the same type of cell with normal or high expression of NNMT gene.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal or high expression of NNMT gene.

In another preferred embodiment, the cell with normal or high expression of NNMT gene comprises the cell that is not sensitive to the compound of formula I, or an optical isomer thereof, or a racemate thereof, or a solvate thereof, or a pharmaceutically acceptable salt thereof.

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same type of cell or a normal cell (e.g., para-tumor tissue cell).

In another preferred embodiment, the tumor with high expression of DNA methylase means the ratio (A1/A0) of the expression level A1 of DNA methylase in the tumor cell to the expression level A0 of DNA methylase in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of DNA methylase.

In another preferred embodiment, the same type of cell comprises the same type of cell with normal or low expression of DNA methylase.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNA methylase.

In another preferred embodiment, A0 refers to the expression level of DNA methylase in the cell with normal or low expression of DNA methylase.

In another preferred embodiment, the cell with normal or low expression of DNA methylase comprises the cell that is not sensitive to the compound of formula I, or an optical isomer thereof, or a racemate thereof, or a solvate thereof, or a pharmaceutically acceptable salt thereof.

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor type of cell is higher than that in the same type of cell or a normal cell (e.g., para-tumor tissue cell).

In another preferred embodiment, the tumor with high expression of DNMT1 means the ratio (B1/B0) of the expression level B1 of DNMT1 in the tumor cell to the expression level B0 of DNMT1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell ) with normal or low expression of DNMT1.

In another preferred embodiment, the same type of cell comprises the same type of cell with normal or low expression of DNMT1.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT1.

In another preferred embodiment, B0 refers to the expression level of DNMT1 in the cell with normal or low expression of DNMT1.

In another preferred embodiment, the cell with normal or low expression of DNMT1 comprises the cell that is not sensitive to the compound of formula I, or an optical isomer thereof, or a racemate thereof, or a solvate thereof, or a pharmaceutically acceptable salt thereof.

In another preferred embodiment, the tumor with high expression of DNMT3a means that DNMT3a protein can be detected in 20 µg of protein extracted from tumor using DNMT3 a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same type of cell or a normal cell (e.g., para-tumor tissue cell).

In another preferred embodiment, the tumor with high expression of DNMT3a means the ratio (C1/C0) of the expression level C1 of DNMT3a in the tumor cell to the expression level C0 of DNMT3a in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell ) with normal or low expression of DNMT3a.

In another preferred embodiment, the same type of cell comprises the same type of cell with normal or low expression of DNMT3a.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT3a.

In another preferred embodiment, C0 refers to the expression level of DNMT3a in the cell with normal or low expression of DNMT3a.

In another preferred embodiment, the cell with normal or low expression of DNMT3a comprises the cell that is not sensitive to the compound of formula I, or an optical isomer thereof, or a racemate thereof, or a solvate thereof, or a pharmaceutically acceptable salt thereof.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor type of cell is higher than that in the same type of cell or a normal cell (e.g., para-tumor tissue cell).

In another preferred embodiment, the tumor with high expression of DNMT3b means the ratio (D1/D0) of the expression level D1 of DNMT3b in the tumor cell to the expression level D0 of DNMT3b in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of DNMT3b.

In another preferred embodiment, the same type of cell comprises the same type of cell with normal or low expression of DNMT3b.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT3b

In another preferred embodiment, D0 refers to the expression level of DNMT3b in the cell with normal or low expression of DNMT3b.

In another preferred embodiment, the cell with normal or low expression of DNMT3b comprises the cell that is not sensitive to the compound of formula I, or an optical isomer thereof, or a racemate thereof, or a solvate thereof, or a pharmaceutically acceptable salt thereof.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF1 means the expression level of UHRF1 in tumor type of cell is higher than that in the same type of cell or a normal cell (e.g., para-tumor tissue cell).

In another preferred embodiment, the tumor with high expression of UHRF1 means the ratio (F1/F0) of the expression level F1 of UHRF1 in the tumor cell to the expression level F0 of UHRF1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of UHRF1.

In another preferred embodiment, the same type of cell comprises the same type of cell with normal or low expression of UHRF1.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of UHRF1

In another preferred embodiment, F0 refers to the expression level of UHRF1 in the cell with normal or low expression of UHRF1.

In another preferred embodiment, the cell with normal or low expression of UHRF1 comprises the cell that is not sensitive to the compound of formula I, or an optical isomer thereof, or a racemate thereof, or a solvate thereof, or a pharmaceutically acceptable salt thereof.

In another preferred embodiment, the high methylation level of nucleotide site of NNMT gene means the methylation level of nucleotide site of NNMT gene in a cell ( e.g., tumor cell ) is higher than that in the same type of cell or a normal cell (e.g., para-tumor tissue cell).

In another preferred embodiment, the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of nucleotide site of NNMT gene means the methylation level of nucleotide site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type cell comprises the same type of tumor cell.

In another preferred embodiment, the same type of cell comprises the cell (e.g., the same type of tumor cell) with normal or low methylation level of nucleotide site of NNMT gene.

In another preferred embodiment, the same type of cell comprises the same type of cell with normal or low methylation level of nucleotide site of NNMT gene.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal methylation level of nucleotide site of NNMT gene.

In another preferred embodiment, the cell with normal or low methylation level of nucleotide site of NNMT gene comprises the cell that is not sensitive to the compound of formula I, or an optical isomer thereof, or a racemate thereof, or a solvate thereof, or a pharmaceutically acceptable salt thereof.

In another preferred embodiment, the high methylation level of nucleotide site of NNMT gene means the methylation level (M%) of nucleotide site of NNMT gene in a cell ( e.g., tumor cell) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide site of one or more (e.g., 2, 3, 4, 5, 6, or 7 ) of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide site of one or more (e.g., 2, 3, 4, 5, 6, or 7 ) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell (e.g., tumor cell) is higher than that in the same type of cell or a normal cell (e.g., para-tumor tissue cell).

In another preferred embodiment, the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of DNA CpG site of NNMT gene means the methylation level of DNA CpG site of NNMT gene in a cell (e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type of cell comprises the same type of tumor cell.

In another preferred embodiment, the same type of cell comprises the cell (e.g., the same type of tumor cell) with normal or low methylation level of DNA CpG site of NNMT gene.

In another preferred embodiment, the same type of cell comprises the same type of cell with normal or low methylation level of DNA CpG site of NNMT gene.

In another preferred embodiment, the normal cell comprises normal tissue cell (e.g., tumor origin cell, tumor-adjacent cell or para-tumor tissue cell) with normal methylation level of DNA CpG site of NNMT gene.

In another preferred embodiment, the cell with normal or low methylation level of DNA CpG site of NNMT gene comprises the cell that is not sensitive to the compound of formula I, or an optical isomer thereof, or a racemate thereof, or a solvate thereof, or a pharmaceutically acceptable salt thereof.

In another preferred embodiment, the high methylation level of DNA CpG site of NNMT gene means the methylation level (M%) of DNA CpG site of NNMT gene in a cell ( e.g., tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated DNA CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated DNA CpG sites to the number of all DNA CpG sites in the NNMT gene.

In another preferred embodiment, the methylation level of DNA CpG site of NNMT gene refers to the ratio of the number of methylated DNA CpG nucleotides to the number of all DNA CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of DNA CpG site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of the site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the mitochondria permeability transition pore inhibitor is administered to achieve low expression, no expression, low activity or no activity of mitochondria permeability transition pore in tumor.

In another preferred embodiment, the peptidyl-prolyl cis-trans isomerase F inhibitor is administered to achieve low expression, no expression, low activity or no activity of peptidyl-prolyl cis-trans isomerase F.

In another preferred embodiment, the inhibitor comprises specific inhibitor.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the NNMT gene inhibitor is administered to achieve low or no expression of the NNMT gene in tumor.

In another preferred embodiment, the DNA methylase promoter is administered to achieve high expression of DNA methylase in tumor.

In another preferred embodiment, the DNMT1 promoter is administered to achieve high expression of DNMT1 in tumor.

In another preferred embodiment, the DNMT3a promoter is administered to achieve high expression of DNMT3a in tumor.

In another preferred embodiment, the DNMT3b promoter is administered to achieve high expression of DNMT3b in tumor.

In another preferred embodiment, the UHRFl promoter is administered to achieve high expression of UHRFl in tumor.

In another preferred embodiment, the methylation promoter of nucleotide site of NNMT gene is administered to achieve high methylation level of nucleotide site of NNMT gene in tumor.

In another preferred embodiment, the methylation promoter of DNA CpG site of NNMT gene is administered to achieve high methylation level of DNA CpG site of NNMT gene in tumor.

In another preferred embodiment, the inhibitor comprises specific inhibitor.

In another preferred embodiment, the promoter comprises specific promoter.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNMT1 promoter comprises promoter that can achieve high expression of DNMT1 in tumor.

In another preferred embodiment, the DNMT3a promoter comprises promoter that can achieve high expression of DNMT3a in tumor.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation promoter of DNA CpG site of NNMT gene in tumor.

In another preferred embodiment, the tumor is selected from the group consisting of lung cancer, renal carcinoma, breast cancer, colon cancer, lymphoma, leukemia, pancreatic cancer, brain tumor, liver cancer, prostate cancer, and combinations thereof.

In another preferred embodiment, the lung cancer is selected from the group consisting of nonsmall cell lung cancer, small cell lung cancer, and combinations thereof.

In another preferred embodiment, the lung cancer cell comprises NCI-H82 cell.

In another preferred embodiment, the colon cancer comprises colon adenocarcinoma.

In another preferred embodiment, the colon cancer cell comprises SW48 cell.

In another preferred embodiment, the breast cancer cell comprises MDA-MB-453 cell.

In another preferred embodiment, the breast cancer comprises triple negative breast cancer.

In another preferred embodiment, the lymphoma is selected from the group consisting of B-cell lymphoma, skin T-cell lymphoma, and combinations thereof.

In another preferred embodiment, the lymphoma comprises diffuse large B-cell lymphoma.

In another preferred embodiment, the brain tumor is selected from the group consisting of brain glioblastoma, neuroglioma, brain medulloblastoma, brain neuroblastoma, and combination thereof.

In another preferred embodiment, the brain medulloblastoma comprises cerebellar medulloblastoma.

In another preferred embodiment, the brain glioblastoma comprises glioblastoma multiforme.

In another preferred embodiment, the brain tumor comprises glioblastoma.

In another preferred embodiment, the brain tumor comprises glioma.

In another preferred embodiment, the brain tumor comprises a malignant glioma in cranial fossa.

In another preferred embodiment, the brain tumor comprises medulloblastoma.

In another preferred embodiment, the brain tumor cell comprises Daoy cell.

In another preferred embodiment, the brain tumor cell comprises one or more of GB-1 cell, and SF126 cell.

In another preferred embodiment, the renal carcinoma is selected from the group consisting of clear cell renal cell adenocarcinoma, renal carcinoma Wilms, and combination thereof.

In another preferred embodiment, the renal carcinoma comprises clear cell renal cell adenocarcinoma.

In another preferred embodiment, the renal carcinoma comprises renal carcinoma Wilms.

In another preferred embodiment, the renal carcinoma cell comprises renal carcinoma Wilms cell.

In another preferred embodiment, the renal carcinoma cell comprises one or more of G-401 cell and 786-0 cell.

In another preferred embodiment, the pancreatic cancer cell comprises CFPAC-1 cell.

In another preferred embodiment, the leukemia is selected from the group consisting of T-lymphocyte leukemia, myeloid leukemia, and combinations thereof.

In another preferred embodiment, the T-lymphocytic leukemia comprises acute T-lymphocytic leukemia.

In another preferred embodiment, the myeloid leukemia comprises type M4 of acute myeloid leukemia.

In another preferred embodiment, the myeloid leukemia comprises FAB type M4 of acute myeloid leukemia.

In another preferred embodiment, the expression comprises protein expression and/or mRNA expression.

In another preferred embodiment, the composition is a pharmaceutical composition.

In another preferred embodiment, the composition or preparation further comprises a pharmaceutically acceptable carrier.

In another preferred embodiment, the expression is mRNA expression or protein expression.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the fourth aspect of the present invention, it provides a marker for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

In another preferred embodiment, the marker comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" comprises "the patient tumor is sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" comprises "the patient tumor is not sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, the tumor with low expression, no expression, low activity or no activity of mitochondria permeability transition pore is as described in the third aspect of the invention.

In another preferred embodiment, the tumor with low expression, no expression, low activity or no activity of peptidyl-prolyl cis-trans isomerase F is as described in the third aspect of the invention.

In another preferred embodiment, the tumor with low or no expression of NNMT gene is as described in the third aspect of the invention.

In another preferred embodiment, the DNA methylase is selected from the group consisting of DNMT1, DNMT3a, DNMT3b, and combinations thereof.

In another preferred embodiment, the tumor with high expression of DNA methylase (e.g., DNMT1, DNMT3a and/or DNMT3b) is as described in the third aspect of the invention.

In another preferred embodiment, the tumor with high expression of UHRFl is as described in the third aspect of the invention.

In another preferred embodiment, the tumor with high methylation level of nucleotide site of NNMT gene is as described in the third aspect of the invention.

In another preferred embodiment, the tumor with high methylation level of DNA CpG site of NNMT gene is as described in the third aspect of the invention.

In another preferred embodiment, the high expression or high activity of mitochondria permeability transition pore means the ratio (H1/H0) of the expression level or activity level H1of mitochondria permeability transition pore in a cell ( e.g., tumor cell ) to the expression level or activity level H0 of mitochondria permeability transition pore in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the high expression or high activity of peptidyl-prolyl cis-trans isomerase F means the ratio (G1/G0) of the expression level or activity level G1of peptidyl-prolyl cis-trans isomerase F in a cell ( e.g., tumor cell) to the expression level or activity level G0 of mitochondria permeability transition pore in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the high expression of NNMT gene means the ratio (E1/E0) of the expression E1 of NNMT gene in a cell ( e.g., tumor cell) to the expression E0 of NNMT gene in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the tumor with low expression of DNA methylase means the ratio (A1/A0) of the expression level A1 of DNA methylase in the tumor cell to the expression level A0 of DNA methylase in the same type of cell or a normal cell (e.g., para-tumor tissue cell) is < 1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT1 means the ratio (B1/B0) of the expression level B1 of DNMT1 in the tumor cell to the expression level B0 of DNMT1 in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3a means the ratio (C1/C0) of the expression level C1 of DNMT3a in the tumor cell to the expression level C0 of DNA methylase in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3b means the ratio (D1/D0) of the expression level D1 of DNMT3b in the tumor cell to the expression level D0 of DNA methylase in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In the fifth aspect of the present invention, it provides a detection kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the expression of NNMT gene is the expression of mRNA and/or protein.

In the sixth aspect of the present invention, it provides a use of the detection kit according to the fifth aspect of the present invention in the preparation of concomitant diagnose kit for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In the seventh aspect of the present invention, it provides a medicine kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In the eighth aspect of the present invention, it provides a method for preventing and/or treating tumor, which comprises administering the compound of formula I, or an optical isomer thereof, or a racemate thereof, or a solvate thereof, or a pharmaceutically acceptable salt thereof, or a deuterated compound thereof according to the first aspect of the present invention to a subject in need.

In another preferred embodiment, the tumor is as described in the third aspect of the invention.

In another preferred embodiment, the subject is human and non-human mammals (rodent, rabbit, monkey, livestock, dog, cat, etc.).

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the third aspect of the invention.

In the ninth aspect of the present invention, it provides a device or system, the device or system comprises:
(i) a detection module, the detection module is used to detect the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene;
(ii) an output module, the output module comprises the information as follows:
   the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In the tenth aspect of the present invention, it provides a use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNA methylase is selected from the group consisting of DNMT1, DNMT3a, DNMT3b, and combinations thereof.

In another preferred embodiment, the DNA methylase promoter comprises DNMT1 promoter.

In another preferred embodiment, the DNMT1 promoter comprises promoter that can achieve high expression of DNMT1 in tumor.

In another preferred embodiment, the DNA methylase promoter comprises DNMT3a promoter.

In another preferred embodiment, the DNMT3a promoter comprises promoter that can achieve high expression of DNMT3a in tumor.

In another preferred embodiment, the DNA methylase promoter comprises DNMT3b promoter.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation level of nucleotide site of NNMT gene in tumor.

In another preferred embodiment, the inhibitor comprises specific inhibitor.

In another preferred embodiment, the promoter comprises specific promoter.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention. In another preferred embodiment, the tumor is as described in the third aspect of the invention. In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the composition or preparation further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the injection preparation is intravenous injection preparation.

In another preferred embodiment, the dosage form of the composition or preparation is tablet, injection, infusion, paste, gel, solution, microsphere or film.

In the eleventh aspect of the present invention, it provides an active ingredient combination, the active ingredient combination comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the molar ratio of the first active ingredient to the second active ingredient is 0.01-600:1, preferably 0.05-500:1, more preferably 0.1-400:1, more preferably 0.2-200:1, more preferably 0.5-100:1, more preferably 0.5-80:1, most preferably 1-50:1.

In another preferred embodiment, at least one active ingredient is independent in the active ingredient combination.

In another preferred embodiment, the first active ingredient and the second active ingredient are independent of each other in the active ingredient combination.

In the twelfth aspect of the present invention, it provides a composition, the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the composition is a pharmaceutical composition.

In another preferred embodiment, the pharmaceutical composition further comprises a pharmaceutically acceptable carrier.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In the thirteenth aspect of the present invention, it provides a medicine kit, the medicine kit comprises:
(A) a first preparation comprising a first active ingredient, the first active ingredient comprises anti-tumor drug; and
(B) a second preparation comprising a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anti-tumor drug.

In another preferred embodiment, the method for using together means the second preparation comprising a second active ingredient is administered firstly, then the first preparation comprising a first active ingredient is administered.

In the fourteenth aspect of the present invention, it provides a method for inhibiting tumor cell, which comprises contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method is vitro method.

In another preferred embodiment, the method is non-therapeutic and non-diagnostic method.

In another preferred embodiment, the contact is performed in vitro culture.

In another preferred embodiment, the tumor is as described in the third aspect of the invention.

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In the fifteenth aspect of the present invention, it provides a use of medicine kit according to the seventh aspect of the present invention in the preparation of medicine box for preventing and/or treating tumor.

In another preferred embodiment, the medicine box further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

It should be understood that, in the present invention, each of the technical features specifically described above and below can be combined with each other, thereby constituting new or preferred technical solutions which need not be redundantly described one-by-one.

### DESCRIPTION OF THE DRAWINGS

Fig.1 shows the expression content of NNMT protein in Con-NCI-H82 cell and ov-NNMT NCI-H82 cell using Western Blot test, wherein, Con-NCI-H82 refers to the expression content of NNMT protein in normal and untreated NCI-H82 cell, which is used as control; ov-NNMT NCI-H82 refers to the expression content of NNMT protein in NCI-H82 cell transfected with virus vector carrying NNMT gene.
Fig.2 shows the relative cell viability of Con-NCI-H82 cell and ov-NNMT NCI-H82 cell, wherein, Con-NCI-H82 refers to the cell viability of normal and untreated NCI-H82 cell, which is used as control; ov-NNMT NCI-H82 refers to the cell viability of NCI-H82 cell transfected with virus vector carrying NNMT gene.
Fig.3 shows the expression of NNMT gene in tumor cells sensitive and insensitive to the compounds prepared in the examples.
Fig.4 shows the methylation level of DNA CpG site of promoter region of NNMT gene in tumor cells sensitive and insensitive to the compounds prepared in the examples.
Fig.5 shows the methylation level of DNA CpG sites from 1050 bp before the transcription start site to 499 bp after the transcription start site in NNMT gene in tumor cells sensitive and insensitive to the compounds prepared in the examples.
Fig.6 shows the methylation level of DNA CpG sites from 1050 bp to 193 bp before the transcription start site in NNMT gene in tumor cells sensitive and insensitive to the compounds prepared in the examples.
Fig.7 shows the methylation level of DNA CpG sites of specific NNMT gene (ie, site 114165695, site 114165730, site 114165769, site 114165804, site 114165938, site 114166050, site 114166066 on human chromosome 11) in tumor cells sensitive and insensitive to the compounds prepared in the examples, black dot indicates that the relevant site is methylated, white white dot indicates that the relevant site is not methylated, SST refers to the transcription starting site, and Chr11 refers to human chromosome 11 according to human genome version GCF_ 00000 1405.25 (GRCh37. p13).
Fig.8 shows the correlation between the expression of NNMT and the expression of DNMT1, UHRF1, DNMT3a and DNMT3b in tumor cells.
Fig.9 shows the expression content of PPIF protein using Western Blot test, wherein, Con shRNA refers to the expression content of PPIF protein in Daoy cell transfected with empty virus vector without carrying shRNA that can specificly induce the degradation of PPIF mRNA; PPIF shRNA refers to the expression content of PPIF protein in Daoy cell transfected with virus vector carrying shRNA that can specificly induce the degradation of PPIF mRNA.
Fig.10 shows the relative cell viability of Daoy cell having inactive mPTP and Daoy cell having active mPTP, wherein, Con shRNA refers to the relative viability of Daoy cell transfected with empty virus vector without carrying shRNA that can specificly induce the degradation of PPIF mRNA; PPIF shRNA refers to the relative viability of Daoy cell transfected with virus vector carrying shRNA that can specificly induce the degradation of PPIF mRNA.

### EMBODIMENTS FOR CARRYING OUT THE INVENTION

Based on an extensive and intensive research, the inventors have unexpectedly developed a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of present invention is suitable for use in the prevention and/or treatment of patient tumor. On this basis, the inventors has completed the present invention.

### Terms

As used herein, the term "comprise", " comprising", and "containing" are used interchangeably, which not only comprise closed definitions, but also semi-closed and open definitions. In other words, the term comprises "consisting of" and "essentially consisting of".

As used herein, the term "anti-tumor cancer" and "anti-tumor drug" are used interchangeably.

As used herein, the term "cancer" and "tumor" are used interchangeably.

As used herein, the term "a cell" refers to a cell (e.g., single tumor cell) or a group of cells containing multiple similar cells ((e.g., a tumor tissue).

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, the term "high methylation level of DNA CpG site", "high level of DNA CpG site methylation" and "high methylation of DNA CpG site" are used interchangeably.

As used herein, the term "high methylation level of nucleotide site", "high level of nucleotide site methylation" and "high methylation of nucleotide site" are used interchangeably.

As used herein, the term "low methylation level of nucleotide site", "low level of nucleotide site methylation" and "low methylation of nucleotide site" are used interchangeably.

As used herein, the term "low methylation level of DNA CpG site", "low level of DNA CpG site methylation" and "low methylation of DNA CpG site" are used interchangeably.

As used herein, the term "methylation of CpG site", "methylation of CpG nucleotide" and "CpG methylation" are used interchangeably.

As used herein, the term "IC50" and "IC₅₀" are used interchangeably, which refers to 50% inhibiting concentration, ie, the concentration of the inhibitor when 50% inhibitory effect is achieved.

As used herein, the term "methylation of CpG site", "methylation of CpG nucleotide" and "CpG methylation" are used interchangeably.

As used herein, the term "P/S" refers to adding penicillin and streptomycin into the culture medium.

As used herein, "the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene" refers to one or more of the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene and the methylation level of DNA CpG site of NNMT gene.

As used herein, "the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene" refers to one or more of the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene and high methylation level of DNA CpG site of NNMT gene.

As used herein, "the high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene" refers to one or more of the high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene and low methylation level of DNA CpG site of NNMT gene.

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_ 000001405.25 (GRCh37. p13).

As used herein, the term "human chromosome 11" refers to human chromosome 11 according to human genome version GCF_ 000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide of site 114165695 on human chromosome 11; "site 114165730 on human chromosome 11" refers to nucleotide of site 114165730 on human chromosome 11; "site 114165769 on human chromosome 11" refers to nucleotide of site 114165769 on human chromosome 11; "site 114165804 on human chromosome 11" refers to nucleotide of site 114165804 on human chromosome 11; "site 114165938 on human chromosome 11" refers to nucleotide of site 114165938 on human chromosome 11; "site 114166050 on human chromosome 11" refers to nucleotide of site 114166050 on human chromosome 11; "site 114166066 on human chromosome 11" refers to nucleotide of site 114166066 on human chromosome 11.

As used herein, mitochondria permeability transition pore is abbreviated as mPTP.

As used herein, peptidyl-prolyl cis-trans isomerase F is abbreviated as PPIF.

As used herein, S-adenosyl methionine is abbreviated as SAM.

As used herein, the gene expression comprises the protein expression of the gene and/or the mRNA expression of the gene.

As used herein, the term "DNMT3a" and "DNMT3A" are used interchangeably, which refers to DNA methyltransferase 3a.

As used herein, the term "DNMT3b" and "DNMT3B" are used interchangeably, which refers to DNA methyltransferase 3b.

As used herein, the term "DNMT1" refers to DNA methyltransferase 1.

As used herein, the term "UHRF1" refers to ubiquitin-like with PHD and ring finger domain 1.

As used herein, the term "deuterated" refers to one or more hydrogen atoms in a compound or group are substituted by deuterium. The deuterated can be mono-substituted, di-substituted, multi-substituted or fully-substituted.

As used herein, the term "solvate" refers to a complex formed by the coordination of a compound with a solvent molecule in a specific ratio.

As used herein, the term "MS-ESI" refers to Electro Spray Ionization-Mass Spectroscopy.

As used herein, the term "1H NMR" refers to H Nuclear Magnetic Resonance Spectra.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc.

As used herein, "R₁", "R1" and "R¹" have the same meaning and can be used interchangeably, the other similar definitions have the same meaning.

As used herein, " 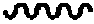 " denotes the linking site of the group.

As used herein, the term "alkyl" refers to a saturated hydrocarbon group with linear chain (ie, unbranched chain) or branched chain, or a combination of linear and branched chains, the alkyl only have carbon and hydrogen atoms. When the number of carbon atoms is limited in front of the alkyl (e.g., C1-C6 alkyl), it refers to the number of carbon atoms (e.g., 1-6) in the alkyl, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the haloalkyl (e.g., C1-C8 haloalkyl), it refers to the number of carbon atoms (e.g., 1-8) in the haloalkyl, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkane ring" refers to a ring having a saturated or partially saturated monocyclic ring, bicyclic ring or polycyclic ring (fused ring, bridged ring or spiro ring). When the number of carbon atoms is limited in front of the cycloalkane ring (e.g., C3-C12 cycloalkane ring), it refers to the number of ring carbon atoms (e.g., 3-12) in the cycloalkane ring. For example, C3-C8 cycloalkane ring refers to a saturated or partially saturated monocycloalkane ring or dicycloalkane ring having 3-8 ring carbon atoms, comprising cyclopropane ring, cyclobutane ring, cyclopentane ring, cycloheptane ring, or the like. The term "spirocycloalkane ring" refers to a bicyclic or polycyclic ring that shares a carbon atom (referred to spiro-atom) between single rings, the spirocycloalkane ring can have one or more double bonds, but no ring has a fully conjugated π electron system. Fused cycloalkane ring refers to all carbon bicyclic or polycyclic ring in which each rings in the system shares an adjacent pair of carbon atoms with other rings, one or more rings can have one or more double bonds, but no ring has a fully conjugated π electron system. Bridged cycloalkane ring refers to all carbon polycyclic ring in which any two rings share two non-directly connected carbon atoms, the bridged cycloalkane ring can have one or more double bonds, but no ring has a fully conjugated π electron system.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it refers to the number of ring carbon atoms (e.g., 3-12) in the cycloalkyl. For example, C3-C8 cycloalkyl refers to a saturated or partially saturated monocycloalkyl or dicycloalkyl having 3-8 ring carbon atoms, comprising cyclopropyl, cyclobutyl, cyclopentyl, cycloheptyl, or the like. The term "spirocycloalkyl" refers to a bicyclic or polycyclic group that shares a carbon atom (referred to spiro-atom) between single rings, the spirocycloalkyl can have one or more double bonds, but no ring has a fully conjugated π electron system. Fused cycloalkyl refers to all carbon bicyclic or polycyclic group in which each rings in the system shares an adjacent pair of carbon atoms with other rings, one or more rings can have one or more double bonds, but no ring has a fully conjugated π electron system. Bridged cycloalkyl refers to all carbon polycyclic group in which any two rings share two non-directly connected carbon atoms, the bridged cycloalkyl can have one or more double bonds, but no ring has a fully conjugated π electron system. The representative examples of cycloalkyl comprise but are not limited to

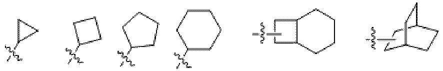

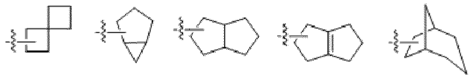

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the halocycloalkyl (e.g, C3-C8 halocycloalkyl), it refers to the number of ring carbon atoms (e.g., 3-8) in the halocycloalkyl, for example, C3-C8 halocycloalkyl refers to an halocycloalkyl having 3-8 ring carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-S- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above, for example, C1-C6 haloalkylthio refers to a haloalkylthio having 1-6 carbon atoms. Representative examples of haloalkylthio comprise but are not limited to monofluoromethylthio, monofluoroethylthio, difluorobutylthio, or the like.

As used herein, the term "cycloalkoxyl" refers to R-O-, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 ring carbon atoms. Representative examples of cycloalkoxyl comprise but are not limited to cyclopropyloxyl, cyclobutoxyl, or the like.

As used herein, the term "cycloalkylthio" refers to R-S- group, wherein R is cycloalkyl, the cycloalkyl is as defined above. When the number of carbon atoms is limited in front of the cycloalkylthio, for example, C3-C8 cycloalkylthio means that the cycloalkyl in the cycloalkylthio has 3-8 ring carbon atoms. Representative examples of cycloalkylthio comprise but are not limited to cyclopropylthio, cyclobutythio, or the like.

As used herein, the term "halocycloalkoxyl" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkoxyl (e.g., C3-C8 halocycloalkoxyl), it refers to the number of ring carbon atoms (e.g., 3-8) in the halocycloalkoxyl, for example, C3-C8 halocycloalkoxyl refers to an halocycloalkoxyl having 3-8 ring carbon atoms. Representative examples comprises but are not limited to monofluorocyclopropyl-O-, monochlorocyclobutyl-O-, monofluorocyclopentyl-O-, difluorocycloheptyl-O -, or similar functional groups, or the like.

As used herein, the term "halocycloalkylthio" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkylthio (e.g., C3-C8 halocycloalkylthio), it refers to the number of ring carbon atoms (e.g., 3-8) in the halocycloalkylthio, for example, C3-C8 halocycloalkylthio refers to an halocycloalkylthio having 3-8 ring carbon atoms. Representative examples comprises but are not limited to monofluorocyclopropyl-S-, monochlorocyclobutyl-S-, monofluorocyclopentyl-S-, difluorocycloheptyl-S-, or similar functional groups, or the like.

As used herein, the term "heterocycloalkane ring" refers to fully saturated or partially unsaturated ring (comprising but not limited to such as 3-7 membered monocyclic ring, 7-11 membered bicyclic ring, or 8-16 membered tricyclic ring), at least one heteroatom is present in a ring with at least one carbon atom. When the number of members is limited in front of the heterocycloalkane ring, it refers to the number of ring atoms in the heterocycloalkane ring, for example, 3-16 membered heterocycloalkane ring refers to a heterocycloalkane ring having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkane ring comprise but are not limited to azetidine ring, oxetane ring, tetrahydrofuran ring, piperidine ring, piperazine ring. Polycyclic heterocycloalkane ring comprises spiro, fused and bridged ring, the spiro, fused and bridged heterocycloalkane ring is optionally linked with other rings by single bond, or further linked with other cycloalkane ring and heterocycloalkane ring by any two or more atoms on the ring.

As used herein, the term "heterocycloalkyl" refers to fully saturated or partially unsaturated cyclic (comprising but not limited to such as 3-7 membered monocyclic ring, 7-11 membered bicyclic ring, or 8-16 membered tricyclic ring) group, at least one heteroatom is present in a ring with at least one carbon atom. When the number of members is limited in front of the heterocycloalkyl, it refers to the number of ring atoms in the heterocycloalkyl, for example, 3-16 membered heterocycloalkyl refers to a heterocycloalkyl having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkyl comprise but are not limited to azetidinyl, oxetanyl, tetrahydrofuranyl, piperidinyl, piperazinyl. Polycyclic heterocycloalkyl comprises spiro, fused and bridged heterocyclyl, the spiro, fused and bridged heterocycloalkyl is optionally linked with other groups by single bond, or further linked with other cycloalkane rings and heterocycloalkane ring by any two or more atoms on the ring.

As used herein, the term "aromatic ring" refers to an full carbon monocyclic ring or fused polycyclic ring (i.e., a ring that shares adjacent carbon atom pairs) with a conjugated π electron system, which is aromatic cyclic hydrocarbon compound. When the number of carbon atoms is limited in front of the aromatic ring, for example, C6-C12 aromatic ring means that the aromatic ring has 6-12 ring carbon atoms, such as benzene ring and naphthalene ring.

As used herein, the term "aryl" refers to an full carbon monocyclic ring or fused polycyclic ring (i.e., a ring that shares adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl.

As used herein, the term "heteroaromatic ring" refers to aromatic heterocyclic ring having one to more (preferably 1, 2, 3 or 4) heteroatoms, at least one heteroatom is present in a ring with at least one carbon atom. The heteroaromatic ring can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaromatic ring, it refers to the number of ring atoms in the heteroaromatic ring, for example, 5-12 membered heteroaromatic ring refers to a heteroaromatic ring having 5-12 ring atoms. Representative examples comprise but are not limited to pyrrole ring, pyrazole ring, imidazole ring, thiazole ring, furan ring, pyridine ring, pyrimidine ring, etc.

As used herein, the term "heteroaryl" refers to aromatic heterocyclic ring group having one to more (preferably 1, 2, 3 or 4) heteroatoms, at least one heteroatom is present in a ring with at least one carbon atom. The heteroaryl can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms in the heteroaryl, for example, 5-12 membered heteroaryl refers to a heteroaryl having 5-12 ring atoms. Representative examples comprise but are not limited to pyrrolyl, pyrazolyl, imidazolyl, thiazolyl, furanyl, pyridyl, pyrimidinyl, etc.

As used herein, the term "carboxyl" refers to -COOH or -alkyl-COOH, the alkyl is as defined above. For example, "C2-C4 carboxyl" refers to -C1-C3 alkyl-COOH. Representative examples of carboxyl comprise but are not limited to -COOH, -CH₂COOH, or the like.

As used herein, the term "ester group" refers to R-C(O)-O- or -C(O)-O-R, wherein, R is alkyl, the alkyl is defined as above. For example, "C₂-C₄ ester group" refers to C₁-C₃ alkyl-C(O)-O- or - C(O)-O-Ci-Cs alkyl. Representative examples of ester group comprise but are not limited to CH₃C(O)O-, C₂H₅C(O)O-, (CH₃)₂CHC(O)O-, -C(O)OCH₃, -C(O)OC₂H₅, or the like.

As used herein, the term "amide group" refers to R-C(O)-N- or -C(O)-N-R, wherein, R is alkyl, the alkyl is defined as above. For example, "C₂-C₄ amide group" refers to C₁-C₃ alkyl-C(O)-N- or - C(O)-N-C₁-C₃ alkyl. Representative examples of amide group comprise but are not limited to CH₃C(O)-N-, C₂H₅C(O)-N-, (CH₃)₂CHC(O)-N-, -C(O)-N-CH₃, -C(O)-N-C₂H₅, or the like.

As used herein, the term "acyl" refers to 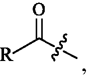 wherein R is alkyl, the alkyl is as defined above. When the number of carbon atoms is limited in front of the acyl (e.g, C2-C6 acyl), it refers to the number of carbon atoms (e.g., 2-6) in the acyl, for example, C2-C6 acyl refers to acyl having 2-6 carbon atoms. For example, the C₂-C₄ acyl refers to C₁-C₃ alkyl -C(O)-, representative examples comprises but are not limited to CHsC(O)-, C₂H₅C(O)- , or the like.

As used herein, the term "-C(O)-" and 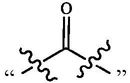 are used interchangeably.

As used alone or as part of other substituent, the term "amino" refers to -NH₂.

As used alone or as part of other substituent, the term 'nitro' refers to -NO₂.

As used alone or as part of other substituent, the term "cyano" refers to -CN.

As used alone or as part of other substituent, the term "hydroxyl" refers to -OH.

As used alone or as part of other substituent, the term "sulfhydryl" refers to -SH.

As used alone or as part of other substituent, the term "sulfuryl" refers to

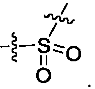

As used alone or as part of other substituent, the term "phenylsulfuryl" refers to

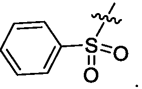

In present invention, it should be understood that all substituents are unsubstituted, unless explicitly described herein as "substituted". The specific substituent is the substituent as described above or the substituent in each Example. The "substituted" means that one or more hydrogen atoms on specific group are substituted by specific substituent. Preferably, each "substituted" means that one or more (preferably 1, 2, 3, 4, 5, 6, 7, or 8) hydrogen atoms on the ring or group are independently substituted by substituent selected from the group consisting of C1-C8 alkyl, C3-C8 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl. Unless otherwise specified, each substituted group can have a substituent selected from a specified group at any substituted position of the group, the substitution can be the same or different at each substituted position.

In the present invention, the term "prevention" refers to a method of preventing the occurrence of disease and/or its accompanying symptoms, or protecting a subject from getting disease. The term 'prevention' also comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100*%* inhibition, elimination and reversal. In some embodiments, compared to the level observed in the absence of the compound of present invention, the compound of present invention alleviates, inhibits and/or reverses related disease (e.g., tumor) and its accompanying symptoms, for example, by at least about 10*%*, at least about 30*%*, at least about 50*%*, at least about 80*%*, at least about 90*%*, or 100*%*.

### Compound

As used herein, the terms "compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof, or a deuterated compound thereof. It should be understood that the term also comprises a mixture of the above components.

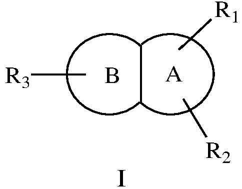

Specifically, the compound of formula I, or an optical isomer thereof, or a racemate thereof, or a solvate thereof, or a pharmaceutically acceptable salt thereof, or a deuterated compound thereof is as described above in the first aspect of the present invention.

It should be understood that, in the present invention, ring B is absent, the compound of formula I has the following structure:

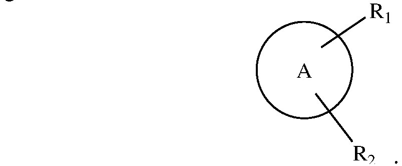

It should be understood that, in the present invention, n is 0, 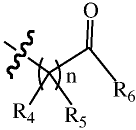 is

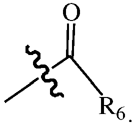

Typically, the compound of formula I of the present invention is the compound prepared in the Examples of the present invention (comprising salt thereof or free form without salt root) prepared in the Examples of the present invention..

The compound of formula I of the present invention can be prepared using the known organic synthesis method in the art.

The study of the prssent invention showed that the compound of present invention has significantly excellen treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is sensitive to the compound of present invention.

The term "pharmaceutically acceptable salt" refers to a salt formed by the compound of the present invention and an acid or a base, the salt is suitable for use as a drug. Pharmaceutically acceptable salt comprises inorganic salt and organic salt. A preferred type of salt is the salt formed by the compound of the present invention and an acid. Acids suitable for salt formation comprise but are not limited to inorganic acid such as hydrochloric acid, hydrobromic acid, hydrofluoric acid, sulfuric acid, nitric acid, phosphoric acid and the like; organic acid such as formic acid, acetic acid, trifluoroacetic acid, trifluoroformic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid and the like; and acidic amino acid such as aspartic acid and glutamic acid. A preferred type of salt is a metal salt formed by the compound of the present invention and a base. Bases suitable for salt formation comprise but are not limited to inorganic base such as sodium hydroxide, potassium hydroxide, sodium carbonate, sodium bicarbonate, sodium phosphate and the like; and organic base such as ammonia, triethylamine, diethylamine and the like.

The compound of formula I in present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

The compounds of the present invention is preferably prepared as described in the Examples of the present invention.

### Mitochondria permeability transition pore

In present invention, the mitochondria permeability transition pore is abbreviated as mPTP.

The compound of present invention has excellent treatment effect on tumor with low expression, no expression, low activity or no activity of mitochondria permeability transition pore, the tumor with low expression, no expression, low activity or no activity of mitochondria permeability transition pore is sensitive to the compound of present invention.

Preferably, the tumor with low expression, no expression, low activity or no activity of mitochondria permeability transition pore is as described above in the third aspect of the invention.

In the present invention, the expression level or activity of mitochondria permeability transition pore can be determined using conventional methods, such as measuring the activity of mPTP, or measuring the expression level of mPTP at the protein or mRNA leve.

### Peptidyl-prolyl cis-trans isomerase F

In present invention, the peptidyl-prolyl cis-trans isomerase F is abbreviated as PPIF.

The compound of present invention has excellent treatment effect on tumor with low expression, no expression, low activity or no activity of peptidyl-prolyl cis-trans isomerase F, the tumor with low expression, no expression, low activity or no activity of peptidyl-prolyl cis-trans isomerase F is sensitive to the compound of present invention.

Preferably, the tumor with low expression, no expression, low activity or no activity of mitochondria permeability transition pore is as described above in the third aspect of the invention.

In the present invention, the expression level or activity of mitochondria permeability transition can be determined using conventional methods, such as measuring the activity of mPTP, or measuring the expression level of mPTP at the protein or mRNA leve.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) and 499 bp after the transcription start site (non-bold section) in NNMT gene, the total length of promoter region of NNMT gene is 2500 bp, The nucleotide sequence of the promoter region of NNMT gene is as shown in SEQ ID NO: 1 as follows:

SEQ ID NO: 1:
TATCCAAGAGCTATCAGCACTCCCATGTTTATTGTAGCACTGTTCACAATAGC
CAAGATTTGGAAGTACTCTAAGTGTCCATTAGCAGATGAATGGATAAAGACAATG
TGGTAATACACATAATGGAGTACTATTCAGTCATAAAGAAGAATTAGATCCTGTC
ATTTGCAATAACATGGATGGAACTGGAGGTCATAATGTTGAGTGAAATAAACCAG
GCACAGAAAGACAAACTTTGCATGTTCTCACTTATTTATGGGAGCTAAAAACTAA
AATAACTGAACTCACAGAGATAGAGAGTAGAAGGATGGTTACGAGAGGATGGGA
AGGGTAGCGAGGTGGGTAGGGGGGATGTGGGGATCATTAATGGGTATAAAAAAT
AGTTAGAGGCCAGGCGCAGTGGCTCACGCCTGTAATCCCAGCACTTTGGGAGGC
CGAGGTAGGCGGAACACCTGAGGAGTTCAAGACCAGCCTGGCCAATATGATGAA
ACCCCGTCTCTACTAAAAATACAAAAATTAGCTGGGCGTGATGGTGTGCACCTGT
AGTCCCAGCTGCTTGGGAGGCTGAGGCAGGAGAATCGCTGGAACCCAAGAGGTG
AAGGTTGCAGTGAGCTGAGATCGCGTCACTGCACTCCAGCCTGGGTGACAGAGT
GAGACTCCACATCAAAAAAAAAAAAAAAAAGTTAGAAAGATTGAATAAGACCTAA
TATTTGCTAGCACAACAGGGTGAATATAGTAAAAAATAATTTATTTGTACCTTCA
AAAATAACTAGACAAGTATAATTGGGTTGTTTGTAACACACAAAAAATAAGTACT
TGAAGTGGTGGATACCCCATTTACCCTGATGTGATTATTTTGTATTGCAGGCCTC
TATCAGAATATCTCATGTAACCCATAAATATATACACCTACTCTGTACCCACAAAA
AGTTTTTAAAAAGAAAAATAAATAGCAACCGAAAAAAAAAGAGAGGGAGAAAAG
AAAAAAGAAAAAAAAATCAAGTGCCTGGCTGGGTAGAATAAATTCTAAGGCCAC
AATGTTACTGACCATGGGTTTTTTGGCTCTCAGTGTATAGAAATTGACACAAGGC
CAATAGTCTTCCCAAACATGCTTTACTGGAACTTACGCCCTGGCATAAGGGCCAC
AACAAAAGAGAGAGCGAATTCTCTGGCTTGCTGACTCCTTGGAAAAAACCGGTA
GGGATTTTTTTATTAGGCAAAGCACAGGAATTGACGTCAGAGGCAGGATGTGCT
GCTGGGCAAAGCATACGAGAAGTGGGGTATGCAGGTCAGCATTACTTGGTTGCA
ATGGTTATCTTGAGGAATGGGCCAACTGGTGGTCTGGCCAGTGGCAACAAGGCT
GTAAATCAATTATTCAGCATTCCTTCCCAAGGTGGGACACCCGGCAACATTGTTT
ATCTCCTAAGGCCAGTTCCTGGAATTAAGTGAAAGGATGACTAATGGACATGTTG
TCAGTGAGGTAGTGGTGTGGGTTTTGTGACCAGTGGGAATGCACGAAAGAATGC
TTTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGACA
GAGTCTTGCTCTGTCGCCCAGGCTGGAGTGCAGTGGCGCCATCTTGGCTCACTG
CAAGCTCCGCCCCCCGGGTTCATGCCATTCTCCTGCCTCAGCCTCCCAAGTAGCT
GGGACTGCAGGTGCCTACCACCAAGCCCAGCTAATTTTTTGTATTTTTTTTTTAG
TAGAGACGGGGTTTCACCATGTTAGCCAGGATGGTCTCGATCTCCTGACCTCGTG
ATCTGCCCGCCTTAGCCTCCCAAAGTGCTGGGATTACAGGCATGAGCCTCCGCC
CCCGGCCTTAAATAATTCTTAAAGGAAGTAAAGTTAACTTTGAAAGAACTATCAG
GATTTGGATTGACTGAAAGGAGTGGGGAAGCTTAGGGAGGAGGTGCTTGCCAGAC
ACTGGGTCATGGCAGTGGTCGGTGAAGCTGCAGTTGCCTAGGGCAGGGATGGAGAGA
GAGTCTGGGCATGAGGAGAGGGTCTCGGGATGTTTGGCTGGACTAGATTTTACAGAA
AGCCTTATCCAGGCTTTTAAAATTACTCTTTCCAGACTTCATCTGAGACTCCTTCTTCA
GCCAACATTCCTTAGCCCTGAATACATTTCCTATCCTCATCTTTCCCTTCTTTTTTTTCC
TTTCTTTTACATGTTTAAATTTAAACCATTCTTCGTGACCCCTTTTCTTGGGAGATTCAT
GGCAAGAACGAGAAGAATGATGGTGCTTGTTAGGGGATGTCCTGTCTCTCTGAACTTT
GGGGTCCTATGCATTAAATAATTTTCCTGACGAGCTCAAGTGCTCCCTCTGGTCTACAA
TCCCTGGCGGCTGGCCTTCATCCCTTGGGCAAGCATTGCATACAGCTCATGGCCCTCCC
TCTACCATACCC.

In the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

In the present invention, the nucleotide sites from 840 bp to 469 bp before the transcription start site in NNMT gene is sites 1161-1532 of nucleotide sequence as shown in SEQ ID NO: 1.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Corresponding to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plants and animals. DNA methylation, as a relatively stable modification state, can be passed on to new generations of DNA during DNA replication process under the action of DNA methyltransferase, which is an important epigenetic mechanism.

There are two types of DNA methylation reactions. One type is that the two unmethylated strands in the DNA are methylated, which is called denovo methylation; the other type is that the unmethylated strand of double-stranded DNA with one methylated strand and one unmethylated strand is methylated, which is called maintenance methylation.

Typically, DNA methylation is the methylation of DNA CpG site. The distribution of CpG binucleotide is very uneven in the human genome, while CpG remains or is higher than normal level in some regions of the genome. The CpG site rich region (also known as CpG island) is mainly located in the promoter region and exon regions of the gene, which is a region rich in CpG dinucleotide. About 60*%* of the promoters of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of the gene. Compared to the regulation of gene expression by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is an ideal biomarker.

### Tumor

The compound of present invention can be used for preventing and/or treating tumor.

As used herein, the term "tumor" and "cancer" are used interchangeably.

In a preferred embodiment of the present invention, the tumor comprises tumor with low expression, no expression, low activity or no activity of mitochondria permeability transition pore. Typically, the tumor with low expression, no expression, low activity or no activity of mitochondria permeability transition pore is as described above in the third aspect of the present invention.

In a preferred embodiment of the present invention, the tumor comprises tumor with low expression, no expression, low activity or no activity of peptidyl-prolyl cis-trans isomerase F. Typically, the tumor with low expression, no expression, low activity or no activity of peptidyl-prolyl cis-trans isomerase F is as described above in the third aspect of the present invention.

In a preferred embodiment of the present invention, the tumor comprises tumor with low or no expression of NNMT gene. Typically, the tumor with low or no expression of NNMT gene is as described above in the third aspect of the present invention.

In a preferred embodiment of the present invention, the tumor comprises tumor with high expression of DNA methylase. Typically, the tumor with high expression of DNA methylase is as described above in the third aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the third aspect of the present invention.

In a preferred embodiment of the present invention, the tumor comprises tumor with high expression of DNMT3a. Typically, the tumor with high expression of DNMT3a is as described above in the third aspect of the present invention.

In a preferred embodiment of the present invention, the tumor comprises tumor with high expression of DNMT3b. Typically, the tumor with high expression of DNMT3b is as described above in the third aspect of the present invention.

In a preferred embodiment of the present invention, the tumor comprises tumor with high expression of UHRF1 (ubiquitin-like with PHD and ring finger domain 1). Typically, the tumor with high expression of UHRF1 is as described above in the third aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the third aspect of the present invention.

In a preferred embodiment of the present invention, the tumor comprises tumor with high methylation level of DNA CpG site of NNMT gene. Typically, the tumor with high methylation level of DNA CpG site of NNMT gene is as described above in the third aspect of the present invention.

Typically, the tumor of present invention is as described above in the third aspect of the present invention.

In present invention, the type of tumors corresponding to tumor cell lines are shown in Table 2

**Table 2**

| Tumor cell line | The corresponding type of tumor |
|---|---|
| NCI-H82 | Human small cell lung cancer cell |
| G-401 | Human renal carcinoma Wilms cell |
| MDA-MB-453 | Breast cancer cell |
| SW48 | Human colon adenocarcinoma cell |
| GB-1 | Human brain glioblastoma cell |
| CFPAC-1 | Human pancreatic cancer cell |
| SF126 | Human glioblastoma multiforme cell |
| 786-O | Clear cell renal cell adenocarcinoma cell |
| Daoy cell | Human medulloblastoma cell |

### Anti-tumor drug

The anti-tumor drug can be the compound of formula I, or an optical isomer thereof, or a racemate thereof, or a solvate thereof, or a pharmaceutically acceptable salt thereof, or a deuterated compound thereof of the present invention.

### Use

A use of the compound of formula I of the present invention for preventing and/or treating tumor is provided.

Specifically, the compound of present invention has more remarkable and excellent prevention and treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. That is, the tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is sensitive to the compound of the present invention. The expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of the present invention is suitable for use in the precision treatment on tumor, the biomarker can be used to effectively identify patient tumor sensitive to the compound of the present invention, improve treatment effects and avoid administrating the compound of the present invention to patient tumor insensitive to the compound of the present invention, thereby achieving precision treatment on tumor.

The present invention further provides a method for preventing and/or treating tumor, which comprises administering the compound of the present invention to a subject in need.

The compound of present invention has more remarkable and excellent precison treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, therefore, during the prevention and/or treatment of tumor, firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of the present invention to prevent and/or treat tumor. Therefore, the present invention develops a compound that can significantly enhance anti-tumor effects when combined with mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene. The compound of the present invention can be combined with mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to significantly enhance the treatment effect of the compound on tumor.

In another preferred embodiment, the subject is human and non-human mammals (rodent, rabbit, monkey, livestock, dog, cat, etc.).

In the present invention, there are no special limitations to the method for achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor. For example, low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene can be specifically achieved using methods such as gene insert, gene knockout, or gene silencing (e.g, shRNA transfection), etc.

In another preferred embodiment, the peptidyl-prolyl cis-trans isomerase F inhibitor comprises shRNA.

Preferably, the nucleotide sequence of shRNA is GTTCTTCATCTGCACCATAAA.

### Marker

The present invention provides a marker for determining whether the compound of present invention is suitable for use in the prevention and/or treatment of patient tumor, the marker comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In a preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether the compound of present invention is suitable for use in the prevention and/or treatment of patient tumor, the method comprises as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

Specifically, the tumor with low expression, no expression, low activity or no activity of mitochondria permeability transition pore, and/or low expression, no expression, low activity or no activity of peptidyl-prolyl cis-trans isomerase F is as described above in the first aspect of the present invention.

Specifically, the tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase (e.g, NNMT1) , high expression of UHRFl, high methylation level of nucleotide site of NNMT gene, and/or high methylation level of DNA CpG site of NNMT gene e is as described above in the third aspect of the present invention.

Specifically, the tumor with high expression or high activity of mitochondria permeability transition pore, high expression or high activity of peptidyl-prolyl cis-trans isomerase F, high expression of NNMT gene, low expression of DNA methylase (e.g, NNMT1), low expression of UHRFl, low methylation level of nucleotide site of NNMT gene, and/or low methylation level of DNA CpG site of NNMT gene is as described above in the fourth aspect of the present invention.

In a preferred embodiment, the tumor with high expression of NNMT gene, low expression of DNA methylase (e.g, NNMT1), low expression of UHRFl, low methylation level of nucleotide site of NNMT gene, and/or low methylation level of DNA CpG site of NNMT gene is as described above in the second aspect of the present invention.

The present invention further provides a use of the marker (or the expression level of marker) or its detection reagent in the preparation of reagent kit for determining whether the compound of the present invention is suitable for use in the prevention and/or treatment of patient tumor.

### Composition or preparation, active ingredient combination, medical kit and administration method

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

Typically, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 10 µg/kg.bw to 1 mg/kg.bw. Of course, the specific dose should also take into account the route of administration, the patient's health and other factors, which are within the skill range of skilled doctors.

### The main advantages of the present invention comprise:

1. The present has developed a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is highly sensitive to the compound of present invention, ie, the compound of present invention has more remarkable and excellent treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. Therefore, the compound of present invention can realize precise treatment on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, thus improving the treatment effect of the compound of present invention on tumor and avoiding administrating the compound of present invention to patient tumor insensitive to such anti-tumor drug. Therefore, the precision treatment of the compound of the present invention on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene has advantages such a more excellent prevention and treatment effect on tumors, low drug dose, and minimal side effects, which improve the precision prevention and treatment effect on tumor, reduce the side effects and improve patient compliance.

The present invention will be further illustrated below with reference to the specific examples. It should be understood that these examples are only to illustrate the invention but are not to limit the scope of the invention. The experimental methods with no specific conditions described in the following examples are generally performed under the conventional conditions, or according to the manufacturer's instructions. Unless indicated otherwise, parts and percentage are calculated by weight.

### Example

Mitochondria permeability transition pore was abbreviated as mPTP.

Peptidyl-prolyl cis-trans isomerase F was abbreviated as PPIF.

DNMT3a refers to DNA methyltransferase 3a, NCBI entrez gene: 1788; Uniprotkb/Swiss-port: Q9Y6K1.

DNMT3b refers to DNA methyltransferase 3b, NCBI entrez gene: 1789; Uniprotkb/Swiss-port: Q9UBC3.

DNMT1 refers to DNA methyltransferase 1, NCBI entrez gene: 1786; Uniprotkb/Swiss-port: P26358.

UHRF1 refers to ubiquitin-like with PHD and ring finger domain 1, NCBI entrez gene: 29128; Uniprotkb/Swis s-port: Q96T88.

NNMT refers to Nicotinamide N-Methyltransferase.

The nucleotide sequence of the promoter region of NNMT gene was as shown in SEQ ID NO: 1.

The nucleotide sites from 1050 bp before the transcription start site to 499 bp after the transcription start site in NNMT gene was sites 951-2500 of nucleotide sequence as shown in SEQ ID NO: 1.

The nucleotide sites from 1050 bp to 193 bp before the transcription start site in NNMT gene was sites 951-1808 of nucleotide sequence as shown in SEQ ID NO: 1.

The nucleotide sites from 840 bp to 469 bp before the transcription start site in NNMT gene was sites 1161-1532 of nucleotide sequence as shown in SEQ ID NO: 1.

### Example 1 Synthesis of compound AB24994

The structure of compound AB24994 was as follows:

### Step 1):

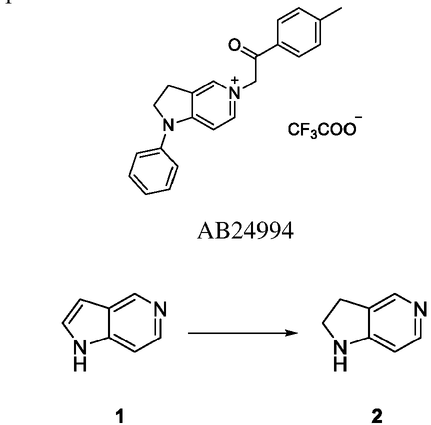

Compound 1 (300 mg, 2.54 mmol) was dissolved in acetic acid (10 mL), and palladium carbon (30 mg) was added. The mixture was stirred at 80 °C for 16 h under H₂, new spot was detected on a plate. The raction mixture was filtered and concentrated under reduced pressure to remove the organic solvent, the residue was purified by preparation chromatography to afford compound 2 (200 mg).

### Step 2):

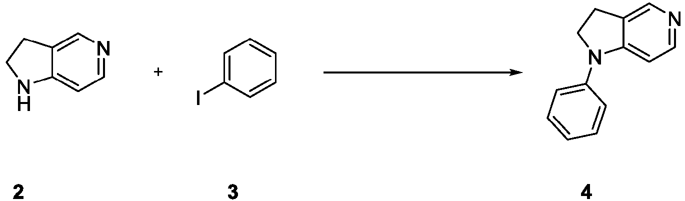

Compound 2 (200 mg, 1.65 mmol) was dissolved in dioxane (10 mL), compound 3 (337 mg, 1.65 mmol) and cyclohexane-1,2-diamine (40 mg, 0.33 mmol) were added, then potassium phosphate (1.75 g, 8.25 mmol) and cuprous iodide (7 mg, 0.033 mmol) were added. The mixture was stirred at 110 °C for 16 h, new spot was detected on a plate. The raction mixture was filtered and concentrated under reduced pressure to remove the organic solvent, the residue was purified by column chromatography (methanol: dichloromethane = 0-10 *%*) to afford compound 4 (100 mg).

### Step 3):

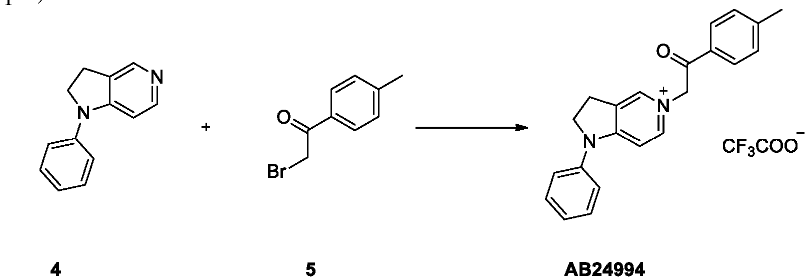

Compound 4 (100 mg, 0.51 mmol) was dissolved in acetonitrile (10 mL), compound 5 (160 mg, 0.76 mmol) was added. The mixture was stirred at room temperature for 16h, new spot was detected on a plate. The raction mixture was filtered and concentrated under reduced pressure to remove the organic solvent, the residue was purified by preparation chromatography (column: Sharpsil-U C18 (250 mm × 30 × 10 um, 100 Å); mobile phase: 0.1*%* aqueous trifluoroacetic acid + acetonitrile; gradient elution: 1-20 min, 5-25*%* ACN; 20-50 min, 25-35*%* ACN) to afford compound AB24994 (10 mg).

MS-ESI and ¹H NMR of the compound AB24994 were as follows:
MS-ESI: calculated [M-CF₃COO⁻]⁺ 329.16, Found: 329.15.
¹H NMR (400 MHz, dmso-d6) δ 8.18 (d, *J =* 6.9 Hz, 1H), 8.13 (s, 1H), 7.91 (d, *J =* 7.5 Hz, 2H), 7.52 (d, *J =* 6.6 Hz, 4H), 7.43 (d, *J =* 7.4 Hz, 2H), 7.34 (s, 1H), 7.12 (d, *J =* 6.8 Hz, 1H), 5.95 (s, 2H), 4.39 (s, 2H), 3.31 (s, 2H), 2.41 (s, 3H) .

### Example 2 Synthesis of compound AB24995

The structure of compound AB24995 was as follows:

### Step 1):

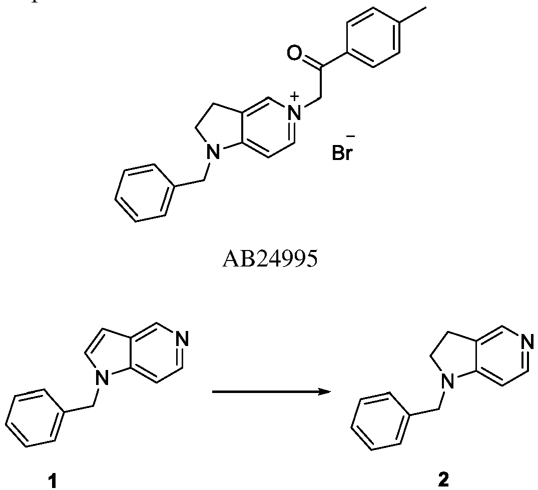

Compound 1 (300 mg, 1.44 mmol) was dissolved in acetic acid (10 mL), and palladium carbon (30 mg) was added. The mixture was stirred at 80 °C for 16 h under H₂, new spot was detected on a plate. The raction mixture was filtered and concentrated under reduced pressure to remove the organic solvent, the residue was purified by column chromatography (methanol: dichloromethane = 10:1) to afford compound 2 (160 mg).

### Step 2):

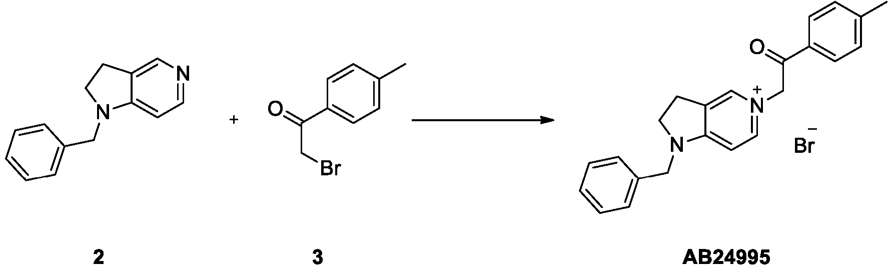

Compound 2 (100 mg, 0.48 mmol) was dissolved in acetonitrile (10 mL), compound 3 (122 mg, 0.57 mmol) was added. The mixture was stirred at room temperature for 16h, new spot was detected on a plate. The raction mixture was concentrated under reduced pressure to remove the organic solvent, the residue was purified by column chromatography (methanol: dichloromethane = 10:1) to afford compound AB24995 (100 mg).

MS-ESI and ¹H NMR of the compound AB24995 were as follows:
MS-ESI: calculated [M-Br⁻]⁺: 343.44, Found: 343.30.
¹H NMR (400 MHz, dmso-d6) δ 8.15 (d, *J =* 5.4 Hz, 1H), 7.91 (d, *J* = 7.8 Hz, 3H), 7.39 (d, *J* = 10.4 Hz, 4H), 7.35 (s, 3H), 7.08 (d, *J =* 6.3 Hz, 1H), 5.88 (s, 2H), 4.71 (s, 2H), 3.83 (d, *J =* 7.9 Hz, 2H), 3.16 (d, *J =* 8.0 Hz, 2H), 2.40 (s, 3H).

### Example 3 Synthesis of compound AB27101

The structure of compound AB27101 was as follows:

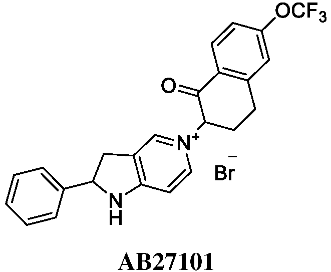

### Step 1):

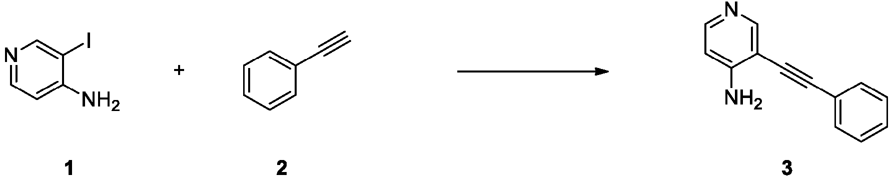

Compound 1 (4.4 g, 20 mmol) was dissolved in dried N, N-dimethylformamide (150 mL), and compound 2 (4.28 g, 42 mmol), bis(triphenylphosphine)dichloropalladium (421 mg, 0.6 mol), N, N-diisopropylethylamine (8.25 g, 64 mmol), and cuprous iodide (190 mg, 1 mmol) were added. The mixture was replaced with N₂ three times and stirred at room temperature for overnight, new spot was detected on a plate. The raction mixture was diluted with water (250 ml) and then extracted with ethyl acetate (100 mL × 3), the combined organic phase was washed with saturated saline (100 mL), dried over anhydrous sodium sulfate, filtered and concentrated under reduced pressure to remove the organic solvent, the residue was purified by column chromatography (ethyl acetate: petroleum ether = 0-50*%*) to afford compound 3 (3.5 g).

### Step 2:

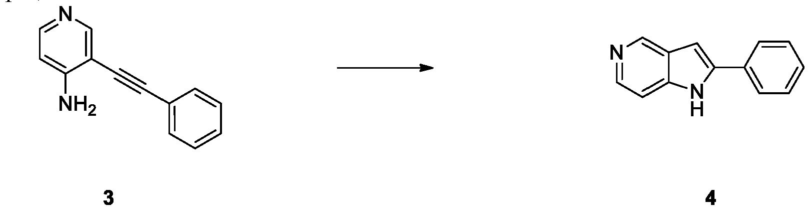

Compound 3 (3.5 g, 17.9 mmol) was dissolved in dioxane (100 mL), and a solution of potassium tert-butanol (53.8 ml, 1 mol) in tetrahydrofuran was added. The mixture was replaced with N₂ three times and stirred at 110 °C for 16 h, new spot was detected on a plate. The raction mixture was diluted with water (200 ml) and then extracted with ethyl acetate (100 mL × 3), the combined organic phase was washed with saturated saline (300 mL), dried over anhydrous sodium sulfate, filtered and concentrated under reduced pressure to remove the organic solvent, the residue was purified by column chromatography (methanol: dichloromethane = 0-10*%*) to afford compound 4 (2.2 g).
MS-ESI calculated [M+1]⁺ 195.23, Found: 195.12

### Step 3):

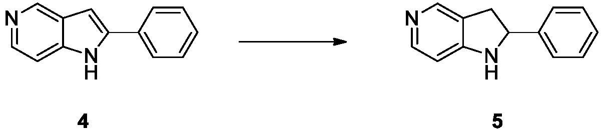

Compound 4 (800 mg, 4.12 mmol) was dissolved in ice acetic acid (50 mL), and palladium carbon (100 mg) was added. The mixture was stirred at 80 °C for 16 h under H₂, new spot was detected on a plate. The raction mixture was filtered with diatomite, the filtrate was rotary dried, the residue was purified by preparation chromatography to afford compound 5 (100 mg).

### Step 4):

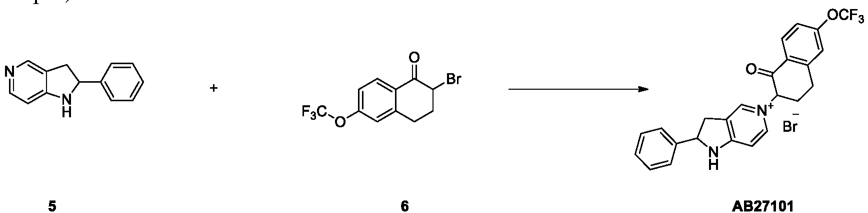

Compound 5 (80 mg, 0.41 mmol) was dissolved in acetone (10 mL), and compound 6 (379 mg, 1.22 mmol) and sodium acetate (168 mg, 2.05 mmol) were added. The mixture was replaced with N₂ three times and stirred at 80 °C for 16 h, new spot was detected on a plate. The raction mixture was concentrated under reduced pressure to remove the organic solvent, the residue was purified by thin layer chromatography to afford compound AB27101 (15.8 mg).

MS-ESI and ¹H NMR of the compound AB27101 were as follows:
MS-ESI: calculated [M-Br⁻]⁺ 425.42, Found: 425.55.
¹H NMR (400 MHz, CD₃OD) δ 8.14 (d, *J=* 8.1 Hz, 1H), 8.08 (d, *J=* 6.3 Hz, 1H), 7.94 (s, 1H), 7.37 (d, *J =* 6.4 Hz, 7H), 6.80 (d, *J =* 6.2 Hz, 1H), 5.50 (d, *J =* 11.5 Hz, 1H), 5.41 (s, 1H), 4.84 - 4.79 (m, 1H), 3.82 - 3.69 (m, 1H), 3.48 - 3.35 (m, 1H), 3.10 (dd, *J =* 17.0, 5.6 Hz, 1H), 2.81 (d, *J =* 11.1 Hz, 1H), 2.64 (s, 1H) .

### Example 4 Synthesis of compound AB24997

The structure of compound AB24997 was as follows:

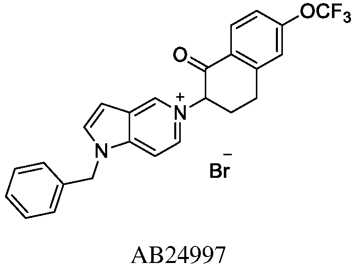

### Step 1):

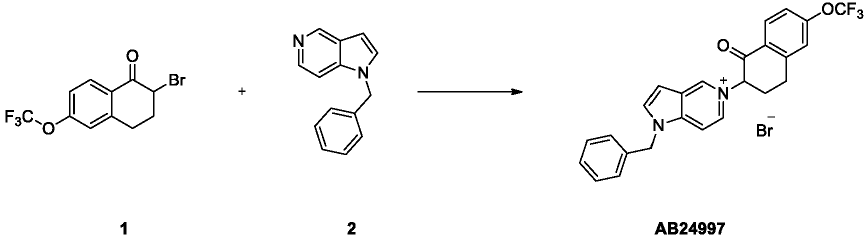

Compound 1 (240 mg, 0.78 mmol) was dissolved in acetonitrile (10 mL), and compound 2 (150 mg, 0.78 mmol) were added. The mixture was replaced with N₂ three times and stirred at room temperature for overnight, new spot was detected on a plate. The raction mixture was concentrated under reduced pressure to remove the organic solvent, the residue was purified by column chromatography (methanol: dichloromethane = 0-30*%*) to afford compound AB24997 (30 mg).

MS-ESI and ¹H NMR of the compound AB24997 were as follows:
MS-ESI: calculated [M-Br⁻]⁺ 437.44, Found: 437.20.
¹H NMR (400 MHz, dmso-d6) δ 9.44 (s, 1H), 8.60 (s, 1H), 8.38 (s, 1H), 8.18 (s, 1H), 8.04 (s, 1H), 7.53 (s, 1H), 7.42 (s, 1H), 7.36 (s, 5H), 7.16 (s, 1H), 6.30 (s, 1H), 5.68 (s, 2H), 3.04 (d, *J=* 12.4 Hz, 1H), 2.66 (s, 1H).

### Example 5 Synthesis of compound AB27171

The structure of compound AB27171 was as follows:

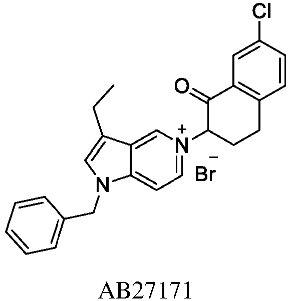

### Step 1):

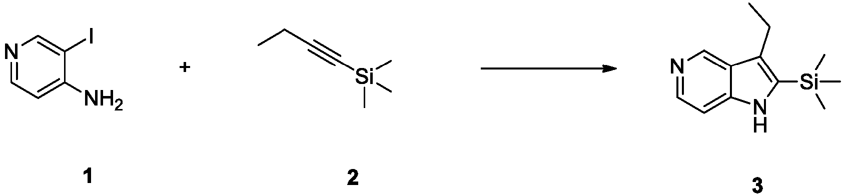

Compound 1 (2.5 g, 11.36 mmol) was dissolved in dried N-N-dimethylformamide (50 mL), and compound 2 (4.3 g, 34.08 mmol), dimethylferrocene palladium dichloride (519 g, 0.56 mmol), lithium chloride (481 mg, 11.36 mmol) and sodium carbonate (2.4 g, 22.72 mmol) were added. The mixture was replaced with N₂ three times and stirred at room temperature for overnight, new spot was detected on a plate. The raction mixture was diluted with water (50 ml) and then extracted with ethyl acetate (100 mL × 3), the combined organic phase was washed with saturated saline (100 mL), dried over anhydrous sodium sulfate, filtered and concentrated under reduced pressure to remove the organic solvent, the residue was purified by thin layer chromatography (ethyl acetate: petroleum ether = 0-50*%*) to afford compound 3 (1.25 g).

### Step 2):

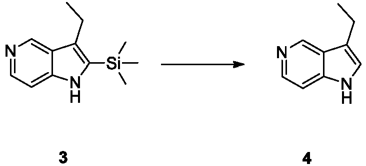

Compound 3 (1.25 g, 5.5 mmol) was dissolved in ethanol (50 mL), and sodium hydroxide (6.6 g, 165 mmol) was added. The mixture was replaced with N₂ three times and stirred at 90°C for overnight, new spot was detected on a plate. The raction mixture was diluted with water (20 ml) and then extracted with dichloromethane (30 mL × 3), the combined organic phase was washed with saturated saline (50 mL), dried over anhydrous sodium sulfate, filtered and concentrated under reduced pressure to remove the organic solvent, the residue was purified by thin layer chromatography (methanol: dichloromethane = 0-10*%*) to afford compound 4 (420 mg).

### Step 3):

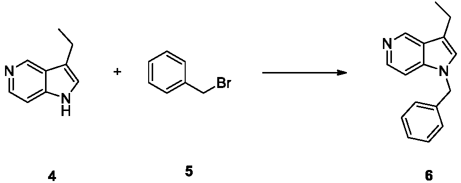

Compound 4 (146 mg, 1 mmol) was dissolved in dried N-N-dimethylformamide (5 mL) and sodium hydride (48 mg, 2 mmol) was added under ice bath. The mixture was replaced with N₂ three times and stirred for 30 min under ice bath, then compound 5 (256 mg, 1.5 mmol) was added slowly. The mixture was replaced with N₂ three times and stirred for 30 min under ice bath, new spot was detected on a plate. The raction mixture was diluted with water (50 ml) and then extracted with ethyl acetate (30 mL × 3), the combined organic phase was washed with saturated saline (50 mL), dried over anhydrous sodium sulfate, filtered and concentrated under reduced pressure to remove the organic solvent, the residue was purified by thin layer chromatography (ethyl acetate: petroleum ether = 0-50*%*) to afford compound 6 (96 mg).

### Step 4):

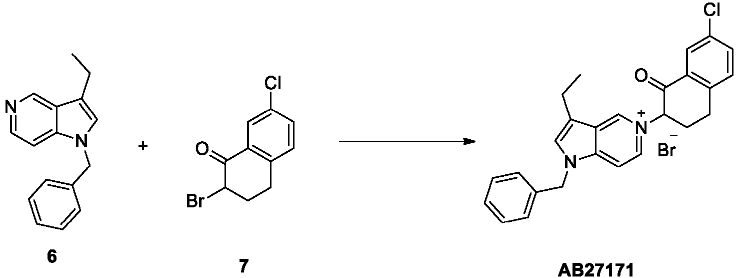

Compound 6 (90 mg, 0.41 mmol) was dissolved in acetonitrile (10 mL), and compound 7 (258 mg, 1.02 mmol) was added. The mixture was replaced with N₂ three times and stirred at room temperature for overnight, new spot was detected on a plate. The raction mixture was filtered and concentrated under reduced pressure to remove the organic solvent, the residue was purified by thin layer chromatography (methanol: dichloromethane = 0-10*%*) to afford compound AB27171 (20.9 mg).

MS-ESI and ¹H NMR of the compound AB27171 were as follows:
MS-ESI: calculated [M-Br⁻] ⁺ 415.15, Found: 415.20.
¹H NMR (400 MHz, dmso-d6) δ 9.42 (s, 1H), 8.51 (s, 1H), 8.29 (d, *J =* 6.4 Hz, 1H), 7.93 (s, 1H), 7.86 (s, 1H), 7.76 (d, *J* = 7.8 Hz, 1H), 7.55 (d, *J* = 7.9 Hz, 1H), 7.34 (s, 5H), 6.13 (d, *J* = 13.4 Hz, 1H), 5.59 (s, 2H), 3.29 - 3.25 (m, 2H), 3.05 (d, *J =* 11.1 Hz, 1H), 2.78 (d, *J =* 6.9 Hz, 2H), 2.65 (s, 1H), 1.27 (t, *J =* 6.7 Hz, 3H).

### Example 6 Synthesis of compound AB27172

The structure of compound AB27172 was as follows:

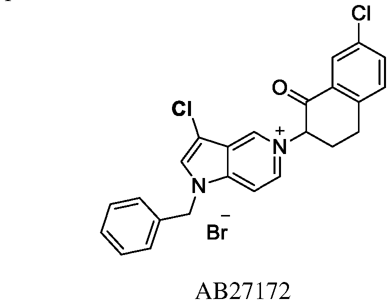

### Step 1:

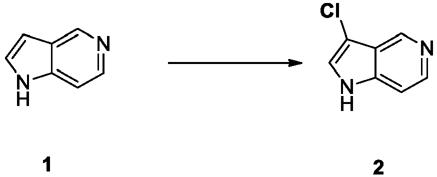

Compound 1 ( 200 mg, 1.69 mmol) was dissolved in dichloromethane (20 mL), and N-chlorosuccinimide (340 mg, 2.50 mmol) was added.The mixture was replaced with N₂ three times and stirred at room temperature for 5 h, new spot was detected on a plate. The raction mixture was diluted with aqueous sodium bicarbonate (20 mL) and then extracted with dichloromethane (30 mL x 3), the combined organic phase was washed with saturated saline (50 mL), dried over anhydrous sodium sulfate, filtered and concentrated under reduced pressure to remove the organic solvent, the residue was purified by thin layer chromatography (methanol: dichloromethane = 0-10*%*) to afford compound 2 (50 mg).

### Step 2):

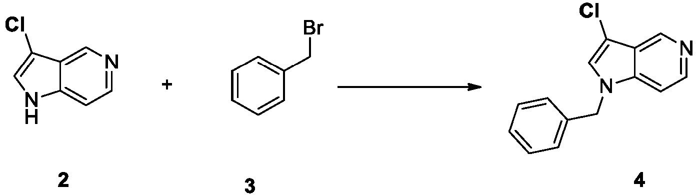

Compound 2 (50 mg, 0.32 mmol) was dissolved in N-N-dimethylformamide (10 mL), sodium hydride (39 mg, 0.96 mmol) was added under ice bath. The mixture was replaced with N₂ three times and stirred for 30 min under ice bath, then compound 3 (68 mg, 0.39 mmol) was injected slowly under ice bath. The mixture was stirred at room temperature for 60 min, new spot was detected on a plate. The raction mixture was quenched with aqueous ammonium chloride (20 mL) and then extracted with ethyl acetate (30 mL × 3), the combined organic phase was washed with saturated saline (50 mL), dried over anhydrous sodium sulfate, filtered and concentrated under reduced pressure to remove the organic solvent, the residue was purified by thin layer chromatography (methanol: dichloromethane = 0-10*%*) to afford compound 4 (60 mg).

### Step 3):

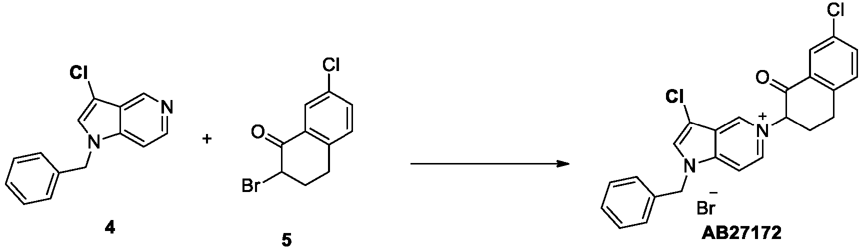

Compound 4 (60 mg, 0.24 mmol) was dissolved in acetonitrile (10 mL), and compound 5 (76 mg, 0.29 mmol) was added.The mixture was replaced with N₂ three times and stirred at 80°C for overnight, new spot was detected on a plate. The raction mixture was concentrated under reduced pressure to remove the organic solvent, the residue was purified by column chromatography (methanol: dichloromethane = 0-30*%*) to afford compound AB27172 (25 mg).

MS-ESI and ¹H NMR of the compound AB27172 were as follows:
MS-ESI: calculated [M-Br⁻]⁺: 421.09, Found: 421.00.
¹H NMR (400 MHz, DMSO) δ 9.52 (s, 1H), 8.68 (s, 1H), 8.47 (d, J = 9.2 Hz, 2H), 7.86 (s, 1H), 7.76 (d, J = 7.8 Hz, 1H), 7.55 (d, J = 7.8 Hz, 1H), 7.38 (d, J = 7.4 Hz, 5H), 6.28 (d, J = 11.3 Hz, 1H), 5.65 (s, 2H), 3.06 (d, J = 11.9 Hz, 3H), 2.64 (s, 1H).

### Example 7 Synthesis of compound AB27173

The structure of compound AB27173 was as follows:

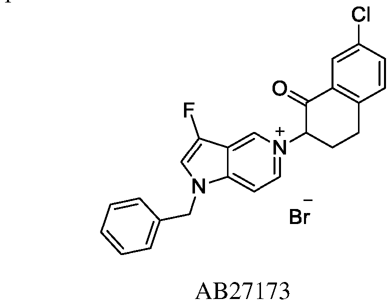

### Step 1:

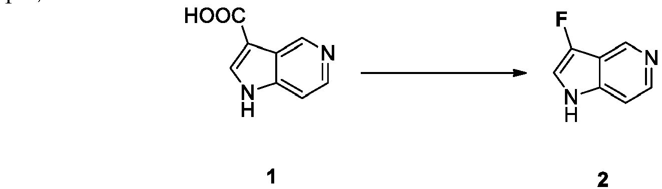

Compound 1 (100 mg, 0.62 mmol) was dissolved in ethyl acetate (4 mL) and water (2 mL), and lithium carbonate (182 mg, 2.50 mmol) was added. The mixture was replaced with N₂ three times and stirred at -20 °C for 1 h, then select fluor (438 mg, 1.24 mmol) was added slowly at -20 °C. The mixture was stirred for 7 h under ice bath, new spot was detected on a plate. The raction mixture was extracted with ethyl acetate (30 mL × 3), the combined organic phase was washed with saturated saline (50 mL), dried over anhydrous sodium sulfate, filtered and concentrated under reduced pressure to remove the organic solvent, the residue was purified by thin layer chromatography (methanol: dichloromethane = 0-10*%*) to afford compound 2 (50 mg).

### Step 2):

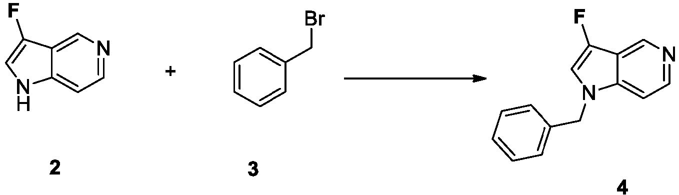

Compound 2 (200 mg, 1.47 mmol) was dissolved in N-N-dimethylformamide (10 mL), sodium hydride (118 mg, 2.94 mmol) was added under ice bath. The mixture was replaced with N₂ three times and stirred for 30 min under ice bath, then compound 3 (252 mg, 1.47 mmol) was injected slowly under ice bath. The mixture was stirred at room temperature for 60 min, new spot was detected on a plate. The raction mixture was quenched with aqueous ammonium chloride (20 mL) and then extracted with ethyl acetate (30 mL × 3), the combined organic phase was washed with saturated saline (50 mL), dried over anhydrous sodium sulfate, filtered and concentrated under reduced pressure to remove the organic solvent, the residue was purified by thin layer chromatography (methanol: dichloromethane = 0-10*%*) to afford compound 4 (70 mg).

### Step 3):

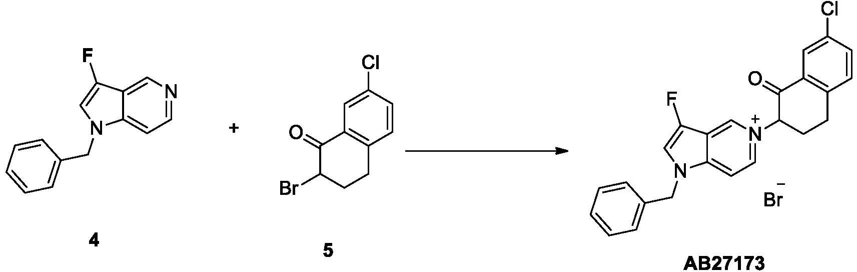

Compound 4 (70 mg, 0.34 mmol) was dissolved in acetonitrile (10 mL), and compound 5 (87 mg, 0.34 mmol) were added. The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromethane = 0-30*%*) to afford compound AB27173 (16 mg).

MS-ESI and ¹H NMR of the compound AB27173 were as follows:
MS-ESI: calculated [M-Br⁻]⁺ 405.87, Found: 405.
¹H NMR (400 MHz, DMSO) δ 9.57 (s, 1H), 8.59 (d, J = 6.0 Hz, 0H), 8.43 (d, J = 6.3 Hz, 0H), 8.26 (s, 0H), 7.86 (s, 0H), 7.77 (d, J = 7.5 Hz, 0H), 7.55 (d, J = 8.3 Hz, 0H), 7.36 (s, 3H), 6.14 (d, J = 14.4 Hz, 1H), 5.60 (s, 2H), 3.03 (d, J = 13.7 Hz, 2H), 2.64 (s, 2H).

### Example 8 Synthesis of compound AB27175

The structure of compound AB27175 was as follows:

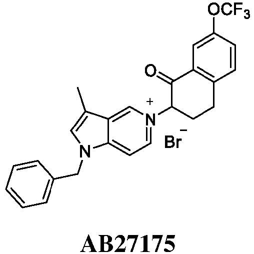

### Step 1):

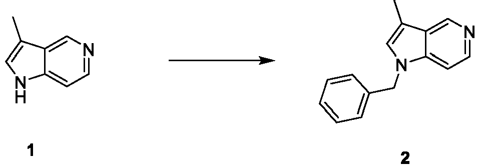

Compound 1 (2.6 g, 20.0 mmol) was dissolved in acetonitrile (50 mL), sodium hydrogen (960 mg, 40 mmol) was added at 0 °C, then benzyl bromide (3.6 g, 21 mmol) was added. The mixture was stirred at room temperature for 2 h, new spot was detected on a plate. The raction mixture was rotary dried, the residue was purified by thin layer chromatography to afford compound 2 (3.6g).

### Step 2):

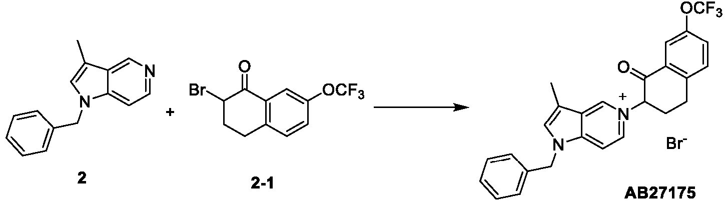

Compound 2 (2.1 g, 9.4 mmol) was dissolved in acetonitrile (50 mL), and compound 2-1 (2.8 g, 10 mmol) was added. The mixture was stirred at 80 °C for overnight, new spot was detected on a plate. The raction mixture was rotary dried, the residue was purified by thin layer chromatography to afford compound AB21715.

MS-ESI and ¹H NMR of the compound AB27175 were as follows:
MS-ESI calculated [M-Br-]⁺ 451.46, Found: 451.15.
¹H NMR (400 MHz, DMSO-d6) δ 9.43 (s, 1H), 8.54 (d, J = 6.2 Hz, 1H), 8.31 (d, J = 6.3 Hz, 1H), 7.90 (s, 1H), 7.80 - 7.71 (m, 2H), 7.67 (d, J = 8.0 Hz, 1H), 7.34 (s, 5H), 6.20 (d, J = 12.1 Hz, 1H), 5.60 (s, 2H), 3.31 - 3.25 (m, 2H), 3.14 - 3.04 (m, 1H), 2.65 (d, J = 7.4 Hz, 1H), 2.35 (s, 3H).

### Example 9 Synthesis of compound AB27177

The structure of compound AB27177 was as follows:

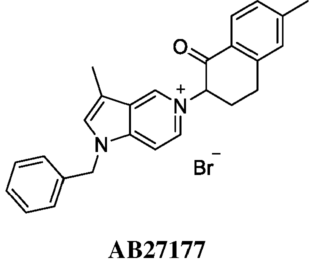

### Step 1):

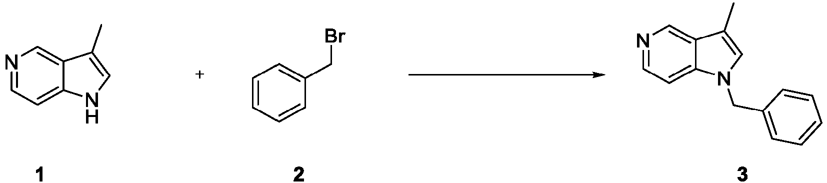

Compound 1 (508 mg, 4 mmol) was dissolved in dried N-N-dimethylformamide (30 mL), sodium hydride (288 mg, 12 mmol) was added under an ice bath. The mixture was stirred for 30 min under ice bath, then compound 2 (547 mg, 3.2 mmol) was added. The mixture was replaced with N₂ three times and stirred at room temperature for 0.5 h, new spot was detected on a plate. The raction mixture was diluted with water and extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (524 mg).

### Step 2):

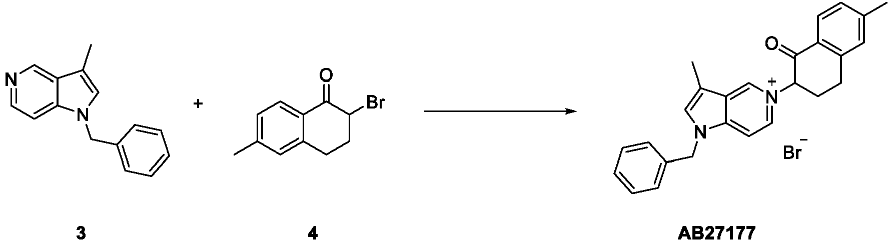

Compound 3 (111 mg, 0.5 mmol) was dissolved in acetonitrile (10 mL), and compound 4 (119 mg, 0.5 mmol) was added. The mixture was replaced with N₂ three times and stirred at room temperature for overnight, new spot was detected on a plate. The raction mixture was filtered and concentrated under reduced pressure to remove the organic solvent, the residue was purified by thin layer chromatography (methanol: dichloromethane = 0-10*%*) to afford compound AB27177 (20 mg).

MS-ESI and ¹H NMR of the compound AB27177 were as follows:
MS-ESI: calculated [M-Br⁻]⁺ 381.50, Found: 381.90.
¹H NMR (400 MHz, CD₃OD) δ 9.23 (s, 1H), 8.36 (d, *J =* 6.8 Hz, 1H), 8.01 (d, *J =* 6.5 Hz, 1H), 7.93 (d, *J =* 7.6 Hz, 1H), 7.67 (s, 1H), 7.35 (d, *J =* 7.1 Hz, 3H), 7.27 (d, *J =* 16.9 Hz, 4H), 5.57 (s, 2H), 3.43 (d, *J* = 15.2 Hz, 1H), 3.28 - 3.22 (m, 2H), 3.00 (d, *J* = 10.9 Hz, 1H), 2.72 (d, *J* = 10.4 Hz, 1H), 2.44 (s, 6H).

### Example 10 Synthesis of compound AB27150

The structure of compound AB27150 was as follows:

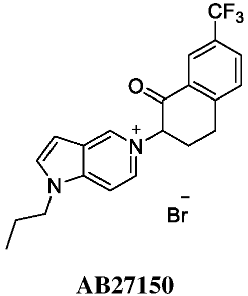

### Step 1):

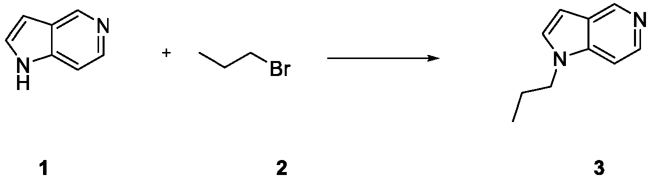

Compound 1 (200 mg, 1.69 mmol) was dissolved in N-N-dimethylformamide (10 mL), and sodium hydride (202.8 mg, 5.07 mmol) was added under ice bath. The mixture was replaced with N₂ three times and stirred for 30 min under ice bath, then compound 2 (310 mg, 2.54 mmol) was injected slowly under ice bath. The mixture was stirred at room temperature for 60 min, new spot was detected on a plate. The raction mixture was quenched with aqueous ammonium chloride and then extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (100 mg).

### Step 2):

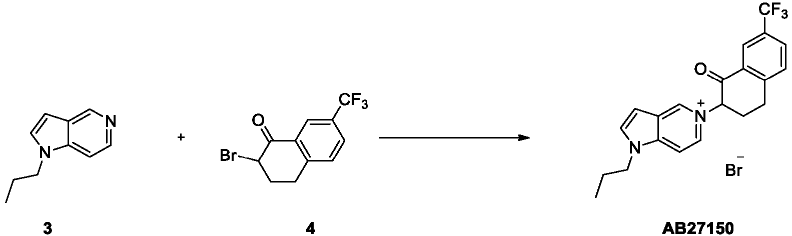

Compound 3 (100 mg, 0.62 mmol) was dissolved in acetonitrile (5 mL), and compound 4 (183 mg, 0.62 mmol) was added. The mixture was replaced with N₂ three times and stirred at 80°C for overnight, new spot was detected on a plate. The raction mixture was concentrated under reduced pressure to remove the organic solvent, the residue was purified by column chromatography (methanol: dichloromethane = 0-30*%*) to afford compound AB27150 (30 mg).

MS-ESI and ¹H NMR of the compound AB27150 were as follows:
MS-ESI: calculated [M-Br⁻]⁺ 373.39, Found373.05.
¹H NMR (400 MHz,DMSO-d6) δ 9.36 (s, 1H), 8.56 (d, J = 6.4 Hz, 1H), 8.34 (d, J = 6.5 Hz, 1H), 8.11 (d, J = 27.0 Hz, 3H), 7.76 (d, J = 7.8 Hz, 1H), 7.13 (s, 1H), 6.21 (d, J = 12.1 Hz, 1H), 4.37 (s, 2H), 3.39 (s, 2H), 3.05 (s, 1H), 2.71 (s, 1H), 1.84 (d, J = 6.4 Hz, 2H), 0.83 (s, 3H).

### Example 11 Synthesis of compound AB27151

The structure of compound AB27151 was as follows:

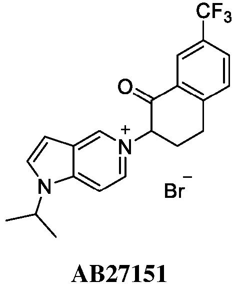

### Step 1):

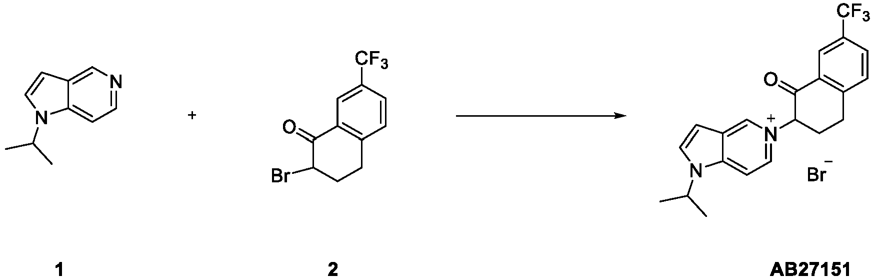

Compound 1 (60 mg, 0.37 mmol) was dissolved in acetonitrile (10 mL), and compound 2 (110 mg, 0.37 mmol) was added. The mixture was replaced with N₂ three times and stirred at 60°C for overnight, new spot was detected on a plate. The raction mixture was concentrated under reduced pressure to remove the organic solvent, the residue was purified by thin layer chromatography to afford compound AB27151 (20 mg).

MS-ESI and ¹H NMR of the compound AB27151 were as follows:
MS-ESI: calculated [M-Br⁻]⁺ 373.40, Found373.30 .
¹H NMR (400 MHz, DMSO -d6) δ 9.37 (s, 1H), 8.57 (d, *J* = 6.8 Hz, 1H), 8.35 (d, *J* = 6.7 Hz, 1H), 8.23 (s, 1H), 8.14 (s, 1H), 8.07 (d, *J* = 7.8 Hz, 1H), 7.77 (d, *J* = 7.9 Hz, 1H), 7.17 (s, 1H), 6.22 (s, 1H), 5.19 - 4.90 (m, 1H), 3.41 (d, *J=* 13.6 Hz, 2H), 3.07 (d, *J* = 9.2 Hz, 1H), 2.70 (s, 1H), 1.52 (s, 6H) .

### Example 12 Synthesis of compound AB27157

The structure of compound AB27157 was as follows:

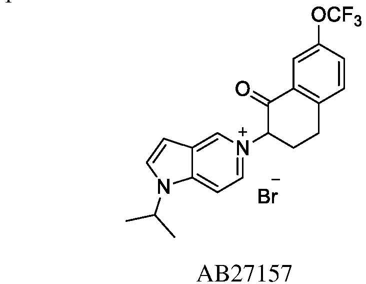

### Step 1):

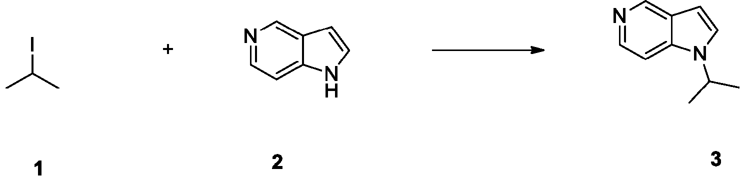

Compound 2 (500 mg, 4.23 mmol) was dissolved in N,N-dimethylformamide (10 mL), and sodium hydrogen (507.6 mg, 12.69 mmol) was added at 0 °C. The mixture was stirred for 30 min, and compound 1 (1.07 g, 6.34 mmol) was added at 0 °C. The mixture was stirred ar room temperature for 2 h, new spot was detected on a plate. The raction mixture was extracted with water and ethyl acetate, the organic phase was rotary dried, the residue was purified by column chromatography to afford compound 3 (300 mg).

### Step 2):

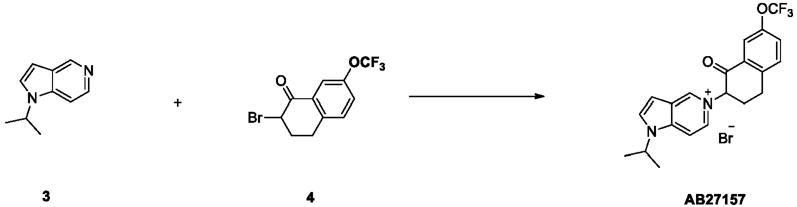

Compound 3 (60 mg, 0.31 mmol) was dissolved in acetonitrile (10 mL), and compound 4 (100 mg, 0.31 mmol) was added. The mixture was replaced with N₂ three times and stirred at 60°C for overnight, new spot was detected on a plate. The raction mixture was concentrated under reduced pressure to remove the organic solvent, the residue was purified by thin layer chromatography to afford compound AB27157 (20 mg).

MS-ESI and ¹H NMR of the compound AB27157 were as follows:
MS-ESI; calculated [M-Br⁻]⁺ 389.15, Found: 389.25.
¹H NMR (400 MHz, dmso-d6) δ 9.36 (s, 1H), 8.56 (d, J = 6.0 Hz, 1H), 8.34 (d, J = 6.7 Hz, 1H), 8.23 (s, 1H), 7.71 (dd, J = 35.0, 15.1 Hz, 3H), 7.16 (s, 1H), 6.19 (d, J = 14.1 Hz, 1H), 5.06 (s, 1H), 3.33 (s, 2H), 3.07 (s, 1H), 2.65 (s, 1H), 1.52 (d, J = 3.9 Hz, 6H).

### Example 13 Synthesis of compound AB27200

The structure of compound AB27200 was as follows:

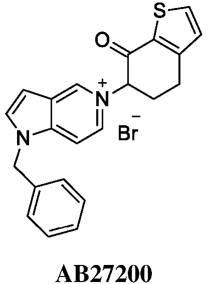

### Step 1):

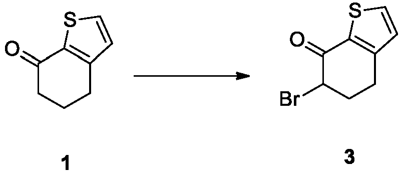

Compound 1 (100 mg, 0.65 mmol) was dissolved in dichloromethane (20 mL), 2 drops of methanol were added, then bis(N,N-dimethylacetamide)hydrogendibromobromate (273 mg, 0.65 mmol) was added. The mixture was stirred at 40 °C for overnight, new spot was detected on a plate. The raction mixture was rotary dried, the residue was purified by thin layer chromatography to afford compound 3 (106 mg).

### Step 2):

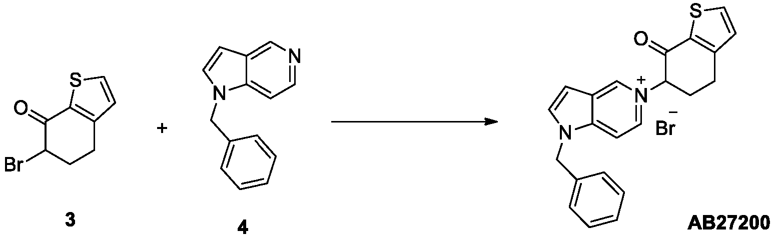

Compound 3 (106 mg, 0.46 mmol) was dissolved in acetonitrile (20 mL), and compound 4 (80 mg, 0.38 mmol) was added. The mixture was stirred at 80 °C for overnight, new spot was detected on a plate. The raction mixture was rotary dried, the residue was purified by thin layer chromatography to afford compound AB27200 (15 mg).

MS-ESI and ¹H NMR of the compound AB27200 were as follows:
MS-ESI calculated [M-Br⁻]⁺ 359.12, Found: 359.20.
¹H NMR (400 MHz, DMSO) δ 9.42 (s, 1H), 8.59 (s, 1H), 8.34 (s, 1H), 8.18 (d, *J* = 10.1 Hz, 2H), 7.35 (s, 4H), 7.26 (s, 2H), 7.14 (s, 1H), 6.15 (d, *J =* 9.0 Hz, 1H), 5.67 (s, 2H), 3.09 (d, *J =* 49.0 Hz, 3H), 2.68 (s, 1H).

### Example 14 Synthesis of compound AB27206

The structure of compound AB27206 was as follows:

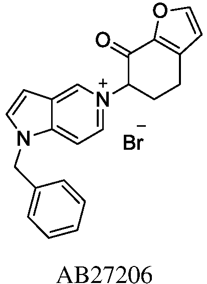

### Step 1):

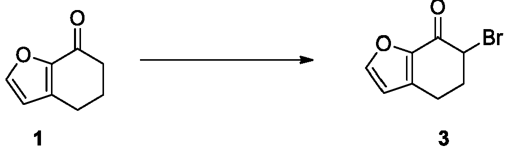

Compound 1 (120 mg, 0.88 mmol) was dissolved in dichloromethane (10 mL), 2 drops of methanol were added, then bis(N,N-dimethylacetamide)hydrogendibromobromate (365 mg, 0.88 mmol) was added. The mixture was stirred at 50 °C for overnight, new spot was detected on a plate. The raction mixture was rotary dried, the residue was purified by thin layer chromatography to afford compound 3 (100 mg).

### Step 2):

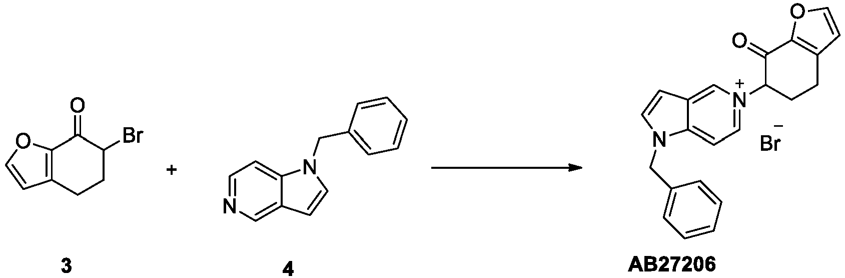

Compound 3 (100 mg, 0.47 mmol) was dissolved in acetonitrile (10 mL), and compound 4 (98 mg, 0.47 mmol) was added. The mixture was replaced with N₂ three times and stirred at 80 °C for overnight, new spot was detected on a plate. The mixture was concentrated under reduced pressure to remove the organic solvent, the residue was purified by thin layer chromatography to afford compound AB27206.

MS-ESI and ¹H NMR of the compound AB27206 were as follows:
MS-ESI: calculated [M-Br⁻]⁺343.14, Found: 343.00.
¹H NMR (400 MHz, dmso-d6) δ 9.41 (s, 1H), 8.59 (d, J = 7.1 Hz, 1H), 8.35 (d, J = 7.0 Hz, 1H), 8.16 (s, 2H), 7.34 (s, 5H), 7.14 (s, 1H), 6.80 (s, 1H), 6.07 (d, J = 9.6 Hz, 1H), 5.66 (s, 2H), 3.03 (s, 2H), 2.95 (d, J = 9.5 Hz, 1H), 2.64 (s, 1H).

### Example 15 Synthesis of compound AB27215

The structure of compound AB27215 was as follows:

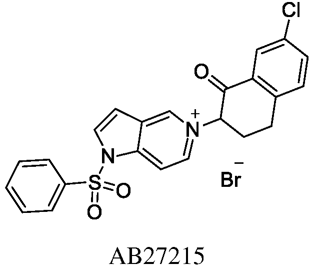

### Step 1):

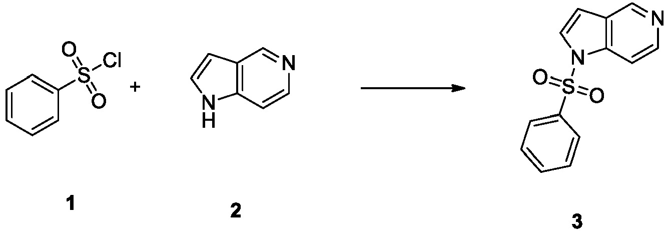

Compound 1 (353 mg, 2 mmol) was dissolved in acetonitrile (10 mL), compound 2 (236 mg, 2 mmol) was added, then triethylamine (401.76 mg, 4 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (methanol: dichloromethane = 0-10%) to afford compound 3 (500 mg).

### Step 2):

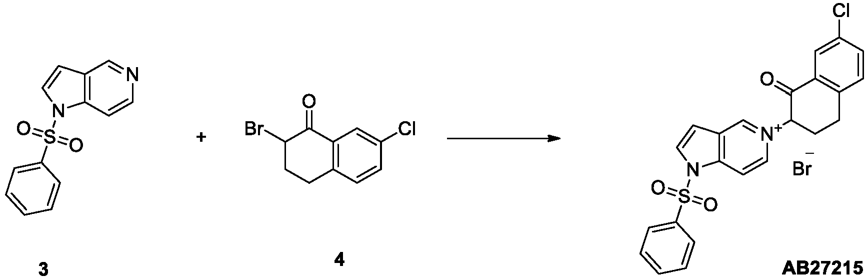

Compound 3 (200 mg, 0.77 mmol) was dissolved in acetonitrile (10 mL), and compound 4 (200 mg, 0.8 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27215.

MS-ESI and ¹H NMR of the compound AB27215 were as follows:
MS-ESI: calculated [M-Br⁻]⁺ 437.92, Found: 437.05.
¹H NMR (400 MHz, cdsod) δ 9.38 (s, 1H), 8.69 (d, *J =* 16.5 Hz, 2H), 8.37 (s, 1H), 8.23 (d, *J =* 4.9 Hz, 2H), 7.97 (s, 1H), 7.80 (s, 1H), 7.68 (s, 3H), 7.49 (s, 1H), 7.26 (s, 1H), 3.41 (s, 3H), 2.99 (s, 1H), 2.77 (s, 1H).

### Example 16 Synthesis of compound AB27219

The structure of compound AB27219 was as follows:

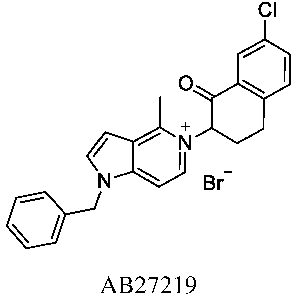

### Step 1):

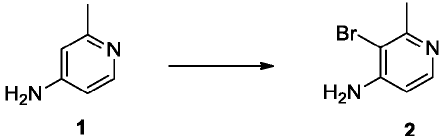

Compound 1 (5 g, 46.2 mmol) was dissolved in acetonitrile (100 mL), and N-bromosuccinimide (9.07 g, 50.9 mmol) was added. The mixture was replaced with N₂ three times and stirred at room temperature for 2 h, new spot was detected on a plate. The raction mixture was concentrated under reduced pressure to remove the organic solvent, the residue was purified by thin layer chromatography (methanol: dichloromethane = 0-10*%*) to afford compound 2 (3.5 g).

### Step 2):

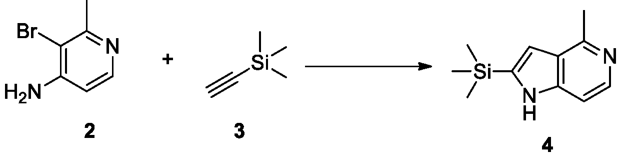

Compound 2 (1.87 g, 10 mmol) was dissolved in dried N-N-dimethylformamide (50 mL), and compound 3 (2.94 g, 10 mmol), dimethylferrocene palladium dichloride (404 mg, 0.5 mmol), lithium chloride (429 mg, 20 mmol) and sodium carbonate (3.18 g, 30 mmol) were added. The mixture was replaced with N₂ three times and stirred at room temperature for overnight, new spot was detected on a plate. The raction mixture was diluted with water (50 ml) and then extracted with ethyl acetate (50 mL x 3), the combined organic phase was washed with saturated saline (100 mL), dried over anhydrous sodium sulfate, filtered and concentrated under reduced pressure to remove the organic solvent, the residue was purified by thin layer chromatography (methanol: dichloromethane = 0-10%) to afford compound 4 (500 g).

### Step 3):

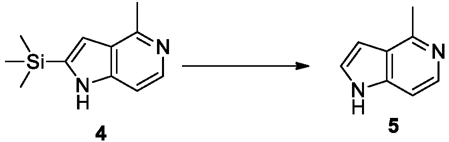

Compound 4 (300 mg, 1.47 mmol) was dissolved in ethanol (20 mL), and sodium hydroxide (1.76 g, 44.11 mmol) was added. The mixture was replaced with N₂ three times and stirred at 90 °C for overnight, new spot was detected on a plate. The raction mixture was concentrated under reduced pressure to remove the organic solvent, diluted with water (50 ml) and then extracted with ethyl acetate (50 mL x 3), the combined organic phase was washed with saturated saline (100 mL), dried over anhydrous sodium sulfate, filtered and concentrated under reduced pressure to remove the organic solvent, the residue was purified by thin layer chromatography (dichloromethane : methanol = 0-10%) to afford compound 5 (130 mg).

### Step 4):

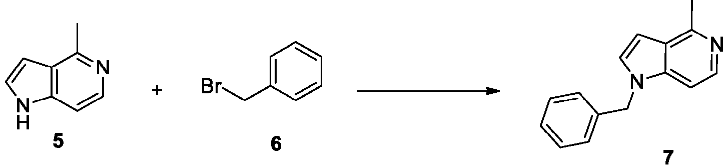

Compound 5 (130 mg, 0.98 mmol) was dissolved in N-N-dimethylformamide (20 mL), sodium hydride (118 mg, 2.93 mmol) was added under ice bath. The mixture was replaced with N₂ three times and stirred for 30 min under ice bath, then compound 6 (167 mg, 0.98 mmol) was injected slowly under ice bath. The mixture was stirred at room temperature for 60 min, new spot was detected on a plate. The raction mixture was diluted with water (10 mL) and then extracted with ethyl acetate (30 mL x 3), the combined organic phase was washed with saturated saline (100 mL), dried over anhydrous sodium sulfate, filtered and concentrated under reduced pressure to remove the organic solvent, the residue was purified by column chromatography (methanol: dichloromethane = 10%) to afford compound 7 (100 mg).

### Step 5:

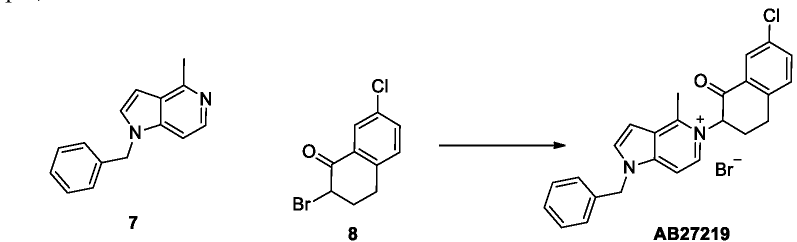

Compound 7 (100 mg, 0.45 mmol) was dissolved in acetonitrile (10 mL), and compound 8 (174 mg, 0.67 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27219 (9.5 mg).

MS-ESI and ¹H NMR of the compound AB27219 were as follows:
MS-ESI calculated [M-Br⁻] ⁺ 401.14, Found: 401.30.
¹H NMR (400 MHz, DMSO) δ 8.51 (d, *J =* 6.7 Hz, 1H), 8.17 (d, *J =* 6.4 Hz, 1H), 8.09 (s, 1H), 7.85 (s, 1H), 7.75 (d, *J* = 7.6 Hz, 1H), 7.54 (d, *J* = 7.9 Hz, 1H), 7.32 (s, 6H), 6.20 (d, *J* = 11.1 Hz, 1H), 5.63 (s, 2H), 3.20 (s, 2H), 3.01 (s, 3H), 2.63 (s, 2H).

### Example 17 Synthesis of compound AB27247

The structure of compound AB27247was as follows:

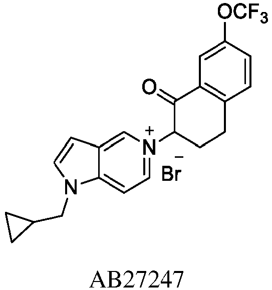

### Step 1):

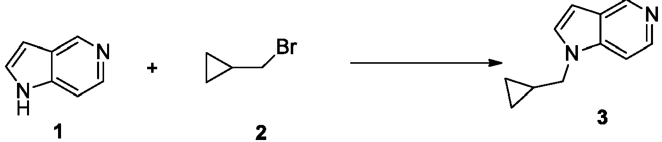

Compound 1 (600 mg, 5.08 mmol) was dissolved in N-N-dimethylformamide (10 mL), and sodium hydride (610 mg, 15.25 mmol) was added under ice bath. The mixture was replaced with N₂ three times and stirred for 30 min under ice bath, then compound 2 (1.0 g, 7.26 mmol) was injected slowly under ice bath. The mixture was stirred at room temperature for 60 min, new spot was detected on a plate. The raction mixture was quenched with aqueous ammonium chloride and then extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (500 mg).

### Step 2):

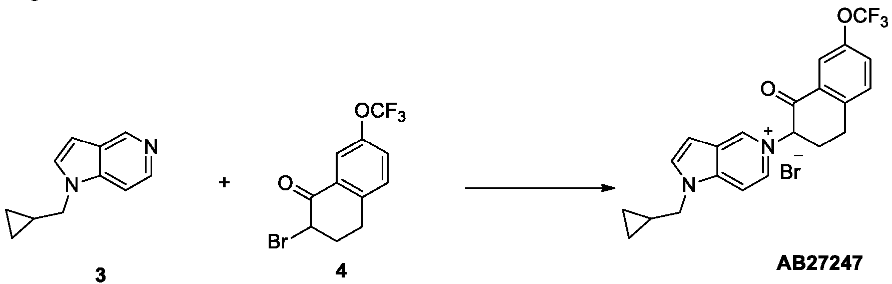

Compound 3 (100 mg, 0.62 mmol) was dissolved in acetonitrile (5 mL), and compound 4 (183 mg, 0.62 mmol) was added.The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromethane = 0-30*%*) to afford compound AB27247.

MS-ESI and ¹H NMR of the compound AB27247 were as follows:
MS-ESI calculated [M-Br⁻]⁺ 401.15, Found: 401.25.
¹H NMR (400 MHz, DMSO) δ 9.36 (s, 1H), 8.56 (d, *J =* 5.6 Hz, 1H), 8.35 (d, *J =* 5.8 Hz, 1H), 8.12 (s, 1H), 7.77 (s, 1H), 7.72 (s, 1H), 7.66 (d, *J =* 7.3 Hz, 1H), 7.13 (s, 1H), 6.19 (d, *J =* 13.4 Hz, 1H), 4.29 (d, *J =* 5.8 Hz, 2H), 3.32 (s, 2H), 3.06 (d, *J =* 8.4 Hz, 1H), 2.69 (s, 1H), 1.32 (s, 1H), 0.54 (d, *J =* 5.3 Hz, 2H), 0.47 (s, 2H).

### Example 18 Synthesis of compound AB27248

The structure of compound AB27248 was as follows:

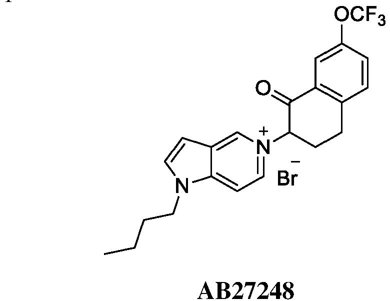

### Step 1):

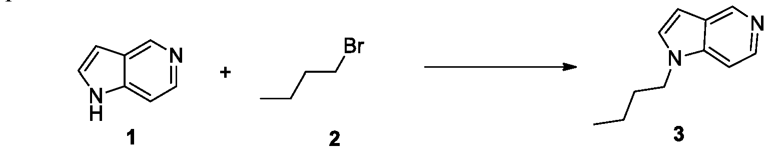

Compound 1 (1.18 g, 10 mmol) was dissolved in N, N-dimethylformamide (50 mL), and sodium hydride (1.2 mg, 30 mmol) was added under ice bath. The mixture was replaced with N₂ three times and stirred for 30 min under ice bath, then compound 2 (2.05 g, 7.26 mmol) was injected slowly under ice bath. The mixture was stirred at room temperature for 60 min, new spot was detected on a plate. The raction mixture was quenched with aqueous ammonium chloride and then extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (870 mg).

### Step 2):

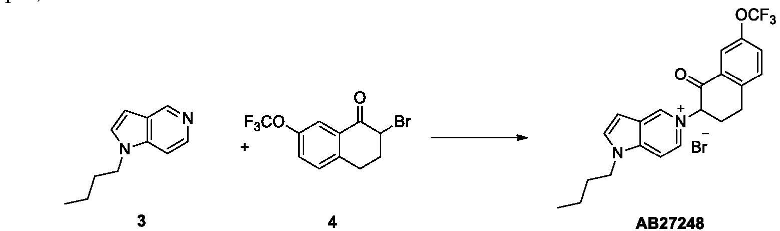

Compound 3 (100 mg, 0.57 mmol) was dissolved in acetonitrile (5 mL), and compound 4 (177 mg, 0.57 mmol) was added. The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromethane = 0-30*%*) to afford compound AB27248 (15 mg, yield: 6.53*%*).

MS-ESI and ¹H NMR of the compound AB27248 were as follows:
MS-ESI calculated [M-Br⁻]⁺ 403.16, Found: 403.
¹H NMR (400 MHz, DMSO) δ 9.34 (s, 1H), 8.55 (s, 1H), 8.32 (s, 1H), 8.06 (s, 1H), 7.77 (s, 1H), 7.72 (s, 1H), 7.66 (s, 1H), 7.12 (s, 1H), 6.16 (d, *J =* 12.7 Hz, 1H), 4.39 (s, 2H), 3.06 (s, 1H), 2.68 (s, 1H), 2.50 (s, 2H), 1.78 (s, 2H), 1.23 (d, *J =* 6.2 Hz, 2H), 0.88 (d, *J =* 6.0 Hz, 3H).

### Example 19 Synthesis of compound AB27249

The structure of compound AB27249 was as follows:

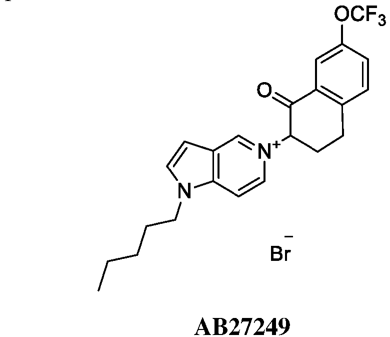

### Step 1:

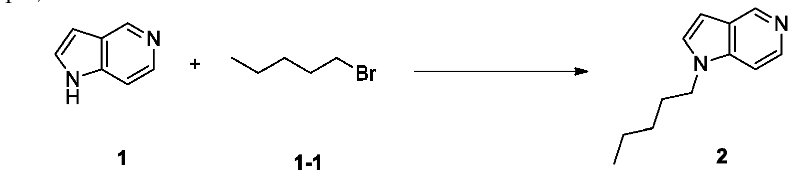

Compound 1 (1.06 g, 9 mmol) was dissolved in N, N-dimethylformamide (20 mL), and sodium hydride (648 mg, 27 mmol) was added under ice bath. The mixture was replaced with N₂ three times and stirred for 30 min under ice bath, then compound 1-1 (1.5 g, 9 mmol) was injected slowly under ice bath. The mixture was stirred at room temperature for 90 min, new spot was detected on a plate. The raction mixture was diluted with water (20 mL) and concentrated under reduced pressure to remove the organic solvent. The crude product was filtered with methanol, the filtrate was concentrated under reduced pressure to remove the organic solvent, the residue was purified by thin layer chromatography (methanol: dichloromethane = 10*%*) to afford compound 2 (1.2 g).

### Step 2):

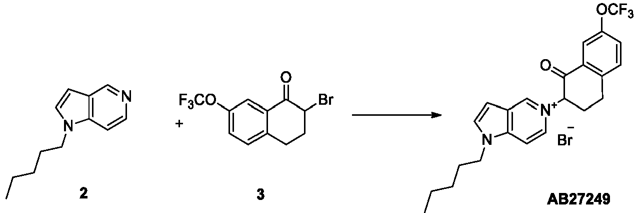

Compound 2 (100 mg, 0.57 mmol) was dissolved in acetonitrile (10 mL), and compound 3 (176 mg, 0.57 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane = 10*%*) to afford compound AB27249.

MS-ESI and ¹H NMR of the compound AB27249 were as follows:
MS-ESI calculated [M-Br⁻] ⁺417.44, Found: 417.
¹H NMR (400 MHz, CD3OD) δ 9.35 (s, 1H), 8.56 (d, *J =* 6.7 Hz, 1H), 8.32 (d, *J =* 6.7 Hz, 1H), 8.07 (s, 1H), 7.77 (s, 2H), 7.72 (s, 1H), 7.66 (d, *J =* 8.3 Hz, 1H), 7.12 (s, 1H), 6.18 (d, *J =* 10.3 Hz, 1H), 4.38 (d, *J =* 6.4 Hz, 2H), 3.37 (s, 1H), 3.29 - 3.26 (m, 1H), 3.08 - 2.99 (m, 1H), 2.67 (d, *J =* 9.5 Hz, 1H), 1.86 - 1.73 (m, 2H), 1.36 - 1.24 (m, 2H), 1.19 (d, *J =* 5.4 Hz, 3H), 0.81 (t, *J =* 6.9 Hz, 3H).

### Example 20 Synthesis of compound AB27250

The structure of compound AB27250 was as follows:

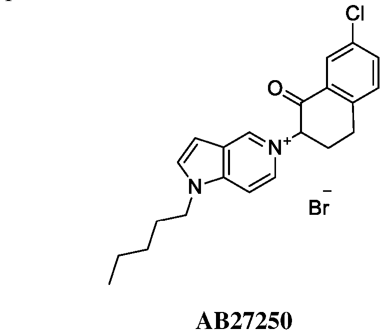

### Step 1):

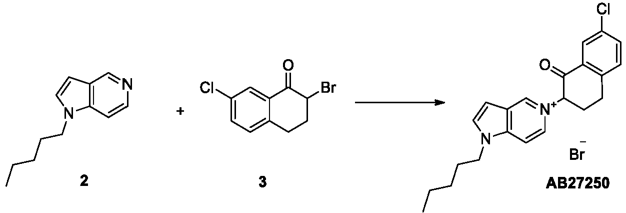

Compound 2 (100 mg, 0.57 mmol) was added to acetonitrile (10 mL), and compound 3 (147 mg, 0.57 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane = 10*%*) to afford compound AB27250.

MS-ESI and ¹H NMR of the compound AB27250 were as follows:
MS-ESI calculated [M-Br⁻]⁺367.89, Found: 367.30.
¹H NMR (400 MHz, DMSO) δ 9.35 (s, 1H), 8.55 (d, *J* = 6.9 Hz, 1H), 8.32 (d, *J* = 7.0 Hz, 1H), 8.08 (d, *J =* 2.7 Hz, 1H), 7.86 (s, 1H), 7.77 (d, *J =* 6.9 Hz, 1H), 7.55 (d, *J =* 8.3 Hz, 1H), 7.12 (d, *J =* 2.5 Hz, 1H), 6.21 - 6.11 (m, 1H), 4.39 (t, *J =* 6.6 Hz, 2H), 3.28 (s, 2H), 3.06-2.97 (m, 1H), 2.66 (d, *J =* 10.2 Hz, 1H), 1.85 - 1.74 (m, 2H), 1.29 (dd, *J =* 14.1, 7.0 Hz, 2H), 1.20 (d, *J =* 7.0 Hz, 2H), 0.82 (t, *J* = 7.0 Hz, 3H).

### Example 21 Synthesis of compound AB27251

The structure of compound AB27251 was as follows:

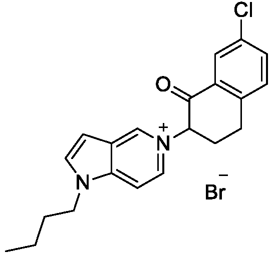

### AB27251

### Step 1:

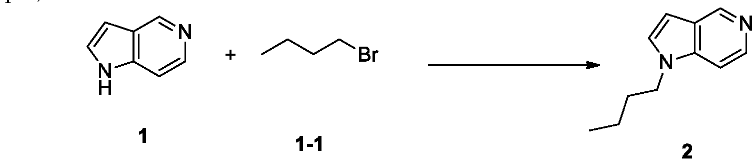

Compound 1 (1.06 mg, 9 mmol) was dissolved in N, N-dimethylformamide (20 mL), and sodium hydride (648 mg, 27 mmol) was added under ice bath. The mixture was replaced with N₂ three times and stirred for 30 min under ice bath, then compound 1-1 (986 mg, 7.2 mmol) was injected slowly under ice bath. The mixture was stirred at room temperature for 90 min, new spot was detected on a plate. The raction mixture was diluted with water (20 mL) and concentrated under reduced pressure to remove the organic solvent to afford crude product. The crude product was triturated with methanol and filtered, the filtrate was concentrated under reduced pressure to afford crude product, the crude product was purified by thin layer chromatography (methanol: dichloromethane =10*%*) to afford compound 2 (870 mg).

### Step 2):

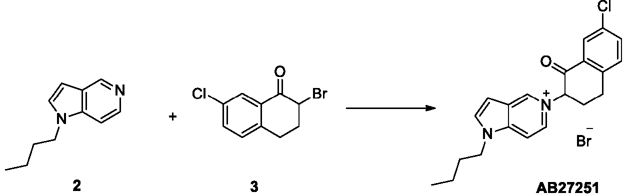

Compound 2 (100 mg, 0.57 mmol) was added to acetonitrile (10 mL), and compound 3 (147 mg, 0.57 mmol) was added. The mixture was stirred at 80 °C for overnight, new spot was detected on a plate. The raction mixture was concentrated under reduced pressure to remove the organic solven to afford crude product. The crude product was triturated with methanol and filtered, the filtrate was concentrated under reduced pressure to remove the organic solvent, the residue was purified by thin layer chromatography (methanol: dichloromethane =10*%*) to afford compound AB27251.

MS-ESI and ¹H NMR of the compound AB27251 were as follows:
MS-ESI calculated [M-Br⁻]⁺353.86, Found: 353.05.
¹H NMR (400 MHz, DMSO) δ 9.34 (s, 1H), 8.53 (s, 1H), 8.32 (s, 1H), 8.07 (s, 1H), 7.86 (s, 1H), 7.76 (d, *J =* 8.3 Hz, 1H), 7.55 (d, *J =* 7.3 Hz, 1H), 7.12 (s, 1H), 6.12 (d, *J =* 10.3 Hz, 1H), 4.40 (s, 2H), 3.02 (d, *J =* 14.0 Hz, 2H), 2.66 (d, *J =* 13.5 Hz, 2H), 1.84 - 1.71 (m, 2H), 1.22 (s, 2H), 0.88 (s, 3H).

### Example 22 Synthesis of compound AB27252

The structure of compound AB27252 was as follows:

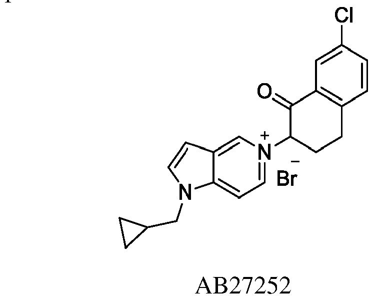

### Step 1):

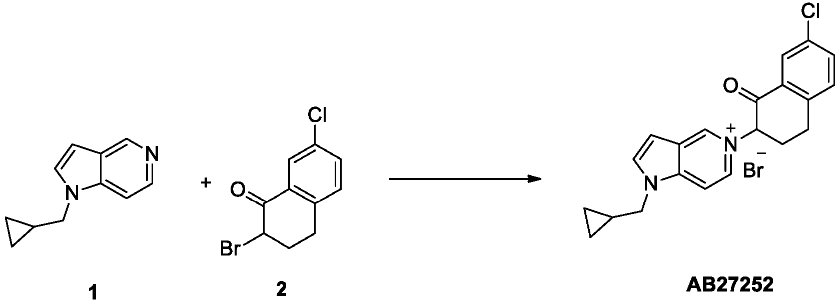

Compound 1 (100 mg, 0.58 mmol) was added to acetonitrile (10 mL), and compound 2 (150 mg, 0.58 mmol) was added. The mixture was stirred at 80 °C for overnight, new spot was detected on a plate. The raction mixture was concentrated under reduced pressure to remove the organic solven to afford crude product. The crude product was triturated with methanol and filtered, the filtrate was concentrated under reduced pressure to remove the organic solvent, the residue was purified by thin layer chromatography (methanol: dichloromethane =10*%*) to afford compound AB27252.

MS-ESI and ¹H NMR of the compound AB27252 were as follows:
MS-ESI calculated [M-Br⁻]⁺ 351.85, Found: 351.25.
¹H NMR (400 MHz, DMSO) δ 9.36 (s, 1H), 8.55 (d, *J =* 5.6 Hz, 1H), 8.35 (d, *J =* 5.6 Hz, 1H), 8.12 (s, 1H), 7.86 (s, 1H), 7.76 (d, *J =* 7.4 Hz, 1H), 7.55 (d, *J =* 7.6 Hz, 1H), 7.13 (s, 1H), 6.18 (d, *J* = 13.2 Hz, 1H), 4.29 (d, *J =* 5.8 Hz, 2H), 3.18 (s, 2H), 3.04 (s, 1H), 2.68 (s, 1H), 1.32 (s, 1H), 0.54 (d, *J* = 5.2 Hz, 2H), 0.47 (s, 2H).

### Example 23 Synthesis of compound AB27253

The structure of compound AB27253 was as follows:

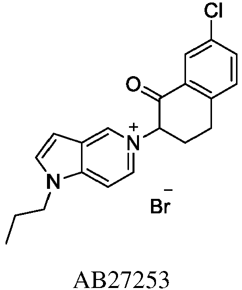

### Step 1):

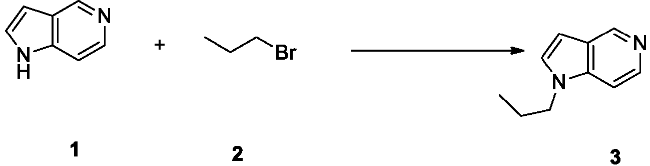

Compound 1 (106 mg, 0.9 mmol) was dissolved in N-N-dimethylformamide (5 mL), and sodium hydride (65 mg, 2.7 mmol) was added under ice bath. The mixture was replaced with N₂ three times and stirred for 30 min under ice bath, then compound 2 (98 mg, 0.72 mmol) was injected slowly under ice bath. The mixture was stirred at room temperature for 90 min, new spot was detected on a plate. The raction mixture was diluted with water (20 mL) and concentrated under reduced pressure to remove the organic solvent to afford crude product. The crude product was filtered with methanol, the filtrate was concentrated under reduced pressure to remove the organic solvent, the residue was purified by thin layer chromatography (methanol: dichloromethane =10%) to afford compound 3 (87 mg).

### Step 2):

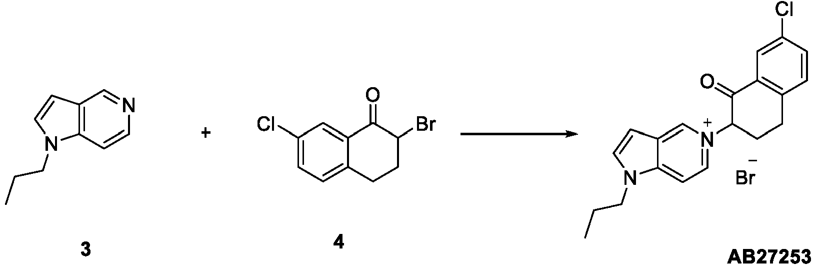

Compound 3 (87 mg, 0.54 mmol) was added to acetonitrile (10 mL), and compound 4 (140 mg, 0.54 mmol) was added. The mixture was stirred at 80 °C for overnight, new spot was detected on a plate. The raction mixture was concentrated under reduced pressure to remove the organic solvent to afford crude product. The crude product was purified by thin layer chromatography (methanol: dichloromethane = 10*%*) to afford compound AB27253 (80 mg).

MS-ESI and ¹H NMR of the compound AB27253 were as follows:
MS-ESI: calculated [M-Br⁻]⁺ 339.84, Found: 339.25 .
1H NMR (400 MHz, dmso) δ 9.37 (s, 1H), 8.56 (d, J = 6.9 Hz, 1H), 8.34 (d, J = 7.0 Hz, 1H), 8.07 (d, J = 3.1 Hz, 1H), 7.86 (d, J = 1.7 Hz, 1H), 7.79 - 7.73 (m, 1H), 7.55 (d, J = 8.3 Hz, 1H), 7.13 (d, J = 2.9 Hz, 1H), 6.19 (dd, J = 13.7, 4.0 Hz, 1H), 4.37 (t, J = 6.8 Hz, 2H), 3.37 (s, 1H), 3.25 (s, 1H), 3.03 (d, J = 8.8 Hz, 1H), 2.66 (d, J = 9.6 Hz, 1H), 1.83 (dd, J = 14.2, 7.1 Hz, 2H), 0.83 (t, J = 7.3 Hz, 3H).

### Example 24 Synthesis of compound AB27356

The structure of compound AB27356 was as follows:

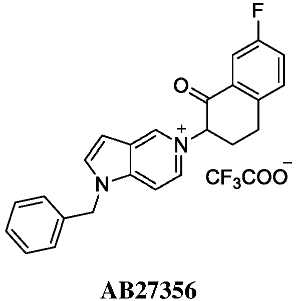

### Step 1):

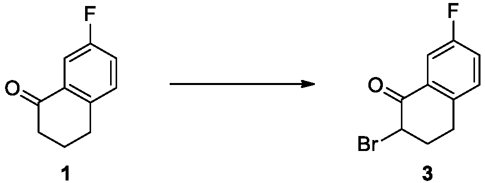

Compound 1 (1.6 g, 10 mmol) was dissolved in dichloromethane (50 mL), 3 drops of methanol were added dropwise, and bis(N,N-dimethylacetamide)hydrogendibromobromate (4.5 g, 10 mmol) was added. The mixture was stirred at 50 °C for overnight, new spot was detected on a plate. The raction mixture was rotary dried, the residue was purified by thin layer chromatography to afford compound 3 (2.2 g).

### Step 2):

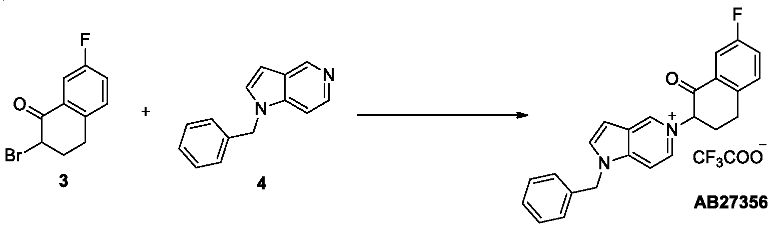

Compound 4 (209 mg, 1 mmol) was dissolved in acetonitrile (10 mL), and compound 3 (243 mg, 1 mmol) and triethylamine (303 mg, 3 mmol) were added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm × 30 × 10 um, 100 Å); mobile phase: 0.1*%* aqueous trifluoroacetic acid + acetonitrile; gradient elution: 1-20 min, 5-25*%* ACN; 20-50 min, 25-35*%* ACN) to afford compound AB27356.

MS-ESI and ¹H NMR of the compound AB27356 were as follows:
MS-ESI calculated [M-CF₃COO⁻]⁺: 371.43, Found: 371.00.
¹H NMR (400 MHz, DMSO) δ 9.37 (s, 1H), 8.55 (d, J = 7.0 Hz, 1H), 8.35 (d, J = 7.0 Hz, 1H), 8.16 (d, J = 2.9 Hz, 1H), 7.58 (dd, J = 17.4, 7.0 Hz, 3H), 7.38 - 7.24 (m, 5H), 7.15 (d, J = 2.7 Hz, 1H), 6.13 (dd, J = 13.7, 3.9 Hz, 1H), 5.66 (s, 2H), 3.28 (d, J = 12.2 Hz, 2H), 3.00 (d, J = 8.2 Hz, 1H), 2.65 (d, J = 9.5 Hz, 1H).

### Example 25 Synthesis of compound AB27470

The structure of compound AB27470 was as follows:

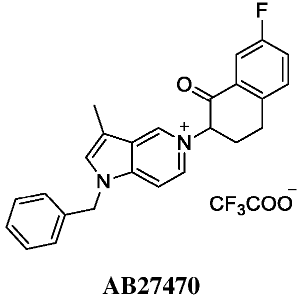

### Step 1):

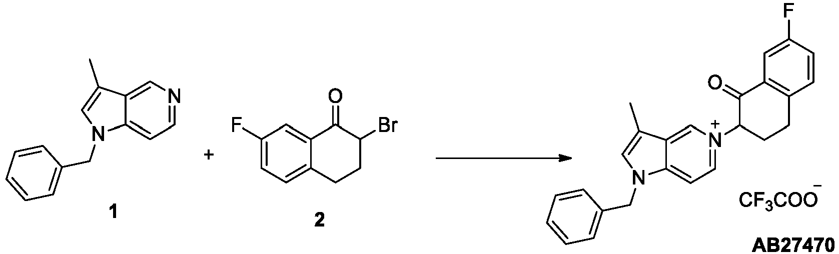

Compound 1 (100 mg, 0.448 mmol) was dissolved in acetonitrile (20 mL), and compound 2 (125 mg, 0.448 mmol) was added. The mixture was replaced with N₂ three times and stirred at 80 °C for overnight, new spot was detected on a plate. The raction mixture was concentrated under reduced pressure to remove the organic solvent, the residue was purified by preparation column chromatography (column: Sharpsil-U C18 (250 mm × 30 × 10 um, 100 Å); mobile phase: 0.1*%* aqueous trifluoroacetic acid + acetonitrile; gradient elution: 1-20 min, 5-25% ACN; 20-50 min, 25-35% ACN) to afford compound AB27470.

MS-ESI calculated [M-CF₃COO⁻]⁺: 385.45, Found: 385.30.

¹H NMR (400 MHz, DMSO-d6) δ 9.37 (s, 1H), 8.49 (d, *J =* 7.2 Hz, 1H), 8.24 (d, *J =* 7.1 Hz, 1H), 7.84 (s, 1H), 7.60 (d, *J =* 8.7 Hz, 3H), 7.55 (d, *J =* 5.6 Hz, 2H), 7.40 - 7.23 (m, 5H), 6.09 (dd, *J* = 13.8, 4.4 Hz, 1H), 5.56 (s, 2H), 3.27 - 3.21 (m, 4H), 3.06 - 2.96 (m, 2H), 2.67 - 2.58 (m, 1H), 2.33 (s, 3H).

### Example 26 Synthesis of compound AB27355

The structure of compound AB27355 was as follows:

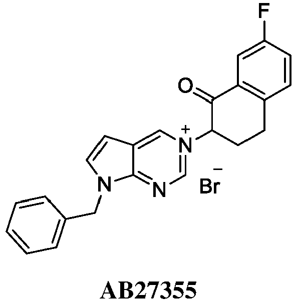

### Step 1):

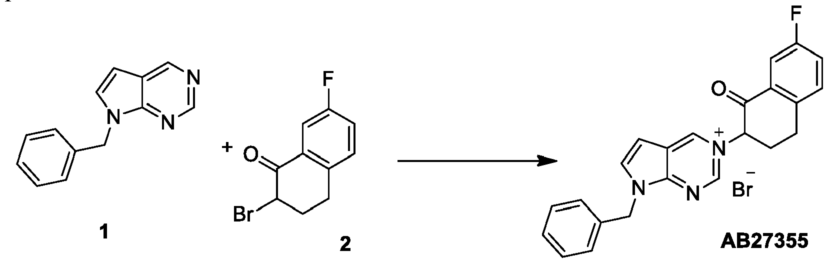

Compound 1 (120 mg, 0.57 mmol) was dissolved in acetonitrile (20 mL), and compound 2 (123 mg, 0.57 mmol) was added. The mixture was replaced with N₂ three times and stirred at 80 °C for overnight, new spot was detected on a plate. The raction mixture was filtered and concentrated under reduced pressure to remove the organic solvent, the residue was purified by thin layer chromatography (dichloromethane: methanol = 10:1) to afford compound AB27355 (20 mg, yield: 9.43*%*).

MS-ESI calculated [M-Br⁻]⁺: 372.15, Found: 372.05.

¹H NMR (399 MHz, dmso) δ 9.62 (s, 1H), 9.35 (s, 1H), 8.28 (d, J = 3.5 Hz, 1H), 7.66 - 7.54 (m, 3H), 7.38 - 7.28 (m, 5H), 7.19 (d, J = 3.5 Hz, 1H), 6.23 (s, 1H), 5.64 (s, 2H), 3.35 (s, 2H), 3.00 (s, 1H), 2.72 (s, 1H).

### Example 27 Synthesis of compound AB27140

The structure of compound AB27140 was as follows:

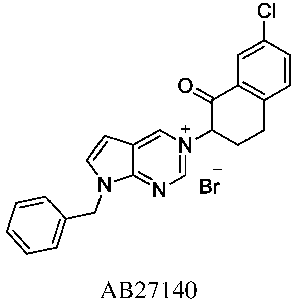

### Step 1):

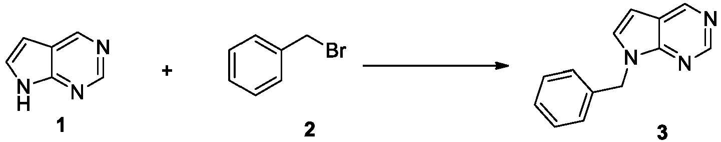

Compound 1 (40 mg, 0.13 mmol) was dissolved in N-N-dimethylformamide (10 mL), sodium hydride (16 mg, 0.40 mmol) was added under ice bath. The mixture was replaced with N₂ three times and stirred for 30 min under ice bath, then compound 2 (34 mg, 0.20 mmol) was injected slowly under ice bath. The mixture was stirred at room temperature for 60 min, new spot was detected on a plate. The raction mixture was quenched with aqueous ammonium chloride (20 mL) and then extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (20 mg).

### Step 2):

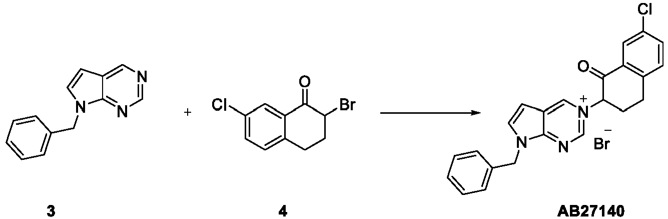

Compound 3 (20 mg, 0.09 mmol) was dissolved in acetonitrile (10 mL), and compound 4 (25 mg, 0.09 mmol) was added. The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromethane=0-30*%*) to afford compound AB27140.

MS-ESI and ¹H NMR of the compound AB27140 were as follows:
MS-ESI: calculated [M-Br⁻]⁺ 388.87, Found: 388.00.
¹H NMR (400 MHz, dmso-d6) δ 9.72 (s, 1H), 9.43 (s, 1H), 8.31 (s, 1H), 7.86 (s, 1H), 7.77 (d, *J =* 8.0 Hz, 1H), 7.55 (d, *J =* 7.8 Hz, 1H), 7.36 (s, 5H), 7.20 (s, 1H), 6.43 (d, *J =* 11.9 Hz, 1H), 5.65 (s, 2H), 3.29 - 3.21 (m, 2H), 3.00 (d, *J =* 9.8 Hz, 1H), 2.71 (d, *J* = 9.0 Hz, 1H).

### Example 28 AB27185

The structure of compound AB27185 was as follows:

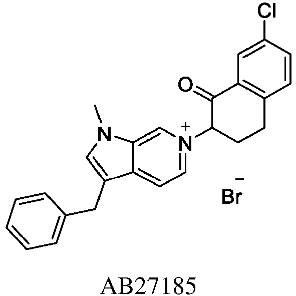

### Step 1):

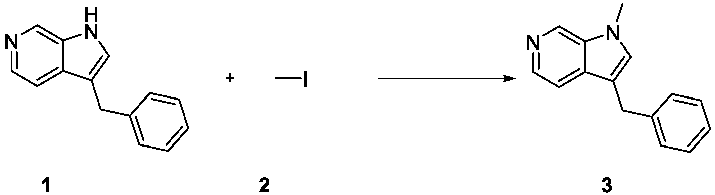

Compound 1 (100 mg, 0.48 mmol) was dissolved in drird N, N-dimethylformamide (5 mL), and sodium hydride (19.2 mg, 0.48 mmol) was added under ice bath. The mixture was replaced with N₂ three times and stirred for 30 min under ice bath, then compound 2 (81 mg, 1.2 mmol) was added. The mixture was replaced with N₂ three times and stirred for 30 min under ice bath, new spot was detected on a plate. The raction mixture was diluted with water and then extracted with ethyl acetate, the combined organic phase was washed with saturated saline, dried over anhydrous sodium sulfate, filtered and concentrated under reduced pressure to remove the organic solvent, the residue was purified by thin layer chromatography (methanol: dichloromethane = 0-10*%*) to afford compound 3 (56 mg).

### Step 2):

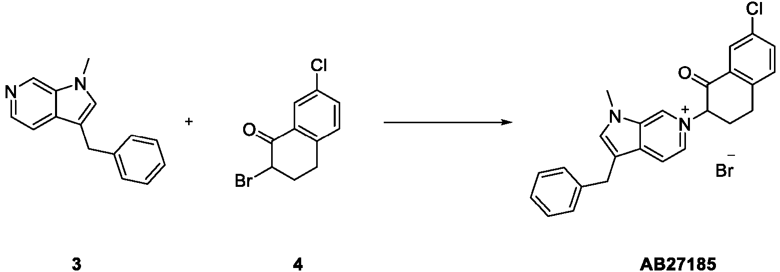

Compound 3 (56 mg, 0.25 mmol) was dissolved in acetonitrile (10 mL), and compound 4 (130 mg, 0.50 mmol) was added. The mixture was replaced with N₂ three times and stirred at 70 °C for overnight, new spot was detected on a plate. The raction mixture was filtered and concentrated under reduced pressure to remove the organic solvent, the residue was purified by thin layer chromatography (methanol: dichloromethane = 0-10*%*) to afford compound AB27185.

MS-ESI and ¹H NMR of the compound AB27185 were as follows:
MS-ESI calculated [M-Br⁻]⁺ 401.91, Found: 401.20.
¹H NMR (400 MHz, DMSO-d6) δ 9.49 (s, 1H), 8.33 (s, 1H), 8.23 (s, 1H), 8.13 (d, J = 4.5 Hz, 1H), 7.86 (s, 1H), 7.76 (s, 1H), 7.56 (d, J = 7.2 Hz, 1H), 7.30 (d, J = 10.5 Hz, 4H), 7.18 (s, 1H), 6.19 (d, J = 11.3 Hz, 1H), 4.17 (s, 2H), 3.98 (s, 3H), 3.30 - 3.26 (m, 2H), 3.05 (d, J = 8.7 Hz, 1H), 2.64 (s, 1H).

### Example 29 Synthesis of compound AB27186

The structure of compound AB27186 was as follows:

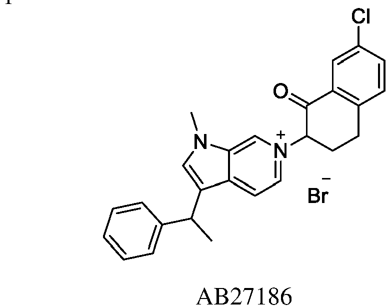

### Step 1):

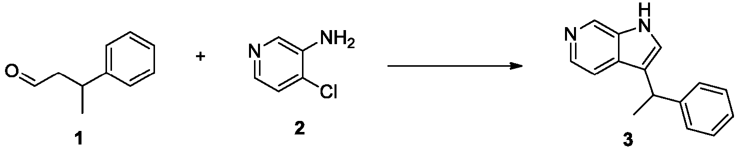

Compound 1 (300 mg, 2.02 mmol) was dissolved in N,N-dimethylacetamide (30 mL), and compound 2 (128 mg, 1 mmol), triethylenediamine (224 mg, 2 mmol), tris(dibenzylideneacetone)dipalladium (18.3 mg, 0.02 mmol ) and tri-tert-butylphosphine tetrafluoroborate ( 17.4 mg, 0.06 mmol) were added in sequence. The mixture was replaced with N₂ three times and stirred at room temperature for overnight, new spot was detected on a plate. The raction mixture was concentrated under reduced pressure to remove the organic solvent, the residue was purified by column chromatography (methanol: dichloromethane = 5*%*) to afford compound 3 (80 mg).

### Step 2):

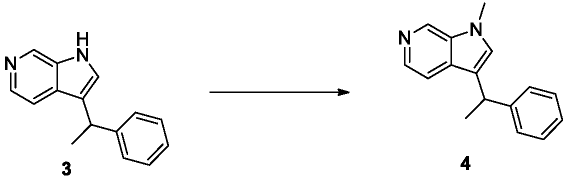

Compound 3 (80 mg, 0.36 mmol) was dissolved in N,N-dimethylformamide (30 mL), and sodium hydrogen (32 mg, 1.35 mmol) was added. The mixture was replaced with N₂ three times and stirred at room temperature for 1 h, and iodomethane (61.56 mg, 0.54 mmol) was added, new spot was detected on a plate. The raction mixture was concentrated under reduced pressure to remove the organic solvent, the residue was purified by thin layer chromatography (methanol: dichloromethane = 10*%*) to afford compound 4 (60 mg).

### Step 3):

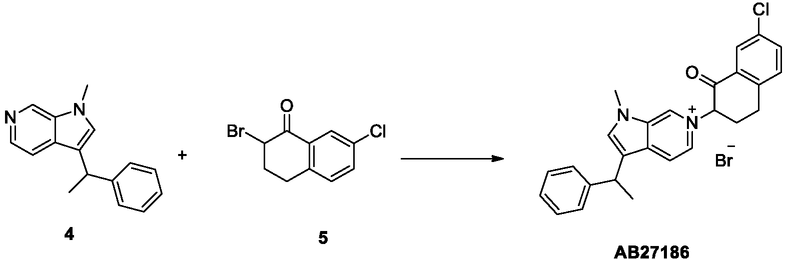

Compound 4 (60 mg, 0.25 mmol) was dissolved in acetonitrile (5 mL), and compound 5 (60 mg, 0.25 mmol) was added. The mixture was replaced with N₂ three times and stirred at room temperature for 48 h, new spot was detected on a plate. The raction mixture was concentrated under reduced pressure to remove the organic solvent, the residue was purified by thin layer chromatography (methanol: dichloromethane = 10*%*) to afford compound AB27186.

MS-ESI and ¹H NMR of the compound AB27186 were as follows:
MS-ESI calculated [M-Br⁻]⁺ 415.93, Found: 415.05.
¹H NMR (400 MHz, cd3od) δ 9.26 (s, 1H), 8.17 (s, 1H), 8.09 (s, 1H), 7.97 (s, 1H), 7.73 - 7.61 (m, 2H), 7.48 (d, J = 7.4 Hz, 1H), 7.25 (d, J = 40.2 Hz, 5H), 4.47 (s, 1H), 4.07 (s, 3H), 3.50 - 3.35 (m, 2H), 3.01 (s, 2H), 2.72 (s, 1H), 1.76 (d, J = 6.1 Hz, 3H).

### Example 30 Synthesis of compound AB27338

The structure of compound AB27338 was as follows:

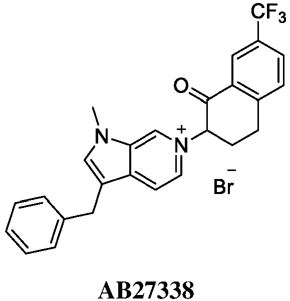

### Step 1):

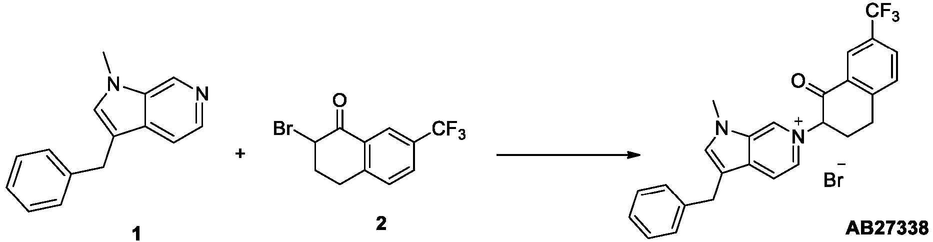

Compound 1 (67 mg, 0.3 mmol) was dissolved in acetonitrile (10 mL), and compound 2 (88 mg, 0.3 mmol) was added. The mixture was stirred at 80°C for overnight, new spot was detected on a plate. The raction mixture was concentrated under reduced pressure to remove the organic solvent, the residue was purified by column chromatography to afford compound AB27338.

MS-ESI calculated [M-Br⁻]⁺: 435.46, Found: 435.20.

¹H NMR (400 MHz, CD3OD) δ 9.29 (s, 1H), 8.29 (s, 1H), 8.20 (d, *J* = 6.7 Hz, 1H), 8.03 (s, 1H), 7.95 (d, *J =* 6.9 Hz, 2H), 7.70 (d, *J =* 8.1 Hz, 1H), 7.32 - 7.19 (m, 5H), 4.23 (s, 2H), 4.05 (s, 3H), 3.57 (t, *J =* 15.8 Hz, 1H), 3.43 (d, *J =* 17.9 Hz, 1H), 3.31 (s, 1H), 3.09 (dd, *J =* 14.3, 10.2 Hz, 1H), 2.79 (d, *J =* 11.8 Hz, 1H).

### Example 31 Synthesis of compound AB27339

The structure of compound AB27339 was as follows:

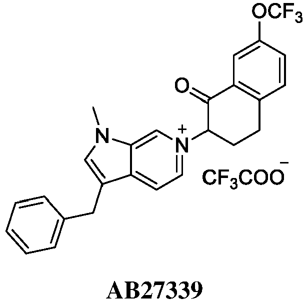

### Step 1):

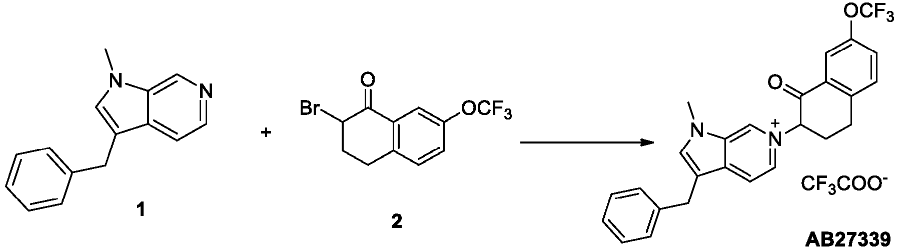

Compound 1 (67 mg, 0.3 mmol) was dissolved in acetonitrile (10 mL), and compound 2 (88 mg, 0.3 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm × 30 × 10 um, 100 Å); mobile phase: 0.1*%* aqueous trifluoroacetic acid + acetonitrile; gradient elution: 1-20 min, 5-25*%* ACN; 20-50 min, 25-35*%* ACN) to afford compound AB27339.

MS-ESI calculated [M-CF₃COO⁻]⁺ :451.46, Found: 451.10.

¹H NMR (400 MHz, CD3OD) δ 9.26 (s, 1H), 8.18 (d, *J =* 5.9 Hz, 1H), 8.00 (s, 1H), 7.92 (d, *J* = 6.8 Hz, 1H), 7.87 (s, 1H), 7.59 (s, 2H), 7.29 (d, *J =* 4.5 Hz, 4H), 7.21 (s, 1H), 5.97 (d, *J =* 14.2 Hz, 1H), 4.21 (s, 2H), 4.03 (s, 3H), 3.30 (s, 2H), 3.28 - 3.28 (m, 2H).

### Example 32 Synthesis of compound AB27121

The structure of compound AB27121 was as follows:

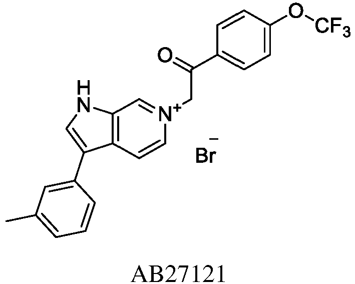

### Step 1):

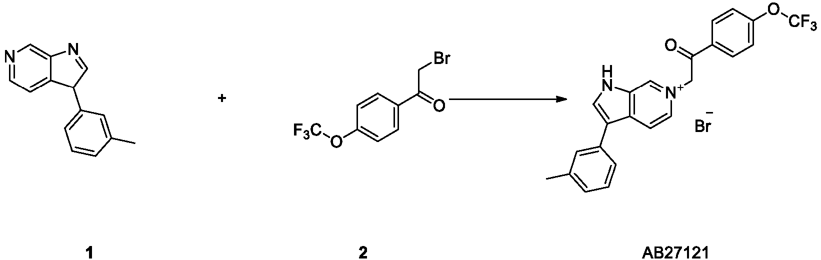

Compound 1 (200 mg, 0.96 mmol) was dissolved in acetonitrile (5 mL), and compound 2 (326 mg, 1.16 mmol) was added. The mixture was replaced with N₂ three times and stirred at room temperature for overnight, new spot was detected on a plate. The raction mixture was filtered, and washed with acetonitrile three times to afford compound AB27121.

MS-ESI calculated [M-Br]⁺: 411.40, Found: 411.10.

1H NMR (400 MHz,DMSO-d6) δ 13.46 (s, 1H), 9.27 (s, 1H), 8.77 (s, 1H), 8.49 (d, J = 6.2 Hz, 1H), 8.35 (d, J = 6.1 Hz, 1H), 8.22 (d, J = 8.1 Hz, 2H), 7.71 - 7.56 (m, 4H), 7.40 (t, J = 7.1 Hz, 1H), 7.20 (d, J = 6.7 Hz, 1H), 6.45 (s, 2H), 2.40 (s, 3H).

### Example 33 Synthesis of compound AB27341

The structure of compound AB27341 was as follows:

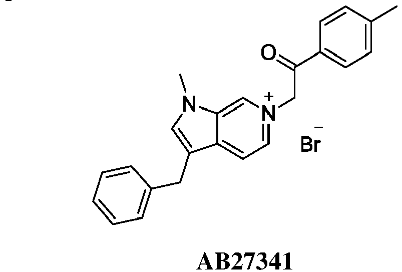

### Step 1):

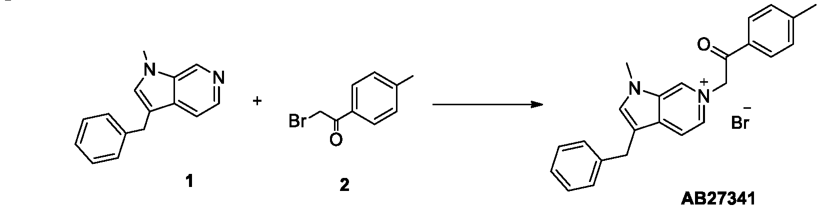

Compound 1 (67 mg, 0.3 mmol) was dissolved in acetonitrile (10 mL), and compound 2 (64 mg, 0.3 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane = 0-10*%*) to afford compound AB27341.

MS-ESI calculated [M-Br⁻] ⁺ 355.45, Found: 355.25.

¹H NMR (400 MHz, dmso) δ 9.42 (s, 1H), 8.29 - 8.20 (m, 2H), 8.14 (d, J = 6.5 Hz, 1H), 7.97 (d, J = 7.7 Hz, 2H), 7.46 (d, J = 7.6 Hz, 2H), 7.35 - 7.27 (m, 4H), 7.20 (d, J = 6.7 Hz, 1H), 6.34 (s, 2H), 4.17 (s, 2H), 3.97 (s, 3H), 2.42 (s, 3H).

### Example 34 Synthesis of compound AB27343

The structure of compound AB27343 was as follows:

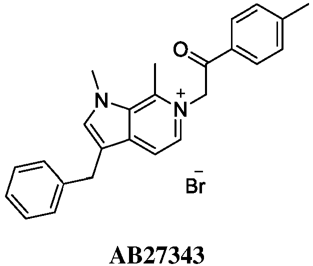

### Step 1:

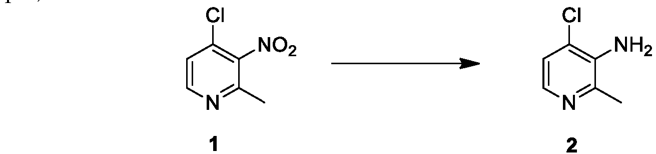

Compound 1 (1.0 g, 5.8 mmol) was dissolved in ethanol (20 mL) and water (10 mL), and iron (650 mg, 11.5 mmol) and ammonium chloride (1.56 g, 29.1 mmol) were added. The mixture was stirred at 80 °C for overnight, new spot was detected on a plate. The raction mixture was filtered and concentrated under reduced pressure to remove the organic solvent to afford compound 2 (400 mg).

### Step 2):

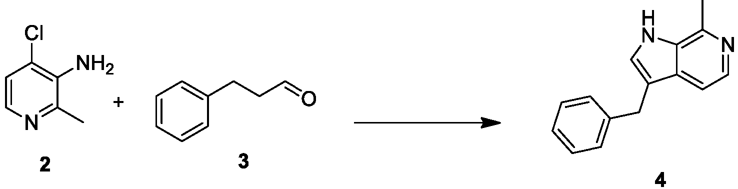

Compound 2 (400 mg, 2.81 mmol) was dissolved in N, N-dimethylacetamide (20 mL), and compound 3 (1.13 g, 8.45 mmol), tris (dibenzylideneacetone) dipalladium (128 mg, 0.14 mmol), tert butylphosphotetrafluoroborate (81.7 mg, 0.28 mmol), and triethylenediamine (948 mg, 8.45 mmol) were added. The mixture was stirred at 120 °C for overnight in a sealed tube, new spot was detected on a plate. The raction mixture was concentrated under reduced pressure to remove the organic solvent, the residue was purified by thin layer chromatography (methanol: dichloromethane = 0-10%) to afford compound 4 (140 mg).

### Step 3):

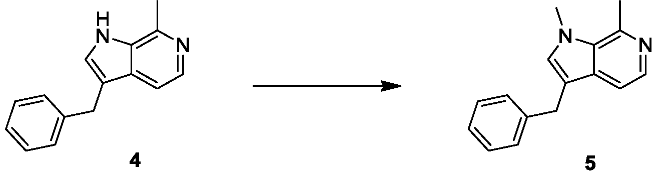

Compound 4 (140 mg, 0.62 mmol) was dissolved in N, N-dimethylacetamide (10 mL), and sodium hydride (75 mg, 3.13 mmol) was added at 0 °C, then iodomethane (98 mg, 0.69 mmol) was added 0.5 h later. The mixture was stirred at room temperature for overnight, new spot was detected on a plate. The raction mixture was concentrated under reduced pressure to remove the organic solvent, the residue was purified by thin layer chromatography (methanol: dichloromethane = 0-10*%*) to afford compound 5 (70 mg).

### Step 4):

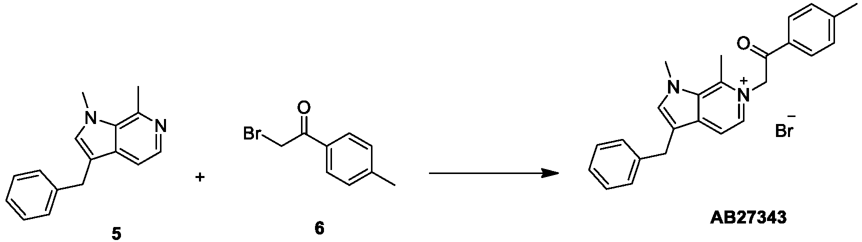

Compound 5 (70 mg, 0.29 mmol) was dissolved in acetonitrile (10 mL), and compound 6 (94 mg, 0.44 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane=0-10%) to afford compound AB27343.

MS-ESI and ¹H NMR of the compound AB27343 were as follows:
MS-ESI calculated [M-Br⁻]⁺: 369.48, Found: 369.15.
¹H NMR (400 MHz, dmso) δ 8.19 (d, J = 6.8 Hz, 1H), 8.10 (s, 1H), 7.97 (t, J = 7.6 Hz, 3H), 7.45 (d, J = 8.0 Hz, 2H), 7.32 - 7.27 (m, 5H), 7.18 (d, J = 5.9 Hz, 1H), 6.46 (s, 2H), 4.21 (s, 3H), 4.12 (s, 2H), 2.94 (s, 3H), 2.42 (s, 2H).

### Example 35 Synthesis of compound AB27475

The structure of compound AB27475 was as follows:

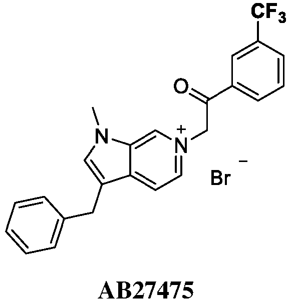

### Step 1):

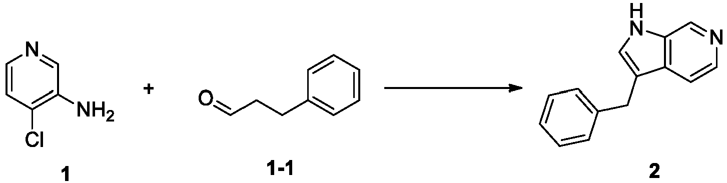

Compound 1 (1 g, 10 mmol) was dissolved in N,N-dimethylacetamide (10 mL), and compound 1-1 (4 g, 30 mmol), tris(dibenzylideneacetone)dipalladium (457 mg, 0.5 mmol), tri-tert-butylphosphonium tetrafluoroborate (290 mg, 1 mmol) were added. The mixture was stirred at 120 °C for overnight in a sealed tube, new spot was detected on a plate. The raction mixture was concentrated under reduced pressure to remove the organic solvent, the residue was purified by thin layer chromatography (methanol: dichloromethane = 0-10*%*) to afford compound 2 (100 mg).

### Step 2):

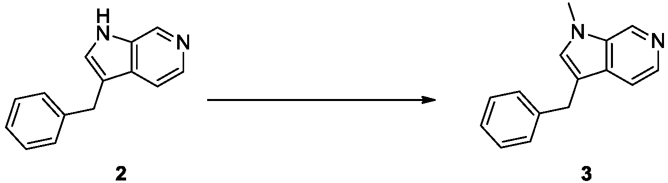

Compound 2 (100 mg, 0.48 mmol) was dissolved in N,N-dimethylformamide (20 mL) . The mixture was replaced with N₂ three times, and sodium hydrogen (60 mg, 2.5 mmol) was added at 0 °C. The mixture was stirred for 0.5 h under ice bath, and iodomethane (80 mg, 0.6 mmol) was added. The mixture was stirred at room temperature for 2 h, new spot was detected on a plate. The raction mixture was concentrated under reduced pressure to remove the organic solvent, the residue was purified by thin layer chromatography (dichloromethane:methanol=10:1) to afford compound 3 (40 mg).

### Step 3):

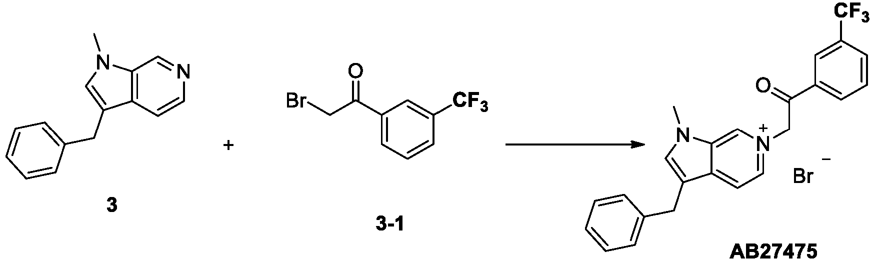

Compound 3 (40 mg, 0.2 mmol) was dissolved in acetonitrile (10 mL), and compound 3-1 (48 mg, 0.2 mmol) was added. The mixture was stirred at room temperature for overnight, new spot was detected on a plate. The raction mixture was concentrated under reduced pressure to remove the organic solvent, the residue was purified by thin layer chromatography (methanol: dichloromethane = 0-10*%*) to afford compound AB27475.

MS-ESI and ¹H NMR of the compound AB27475 were as follows:
MS-ESI calculated [M-Br⁻]⁺: 409.42, Found: 409.00.
¹H NMR (400 MHz, DMSO-d6) δ 9.36 (s, 1H), 8.34 (d, *J =* 6.5 Hz, 2H), 8.26 - 8.20 (m, 2H), 8.14 (dd, *J* = 7.1, 2.9 Hz, 2H), 7.89 (t, *J =* 8.2 Hz, 1H), 7.33 - 7.26 (m, 4H), 7.21 - 7.16 (m, 1H), 6.42 (s, 2H), 4.16 (s, 2H), 3.96 (s, 3H).

### Example 36 Synthesis of compound AB27476

The structure of compound AB27476 was as follows:

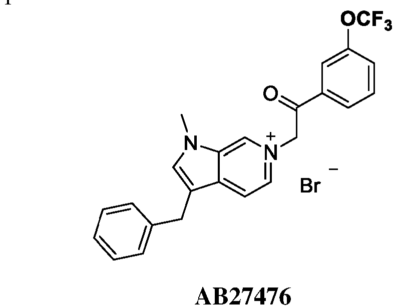

### Step 1):

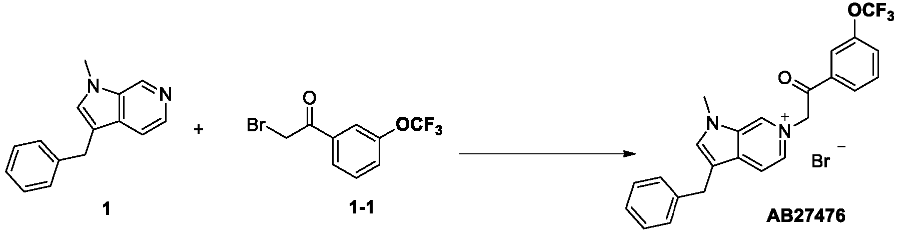

Compound 1 (150 mg, 0.67 mmol) was dissolved in acetonitrile (10 mL), and compound 1-1 (190 mg, 0.67 mmol) was added. The mixture was stirred at room temperature for overnight, new spot was detected on a plate. The raction mixture was concentrated under reduced pressure to remove the organic solvent, the residue was purified by column chromatography (methanol: dichloromethane = 0-10%) to afford compound AB27476.

MS-ESI calculated [M-Br⁻]⁺: 425.42, Found: 425.00.

¹H NMR (400 MHz, DMSO-d6) δ 9.37 (s, 1H), 8.27 - 8.20 (m, 2H), 8.12 (dd, *J* = 12.2, 6.0 Hz, 2H), 7.97 (s, 1H), 7.80 (d, *J* = 4.7 Hz, 2H), 7.33 - 7.26 (m, 4H), 7.18 (t, *J* = 7.1 Hz, 1H), 6.38 (s, 2H), 4.16 (s, 2H), 3.96 (s, 3H).

### Example 37 Synthesis of compound AB27482

The structure of compound AB27482 was as follows:

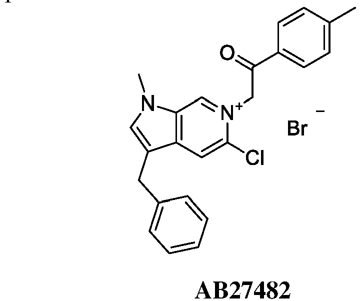

### Step 1):

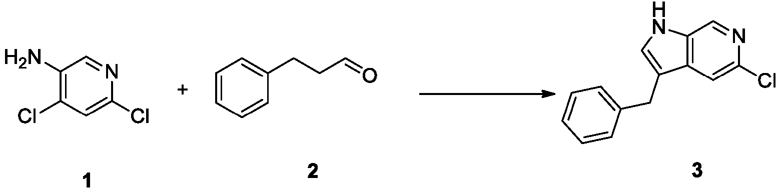

Compound 1 (815 mg, 5 mmol) was dissolved in N, N-dimethylacetamide (30 mL), and compound 2 (2 g, 15 mmol), tris(dibenzylideneacetone)dipalladium (229 mg, 0.25 mmol), tri-tert-butylphosphonium tetrafluoroborate (145 mg, 0.5 mmol) and triethylenediamine (1.68 g, 15 mmol) were added. The mixture was stirred at 120 °C for overnight in a sealed tube, new spot was detected on a plate. The raction mixture was concentrated under reduced pressure to remove the organic solvent, the residue was purified by thin layer chromatography (methanol: dichloromethane = 0-10%) to afford compound 3 (110 mg).

MS-ESI calculated [M+1⁻]⁺: 243.70, Found: 243.

### Step 2):

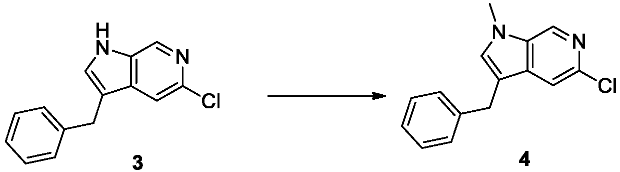

Compound 3 (110 mg, 0.46 mmol) was dissolved in N,N-dimethylacetamide (30 mL), sodium hydride (56 mg, 2.34 mmol) was added at 0 °C, then iodomethane (73.3 mg, 0.516 mmol) was added 0.5 h later. The mixture was stirred at room temperature for overnight, new spot was detected on a plate. The raction mixture was concentrated under reduced pressure to remove the organic solvent, the residue was purified by thin layer chromatography (methanol: dichloromethane = 0-10%) to afford compound 4 (90 mg).

MS-ESI calculated [M+1⁻]⁺: 257.73, Found: 257.

### Step 3):

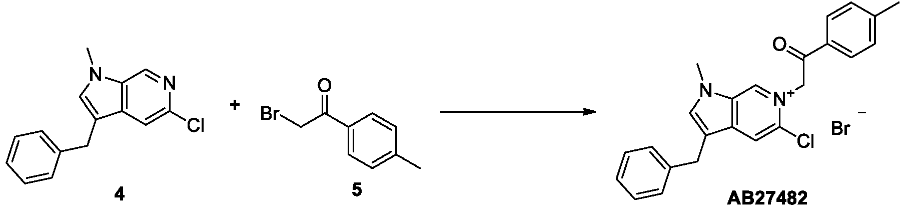

Compound 4 (90 mg, 0.35 mmol) was dissolved in acetonitrile (30 mL), and compound 5 (74.8 mg, 0.35 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane=0-10%) to afford compound AB27482.

MS-ESI and ¹H NMR of the compound AB27482 were as follows:
MS-ESI calculated [M-Br⁻]⁺: 389.90, Found: 389.25.
¹H NMR (400 MHz, DMSO-d6) δ 9.56 (s, 1H), 8.46 (s, 1H), 8.29 (s, 1H), 8.00 (d, *J =* 8.3 Hz, 2H), 7.45 (d, *J =* 8.2 Hz, 2H), 7.34-7.27 (m, 4H), 7.19 (t, *J =* 7.1 Hz, 1H), 6.47 (s, 2H), 4.15 (s, 2H), 3.95 (s, 3H), 2.41 (s, 3H).

### Example 38 Synthesis of compound AB27204

The structure of compound AB27204 was as follows:

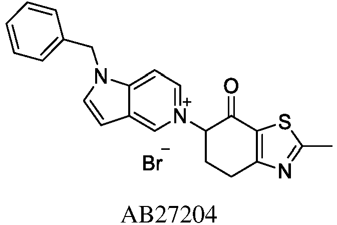

### Step 1):

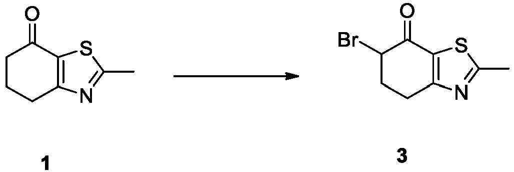

Compound 1 (84 mg, 0.5 mmol) was dissolved in dichloromethane (10 mL), 2 drops of methanol were added, and then bis(N,N-dimethylacetamide)hydrogendibromobromate (207 mg, 0.5 mmol) was added. The mixture was stirred at 80 °C for overnight, new spot was detected on a plate. The raction mixture was rotary dried, the residue was purified by thin layer chromatography to afford compound 3 (70 mg).

### Step 2):

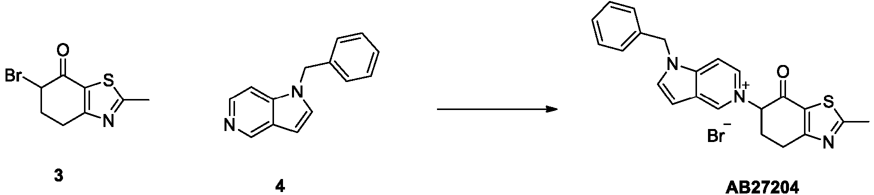

Compound 3 (70 mg, 0.28 mmol) was dissolved in acetonitrile (20 mL), and compound 4 (60 mg, 0.28 mmol) was added. The mixture was stirred at 80 °C for overnight, new spot was detected on a plate. The raction mixture was rotary dried, the residue was purified by thin layer chromatography to afford compound AB27204.

MS-ESI calculated [M-Br⁻] ⁺ 374.48, Found: 374.25.

1H NMR (400 MHz, dmso-d6) δ 9.42 (s, 1H), 8.59 (d, J = 6.0 Hz, 1H), 8.36 (d, J = 5.9 Hz, 1H), 8.17 (s, 1H), 7.35 (s, 5H), 7.15 (s, 1H), 6.19 (d, J = 12.7 Hz, 1H), 5.66 (s, 2H), 3.30 - 3.24 (m, 2H), 3.02 (s, 1H), 2.77 (s, 3H), 2.72 (s, 1H).

### Example 39 Synthesis of compound AB27254

The structure of compound AB27254 was as follows:

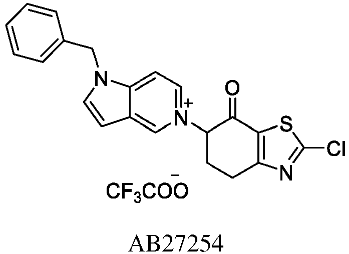

### Step 1):

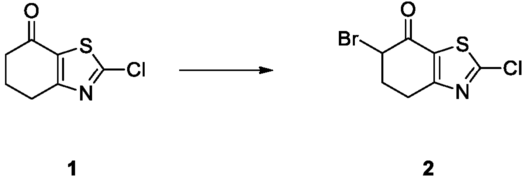

Compound 1 (94 mg, 0.5 mmol) was dissolved in dichloromethane (10 mL), two drops of methanol were added, and then bis(N,N-dimethylacetamide)hydrogendibromobromate (210 mg, 0.5 mmol) was added. The mixture was stirred at 80 °C for overnight, new spot was detected on a plate. The raction mixture was rotary dried, the residue was purified by thin layer chromatography to afford compound 2 (112 mg).

### Step 2):

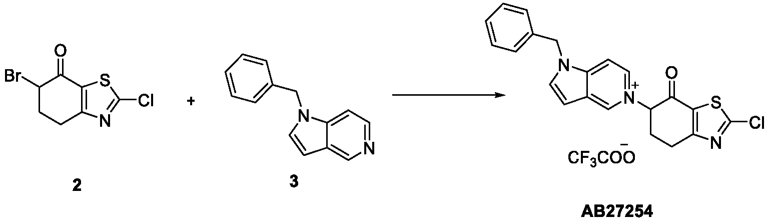

Compound 2 (112 mg, 0.41 mmol) was dissolved in acetonitrile (5 mL), and compound 3 (91.6 mg, 0.41 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27254 (12 mg).

MS-ESI and ¹H NMR of the compound AB27254 were as follows:
MS-ESI calculated [M-CF₃COO⁻]⁺ 394.90, Found: 394.08.
¹H NMR (400 MHz, CD3OD) δ 10.03 (s, 1H), 9.16 (d, *J =* 6.1 Hz, 1H), 8.26 (d, *J =* 6.5 Hz, 1H), 8.04 (s, 1H), 7.32 (d, *J =* 18.4 Hz, 6H), 5.70 (s, 2H), 4.86 (s, 1H), 3.32 (s, 1H), 3.22 (s, 1H), 2.74 (s, 1H), 2.59 (s, 1H).

### Example 40 Synthesis of compound AB27102

The structure of compound AB27102 was as follows:

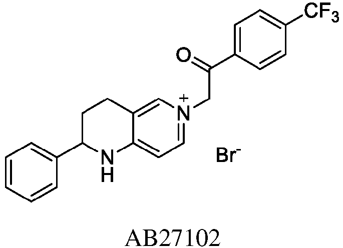

### Step 1):

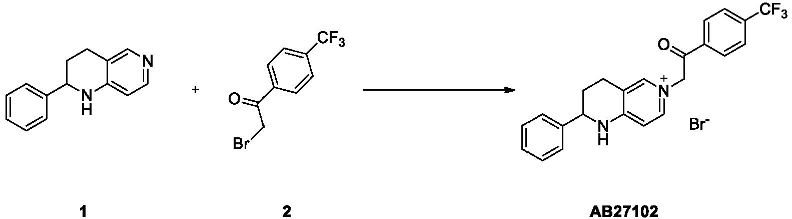

Compound 1 (100 mg, 0.48 mmol) was dissolved in acetonitrile (10 mL), compound 2 (153 mg, 0.57 mmol) and potassium carbonate (66 mg, 0.48 mmol) were added. The mixture was stirred at 80 °C for 16 h, new spot was detected on a plate. The raction mixture was concentrated under reduced pressure to remove the organic solvent, the residue was purified by column chromatography (methanol: dichloromethane= 0-10%) to afford compound AB27102.

MS-ESI calculated [M-Br⁻]⁺ 397.41, Found: 397.20.

¹H NMR (400 MHz, dmso-d6) δ 9.93 (s, 1H), 8.82 (d, *J =* 7.3 Hz, 2H), 8.64 (d, *J =* 20.5 Hz, 4H), 8.06 - 7.93 (m, 5H), 7.54 (d, *J =* 5.8 Hz, 1H), 6.57 (s, 2H), 5.39 (s, 1H), 3.40 (s, 1H), 3.20 (d, *J =* 13.9 Hz, 1H), 2.71 (s, 1H), 2.49 (s, 1H).

### Example 41 Synthesis of compound AB27103

The structure of compound AB27103 was as follows:

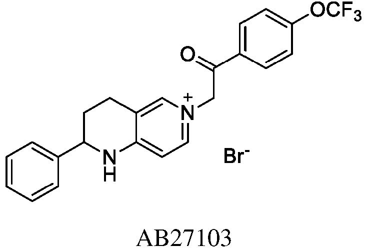

### Step 1):

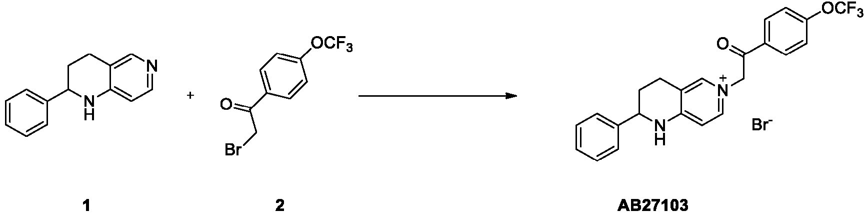

Compound 1 (100 mg, 0.48 mmol) was dissolved in acetonitrile (10 mL), compound 2 (162 mg, 0.57 mmol) and potassium carbonate (66 mg, 0.48 mmol) were added. The mixture was stirred at 80 °C for 16 h, new spot was detected on a plate. The raction mixture was concentrated under reduced pressure to remove the organic solvent, the residue was purified by column chromatography (methanol: dichloromethane= 0-10%) to afford compound AB27103.

MS-ESI calculated [M-Br⁻]⁺ 413.41, Found: 413.10.

¹H NMR (400 MHz, dmso-d6) δ 9.35 (s, 1H), 8.15 (d, *J =* 7.3 Hz, 2H), 8.08 - 7.97 (m, 2H), 7.62 (d, *J* = 6.9 Hz, 2H), 7.44 - 7.31 (m, 5H), 6.94 (d, *J* = 6.0 Hz, 1H), 5.94 (s, 2H), 4.77 (s, 1H), 2.79 (s, 1H), 2.57 (d, *J =* 15.6 Hz, 1H), 2.09 (s, 1H), 1.89 (s, 1H).

### Example 42 Synthesis of compound AB27146

The structure of compound AB27146 was as follows:

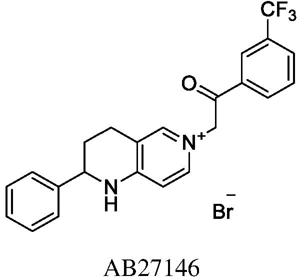

### Step 1):

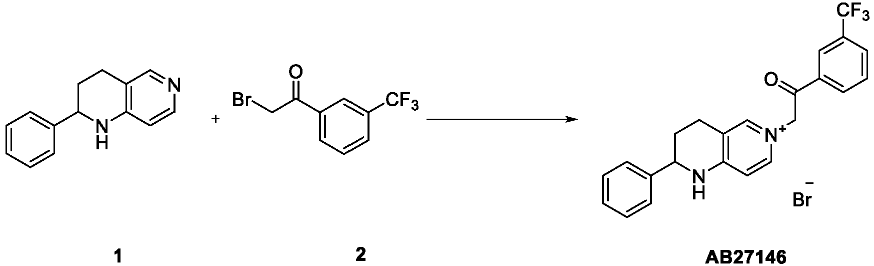

Compound 1 (210 mg, 1 mmol) was dissolved in acetonitrile (5 mL), compound 2 (283 mg, 1 mmol) was added. The mixture was replaced with N₂ three times and stirred at room temperature for overnight, new spot was detected on a plate. The raction mixture was filtered and concentrated under reduced pressure to remove the organic solvent, the residue was purified by column chromatography to afford compound AB27146.

MS-ESI calculated [M-Br⁻]⁺ 397.15, Found: 397.30.

¹H NMR (400 MHz, DMSO) δ 9.38 (s, 1H), 8.29 (s, 2H), 8.16 - 7.99 (m, 3H), 7.88 (d, J = 6.8 Hz, 1H), 7.45 - 7.31 (m, 5H), 6.95 (d, J = 6.6 Hz, 1H), 6.00 (s, 2H), 4.78 (s, 1H), 2.79 (s, 1H), 2.60 (s, 1H), 2.10 (s, 1H), 1.89 (s, 1H).

### Example 43 Synthesis of compound AB27147

The structure of compound AB27147 was as follows:

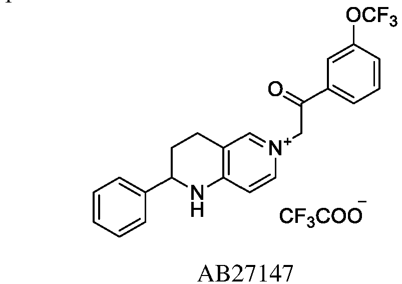

### Step 1):

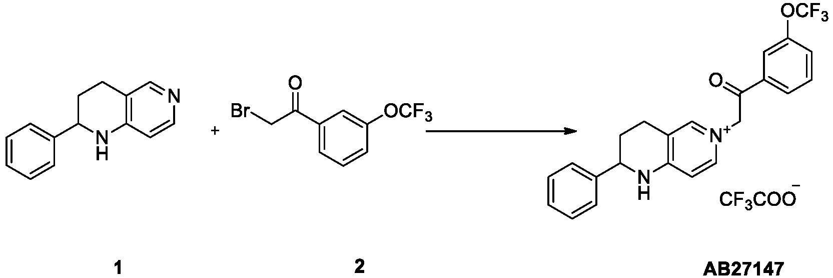

Compound 1 (210 mg, 1 mmol) was dissolved in acetonitrile (5 mL), and compound 2 (283 mg, 1 mmol) was added. The mixture was replaced with N₂ three times and stirred at room temperature for overnight, new spot was detected on a plate. The raction mixture was filtered and concentrated under reduced pressure to remove the organic solvent, the residue was purified by preparation chromatography (column: Sharpsil-U C18 (250 mm x 30 x 10 um, 100 A); mobile phase: 0.1% aqueous trifluoroacetic acid + acetonitrile; gradient elution: 1-20 min, 5-25% ACN; 20-50 min, 25-35% ACN) to afford compound AB27147.

MS-ESI calculated [M-CF₃COO⁻] ⁺ 413.15, Found: 413.05.

1H NMR (400 MHz, DMSO) δ 9.26 (s, 1H), 8.04 (s, 2H), 7.97 (s, 1H), 7.93 (s, 1H), 7.78 (s, 2H), 7.40 (s, 2H), 7.36 (s, 3H), 6.89 (s, 1H), 5.91 (s, 2H), 4.77 (s, 1H), 2.78 (s, 1H), 2.59 (d, J = 14.8 Hz, 1H), 2.10 (s, 1H), 1.88 (s, 1H).

### Example 44 Synthesis of compound AB27105

The structure of compound AB27105 was as follows:

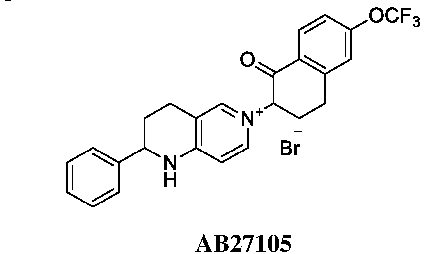

### Step 1):

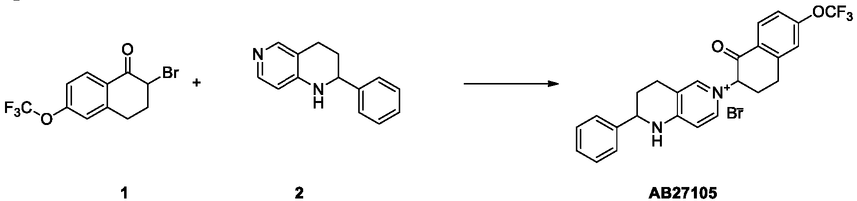

Compound 1 (240 mg, 0.78 mmol) was dissolved in acetonitrile (10 mL), and compound 2 (150 mg, 0.78 mmol) was added. The mixture was replaced with N₂ three times and stirred at room temperature for overnight, new spot was detected on a plate. The raction mixture was concentrated under reduced pressure to remove the organic solvent, the residue was purified by column chromatography (methanol: dichloromethane = 0-30%) to afford compound AB27105.

MS-ESI calculated [M-Br⁻]+ 439.45, Found: 439.20.

1H NMR (400 MHz, DMSO-d6) δ 9.49 (s, 1H), 8.20 (s, 1H), 8.12 (d, J = 6.7 Hz, 1H), 8.04 (d, J = 8.5 Hz, 1H), 7.50 (s, 1H), 7.45 - 7.37 (m, 3H), 7.34 (d, J = 6.2 Hz, 3H), 6.98 (d, J = 6.9 Hz, 1H), 5.78 (d, J = 13.3 Hz, 1H), 4.77 (s, 1H), 3.27 (s, 1H), 2.80 (d, J = 10.3 Hz, 2H), 2.56 (s, 1H), 2.08 (s, 1H), 1.88 (s, 1H).

### Example 45 Synthesis of compound AB27132

The structure of compound AB27132 was as follows:

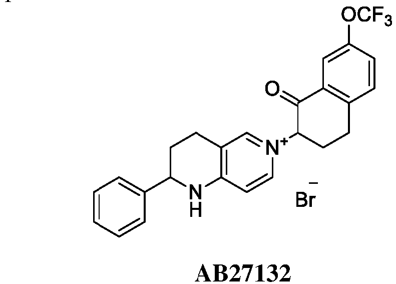

### Step 1):

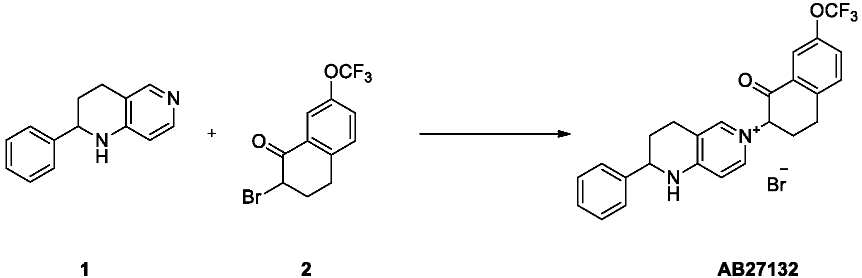

Compound 1 (60 mg, 0.27 mmol) and compound 2 (100 mg, 0.32 mmol) were dissolved in acetonitrile (10 mL), and potassium carbonate (45 mg, 0.32 mmol) was added. The mixture was stirred at room temperature for 3 h, new spot was detected on a plate. The raction mixture was concentrated under reduced pressure to remove the organic solvent, the residue was purified by thin layer chromatography (methanol: dichloromethane = 10:1) to afford compound AB27132.

MS-ESI calculated [M-Br⁻]⁺ 439.46, Found: 439.25.

¹H NMR (400 MHz, dmso-d6) δ 9.32 (s, 1H), 8.17 (s, 1H), 8.08 (d, *J =* 6.9 Hz, 1H), 7.68 (dd, *J =* 39.8, 17.7 Hz, 3H), 7.43 - 7.31 (m, 5H), 6.91 (d, *J =* 7.0 Hz, 1H), 5.69 (d, *J =* 14.4 Hz, 1H), 4.77 (s, 1H), 3.27 - 3.18 (m, 3H), 2.80 (s, 2H), 2.58 (s, 1H), 2.08 (s, 1H), 1.89 (s, 1H).

### Example 46 Synthesis of compound AB27134

The structure of compound AB27134 was as follows:

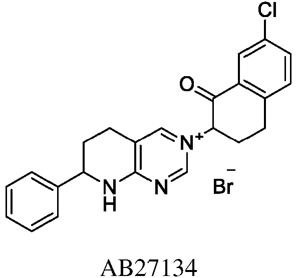

### Step 1):

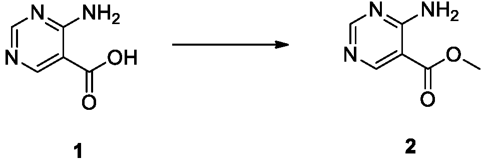

Compound 1 (6 g, 43.2 mmol) was dissolved in methanol (80 mL), concentrated sulfuric acid (8 mL) was slowly added dropwise under ice water. The mixture was reflux stirred at 85 °C for 12 h, new spot was detected on a plate. The raction mixture was concentrated under reduced pressure to remove the organic solvent, water (30 mL) was added slowly unde ice water bath, sodium bicarbonate was added to adjust the base, and then the mixture was extracted with ethyl acetate (30 mL x 3), the combined organic phase was washed with saturated saline (50 mL), dried over anhydrous sodium sulfate, filtered and concentrated under reduced pressure to remove the organic solvent to afford compound 2 (3.2 g).

### Step 2):

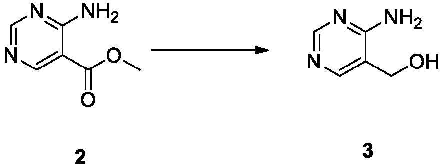

Compound 2 (3.2 g, 20.9 mmol) was dissolved in tetrahydrofuran (50 mL), and 2.5 mol/L lithium tetrahydroaluminate solution (26 mL) was slowly added dropwise under ice water. The mixture was stirred at room temperature for 1h, new spot was detected on a plate, water (26 mL) was added slowly unde ice water bath, and 15% aqueous sodium hydroxide solution (26 mL) was added dropwise, then water (78 mL) was added, the mixture was extracted with ethyl acetate (30 mL x 3), the combined organic phase was washed with saturated saline (50 mL), dried over anhydrous sodium sulfate, filtered and concentrated under reduced pressure to remove the organic solvent, the residue was purified by column chromatography (methanol: dichloromethane = 0-30%) to afford compound 3 (1.21 g).

### Step 3):

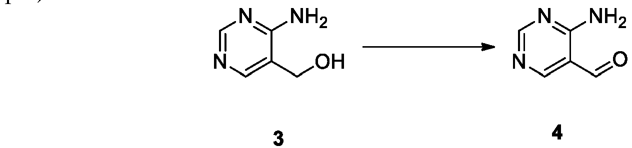

Compound 3 (1.21 g, 9.68 mmol) was dissolved in dichloromethane (20 mL), and Dess-Martin periodinane (4.93 g, 11.6 mmol) was added. The mixture was stirred at room temperature for 1h, new spot was detected on a plate. The raction mixture was filtered with diatomite, the filtrate was concentrated under reduced pressure to remove the organic solvent, the residue was purified by column chromatography (methanol: dichloromethane = 0-30%) to afford compound 4 (900 mg).

### Step 4):

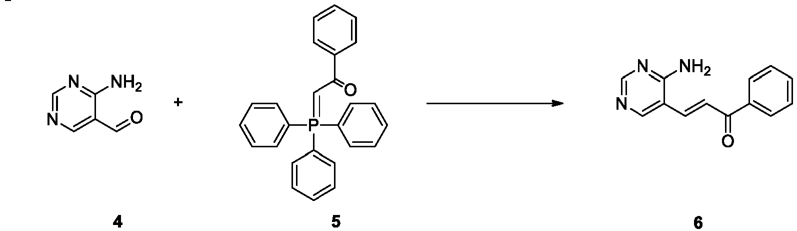

Compound 4 (800 mg, 6.5 mmol) was dissolved in dichloromethane (50 mL), compound 5 (3 g, 7.8 mmol) was added, and then triethylamine (2 g, 19.5 mmol) was added. The mixture was replaced with N₂ three times and stirred at 40°C for overnight, new spot was detected on a plate. The raction mixture was filetered to afford compound 6 (800 mg).

### Step 5):

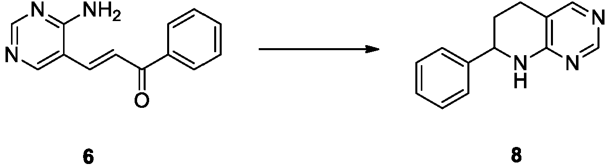

Compound 6 (700 mg, 3.11 mmol) was dissolved in ice acetic acid (20 mL), and palladium carbon (100 mg) was added. The mixture was replaced with N₂ three times and stirred at 60°C for 5h, new spot was detected on a plate. The raction mixture was filtered with diatomite, the filtrate was concentrated under reduced pressure to remove the organic solvent, the residue was purified by column chromatography (methanol: dichloromethane = 0-30%) to afford compound 8 (440 mg).

### Step 6):

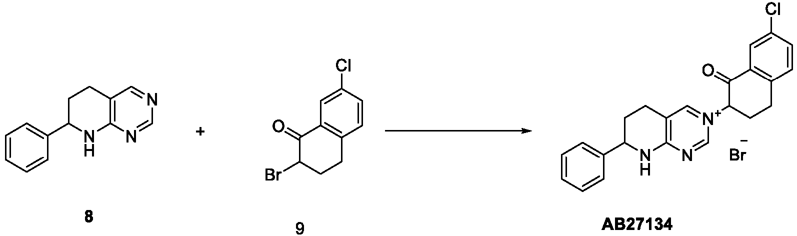

Compound 8 (60 mg, 0.28 mmol) was dissolved in acetonitrile (10 mL), compound 9 (94 mg, 0.36 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (methanol: dichloromethane = 10%) to afford compound AB27134.

MS-ESI and ¹H NMR of the compound AB27134 were as follows:
MS-ESI calculated [M-Br⁻] ⁺390.89, Found: 390.05.
¹H NMR (400 MHz, dmso-d6) δ 10.31 (s, 1H), 8.74 (s, 1H), 8.30 (s, 1H), 7.86 (s, 1H), 7.75 (d, J = 8.0 Hz, 1H), 7.52 (d, J = 7.9 Hz, 1H), 7.36 (d, J = 30.6 Hz, 5H), 5.75 (d, J = 12.0 Hz, 1H), 4.91 (s, 1H), 3.23 (s, 2H), 2.76 (s, 2H), 2.51 (s, 2H), 2.09 (s, 1H), 1.91 (s, 1H).

### Example 47 Synthesis of compound AB27136

The structure of compound AB27136 was as follows:

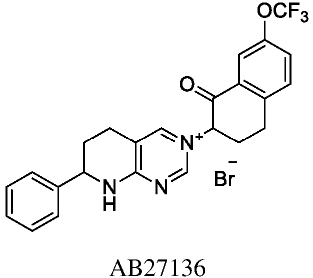

### Step 1):

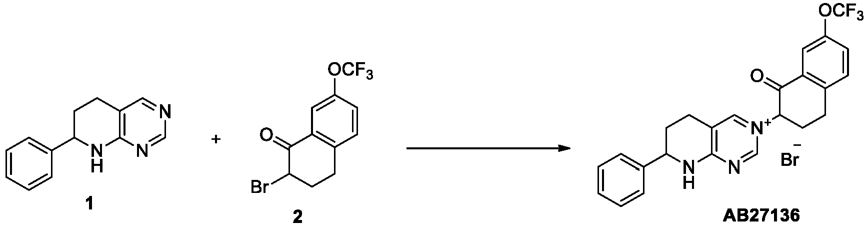

Compound 1 (60 mg, 0.28 mmol) was dissolved in acetonitrile (10 mL), and compound 2 (88 mg, 0.28 mmol) were added. The mixture was replaced with N₂ three times and stirred at room temperature for overnight, new spot was detected on a plate. The raction mixture was concentrated under reduced pressure to remove the organic solvent, the residue was purified by thin layer chromatography (methanol: dichloromethane = 10%) to afford compound AB27136.

MS-ESI calculated [M-Br⁻]⁺440.16, Found: 440.10.

1H NMR (400 MHz, dmso-d6) δ 10.31 (s, 1H), 8.74 (s, 1H), 8.31 (s, 1H), 7.77 (s, 1H), 7.70 (s, 1H), 7.63 (d, J = 8.0 Hz, 1H), 7.36 (d, J = 30.6 Hz, 5H), 5.79 (d, J = 15.5 Hz, 1H), 4.91 (s, 1H), 2.79 (s, 3H), 2.51 (s, 3H), 2.10 (s, 1H), 1.90 (s, 1H).

### Example 48 Synthesis of compound AB27137

The structure of compound AB27137 was as follows:

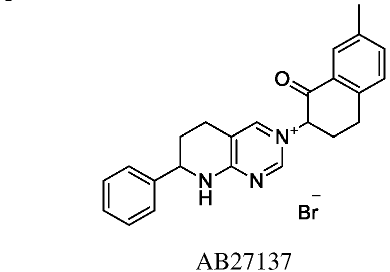

### Step 1):

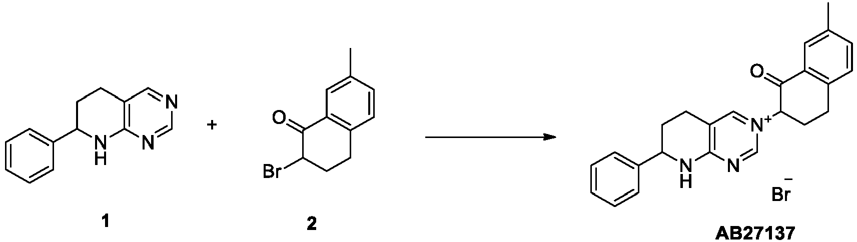

Compound 1 (50 mg, 0.24 mmol) was dissolved in acetonitrile (10 mL), and compound 2 (62 mg, 0.26 mmol) were added. The mixture was replaced with N₂ three times and stirred at room temperature for overnight, new spot was detected on a plate. The raction mixture was concentrated under reduced pressure to remove the organic solvent, the residue was purified by thin layer chromatography (methanol: dichloromethane = 10%) to afford compound AB27137.

MS-ESI calculated [M-Br⁻]⁺ 370.47, Found: 370.15.

¹H NMR (400 MHz, dmso) δ 10.30 (s, 1H), 8.78 (s, 1H), 8.34 (s, 1H), 7.73 (s, 1H), 7.49 (d, J = 7.3 Hz, 1H), 7.40 (s, 2H), 7.32 (s, 4H), 5.77 (d, J = 14.2 Hz, 1H), 4.91 (s, 1H), 3.19 (dd, J = 32.4, 14.1 Hz, 3H), 2.75 (s, 2H), 2.52 (s, 1H), 2.34 (s, 3H), 2.09 (s, 1H), 1.91 (s, 1H).

### Example 49 Synthesis of compound AB27198

The structure of compound AB27198 was as follows:

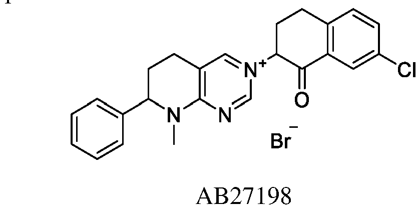

### Step 1:

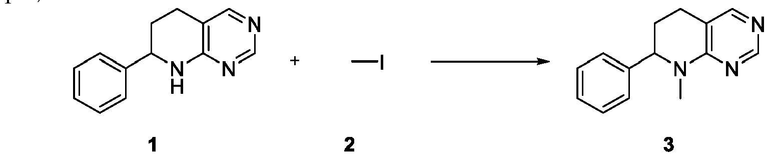

Compound 1 (50 mg, 0.23 mmol) was dissolved in N-N-dimethylformamide (10 mL), sodium hydride (30 mg, 0.71 mmol) was added under ice bath. The mixture was replaced with N₂ three times and stirred for 30 min under ice bath, then compound 2 (40 mg, 0.28 mmol) was injected slowly under ice bath. The mixture was stirred at room temperature for 60 min, new spot was detected on a plate. The raction mixture was quenched with aqueous ammonium chloride and then extracted with ethyl acetate, the combined organic phase was washed with saturated saline, dried over anhydrous sodium sulfate, filtered and concentrated under reduced pressure to remove the organic solvent. The residue was purified by thin layer chromatography (methanol: dichloromethane = 0-10%) to afford compound 2 (50 mg).

### Step 2):

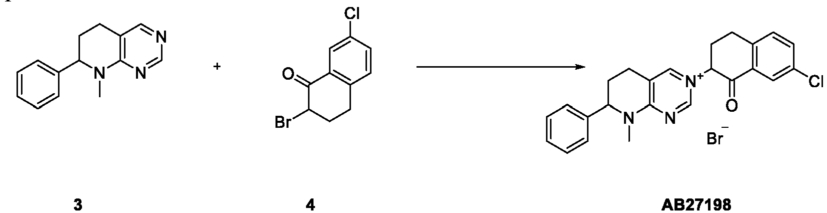

Compound 3 (50 mg, 0.22 mmol) was dissolved in acetonitrile (10 mL), and compound 4 (57 mg, 0.22 mmol) was added. The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromethane = 0-30%) to afford compound AB27198.

MS-ESI and ¹H NMR of the compound AB27198 were as follows:
MS-ESI calculated [M-Br⁻]⁺ 404.92, Found: 404.25.
¹H NMR (400 MHz, dmso-d6) δ 8.98 (s, 1H), 8.38 (s, 1H), 7.84 (s, 1H), 7.74 (d, *J =* 7.3 Hz, 1H), 7.52 (d, *J =* 7.2 Hz, 1H), 7.37 (d, *J =* 20.4 Hz, 3H), 7.20 (d, *J =* 5.6 Hz, 2H), 6.00 (s, 1H), 5.12 (s, 1H), 3.25 (d, *J =* 30.4 Hz, 5H), 2.74 (d, *J =* 47.6 Hz, 2H), 2.55 (s, 1H), 2.31 (s, 1H), 2.11 (d, *J* = 39.4 Hz, 2H).

### Example 50 Synthesis of compound AB27222

The structure of compound AB27222 was as follows:

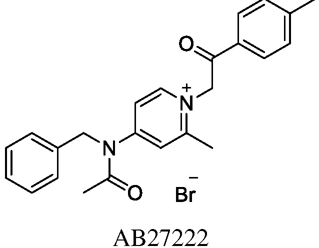

### Step 1):

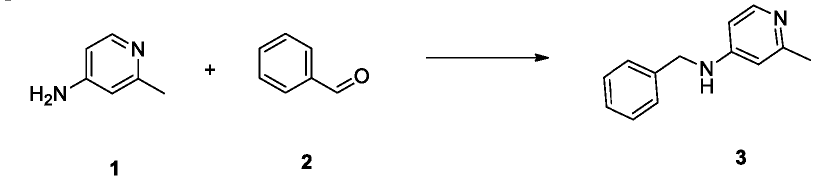

Compound 1 (1 g, 9.26 mmol) was dissolved in ethanol (10 mL), and compound 2 (2.9 g, 27.78 mmol) was added. The mixture was replaced with N₂ three times and stirred at 90 °C for overnight, new spot was detected on a plate. The raction temperature was lowered to 0 °C, sodium borohydride (422 mg, 11.11 mmol) was added slowly, and the mixture was naturally heated to room temperature and stirred for 1 h, new spot was detected on a plate. The raction mixture was diluted with water (30 ml) and then extracted with ethyl acetate (20 mL x 3), the combined organic phase was washed with saturated saline (20 mL), dried over anhydrous sodium sulfate, filtered and concentrated under reduced pressure to remove the organic solvent. The residue was purified by column chromatography (methanol: dichloromethane = 10:1) to afford compound 3 (1.5 g).

### Step 2):

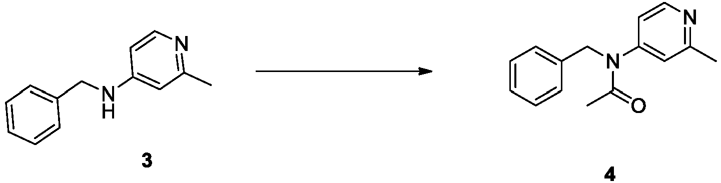

Compound 3 (200 mg, 1.01 mmol) was dissolved in dichloromethane (10 mL), and acetyl chloride (159 mg, 2.02 mmol) and triethylamine (306 mg, 303 mmol) were added under ice bath. The mixture was naturally heated to room temperature and stirred for 2 h, new spot was detected on a plate. The raction mixture was rotary dried, the residue was purified by column chromatography (methanol: dichloromethane =0-15%) to afford compound 4 (90 mg).

### Step 3):

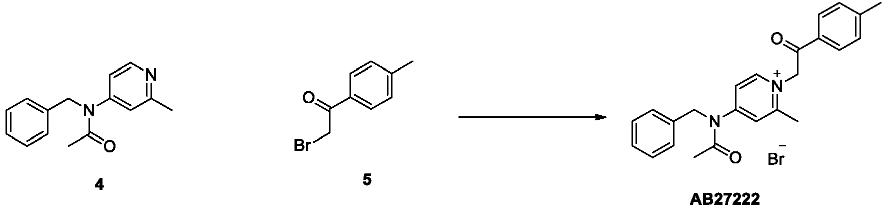

Compound 4 (90 mg, 0.38 mmol) was dissolved in acetonitrile (5 mL), and compound 5 (80 mg, 0.38 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27222.

MS-ESI and ¹H NMR of the compound AB27222 were as follows:
MS-ESI calculated [M-Br⁻]⁺ 373.19, Found: 373.25.
¹H NMR (400 MHz, dmso-d6) δ 8.66 (d, J = 6.2 Hz, 1H), 8.11 (s, 1H), 8.01 (s, 1H), 7.93 (d, J = 6.7 Hz, 2H), 7.44 (d, J = 7.0 Hz, 2H), 7.34 (d, J = 6.3 Hz, 2H), 7.26 (s, 3H), 6.28 (s, 2H), 5.28 (s, 2H), 2.53 (s, 3H), 2.40 (s, 3H), 2.35 (s, 3H).

### Example 51 Synthesis of compound AB27232

The structure of compound AB27232 was as follows:

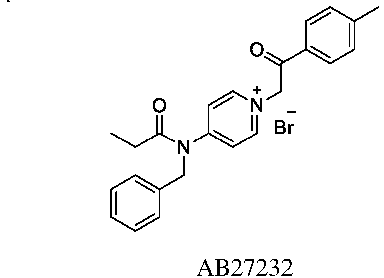

### Step 1):

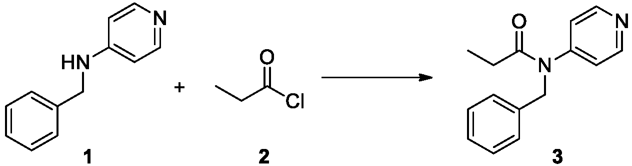

Compound 1 (184 mg, 1 mmol) was dissolved in dichloromethane (5 mL), and triethylamine (303 mg, 3 mmol) was added. The mixture was replaced with N₂ three times and stirred at 0°C for 30 min, and compound 2 (185 mg, 2 mmol) was added at 0°C, then the mixture was reacted at room temperature for 30 min, new spot was detected on a plate. The raction mixture was concentrated under reduced pressure to remove the organic solvent, the residue was purified by thin layer chromatography (methanol: dichloromethane =0-10%) to afford compound 3 (100 mg).

### Step 2):

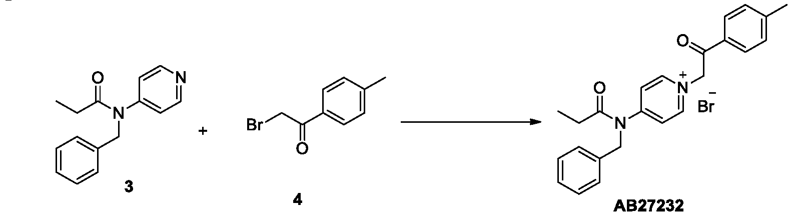

Compound 3 (100 mg, 0.42 mmol) was dissolved in acetonitrile (5 mL), and compound 4 (134 mg, 0.63 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27232.

MS-ESI and ¹H NMR of the compound AB27232 were as follows:
MS-ESI calculated [M-Br⁻]⁺ 373.47, Found: 373.30.
¹H NMR (400 MHz, DMSO) δ 8.70 (d, *J* = 6.6 Hz, 2H), 8.14 (d, *J* = 6.9 Hz, 2H), 7.90 (d, *J =* 8.0 Hz, 2H), 7.43 (d, *J* = 7.9 Hz, 2H), 7.36 (t, *J* = 7.4 Hz, 2H), 7.29 (d, *J* = 7.1 Hz, 1H), 7.25 (d, *J =* 7.6 Hz, 2H), 6.20 (s, 2H), 5.31 (s, 2H), 2.68 (dd, *J* = 12.1, 4.9 Hz, 1H), 2.40 (s, 4H), 1.06 (s, 2H).

### Example 52 Synthesis of compound AB27234

The structure of compound AB27234 was as follows:

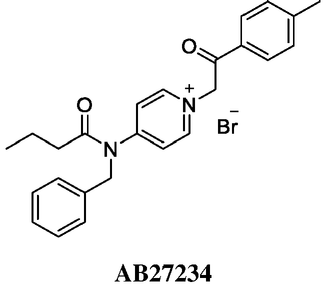

### Step 1):

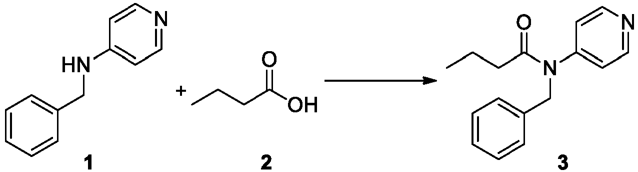

Compound 1 (370 mg, 2 mmol) was dissolved in N,N-dimethylformamide (20 mL), and compound 2 (264 mg, 3 mmol) was added, then triethylamine (505 mg, 5 mmol) was added, then HATU (1.14 g, 3 mmol) was added. The mixture was replaced with N₂ three times and stirred at room temperature for 30 min, new spot was detected on a plate. The raction mixture was concentrated under reduced pressure to remove the organic solvent, the residue was purified by thin layer chromatography (methanol: dichloromethane =0-10%) to afford compound 3 (200 mg).
MS-ESI calculated [M+1]⁺ 255, Found: 255

### Step 2):

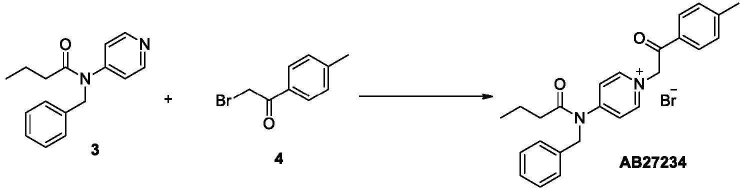

Compound 3 (200 mg, 0.78 mmol) was dissolved in acetonitrile (10 mL), and compound 4 (165 mg, 0.78 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane =0-10%) to afford compound AB27234 (260 mg).

MS-ESI and ¹H NMR of the compound AB27234 were as follows:
MS-ESI calculated [M-Br⁻]⁺ 387.49, Found: 387.
¹H NMR (400 MHz, DMSO) δ 8.71 (s, 2H), 8.13 (s, 2H), 7.91 (d, *J =* 6.6 Hz, 2H), 7.43 (d, *J* = 6.4 Hz, 4H), 7.36 (d, *J =* 6.0 Hz, 1H), 7.29 (s, 3H), 7.25 (d, *J =* 5.9 Hz, 1H), 6.21 (s, 1H), 5.32 (s, 1H), 2.66 (s, 1H), 2.41 (s, 2H), 1.61 (s, 1H), 0.87 (s, 2H).

### Example 53 Synthesis of compound AB27235

The structure of compound AB27235 was as follows:

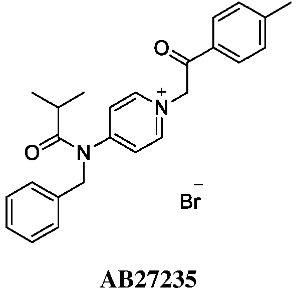

### Step 1):

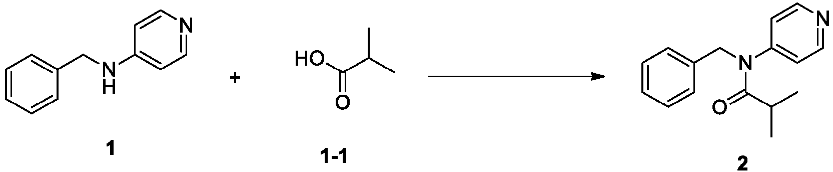

Compound 1 (200 mg, 1.09 mmol) was dissolved in N, N-dimethylformamide (10 mL), compound 1-1 (115 mg, 1.30 mmol), 2-(7-azobenzotriazolyl)-N,N,N',N'-tetramethyluronium hexafluorophosphate (620 mg, 1.63 mmol) and N,N-diisopropylethylamine ( 421 mg, 3.26 mmol) were added. The mixture was replaced with N₂ three times and stirred at room temperature for overnight, new spot was detected on a plate. The raction mixture was concentrated under reduced pressure to remove the organic solvent, the residue was purified by column chromatography to afford compound 2 (70 mg).

### Step 2):

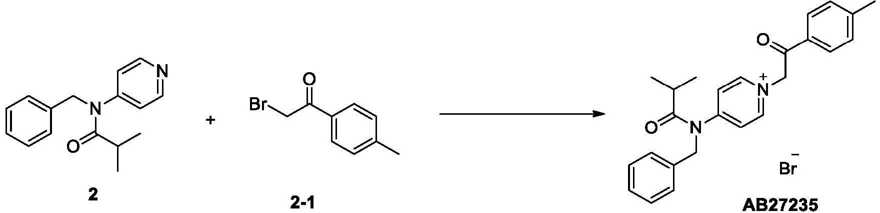

Compound 2 (70 mg, 0.28 mmol) was dissolved in acetonitrile (10 mL), and compound 2-1 (59 mg, 0.28 mmol) was added. The mixture was stirred at 80 °C for overnight, new spot was detected on a plate. The raction mixture was concentrated under reduced pressure to remove the organic solvent, the residue was purified by column chromatography to afford compound AB27235 (79 mg).

MS-ESI and ¹H NMR of the compound AB27235 were as follows:
MS-ESI calculated [M-Br⁻]⁺ 387.49, Found: 387.
¹H NMR (400 MHz, DMSO) δ 8.74 (d, *J* = 6.5 Hz, 2H), 8.10 (d, *J* = 6.4 Hz, 2H), 7.90 (d, *J =* 7.7 Hz, 2H), 7.43 (d, *J* = 7.7 Hz, 1H), 7.36 (t, *J* = 7.1 Hz, 1H), 7.29 (d, *J* = 6.9 Hz, 0H), 7.24 (d, *J =* 7.2 Hz, 1H), 6.24 (s, 1H), 5.31 (s, 1H), 3.07 - 2.99 (m, 1H), 2.40 (s, 3H), 1.11 (d, *J* = 6.3 Hz, 3H).

### Example 54 Synthesis of compound AB27236

The structure of compound AB27236 was as follows:

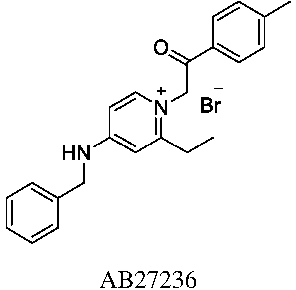

### Step 1):

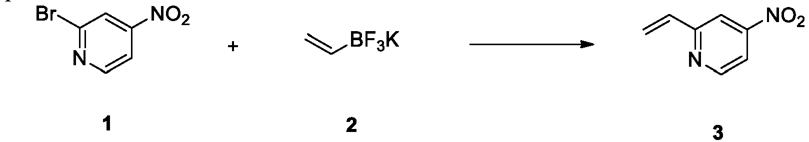

Compound 1 (404 mg, 2 mmol) was dissolved in dried N, N-dimethylformamide (50 mL), compound 2 (804 mg, 6 mmol), dimethylferrocene palladium dichloride (75.1 mg, 0.1 mmol) and sodium carbonate (636 mg, 6 mmol) were added. The mixture was replaced with N₂ three times and stirred at room temperature for overnight, new spot was detected on a plate. The raction mixture was diluted with water (10 ml) and extracted with ethyl acetate (10 mL x 3), the combined organic phases were washed with saturated saline (100 mL), dried over anhydrous sodium sulfate, filtered and concentrated under reduced pressure to remove the organic solvent. The residue was purified by thin layer chromatography (ethyl acetate: petroleum ether = 0-50%) to afford compound 3 (120 mg).

### Step 2):

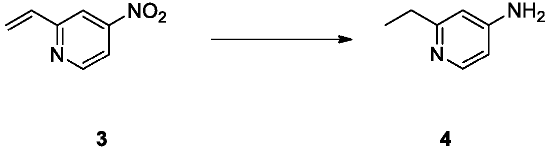

Compound 3 (120 mg, 0.79 mmol) was dissolved in tetrahydrofuran (20 mL), palladium carbon (24 mg, 20%) was added. The mixture was replaced with N₂ three times and stirred at room temperature for overnight, new spot was detected on a plate. The raction mixture was filtered and concentrated under reduced pressure to remove the organic solvent to afford compound 4 (90 mg).

### Step 3):

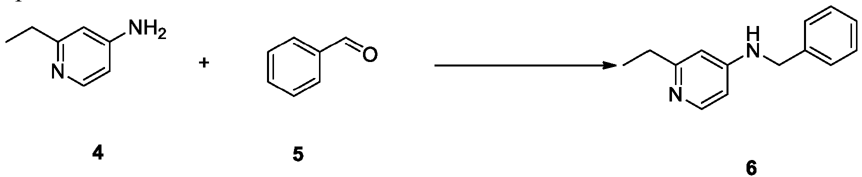

Compound 4 (90 mg, 0.73 mmol) was dissolved in ethanol (20 mL), and compound 5 (234 mg, 62.21 mmol) was added. The mixture was replaced with N₂ three times and stirred at 90 °C for overnight, new spot was detected on a plate, and sodium borohydride (56 mg 1.47 mmoL) was added, then the mixture was stirred at room temperature for overnight. The raction mixture was filtered and concentrated under reduced pressure to remove the organic solvent, the residue was purified by thin layer chromatography (dichloromethane: methanol =0-10%) to afford compound 6 (26 mg).

### Step 4):

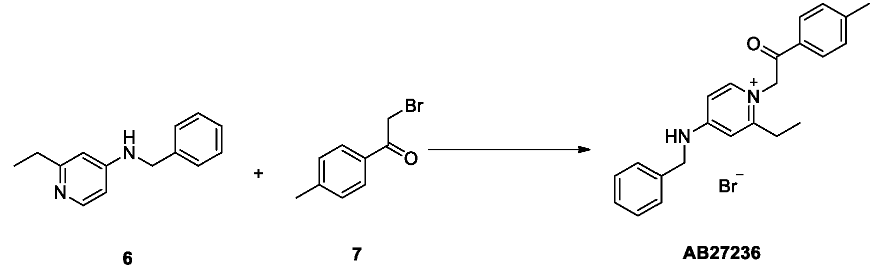

Compound 6 (106 mg, 0.5 mmol) was dissolved in acetonitrile (5 mL), and compound 7 (107 mg, 0.5 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27236.

MS-ESI and ¹H NMR of the compound AB27236 were as follows:
MS-ESI calculated [M-Br⁻]⁺ 345.47, Found: 345.47.
¹H NMR (400 MHz, CD3OD) δ 7.99 (d, *J =* 5.9 Hz, 1H), 7.90 (d, *J =* 5.7 Hz, 0H), 7.40 (s, 7H), 7.33 (s, 1H), 6.96 - 6.75 (m, 2H), 4.60 (d, *J* = 7.8 Hz, 2H), 2.65 (d, *J* = 6.0 Hz, 2H), 2.45 (s, 3H), 1.22 (d, *J =* 32.4 Hz, 3H).

### Example 55 Synthesis of compound AB27237

The structure of compound AB27237 was as follows:

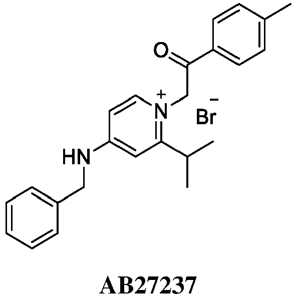

### Step 1:

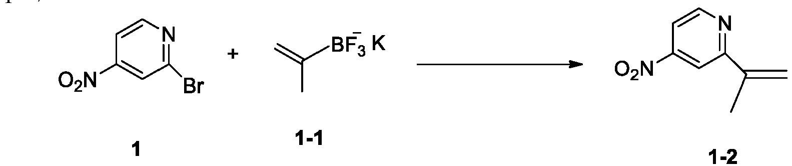

Compound 1 (2.03 g, 10 mmol) was dissolved in toluene (25 mL) and water (5 mL), compound 1-1 (1.62 g, 11 mmol), palladium acetate (11 mg, 0.5 mmol), cesium carbonate (9.78 g, 30 mmol), and n-butylbis(1-adamantyl)phosphine (358 mg, 1 mmol) were added. The mixture was replaced with N₂ three times and stirred at 98°C for overnight, new spot was detected on a plate. The raction mixture was concentrated under reduced pressure to remove the organic solvent, the residue was purified by thin layer chromatography (petroleum ether: ethyl acetate = 0-1 %) to afford compound 1-2 (970 mg).

### Step 2):

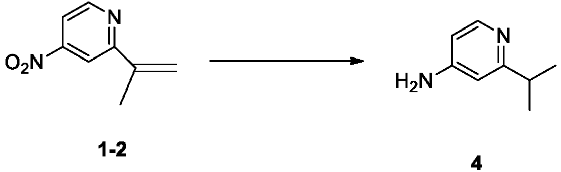

Compound 1-2 (970 mg, 5.91 mmol) was dissolved in methanol (20 mL), palladium carbon (240 mg, 20%) was added. The mixture was replaced with H₂ three times and stirred at room temperature for overnight, new spot was detected on a plate. The raction mixture was filtered and concentrated under reduced pressure to remove the organic solvent to afford compound 4 (800 mg).

### Step 3):

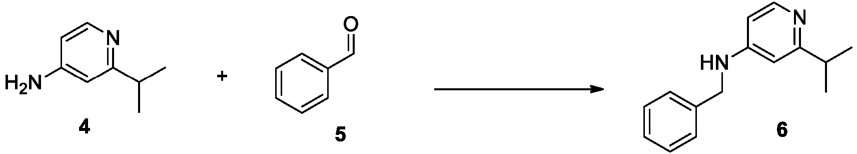

Compound 4 (800 mg, 6.06 mmol) was dissolved in ethanol (10 mL), and compound 5 (1.28 g, 12.12 mmol) was added. The mixture was replaced with N₂ three times and stirred at 90 °C for 2 h, new spot was detected on a plate. The raction temperature was lowered to 0 °C, sodium borohydride (458 mg, 12.12 mmol) was added slowly, and the mixture was naturally heated to room temperature and stirred for 1 h, new spot was detected on a plate. The raction mixture was diluted with water (50 ml) and then extracted with ethyl acetate (20 mL x 3), the combined organic phase was washed with saturated saline (50 mL), dried over anhydrous sodium sulfate, filtered and concentrated under reduced pressure to remove the organic solvent. The residue was purified by column chromatography (methanol: dichloromethane = 10:1) to afford compound 6 (200 mg).

### Step 4):

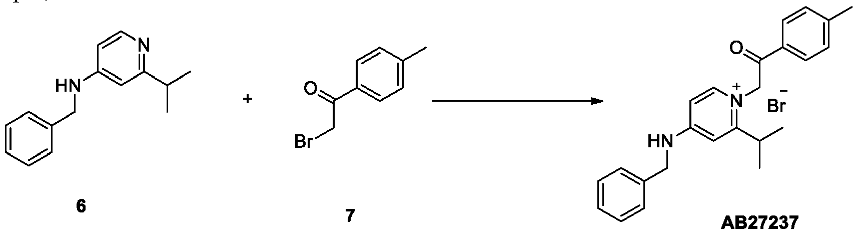

Compound 6 (113 mg, 0.5 mmol) was dissolved in acetonitrile (5 mL), and compound 7 (213 mg, 1.0 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography (dichloromethane: methanol = 10:1) to afford compound AB27237.

MS-ESI and ¹H NMR of the compound AB27237 were as follows:
MS-ESI calculated [M-Br⁻]⁺: 359.48, Found: 359.30.
¹H NMR (400 MHz, DMSO) δ 9.07 (d, *J =* 15.6 Hz, 1H), 8.13 (d, *J* = 9.1 Hz, 0H), 7.94 (t, *J* = 6.2 Hz, 2H), 7.43 (s, 1H), 7.40 (dd, *J =* 10.5, 5.1 Hz, 5H), 7.31 (s, 1H), 6.96 (s, 1H), 6.84 (s, 1H), 6.01 (d, *J =* 6.0 Hz, 2H), 4.59 (dd, *J =* 25.3, 5.9 Hz, 2H), 2.95 - 2.80 (m, 1H), 2.41 (s, 3H), 1.13 (t, *J* = 7.3 Hz, 6H).

### Example 56 Synthesis of compound AB27238

The structure of compound AB27238 was as follows:

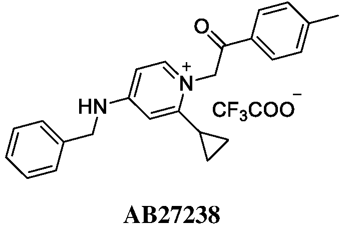

### Step 1):

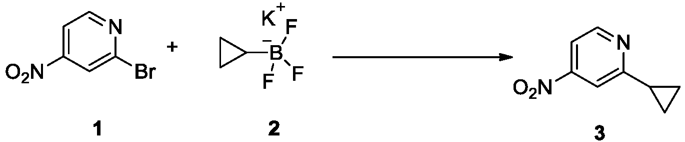

Compound 1 (1.5 g, 7.43 mmol) was dissolved in toluene (20 mL) and water (4 mL), compound 2 (2.2 g, 14.86 mmol), palladium acetate (84 mg, 0.37 mmol), n-butyldi(1-adamantyl)phosphine (267 mg, 0.74 mmol) and cesium carbonate (7.26 g, 22.29 mmol) were added. The mixture was replaced with N₂ three times and stirred at 100°C for overnight, new spot was detected on a plate. The raction mixture was diluted with water (20 ml) and then extracted with ethyl acetate (20 mL x 3), the combined organic phase was washed with saturated saline (10 mL), dried over anhydrous sodium sulfate, filtered and concentrated under reduced pressure to remove the organic solvent to afford compound 3 (900 mg).

### Step 2):

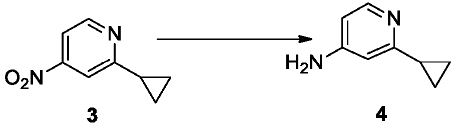

Compound 3 (900 mg, 5.48 mmol) was dissolved in methanol (20 mL), and palladium carbon (100 mg) was added. The mixture was replaced with N₂ three times and stirred at 100°C for overnight, new spot was detected on a plate. The raction mixture was filtered to remove palladium carbon, the filtrate was rotary dried to obtain compound 4 (900 mg).

### Step 3):

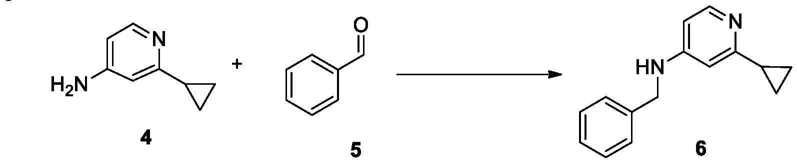

Compound 4 (400 mg, 2.98 mmol) was dissolved in ethanol (20 mL), and compound 5 (380 mg, 3.58 mmol) was added. The mixture was replaced with N₂ three times and stirred at 90 °C for overnight, and sodium borohydride (340 mg, 9.1 mmol) was added, then the mixture was replaced with N₂ three times and stirred at room temperature for 3h, new spot was detected on a plate. The raction mixture was diluted with water (20 ml) and then extracted with ethyl acetate (20 mL x 3), the combined organic phase was washed with saturated saline (10 mL), dried over anhydrous sodium sulfate, filtered and concentrated under reduced pressure to remove the organic solvent to afford compound 6 (120 mg).

### Step 3):

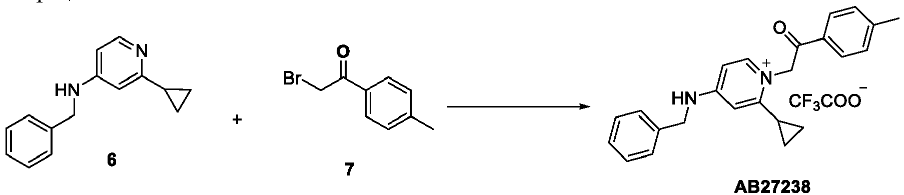

Compound 6 (120 mg, 0.53 mmol) was dissolved in acetonitrile (10 mL), and compound 7 (115 mg, 0.53 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27238.

MS-ESI and ¹H NMR of the compound AB27238 were as follows:
MS-ESI calculated [M-CF₃COO⁻]⁺ 357.20, Found: 357.05.
¹H NMR (400 MHz, DMSO) δ 9.10 (dd, *J* = 10.6, 5.9 Hz, 1H), 8.20 - 7.93 (m, 3H), 7.46 - 7.29 (m, 7H), 6.94-6.87 (m, 1H), 6.76-6.57 (m, 1H), 6.07 (s, 2H), 4.57 (dd, *J* = 16.8, 5.6 Hz, 2H), 2.40 (s, 3H), 1.95 (d, *J* = 3.1 Hz, 1H), 0.94 (d, *J =* 7.7 Hz, 2H), 0.79 (d, *J =* 3.9 Hz, 1H), 0.69 (d, *J =* 4.3 Hz, 1H).

### Example 57 Synthesis of compound AB27272

The structure of compound AB27272 was as follows:

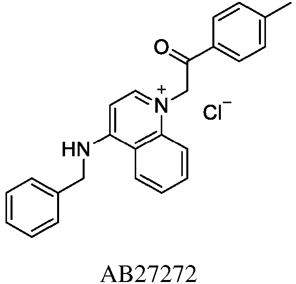

### Step 1):

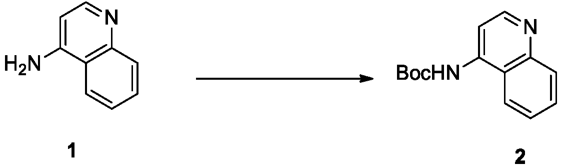

Compound 1 (200 mg, 1.38 mmol) was dissolved in dried tetrahydrofuran (20 mL), and sodium bis(trimethylsilyl)amide (1.38 mL, 2.76 mmol) and BOC anhydride (288 mg, 1.32 mmol) were added under ice water bath. The mixture was replaced with N₂ three times and stirred at room temperature for 2 h, new spot was detected on a plate. The raction mixture was diluted with water (10 ml) and extracted with ethyl acetate (10 mL x 3), the combined organic phases were washed with saturated saline (100 mL), dried over anhydrous sodium sulfate, filtered and concentrated under reduced pressure to remove the organic solvent. The residue was purified by thin layer chromatography (ethyl acetate: petroleum ether = 0-50%) to afford compound 2 (200 mg).

### Step 2):

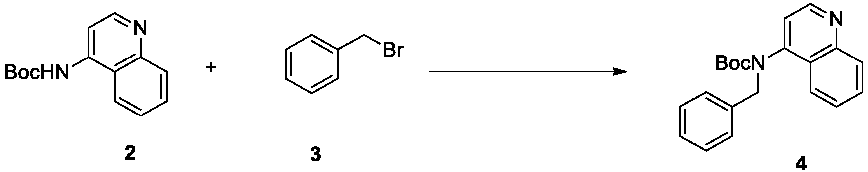

Compound 2 (200 mg, 0.82 mmol) was dissolved in N-N-dimethylformamide (20 mL), and sodium hydride (100 mg, 2.45 mmol) was added under ice bath. The mixture was replaced with N₂ three times and stirred for 30 min under ice bath, then compound 3 (169 mg, 0.98 mmol) was injected slowly under ice bath. The mixture was stirred at room temperature for 60 min, new spot was detected on a plate. The raction mixture was diluted with water (10 ml) and extracted with ethyl acetate (30 mL x 3), the combined organic phases were washed with saturated saline (100 mL), dried over anhydrous sodium sulfate, filtered and concentrated under reduced pressure to remove the organic solvent. The residue was purified by column chromatography (methanol: dichloromethane = 0-10%) to afford compound 4 (100 mg).

### Step 3):

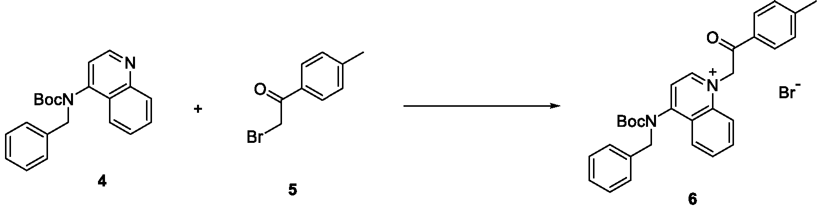

Compound 4 (100 mg, 0.30 mmol) was dissolved in acetonitrile (10 mL), and compound 5 (128 mg, 0.6 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound 6 (120 mg).

### Step 4):

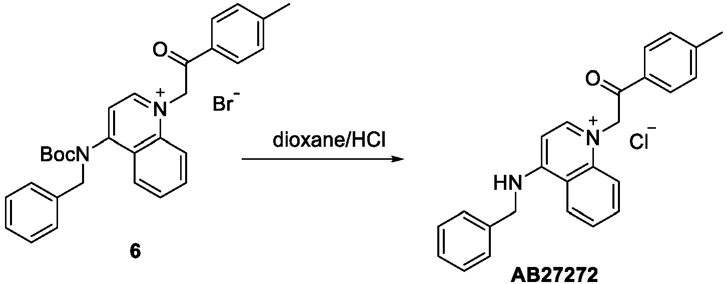

Compound 6 (120 mg, 0.25 mmol) was dissolved in dioxane hydrochloride (5 mL, 0.122 mmol). The mixture was replaced with N₂ three times and stirred at room tempetature for 2 h, new spot was detected on a plate. The raction mixture was filtered to afford compound AB27272.

MS-ESI and ¹H NMR of the compound AB27272 were as follows:
MS-ESI calculated [M-Cl⁻]⁺=367.18, Found: 367.30.
¹H NMR (400 MHz, DMSO) δ 10.31 (s, 1H), 8.75 (d, *J =* 5.9 Hz, 1H), 8.50 (d, *J =* 5.9 Hz, 1H), 8.00-7.87 (m, 4H), 7.76 (s, 1H), 7.44 (s, 4H), 7.36 (s, 2H), 7.28 (s, 1H), 6.95 (d, *J =* 5.3 Hz, 1H), 6.35 (s, 2H), 4.84 (s, 2H), 2.41 (s, 3H).

### Example 58 Synthesis of compound AB27273

The structure of compound AB27273 was as follows:

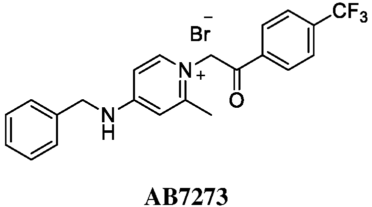

### Step 1:

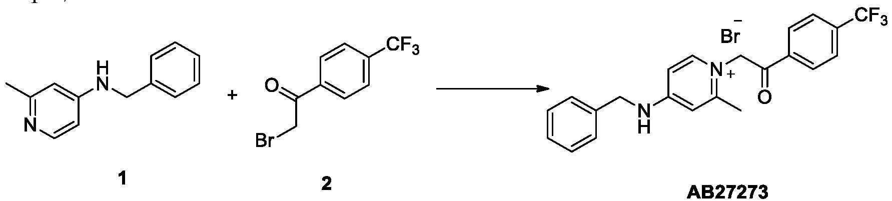

Compound 1 (100 mg, 0.51 mmol) was dissolved in acetonitrile (10 mL), and compound 2 (135 mg, 0.51 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27273.

MS-ESI calculated [M-Br⁻]⁺ 385.15, Found: 385.20.

¹H NMR (400 MHz, DMSO) δ 9.19 (d, *J =* 25.2 Hz, 1H), 8.25 (d, *J =* 7.0 Hz, 2H), 8.08 (dt, *J* = 7.0, 6.0 Hz, 3H), 7.43 - 7.31 (m, 5H), 7.08 - 6.96 (m, 1H), 6.95 - 6.89 (m, 1H), 6.07 (d, *J =* 4.3 Hz, 2H), 4.65 - 4.52 (m, 2H), 2.38 (d, *J =* 8.4 Hz, 3H).

### Example 59 Synthesis of compound AB27274

The structure of compound AB27274 was as follows:

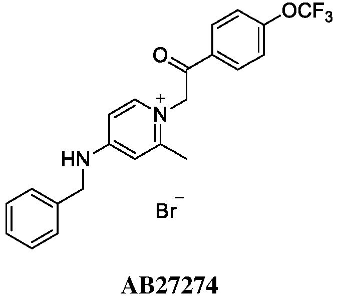

### Step 1):

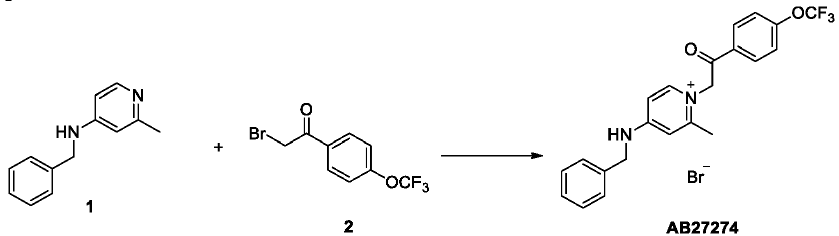

Compound 1 (198 mg, 1 mmol) and triethylamine (303 mg, 3 mmol) were dissolved in acetonitrile (5 mL), and compound 2 (283 mg, 1 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27274.

MS-ESI calculated [M-Br-t 401.40, Found: 401.10.

¹H NMR (400 MHz, DMSO) δ 9.14 (d, *J =* 26.3 Hz, 1H), 8.16 (d, *J =* 7.1 Hz, 2H), 8.05 (dd, *J* = 48.8, 7.3 Hz, 1H), 7.64 (d, *J* = 8.1 Hz, 2H), 7.41 - 7.34 (m, 4H), 7.31 (d, *J* = 6.4 Hz, 1H), 7.04 - 6.85 (m, 2H), 6.00 (d, *J =* 4.2 Hz, 2H), 4.56 (s, 2H), 2.34 (d, *J =* 8.0 Hz, 3H).

### Example 60 Synthesis of compound AB27275

The structure of compound AB27275 was as follows:

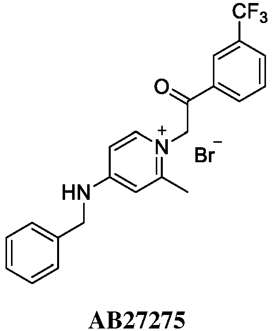

### Step 1):

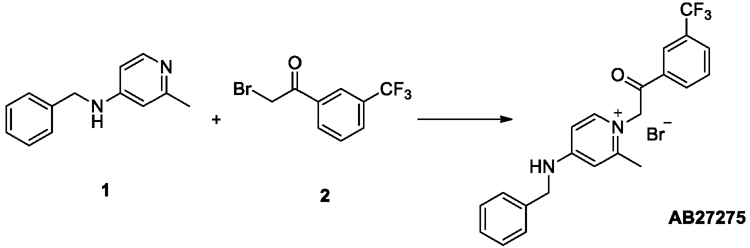

Compound 1 (100 mg, 0.51 mmol) was dissolved in acetonitrile (10 mL), and compound 2 (135 mg, 0.51 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27275.

MS-ESI calculated [M-Br⁻]⁺ 385.41, Found: 385.75.

¹H NMR (400 MHz, DMSO) δ 9.25 (d, *J* = 25.0 Hz, 1H), 8.32 (s, 2H), 8.18 - 8.02 (m, 2H), 7.88 (t, *J* = 7.7 Hz, 1H), 7.46 - 7.27 (m, 6H), 7.06-6.92 (m, 2H), 6.14 (s, 2H), 4.57 (d, *J* = 5.4 Hz, 2H), 2.37 (d, *J =* 10.0 Hz, 4H).

### Example 61 Synthesis of compound AB27276

The structure of compound AB27276 was as follows:

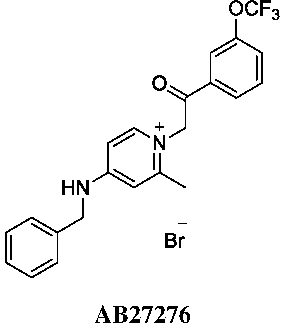

### Step 1):

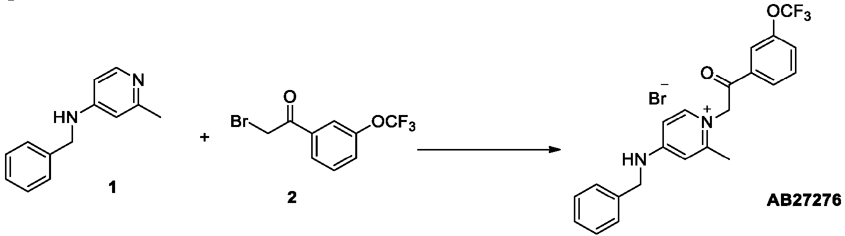

Compound 1 (100 mg, 0.5 mmol) was dissolved in acetonitrile (10 mL), and compound 2 (157 mg, 0.55 mmol) was added. The mixture was stirred at 70 °C for overnight, new spot was detected on a plate. The raction mixture was concentrated under reduced pressure to remove the organic solvent, the residue was purified by column chromatography to afford compound AB27276.

MS-ESI calculated [M-Br⁻]⁺: 401.41, Found: 401.10.

¹H NMR (400 MHz, CD3OD) δ 8.11 (s, 1H), 8.32 (s, 2H), 8.01 -7.90 (m, 2H), 7.68 (t, *J =* 7.7 Hz, 2H), 7.39 - 7.33 (m, 5H), 6.90-6.85 (m, 2H), 5.93 (d, *J =* 8.0 Hz, 2H), 4.58 (s, 2H), 2.40 (s, 3H).

### Example 62 Synthesis of compound AB27278

The structure of compound AB27278 was as follows:

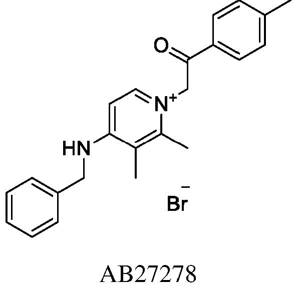

### Step 1):

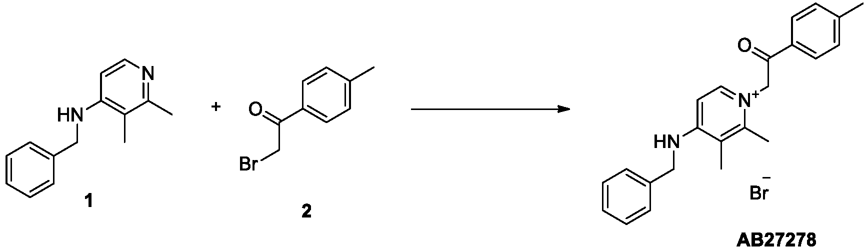

Compound 1 (50 mg, 0.24 mmol) was dissolved in acetonitrile (5 mL), and compound 2 (60 mg, 0.24 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27278.

MS-ESI calculated [M-Br⁻] ⁺ 345.46, Found: 345.10.

¹H NMR (400 MHz, DMSO) δ 8.41 (s, 1H), 8.01 (s, 4H), 7.88 (s, 1H), 7.38 (s, 7H), 7.30 (s, 2H), 7.23 (s, 1H), 6.79 (s, 1H), 5.98 (s, 2H), 4.60 (s, 2H), 2.35 (s, 4H), 2.27 (s, 4H), 2.18 (s, 4H).

### Example 63 Synthesis of compound AB27325

The structure of compound AB27325 was as follows:

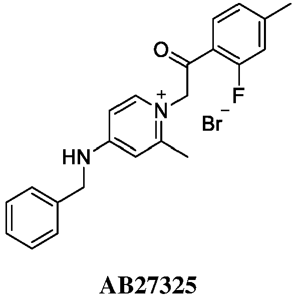

### Step 1):

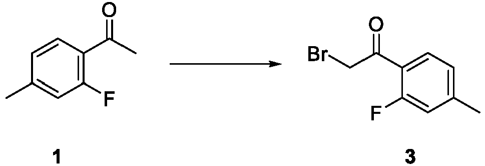

Compound 1 (150 mg, 1 mmol) was dissolved in dichloromethane (50 mL), three drops of methanol were added dropwise, and bis(N,N-dimethylacetamido)hydrogendibromobromate(414 mg, 1 mmol) was added. The mixture was stirred at 50 °C for overnight, new spot was detected on a plate. The raction mixture was rotary dried, the residue was purified by thin layer chromatography (petroleum ether: ethyl acetate = 50:1) to afford compound 3 (190 mg).

### Step 2):

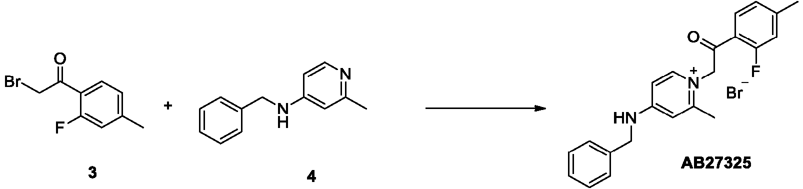

Compound 3 (190 mg, 0.82 mmol) was dissolved in acetonitrile (10 mL), and compound 4 (182 mg, 0.82 mol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27325.

MS-ESI and ¹H NMR of the compound AB27325 were as follows:
MS-ESI calculated [M-Br⁻]⁺: 349.42, Found: 349.10.
¹H NMR (400 MHz, DMSO) δ 9.13 (d, *J =* 25.2 Hz, 1H), 8.05 (dd, *J =* 49.2, 7.3 Hz, 1H), 7.83 - 7.69 (m, 2H), 7.55 (t, *J* = 7.7 Hz, 1H), 7.46 - 7.24 (m, 5H), 7.08 - 6.81 (m, 2H), 5.96 (d, *J* = 4.2 Hz, 2H), 4.68 - 4.44 (m, 2H), 2.32 (d, *J* = 8.0 Hz, 6H).

### Example 64 Synthesis of compound AB27327

The structure of compound AB27327 was as follows:

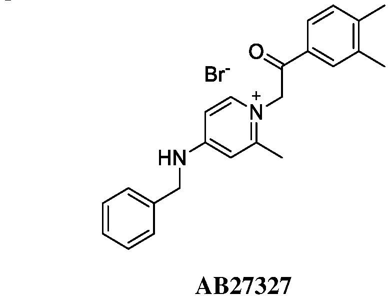

### Step 1):

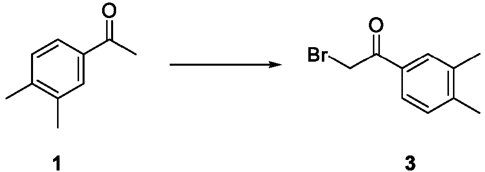

Compound 1 (148.20 g, 11 mmol) was dissolved in dichloromethane (50 mL), three drops of methanol were added, and bis(N,N-dimethylacetamide)hydrogendibromobromate(414 mg, 1 mmol) was added. The mixture was stirred at 50 °C for overnight, new spot was detected on a plate. The raction mixture was rotary dried, the residue was purified by thin layer chromatography to afford compound 3 (170 mg).

MS-ESI calculated [M+1]⁺ 227, Found: 227.

### Step 2):

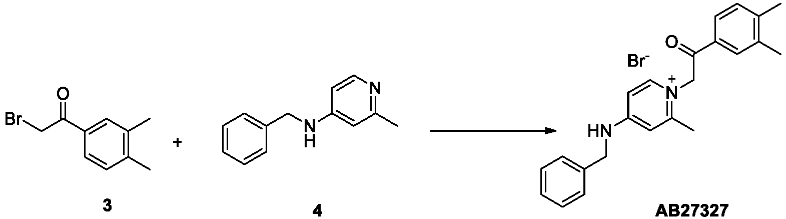

Compound 3 (74.56 mg, 0.74 mmol) was dissolved in acetonitrile (10 mL) and compound 4 (147 mg, 0.74 mol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27327 (146mg).

MS-ESI and ¹H NMR of the compound AB27327 were as follows:
MS-ESI calculated [M-Br⁻]⁺: 345.46, Found: 345.25.
¹H NMR (400 MHz, DMSO) δ 9.14 (d, *J =* 23.9 Hz, 1H), 8.07 (dd, *J =* 49.2, 7.3 Hz, 1H), 7.84 - 7.74 (m, 2H), 7.37 (d, *J =* 7.5 Hz, 5H), 7.34 - 7.27 (m, 1H), 6.94 (dd, *J =* 33.0, 24.4 Hz, 2H), 5.96 (d, *J =* 3.0 Hz, 2H), 4.55 (s, 2H), 2.31 (s, 9H).

### Example 65 Synthesis of compound AB27328

The structure of compound AB27328 was as follows:

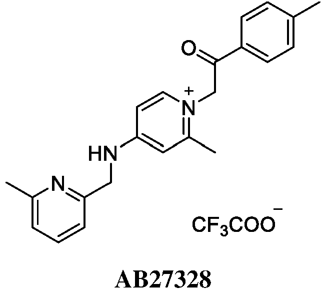

### Step 1):

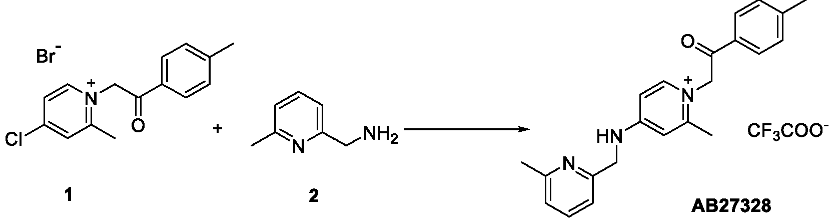

Compound 1 (153 mg, 0.45 mmol) was dissolved in acetonitrile (10 mL), compound 2 (50 mg, 0.41 mmol) and triethylamine (0.1 ml) were added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27328 (35mg).

MS-ESI calculated [M-CF₃COO⁻]⁺: 346.44, Found: 346.05.

¹H NMR (400 MHz, CDsOD) δ 8.26 (t, *J =* 7.1 Hz, 1H), 8.06 (dd, *J =* 31.0, 6.9 Hz, 1H), 7.97 (d, *J =* 8.2 Hz, 2H), 7.68 (t, *J =* 7.8 Hz, 2H), 7.40 (d, *J =* 8.1 Hz, 2H), 5.93 (s, 2H), 3.29 (dt, *J =* 3.1, 1.5 Hz, 1H), 2.44 (s, 3H), 2.40 (s, 3H).

### Example 66 Synthesis of compound AB27330

The structure of compound AB27330 was as follows:

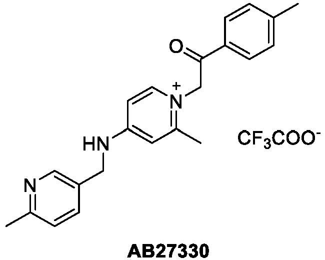

### Step 1:

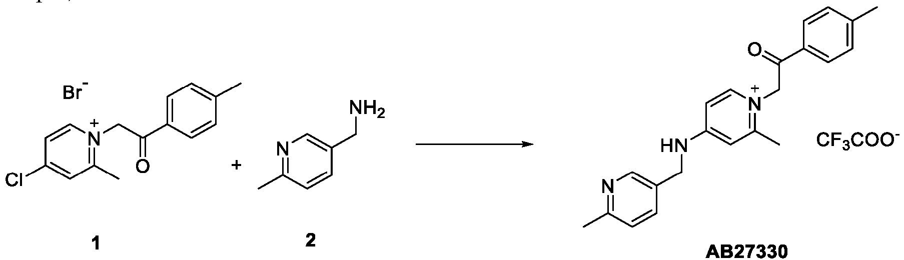

Compound 1 (85 mg, 0.25 mmol) was dissolved in acetonitrile (10 mL), compound 2 (31 mg, 0.6 mmol) and triethylamine (0.1 ml) were added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27330.

MS-ESI calculated [M-CF₃COO⁻]⁺: 346.44, Found: 346.10.

¹H NMR (400 MHz, CD3OD) δ 8.69 (s, 1H), 8.31 (s, 1H), 8.03 (dd, *J =* 45.1, 7.5 Hz, 3H), 7.80 (d, *J =* 7.9 Hz, 1H), 7.41 (d, *J =* 7.9 Hz, 2H), 6.94 (d, *J =* 12.3 Hz, 2H), 5.91 (s, 2H), 4.76 (s, 2H), 2.73 (s, 3H), 2.45 (s, 3H), 2.40 (s, 3H).

### Example 67 Synthesis of compound AB27225

The structure of compound AB27225 was as follows:

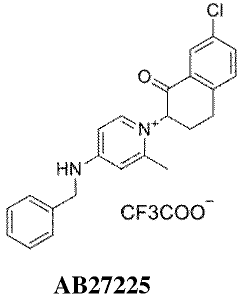

### Step 1):

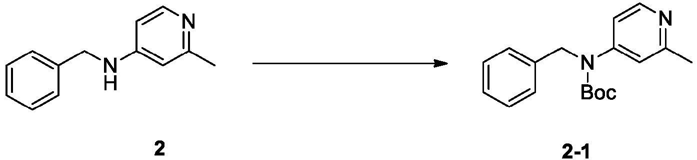

Compound 2 (460 mg, 2.32 mmol) was dissolved in dichloromethane (10 mL), tert-butyl carbonate (760 mg, 3.49 mmol), 4-dimethylaminopyridine (57 mg, 0.47 mmol) and triethylamine (704 mg, 6.97 mmol) were added. The mixture was replaced with N₂ three times and stirred at room temperature for overnight, new spot was detected on a plate. The raction mixture was concentrated under reduced pressure to remove the organic solvent, the residue was purified by column chromatography to afford compound 2-1 (450 mg).

### Step 2):

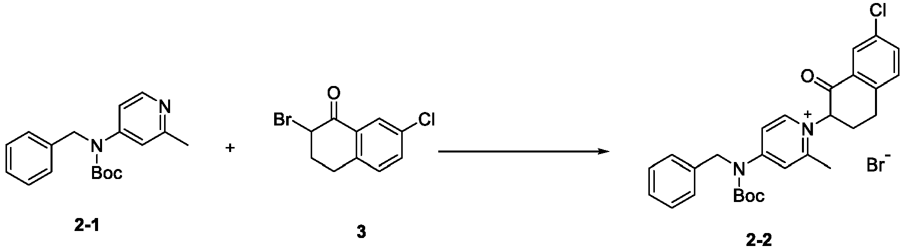

Compound 2-1 (450 mg, 1.51 mmol) was dissolved in acetonitrile (10 mL), compound 3 (390 mg, 1.51 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound 2-2 (25 mg)

### Step 3):

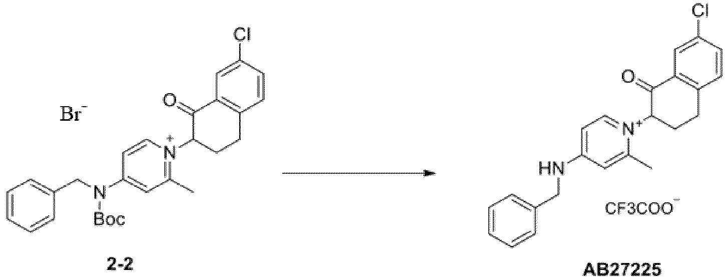

Compound 2-2 (40 mg, 0.08 mmol) was dissolved in ethyl acetate hydrochloride (10 mL). The mixture was replaced with N₂ three times, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27225.

MS-ESI and ¹H NMR of the compound AB27225 were as follows:
MS-ESI calculated [M-CF₃COO⁻] ⁺377.89, Found: 377.
¹H NMR (400 MHz, CD3OD) δ 8.08 - 7.93 (m, 2H), 7.62 (d, *J =* 8.0 Hz, 1H), 7.44 (d, *J =* 8.2 Hz, 1H), 7.35 (dt, *J =* 8.5, 3.8 Hz, 5H), 6.85 (dd, *J* = 20.9, 5.7 Hz, 2H), 5.69 (dd, *J* = 13.6, 4.2 Hz, 1H), 4.58 (d, *J =* 3.9 Hz, 2H), 3.47 - 3.36 (m, 1H), 3.25-3.20 (m, 1H), 2.85 - 2.74 (m, 1H), 2.58 (s, 1H), 2.54 (d, *J* = 6.9 Hz, 3H).

### Example 68 Synthesis of compound AB27110

The structure of compound AB27110 was as follows:

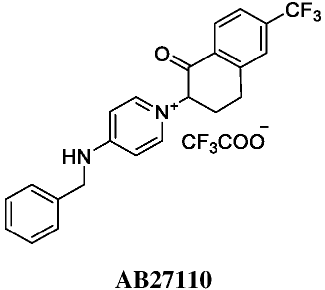

### Step 1):

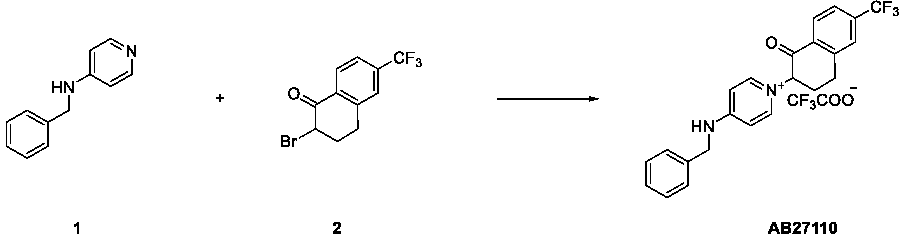

Compound 1 (90 mg, 0.49 mmol) was dissolved in acetonitrile (5 mL), and compound 2 (287 mg, 0.98 mmol) was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 A); mobile phase: 0.1% aqueous trifluoroacetic acid + acetonitrile; gradient elution: 1-20 min, 5-25% ACN; 20-50 min, 25-35% ACN) to afford compound AB27110.

MS-ESI calculated [M-CF₃COO⁻]⁺ 397.42, Found: 397.25.

¹H NMR (400 MHz, DMSO) δ 9.72 (s, 1H), 8.28 (d, *J* = 6.8 Hz, 1H), 8.11 (d, *J* = 6.7 Hz, 1H), 8.07 (d, *J* = 8.5 Hz, 1H), 7.89 (s, 1H), 7.78 (s, 1H), 7.76 (d, *J* = 5.7 Hz, 1H),7.31 (d, *J* = 11.9 Hz, 5H), 7.01 (d, *J =* 5.6 Hz, 1H), 5.72 (d, *J =* 10.7 Hz, 1H), 4.58 (d, *J =* 4.9 Hz, 2H), 3.34 (s, 2H), 2.82 (s, 1H), 2.47 (d, *J* = 9.3 Hz, 1H).

### Example 69 Synthesis of compound AB27230

The structure of compound AB27230 was as follows:

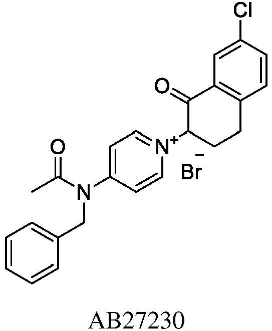

### Step 1):

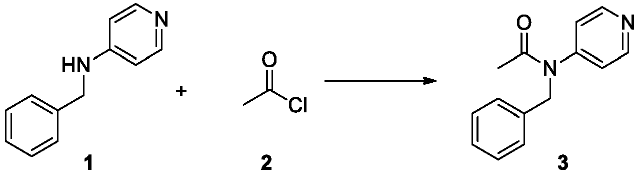

Compound 1 (184 mg, 1 mmol) was dissolved in dichloromethane (5 mL), and triethylamine (303 mg, 3 mmol) was added. The mixture was replaced with N₂ three times and stirred at 0 °C for 30 min, and compound 2 (157 mg, 2 mmol) was added at 0°C. The mixture was reacted at room temperature, new spot was detected on a plate. The raction mixture was concentrated under reduced pressure to remove the organic solvent, the residue was purified by thin layer chromatography (methanol:dichloromethane= 0-10%) to afford compound 3 (120 mg).

### Step 2):

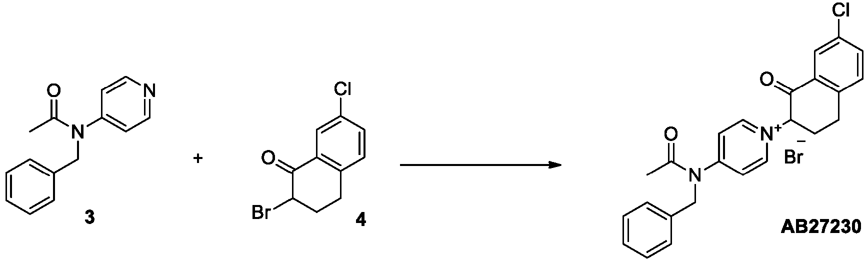

Compound 3 (120 mg, 0.52 mmol) was dissolved in acetonitrile (5 mL), and compound 4 (131 mg, 0.52 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27230.

MS-ESI and ¹H NMR of the compound AB27230 were as follows:
MS-ESI calculated [M-Br⁻] ⁺ 405.90, Found: 405.10.
¹H NMR (400 MHz, dmso) δ 8.82 (s, 2H), 8.15 (d, J = 4.7 Hz, 2H), 7.82 (s, 1H), 7.75 (d, J = 7.7 Hz, 1H), 7.52 (d, J = 7.3 Hz, 1H), 7.36 (d, J = 6.1 Hz, 2H), 7.27 (s, 3H), 6.12 (d, J = 13.1 Hz, 1H), 5.33 (s, 2H), 3.23 (s, 2H), 2.90 (s, 1H), 2.61 (s, 1H), 2.38 (s, 3H).

### Example 70 Synthesis of compound AB27138

The structure of compound AB27138 was as follows:

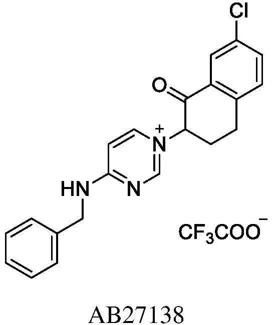

### Step 1):

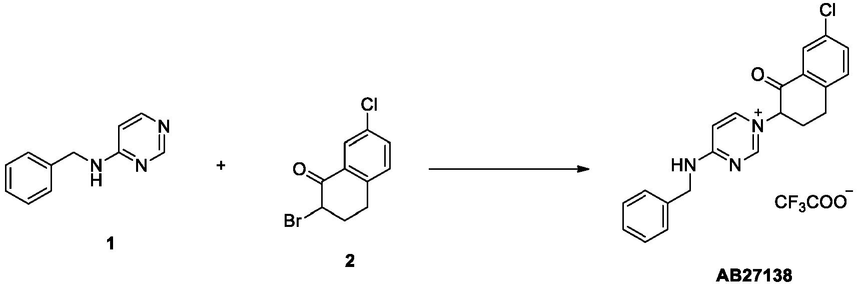

Compound 1 (100 mg, 0.54 mmol) was dissolved in acetonitrile (10 mL), compound 2 (140 mg, 0.54 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27138.

MS-ESI calculated [M-CF₃COO⁻] ⁺ 364.85, Found: 364.10.

¹H NMR (400 MHz, dmso-d6) δ 10.15 (s, 1H), 8.84 (s, 1H), 8.22 (s, 1H), 7.84 (s, 1H), 7.73 (s, 1H), 7.52 (s, 1H), 7.36 (s, 5H), 6.94 (s, 1H), 5.69 (d, J = 17.4 Hz, 1H), 4.74 (s, 2H), 3.14 (s, 2H), 2.76 (s, 1H), 1.91 (d, J = 24.7 Hz, 1H).

### Example 71 Synthesis of compound AB27160

The structure of compound AB27160 was as follows:

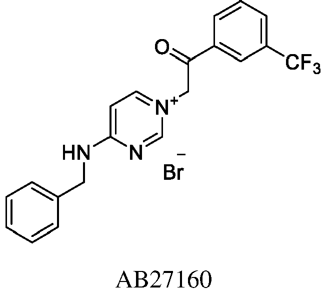

### Step 1):

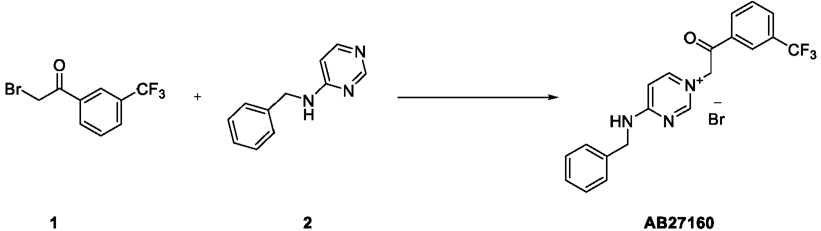

Compound 1 (133 mg, 0.5 mmol) was dissolved in acetonitrile (10 mL), and compound 2 (90 mg, 0.5 mmol) was added. The mixture was replaced with N₂ three times and stirred at room temperature for overnight, new spot was detected on a plate. The raction mixture was concentrated under reduced pressure to remove the organic solvent, the residue was purified by column chromatography (methanol: dichloromethane = 0-30 %) to afford compound AB27160.

MS-ESI calculated [M-Br⁻]⁺ 372.37, Found: 372.20.

¹H NMR (400 MHz, dmso-d6) δ 10.47 (d, *J =* 83.6 Hz, 1H), 8.79 (s, 1H), 8.30 (d, *J=* 13.5 Hz, 2H), 8.15 (dd, *J =* 14.7, 7.4 Hz, 2H), 7.88 (s, 1H), 7.35 (t, *J =* 12.8 Hz, 5H), 7.08 (d, *J =* 7.2 Hz, 1H), 6.01 (s, 2H), 4.73 (s, 2H).

### Example 72 Synthesis of compound AB27283

The structure of compound AB27283 was as follows:

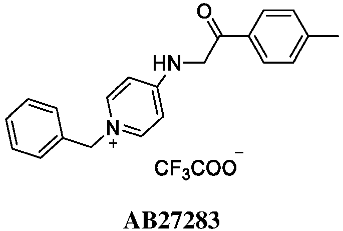

### Step 1:

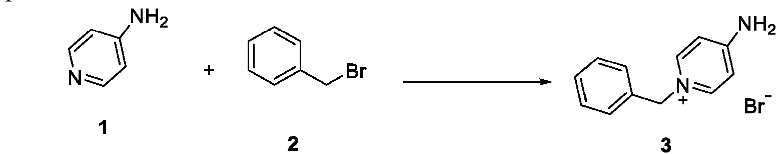

Compound 1 (3 g, 32 mmol) was dissolved in acetonitrile (50 mL), and compound 2 (6.54 g, 38 mmol) was added. The mixture was stirred at 80 °C for 2 h, new spot was detected on a plate. The raction mixture was filtered to afford compound 3 (5.2 g).

### Step 2):

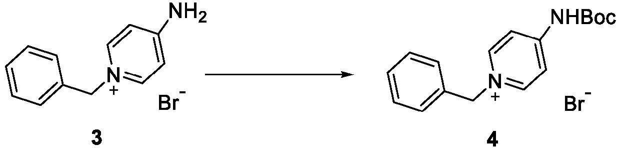

Compound 3 (3 g, 16 mmol) was dissolved in dichloromethane (50 mL), triethylamine (4.8 g, 48 mmol), tert-butyl carbonate (3.56 mg, 16 mmol), and DMAP (195 mg, 1.6 mmol) were added. The mixture was replaced with N₂ three times and stirred at room temperature for 2h, new spot was detected on a plate. The raction mixture was diluted with water (30 ml) and extracted with dichloromethane (30 mL x 3), the combined organic phases were washed with saturated saline (100 mL), dried over anhydrous sodium sulfate, filtered and concentrated under reduced pressure to remove the organic solvent. The residue was purified by thin layer chromatography (dichloromethane: methanol = 0-5%) to afford compound 4 (700 mg).

### Step 3):

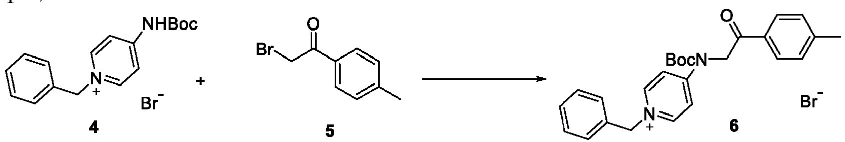

Compound 4 (200 mg, 0.70 mmol) was dissolved in acetonitrile (10 mL), compound 5 (150 mg, 0.70 mmol) and triethylamine (140 mg, 1.4 mmol) were added. The mixture was stirred at 40 °C for overnight, new spot was detected on a plate. The raction mixture was concentrated under reduced pressure to remove the organic solvent, the residue was purified by preparation chromatography to afford compound 6 (100 mg).

### Step 4):

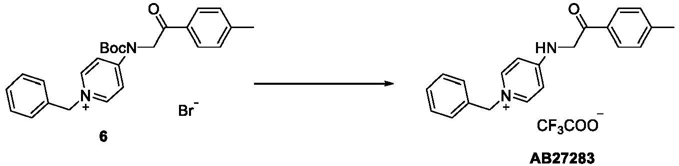

Compound 6 (100 mg, 0.23 mmol) was dissolved in dioxane hydrochloride (10 mL). The mixture was stirred at room temperature for 2 h, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27283.

MS-ESI and ¹H NMR of the compound AB27283 were as follows:
MS-ESI calculated [M-CF₃COO⁻]⁺ 317.16, Found: 317.00
¹H NMR (400 MHz, DMSO) δ 8.86 (s, 1H), 8.43 (d, *J =* 7.1 Hz, 1H), 8.30 (d, *J =* 7.0 Hz, 1H), 7.94 (d, *J* = 8.0 Hz, 2H), 7.42 - 7.34 (m, 7H), 7.19 - 7.14 (m, 1H), 7.02 - 6.92 (m, 1H), 5.36 (s, 2H), 5.02 (d, *J =* 5.3 Hz, 2H), 2.39 (s, 3H).

### Example 73 Synthesis of compound AB27285

The structure of compound AB27285 was as follows:

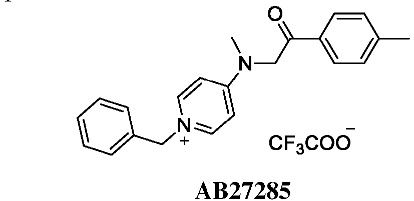

### Step 1):

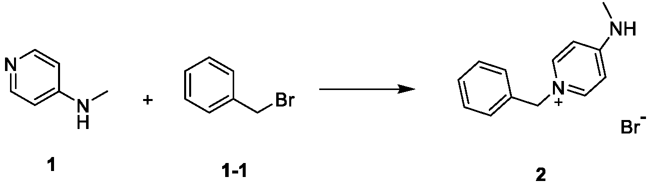

Compound 1 (500 mg, 4.62 mmol) and compound 1-1 (949 mg, 5.55 mmol) were dissolved in acetonitrile (10 mL). The mixture was at room temperature for 2 h, new spot was detected on a plate. The raction mixture was filtered, and the solid was rotary dried to afford compound 2 (750 mg).

### Step 2):

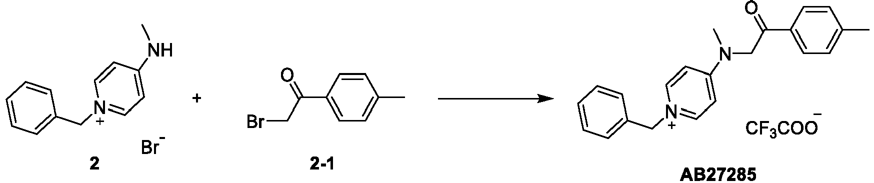

Compound 2 (100 mg, 0.5 mmol), compound 2-1 (127.84 mg, 0.6 mmol) and triethylamine (151.5 mg, 1.5 mmol) were dissolved in acetonitrile (10 mL). The mixture was stirred at 30 °C for 16 h, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27285.

MS-ESI and ¹H NMR of the compound AB27285 were as follows:
MS-ESI calculated [M-CF₃COO⁻] ⁺ 331.43, Found: 331.20.
¹H NMR (400 MHz, DMSO) δ 8.48 (d, *J* = 7.2 Hz, 1H), 8.31 (d, *J* = 7.5 Hz, 1H), 7.88 (d, *J =* 7.8 Hz, 2H), 7.39 (s, 7H), 7.18 (d, *J =* 4.7 Hz, 1H), 6.99 (d, *J =* 4.6 Hz, 1H), 5.37 (d, *J =* 11.5 Hz, 4H), 3.17 (s, 3H), 2.39 (s, 3H).

### Example 74 Synthesis of compound AB27299

The structure of compound AB27299 was as follows:

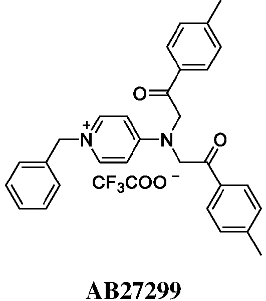

### Step 1):

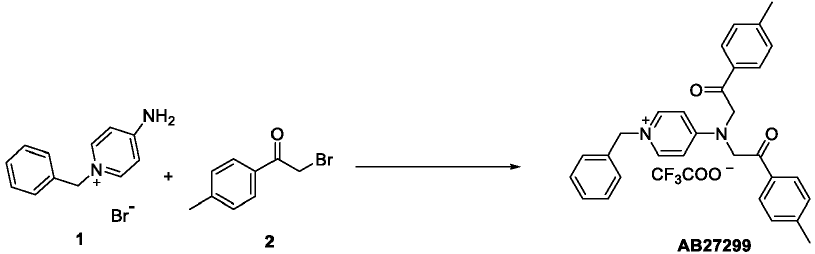

Compound 1 (200 mg, 1.08 mmol) was dissolved in acetonitrile (10 mL), and compound 2 (464 mg, 2.17 mmol) was added. The mixture was stirred at room tempertuare for 2 h, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27299.

MS-ESI calculated [M-CF₃COO⁻]⁺: 449.56, Found: 449.25.

¹H NMR (400 MHz, DMSO) δ 8.98 (s, 1H), 8.48 (s, 1H), 8.26 (d, *J=* 6.2 Hz, 1H), 7.87 (dd, *J* = 27.5, 7.2 Hz, 4H), 7.37 (d, *J* = 8.6 Hz, 6H), 7.30 (d, *J* = 7.3 Hz, 2H), 7.26 (s, 1H), 6.97 (d, *J* = 5.8 Hz, 1H), 5.37 (s, 2H), 3.74 (s, 4H), 2.36 (d, *J=* 4.5 Hz, 6H).

### Example 75 Synthesis of compound AB27306

The structure of compound AB27306 was as follows:

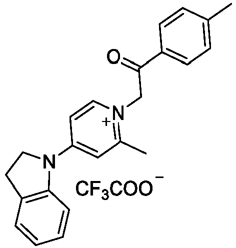

### AB27306

### Step 1):

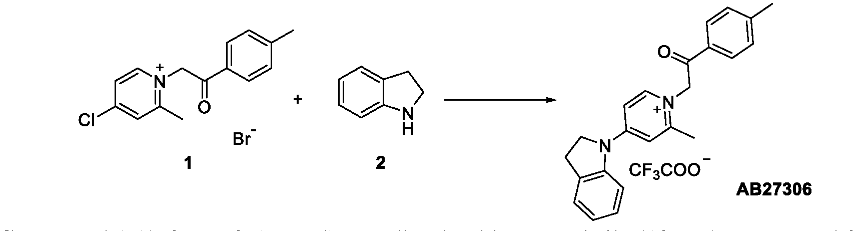

Compound 1 (170 mg, 0.5 mmol) was dissolved in acetonitrile (10 mL), compound 2 (60 mg, 0.5 mmol) was added, and triethylamine (0.5 was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27306.

MS-ESI calculated [M-CF₃COO⁻]⁺: 343.44, Found: 343.10.

¹H NMR (400 MHz,DMSO) δ 8.33 (d, *J* = 7.4 Hz, 1H), 7.96 (d, *J* = 8.0 Hz, 2H), 7.73 (d, *J* = 8.1 Hz, 1H), 7.65 - 7.37 (m, 5H), 7.30 (t, *J=* 7.7 Hz, 1H), 7.15 (t, *J=* 7.4 Hz, 1H), 6.12 (s, 2H), 4.22 (t, *J =* 8.0 Hz, 2H), 3.23 (t, *J =* 7.9 Hz, 2H), 2.49 - 2.45 (m, 3H), 2.42 (s, 3H).

### Example 76 Synthesis of compound AB27307

The structure of compound AB27307 was as follows:

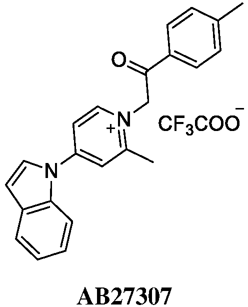

### Step 1):

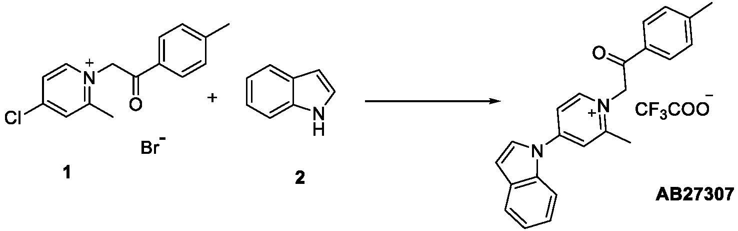

Compound 1 (170 mg, 0.5 mmol) was dissolved in acetonitrile (10 mL), compound 2 (60 mg, 0.5 mmol) was added, and triethylamine (0.5 was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27307.

MS-ESI calculated [M-CF₃COO⁻]⁺: 341.43, Found: 341.10.

¹H NMR (400 MHz,CD₃OD) δ 8.69 (d, *J =* 6.6 Hz, 1H), 8.29 (s, 1H), 8.22 (s, 1H), 8.05 (t, *J =* 8.7 Hz, 3H), 7.90 (s, 1H), 7.71 (d, *J =* 8.1 Hz, 1H), 7.43 (t, *J =* 9.1 Hz, 3H), 7.35 (d, *J =* 8.0 Hz, 1H), 6.98 (s, 1H), 6.29 (s, 2H), 2.73 (s, 3H), 2.47 (s, 3H).

### Example 77 Synthesis of compound AB27309

The structure of compound AB27309 was as follows:

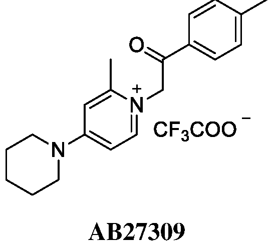

### Step 1:

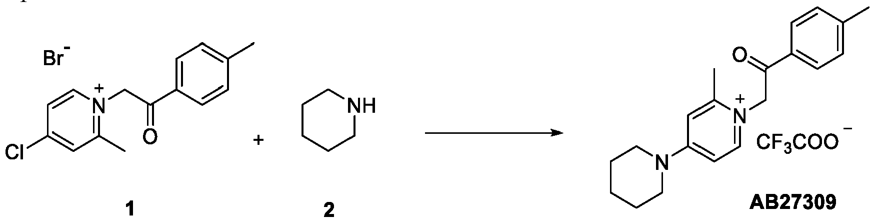

Compound 1 (102 mg, 0.3 mmol) was dissolved in acetonitrile (10 mL), compound 2 (30 mg, 0.3 mmol) was added, and triethylamine (0.1 was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27309.

MS-ESI calculated [M-CF₃COO⁻]⁺: 309.42, Found: 309.10.

¹H NMR (400 MHz,DMSO) δ 8.06 (d, *J* = 7.6 Hz, 1H), 7.92 (d, *J* = 8.0 Hz, 2H), 7.41 (d, *J* = 7.9 Hz, 2H), 7.20 (s, 2H), 5.93 (s, 2H), 3.66 (s, 21H), 2.35 (d, *J =* 29.4 Hz, 6H), 1.62 (d, *J =* 25.4 Hz, 7H).

### Example 78 Synthesis of compound AB27178

The structure of compound AB27178 was as follows:

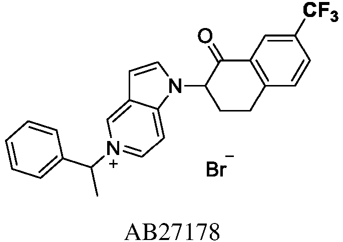

### Step 1):

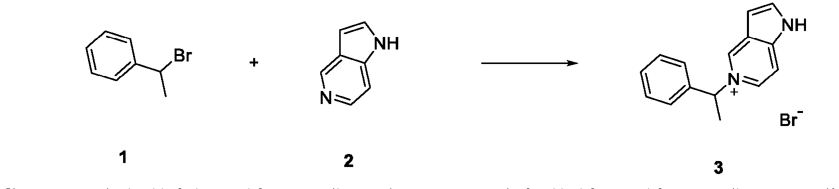

Compound 1 (1.85 g, 10 mmol) and compound 2 (1.18 g, 10 mmol) were dissolved in acetonitrile (50 mL). The mixture was stirred at room temperature for 16 h, new spot was detected on a plate. The raction mixture was filtered, and the solid was rotary dried to afford compound 3 (2 g).

### Step 2):

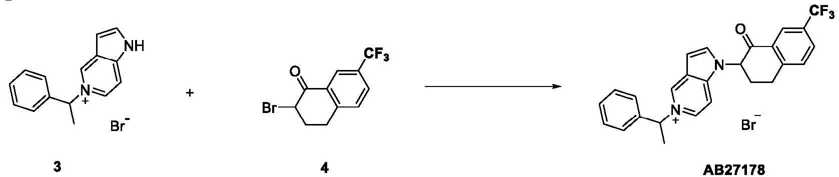

Compound 3 (100 mg, 0.44 mmol) and N,N-diisopropylethylamine (173 mg, 1.34 mmol) were dissolved in acetonitrile (5 mL), and compound 4 (128.92 mg, 0.44 mmol) was added. The mixture was stirred at room temperature for 4 h, new spot was detected on a plate. The raction mixture was concentrated under reduced pressure to remove the organic solvent, the residue was purified by column chromatography (methanol: dichloromethane = 0-10%) to afford compound AB27178.

MS-ESI and ¹H NMR of the compound AB27178 were as follows:
MS-ESI calculated [M-Br⁻]⁺ 435.46, Found: 435.25.
¹H NMR (400 MHz, dmso-d6) δ 9.66 (s, 1H), 8.74 (d, J = 5.9 Hz, 1H), 8.21 (s, 1H), 8.11 - 8.00 (m, 3H), 7.75 (d, J = 7.6 Hz, 1H), 7.52 (d, J = 6.0 Hz, 2H), 7.41 (d, J = 7.3 Hz, 3H), 7.15 (s, 1H), 6.20 (s, 2H), 3.43 (s, 2H), 2.94 (t, J = 28.4 Hz, 2H), 2.06 (d, J = 5.8 Hz, 3H).

### Example 79 Synthesis of compound AB27180

The structure of compound AB27180 was as follows:

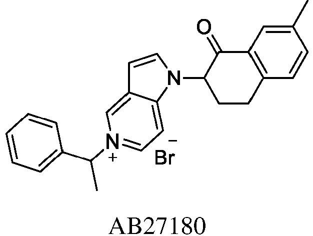

### Step 1):

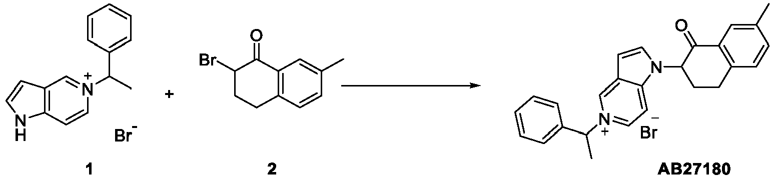

Compound 1 (100 mg, 0.45 mmol) was dissolved in acetonitrile (5 mL), and compound 2 (160 mg, 0.67 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27180.

MS-ESI calculated [M-Br⁻]⁺ 381.49, Found: 381.20.

1H NMR (400 MHz, dmso-d6) δ 9.65 (s, 1H), 8.72 (d, J = 5.6 Hz, 1H), 8.19 (d, J = 5.4 Hz, 1H), 8.05 (s, 1H), 7.68 (s, 1H), 7.51 (s, 2H), 7.48 (d, J = 7.0 Hz, 1H), 7.44 - 7.34 (m, 4H), 7.13 (s, 1H), 6.19 (d, J = 5.8 Hz, 1H), 6.10 (d, J = 13.2 Hz, 1H), 3.14 (d, J = 15.8 Hz, 1H), 2.90 (d, J = 12.1 Hz, 1H), 2.47 - 2.41 (m, 2H), 2.32 (s, 3H), 2.06 (s, 3H).

### Example 80 Synthesis of compound AB27181

The structure of compound AB27181 was as follows:

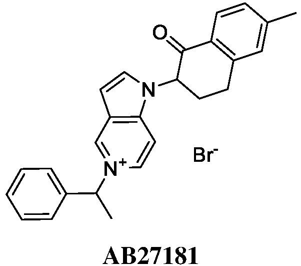

### Step 1):

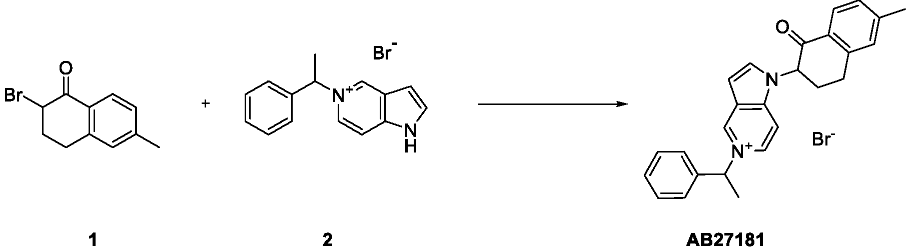

Compound 1 (116 mg, 0.5 mmol) was dissolved in acetonitrile (10 mL), and triethylamine (101.19 mg, 1 mmol) and compound 2 (111 mg, 0.5 mmol) were added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27181.

MS-ESI calculated [M-Br⁻]⁺ 381.50, Found: 381.10.

¹H NMR (400 MHz, dmso-d6) δ 9.62 (s, 1H), 8.71 (d, *J =* 5.7 Hz, 1H), 8.17 (d, *J =* 5.5 Hz, 1H), 8.05 (s, 1H), 7.78 (d, *J =* 7.3 Hz, 1H), 7.50 (s, 2H), 7.41 (d, *J =* 7.2 Hz, 3H), 7.32 - 7.19 (m, 2H), 7.13 (s, 1H), 6.17 (d, *J* = 5.8 Hz, 1H), 6.05 (d, *J* = 13.0 Hz, 1H), 3.14 (d, *J* = 16.4 Hz, 1H), 2.99 - 2.81 (m, 1H), 2.46 - 2.40 (m, 2H), 2.37 (s, 3H), 2.06 (d, *J =* 5.6 Hz, 4H).

### Example 81 Synthesis of compound AB27182

The structure of compound AB27182 was as follows:

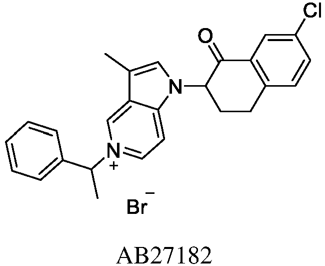

### Step 1):

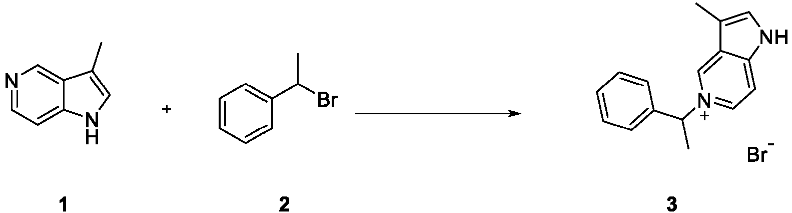

Compound 1 (200 mg, 1.51 mmol) was dissolved in dried acetonitrile (5 mL), and compound 2 (280 mg, 1.51 mmol) was added. The mixture was replaced with N₂ three times and stirred at 105°C for 16 h, new spot was detected on a plate. The raction mixture was concentrated under reduced pressure to remove the organic solvent, the residue was purified by thin layer chromatography (methanol: dichloromethane = 0-10%) to afford compound 3 (315 mg).

### Step 2):

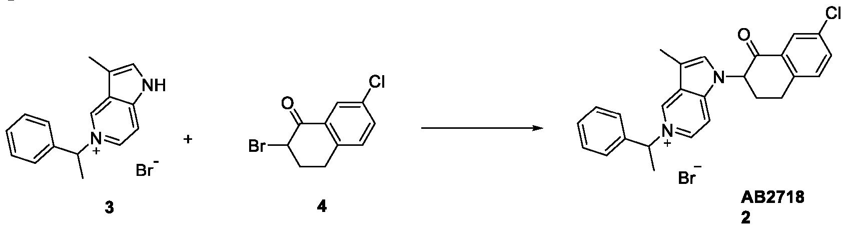

Compound 3 (315 mg, 1.33 mmol) was dissolved in acetonitrile (10 mL), and triethylamine (0.4 mL) and compound 4 (380 mg, 1.33 mmol) were added. The mixture was replaced with N₂ three times and stirred at room temperature for overnight, new spot was detected on a plate. The raction mixture was filtered and concentrated under reduced pressure to remove the organic solvent, the residue was purified by thin layer chromatography (methanol: dichloromethane = 0-10%) to afford compound AB27182.

MS-ESI and ¹H NMR of the compound AB27182 were as follows:
MS-ESI calculated [M-Br⁻]⁺ 415.94, Found: 415.20.
¹H NMR (400 MHz, CD₃OD) δ 9.43 (s, 1H), 8.43 (d, *J =* 5.8 Hz, 1H), 7.93 (d, *J =* 8.0 Hz, 2H), 7.63 (d, *J =* 19.4 Hz, 2H), 7.45 (s, 6H), 5.91 - 5.82 (m, 1H), 5.79 (s, 2H), 3.45 (t, *J =* 13.8 Hz, 1H), 3.24 (s, 1H), 2.96 - 2.82 (m, 1H), 2.60 (s, 1H), 2.47 (s, 3H).

### Example 82 Synthesis of compound AB27187

The structure of compound AB27187 was as follows:

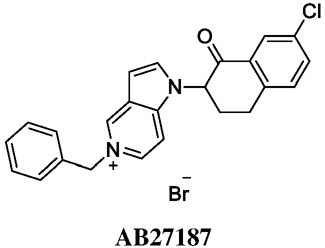

### Step 1):

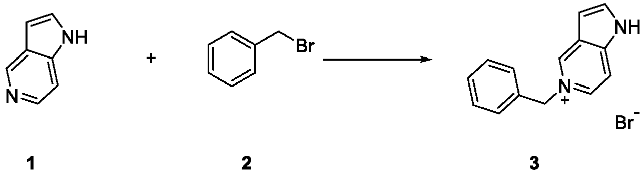

Compound 1 (1 g, 8.4 mmol) was dissolved in dried toluene (20 mL), and compound 2 (1.45 g, 8.4 mmol) was added. The mixture was replaced with N₂ three times and stirred at 105°C for 16 h, new spot was detected on a plate. The raction mixture was concentrated under reduced pressure to remove the organic solvent, the residue was purified by thin layer chromatography (methanol: dichloromethane = 0-10%) afford compound 3 (1.2 g).

### Step 2):

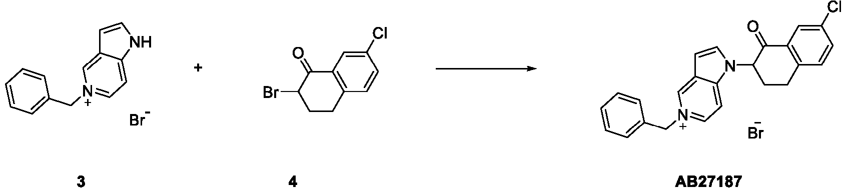

Compound 3 (100 mg, 0.48 mmol) was dissolved in acetonitrile (10 mL), and compound 4 (136 mg, 0.53 mmol) and triethylamine (97 mg, 0.96 mmol) were added. The mixture was replaced with N₂ three times and stirred at 40°C for overnight, new spot was detected on a plate. The raction mixture was concentrated under reduced pressure to remove the organic solvent, the residue was purified by column chromatography (methanol: dichloromethane = 0-30%) to afford compound AB27187.

MS-ESI and ¹H NMR of the compound AB27187 were as follows:
MS-ESI calculated [M-Br⁻]⁺ 387.88, Found: 387.15.
¹H NMR (400 MHz,DMSO-d6) δ 9.60 (s, 1H), 8.72 (d, J = 5.7 Hz, 1H), 8.21 (d, J = 5.9 Hz, 1H), 8.05 (s, 1H), 7.80 (s, 1H), 7.73 (d, J = 7.6 Hz, 1H), 7.56 - 7.47 (m, 3H), 7.40 (d, J = 7.2 Hz, 3H), 7.16 (s, 1H), 6.14 (d, J = 12.6 Hz, 1H), 5.81 (s, 2H), 3.42 (s, 1H), 3.23 (s, 2H), 2.94 (d, J = 11.9 Hz, 1H).

### Example 83 Synthesis of compound AB27188

The structure of compound AB27188 was as follows:

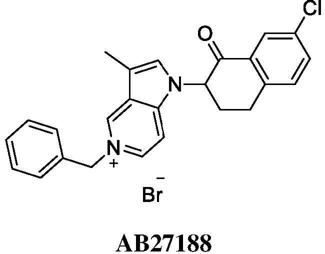

### Step 1):

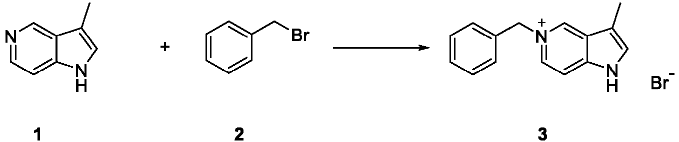

Compound 1 (100 mg, 0.75 mmol) was dissolved in acetonitrile (25 mL), and compound 2 (130 mg, 0.75 mmol) was added. The mixture was replaced with N₂ three times and stirred at 90°C for overnight, new spot was detected on a plate. The raction mixture was concentrated under reduced pressure to remove the organic solvent, the residue was purified by thin layer chromatography (dichloromethane:methanol = 0-10%) afford compound 3 (110 mg).

### Step 2):

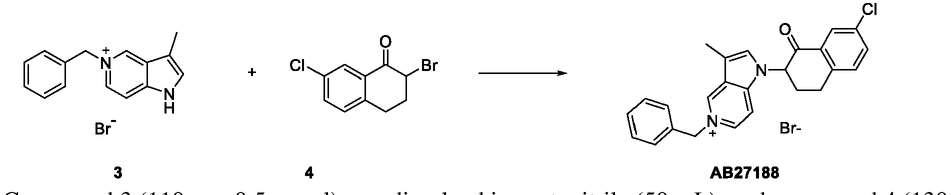

Compound 3 (110 mg, 0.5 mmol) was dissolved in acetonitrile (50 mL), and compound 4 (130 mg, 0.5 mmol) and triethylamine (0.13 mL) were added. The mixture was replaced with N₂ three times and stirred at room temperature for overnight, new spot was detected on a plate. The raction mixture was filtered and concentrated under reduced pressure to remove the organic solvent, the residue was purified by thin layer chromatography (methanol: dichloromethane = 0-10%) to afford compound AB27188.

MS-ESI and ¹H NMR of the compound AB27188 were as follows:
MS-ESI calculated [M-Br⁻]+ 401.91, Found: 401.30.
¹H NMR (400 MHz, CD₃OD) δ 9.43 (s, 1H), 8.43 (d, J = 5.8 Hz, 1H), 7.93 (d, J = 8.0 Hz, 2H), 7.63 (d, J = 19.4 Hz, 2H), 7.45 (s, 6H), 5.91 - 5.82 (m, 1H), 5.79 (s, 2H), 3.45 (t, J = 13.8 Hz, 1H), 3.24 (s, 1H), 2.96 - 2.82 (m, 1H), 2.60 (s, 1H), 2.47 (s, 3H).

### Example 84 Synthesis of compound AB27190

The structure of compound AB27190 was as follows:

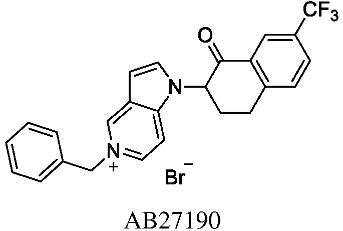

### Step 1):

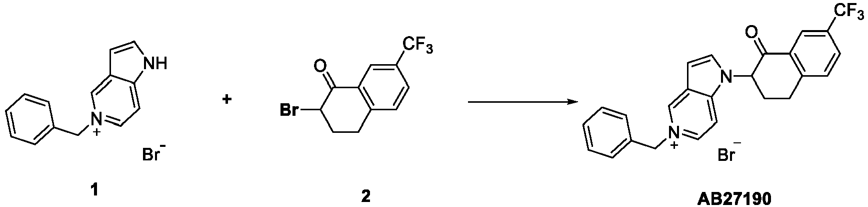

Compound 1 (209 mg, 1 mmol) and triethylamine (303 mg, 3 mmol) were dissolved in acetonitrile (5 mL), and compound 2 (293 mg, 1 mmol) was added. The mixture was replaced with N₂ three times and stirred at 40°C for overnight, new spot was detected on a plate. The raction mixture was concentrated under reduced pressure to remove the organic solvent, the residue was purified by thin layer chromatography to afford compound AB27190 (20 mg).

MS-ESI calculated [M-Br⁻]⁺ 421.15, Found: 421.

¹H NMR (400 MHz, CD3OD) δ 9.38 (s, 1H), 8.49 (d, *J=* 7.1 Hz, 1H), 8.23 (s, 1H), 8.03 (d, *J* = 7.0 Hz, 1H), 7.91 (dd, *J =* 7.6, 2.6 Hz, 2H), 7.67 (d, *J=* 8.1 Hz, 1H), 7.43 (dd, *J =* 6.9, 3.3 Hz, 5H), 7.15 (d, *J=* 3.4 Hz, 1H), 5.98 (dd, *J* = 14.2, 4.7 Hz, 1H), 5.80 (s, 2H), 3.56 (t, *J =* 13.1 Hz, 1H), 3.37 (d, *J=* 19.9 Hz, 1H), 2.98 (qd, *J* = 12.7, 4.4 Hz, 1H), 2.65 (ddd, *J* = 9.3, 7.1, 4.6 Hz, 1H).

### Example 85 Synthesis of compound AB27191

The structure of compound AB27191 was as follows:

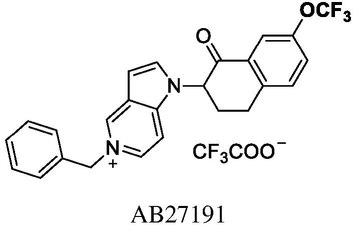

### Step 1):

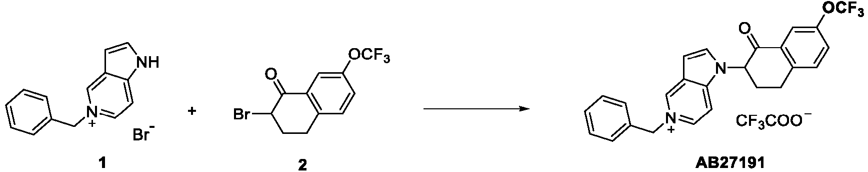

Compound 1 (100 mg, 0.48 mmol) was dissolved in acetonitrile (10 mL), and compound 2 (147 mg, 0.48 mmol) and triethylamine (97 mg, 0.96 mmol) were added. The mixture was replaced with N₂ three times and stirred at 40°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27191.

MS-ESI calculated [M-CF₃COO⁻]+437.15, Found: 437.10.

¹H NMR (400 MHz, dmso-d6) δ 9.57 (s, 1H), 8.70 (d, J = 6.8 Hz, 1H), 8.17 (d, J = 6.9 Hz, 1H), 8.05 (s, 1H), 7.67 (dd, J = 21.9, 7.7 Hz, 3H), 7.48 (d, J = 6.5 Hz, 2H), 7.40 (d, J = 7.3 Hz, 3H), 7.16 (s, 1H), 6.11 (d, J = 12.4 Hz, 1H), 5.79 (s, 2H), 3.25 (d, J = 16.5 Hz, 2H), 2.98 (d, J = 7.8 Hz, 1H), 2.44 - 2.38 (m, 1H).

### Example 86 Synthesis of compound AB27192

The structure of compound AB27192 was as follows:

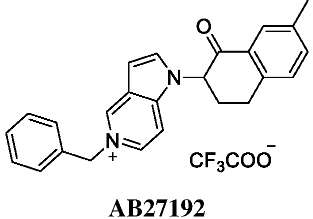

### Step 1):

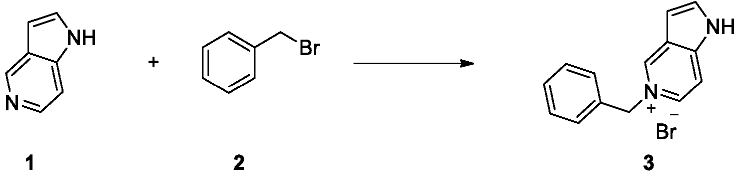

Compound 1 (1 g, 8.4 mmol) was dissolved in dried toluene (20 mL), and compound 2 (1.45 g, 8.4 mmol) was added. The mixture was replaced with N₂ three times and stirred at 105°C for 16 h, new spot was detected on a plate. The raction mixture was concentrated under reduced pressure to remove the organic solvent, the residue was purified by thin layer chromatography (methanol: dichloromethane = 0-10%) to afford compound 3 (1.2 g).

### Step 2):

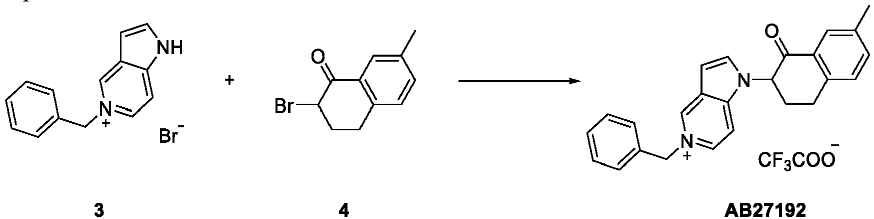

Compound 3 (100 mg, 0.48 mmol) was dissolved in acetonitrile (10 mL), and triethylamine (200 mg, 2 mmol) and compound 4 (285 mg, 1.20 mmol) were added. The mixture was replaced with N₂ three times and stirred at 40°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27192.

MS-ESI and ¹H NMR of the compound AB27192 were as follows:
MS-ESI calculated [M- CF₃COO⁻]⁺367.18, Found: 367.20
¹H NMR (400 MHz, DMSO-d6) δ 9.56 (s, 1H), 8.68 (d, *J =* 6.7 Hz, 1H), 8.17 (d, *J =* 6.2 Hz, 1H), 8.05 (s, 1H), 7.68 (s, 1H), 7.48 (s, 3H), 7.44 - 7.35 (m, 4H), 7.15 (s, 1H), 6.04 (d, *J =* 13.2 Hz, 1H), 5.79 (s, 2H), 3.15 (d, *J =* 12.7 Hz, 2H), 2.91 (d, *J =* 11.2 Hz, 2H), 2.32 (s, 3H).

### Example 87 Synthesis of compound AB27193

The structure of compound AB27193 was as follows:

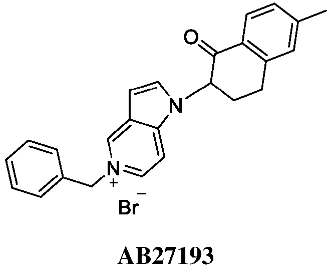

### Step 1):

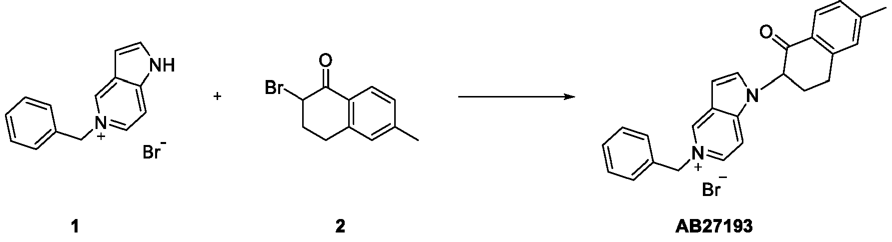

Compound 1 (104 mg, 0.5 mmol) was dissolved in acetonitrile (10 mL), and triethylamine (101.19 mg, 1 mmol) and compound 2 (119 mg, 0.5 mmol) were added. The mixture was replaced with N₂ three times and stirred at room temperature for overnight, new spot was detected on a plate. The raction mixture was concentrated under reduced pressure to remove the organic solvent, the residue was purified by thin layer chromatography to afford compound AB27193.
MS-ESI calculated [M-Br⁻]⁺ 367.47, Found: 367.10
¹H NMR (400 MHz, CD₃OD) δ 9.37 (s, 1H), 8.47 (s, 1H), 8.01 (d, *J =* 6.6 Hz, 1H), 7.90 (s, 2H), 7.45 (s, 5H), 7.30 - 7.18 (m, 2H), 7.14 (s, 1H), 5.79 (s, 2H), 3.45 (s, 1H), 3.21 (s, 2H), 2.89 (s, 1H), 2.60 (d, *J =* 11.9 Hz, 1H), 2.42 (s, 3H).

### Example 88 Synthesis of compound AB27199

The structure of compound AB27199 was as follows:

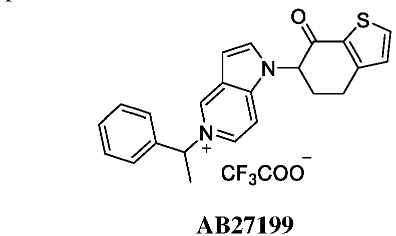

### Step 1):

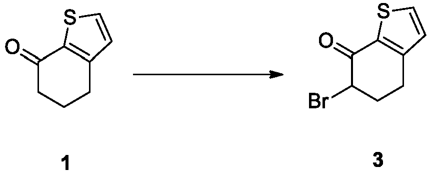

Compound 1 (200 mg, 1.3 mmol) was dissolved in dichloromethane (20 mL), two drops of methanol were added dropwise, and bis(N,N-dimethylacetamide)hydrogendibromobromate (540 mg, 1.3 mmol) was added. The mixture was stirred at 40 °C for overnight, new spot was detected on a plate. The raction mixture was rotary dried, the residue was purified by thin layer chromatography to afford compound 3 (200 mg).

### Step 2):

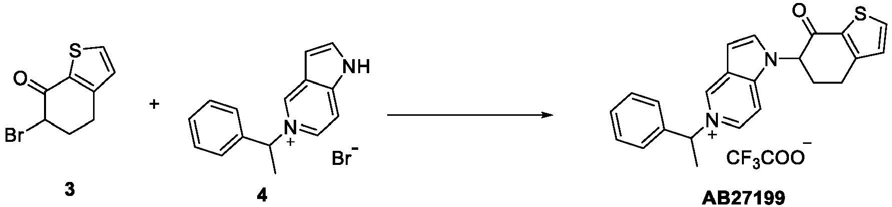

Compound 3 (200 mg, 1.3 mmol) was dissolved in acetonitrile (20 mL), and compound 4 (290 mg, 1.3 mmol) was added. The mixture was stirred at 80°C for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27199.

MS-ESI and ¹H NMR of the compound AB27199 were as follows:
MS-ESI calculated [M- CF₃COO⁻] ⁺ 373.14, Found: 373.05.
¹H NMR (400 MHz, DMSO) δ 9.62 (s, 1H), 8.73 (d, *J* = 6.9 Hz, 1H), 8.17 (dd, *J* = 13.2, 5.7 Hz, 2H), 8.10 (d, *J =* 2.8 Hz, 1H), 7.52 (d, *J =* 7.2 Hz, 2H), 7.47 - 7.36 (m, 3H), 7.25 (d, *J =* 4.7 Hz, 1H), 7.14 (s, 1H), 6.21 - 6.06 (m, 2H), 3.18 (d, *J =* 12.0 Hz, 2H), 3.03 - 2.93 (m, 1H), 2.49 (s, 1H), 2.06 (d, *J =* 6.7 Hz, 3H).

### Example 89 Synthesis of compound AB27214

The structure of compound AB27214 was as follows:

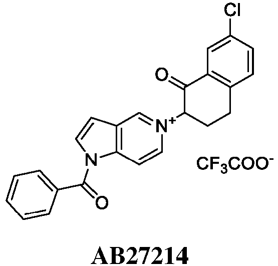

### Step 1):

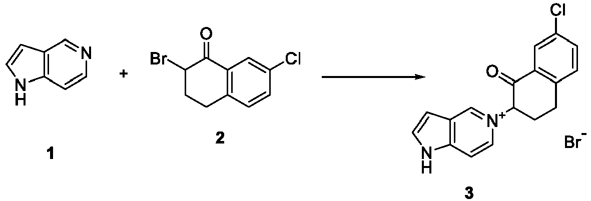

Compound 1 (236 mg, 2 mmol) was dissolved in acetonitrile (10 mL), compound 2 (516 mg, 2 mmol) was added. The mixture was stirred at 80 °C for 2 h, new spot was detected on a plate. The mixture was filtered to afford compound 3 (150 mg).

### Step 2):

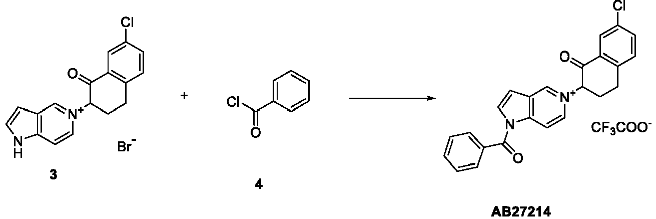

Compound 3 (100 mg, 0.34 mmol) was dissolved in dichloromethane (10 mL), compound 4 (47.6 mg, 0.34 mmol) and triethylamine (103 mg, 1.02 mmol) were added. The mixture was stirred at room temperature for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27214.

MS-ESI calculated [M-CF₃COO⁻]⁺: 401.86, Found: 401.

¹H NMR (399 MHz, CD3OD) δ 9.42 (s, 1H), 8.52 (d, *J* = 7.0 Hz, 1H), 7.98 - 7.93 (m, 3H), 7.82 (d, *J =* 3.3 Hz, 1H), 7.64 (t, *J =* 7.4 Hz, 1H), 7.46 (t, *J =* 7.7 Hz, 2H), 7.42 - 7.36 (m, 2H), 7.24 (s, 1H), 7.04 (d, *J=* 3.1 Hz, 1H), 3.33 (s, 3H), 3.19 (t, *J=* 8.1 Hz, 2H).

### Example 90 Synthesis of compound AB27203

The structure of compound AB27203 was as follows:

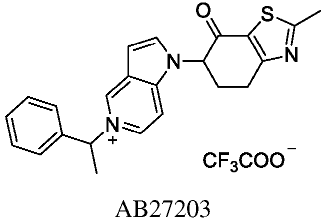

### Step 1):

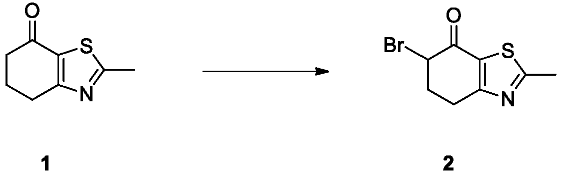

Compound 1 (167 mg, 1 mmol) was dissolved in dichloromethane (10 mL), two drops of methanol were added, and bis(N,N-dimethylacetamide)hydrogendibromobromate (207 mg, 0.5 mmol) was added. The mixture was stirred at 50 °C for overnight, new spot was detected on a plate. The raction mixture was rotary dried, the residue was purified by thin layer chromatography to afford compound 3 (170 mg).

### Step 2):

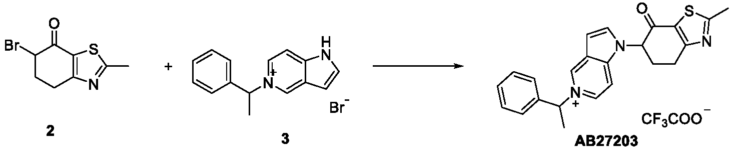

Compound 2 (170 mg, 0.68 mmol) was dissolved in acetonitrile (20 mL), compound 3 (153 mg, 0.68 mmol) and triethylamine (0.3 mL) were added. The mixture was stirred at 40 °C for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27203 (10 mg).

MS-ESI and ¹H NMR of the compound AB27203 were as follows:
MS-ESI calculated [M-CF₃COO⁻]⁺ 388.51, Found: 388.51.
¹H NMR (400 MHz, DMSO) δ 9.61 (s, 1H), 8.72 (d, *J =* 6.2 Hz, 1H), 8.15 (d, *J =* 5.0 Hz, 1H), 8.07 (s, 1H), 7.49 (s, 2H), 7.40 (d, *J* = 7.1 Hz, 3H), 7.13 (s, 1H), 6.13 (dd, *J* = 20.2, 10.7 Hz, 2H), 3.26 (s, 3H), 2.98 (s, 1H), 2.76 (s, 3H), 2.05 (d, *J =* 5.2 Hz, 3H).

### Example 91 Synthesis of compound AB27205

The structure of compound AB27205 was as follows:

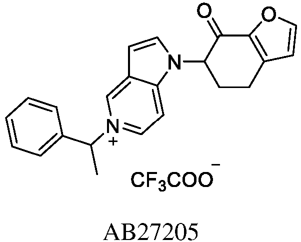

### Step 1):

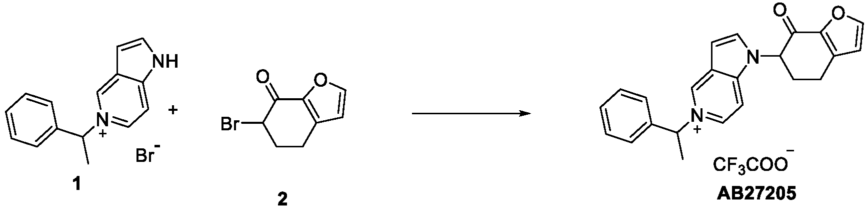

Compound 1 (80 mg, 0.36 mmol) was dissolved in acetonitrile (20 mL), and compound 2 (84 mg, 0.36 mmol) was added. The mixture was stirred at 80 °C for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27205.

MS-ESI calculated [M-CF₃COO⁻] ⁺ 357.16, Found: 357.10.

¹H NMR (400 MHz, DMSO) δ 9.60 (s, 1H), 8.70 (s, 1H), 8.16 (s, 3H), 8.08 (d, *J* = 12.2 Hz, 1H), 7.49 (s, 2H), 7.40 (s, 3H), 7.12 (s, 1H), 6.77 (s, 1H), 6.16 (s, 1H), 6.02 (s, 1H), 2.99 (d, *J=* 1.6 Hz, 1H), 2.91 (s, 0H), 2.41 - 2.34 (m, 0H), 2.04 (s, 3H).

### Example 92 Synthesis of compound AB27314

The structure of compound AB27314 was as follows:

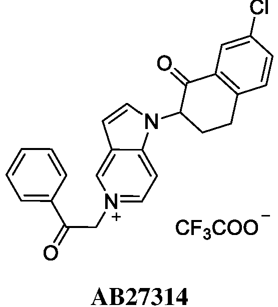

### Step 1):

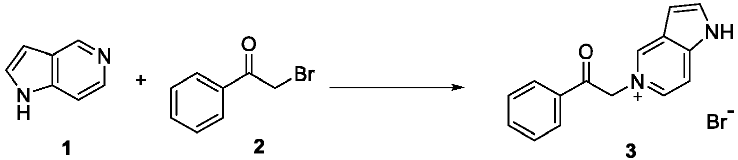

Compound 1 (236 mg, 2 mmol) was dissolved in acetonitrile (10 mL), and compound 2 (740 mg, 4 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound 3 (200 mg).

### Step 2):

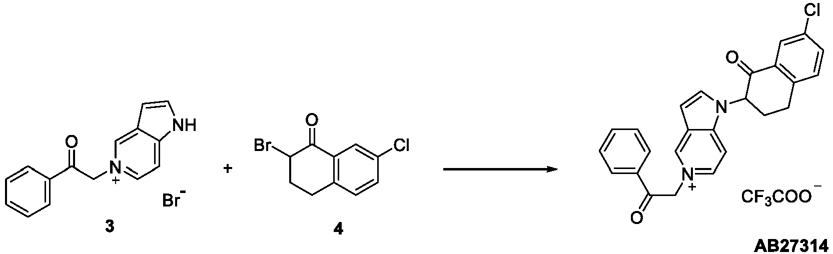

Compound 3 (300 mg, 1.35 mmol) was dissolved in acetonitrile (10 mL), compound 4 (348 mg, 1.35 mmol) was added, and triethylamine (410 mg, 4.05 mmol) was added. The mixture was replaced with N₂ three times and stirred at 40 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27314.

MS-ESI and ¹H NMR of the compound AB27314 were as follows:
MS-ESI calculated [M-CF₃COO⁻] ⁺ 415.90, Found: 415.10.
¹H NMR (400 MH_{Z},CD₃OD) δ 9.16 (s, 1H), 8.34 (d, *J =* 6.5 Hz, 1H), 8.12 (d, *J =* 7.6 Hz, 2H), 8.07 (d, *J =* 6.9 Hz, 1H), 7.97 - 7.91 (m, 2H), 7.74 (t, *J =* 7.3 Hz, 1H), 7.61 (t, *J =* 7.8 Hz, 3H), 7.47 (d, *J =* 8.3 Hz, 1H), 7.17 (d, *J =* 3.2 Hz, 1H), 6.34 (s, 2H), 5.95 (dd, *J =* 14.0, 4.4 Hz, 1H), 3.46 (d, *J* = 12.7 Hz, 1H), 3.31 (s, 1H), 2.96 (d, *J =* 7.8 Hz, 1H), 2.67 (s, 1H).

### Example 93 Synthesis of compound AB27240

The structure of compound AB27240 was as follows:

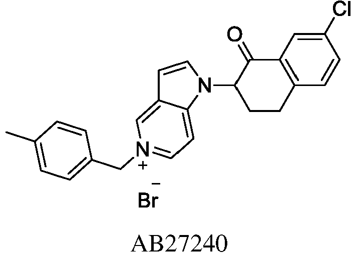

### Step 1):

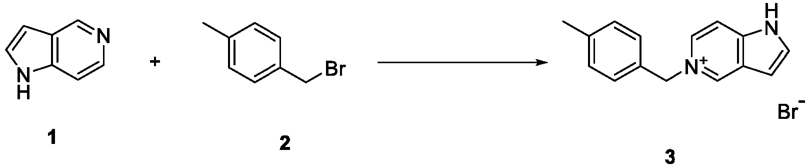

Compound 1 (236 mg, 2 mmol) was dissolved in acetonitrile (10 mL), and compound 2 (370 mg, 2 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound 3 (275 mg).

MS-ESI calculated [M+1]⁺: 222, Found: 222.

### Step 2):

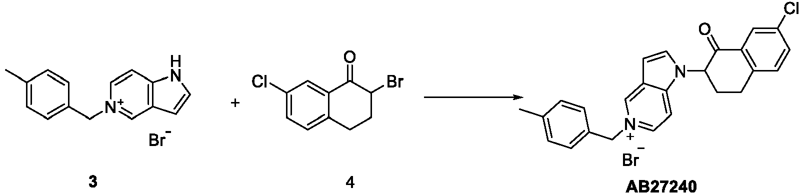

Compound 3 (111 mg, 0.5 mmol) was dissolved in acetonitrile (10 mL), compound 4 (129 mg, 0.5 mmol) was added, and triethylamine (101.19 mg, 1 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (dichloromethane: methanol = 10:1) to afford compound AB27240.

MS-ESI and ¹H NMR of the compound AB27240 were as follows:
MS-ESI calculated [M-Br⁻]⁺: 401.91, Found: 401.25.
¹H NMR (400 MHz, DMSO) δ 9.54 (s, 1H), 8.67 (d, *J =* 6.7 Hz, 1H), 8.15 (d, *J =* 6.7 Hz, 1H), 8.03 (s, 1H), 7.86 - 7.68 (m, 2H), 7.54 (d, *J =* 8.2 Hz, 1H), 7.39 (d, *J =* 7.4 Hz, 2H), 7.24 - 7.13 (m, 3H), 6.08 (d, *J* = 10.4 Hz, 1H), 5.74 (s, 2H), 3.25 - 3.13 (m, 3H), 3.01 - 2.86 (m, 1H), 2.27 (s, 3H).

### Example 94 Synthesis of compound AB27241

The structure of compound AB27241 was as follows:

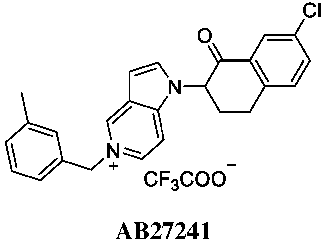

### Step 1):

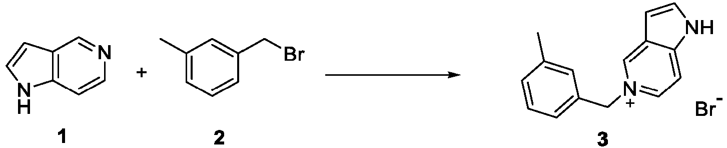

Compound 1 (236 mg, 2 mmol) was dissolved in acetonitrile (10 mL), and compound 2 (740 mg, 4 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound 3 (300 mg).

### Step 2):

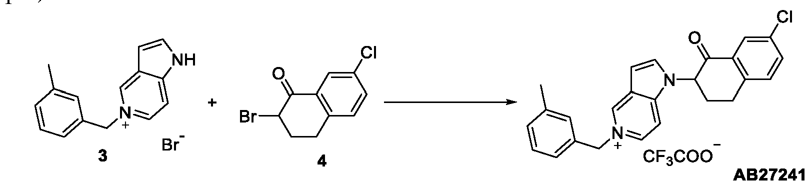

Compound 3 (300 mg, 1.35 mmol) was dissolved in acetonitrile (10 mL), compound 4 (348 mg, 1.35 mmol) was added, and triethylamine (410 mg, 4.05 mmol) was added. The mixture was replaced with N₂ three times and stirred at 40 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27241.

MS-ESI and ¹H NMR of the compound AB27241 were as follows:
MS-ESI calculated [M-CF₃COO⁻] ⁺ 401.14, Found: 401.25.
¹H NMR (400 MHz, DMSO) δ 9.56 (s, 1H), 8.68 (s, 1H), 8.17 (s, 1H), 8.04 (s, 1H), 7.77 (d, *J* = 30.6 Hz, 2H), 7.54 (d, *J =* 7.2 Hz, 1H), 7.30 (d, *J =* 15.8 Hz, 3H), 7.17 (s, 2H), 6.09 (d, *J =* 9.7 Hz, 1H), 5.75 (s, 2H), 3.59-3.19 (m, 3H), 2.94 (s, 1H), 2.28 (s, 3H).

### Example 95 Synthesis of compound AB27242

The structure of compound AB27242 was as follows:

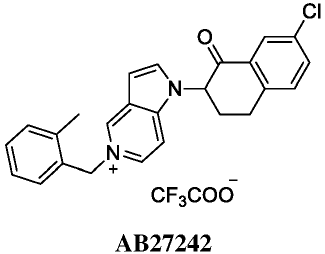

### Step 1):

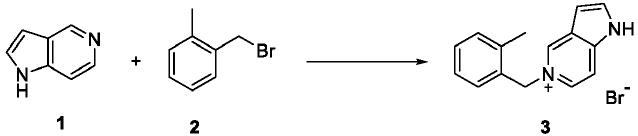

Compound 1 (236 mg, 2 mmol) was dissolved in acetonitrile (10 mL), and compound 2 (740 mg, 4 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound 3 (300 mg).

MS-ESI calculated [M+1]⁺ 223.29, Found: 223.

### Step 2):

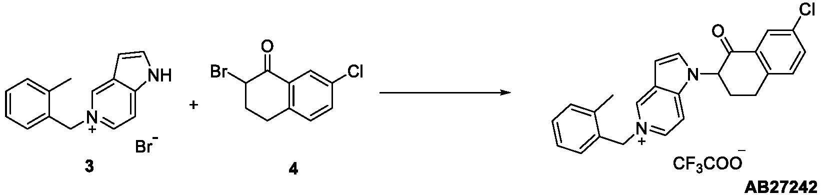

Compound 3 (300 mg, 1.35 mmol) was dissolved in acetonitrile (10 mL), compound 4 (348 mg, 1.35 mmol) was added, and triethylamine (410 mg, 4.05 mmol) was added. The mixture was replaced with N₂ three times and stirred at 40 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27242 (10 mg).

MS-ESI and ¹H NMR of the compound AB27242 were as follows:
MS-ESI calculated [M-CF₃COO⁻]⁺: 401.14, Found: 401.25.
¹H NMR (400 MHz, CD3OD) δ 9.24 (s, 1H), 8.40 (d, *J* = 6.8 Hz, 1H), 8.03 (d, *J =* 7.0 Hz, 1H), 7.96 - 7.91 (m, 2H), 7.63 (d, *J* = 6.8 Hz, 1H), 7.48 (d, *J* = 8.2 Hz, 1H), 7.37 - 7.28 (m, 3H), 7.16 (dd, *J* = 16.3, 50 Hz, 2H), 5.94 (dd, *J =* 14.0, 4.2 Hz, 1H), 5.87 (s, 2H), 3.45 (d, *J =* 13.0 Hz, 1H), 3.28 - 3.24 (m, 1H), 3.00 - 2.92 (m, 1H), 2.64 (d, *J=* 9.4 Hz, 1H), 2.32 (s, 3H).

### Example 96 Synthesis of compound AB27243

The structure of compound AB27243 was as follows:

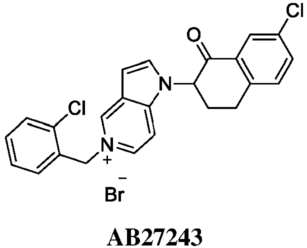

### Step 1):

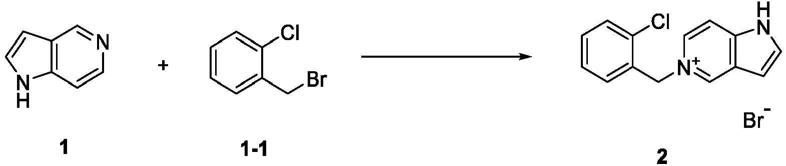

Compound 1 (118.14 mg, 1 mmol) was dissolved in acetonitrile (10 mL), and compound 1-1 (205 mg, 2 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound 2 (203 mg).

### Step 2):

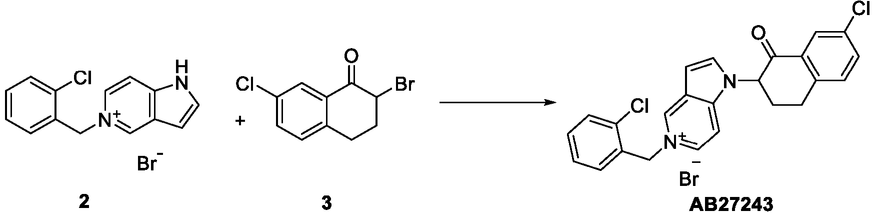

Compound 2 (100 mg, 0.41 mmol) was dissolved in acetonitrile (10 mL), compound 3 (105 mg, 0.41 mmol) was added, and triethylamine (83 mg, 0.82 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (dichloromethane: methanol = 10:1) to afford compound AB27243.

MS-ESI and ¹H NMR of the compound AB27243 were as follows:
MS-ESI calculated [M-Br]⁺: 422.33, Found: 421.
¹H NMR (400 MHz, CD3OD) δ 9.35 (s, 1H), 8.46 (s, 1H), 7.99 (d, *J* = 44.6 Hz, 3H), 7.63 (d, *J* = 4.1 Hz, 1H), 7.51 (d, *J =* 24.2 Hz, 5H), 7.16 (s, 1H), 5.95 (s, 3H), 3.45 (d, *J =* 13.5 Hz, 2H), 2.95 (d, *J =* 10.7 Hz, 1H), 2.64 (d, *J =* 4.1 Hz, 1H).

### Example 97 Synthesis of compound AB27244

The structure of compound AB27244 was as follows:

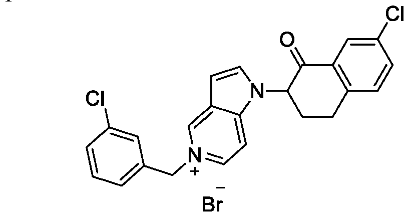

### AB27244

### Step 1):

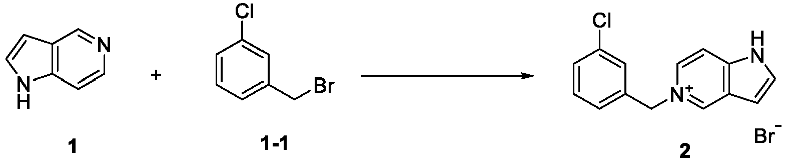

Compound 1 (118.14 mg, 1 mmol) was dissolved in acetonitrile (10 mL), and compound 1-1 (205 mg, 1 mmol) was added. The mixture was stirred at room temperature for 90 min, new spot was detected on a plate. The raction mixture was concentrated under reduced pressure to remove the organic solvent, the residue was purified by thin layer chromatography (methanol: dichloromethane = 10 %) to afford compound 2 (200 mg).

### Step 2):

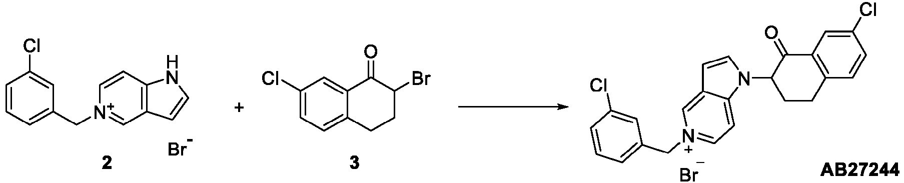

Compound 2 (122 mg, 0.5 mmol) was added to acetonitrile (10 mL), and compound 3 (129 mg, 0.5 mmol) was added. The mixture was stirred at room temperature for overnight, new spot was detected on a plate. The raction mixture was concentrated under reduced pressure to remove the organic solvent to afford crude product. The crude product was triturated with methanol and filtered, the filtrate was concentrated under reduced pressure to remove the organic solvent, the residue was purified by thin layer chromatography (methanol: dichloromethane =10%) to afford compound AB 27244.

MS-ESI and ¹H NMR of the compound AB27244 were as follows:
MS-ESI calculated [M-Br]⁺: 422.33, Found: 422.
¹H NMR (400 MHz, CD3OD) δ 9.41 (s, 1H), 8.52 (d, *J* = 5.3 Hz, 1H), 8.06 (d, *J* = 5.8 Hz, 1H), 7.94 (s, 2H), 7.63 (d, *J =* 7.4 Hz, 1H), 7.55 (s, 1H), 7.44 (t, *J =* 14.6 Hz, 4H), 7.17 (s, 1H), 5.96 (d, *J* = 13.1 Hz, 1H), 5.81 (s, 2H), 3.47 (t, *J =* 14.4 Hz, 1H), 3.28 - 3.25 (m, 1H), 2.98-2.89 (m, 1H), 2.63 (d, *J =* 10.3 Hz, 1H).

### Example 98 Synthesis of compound AB27245

The structure of compound AB27245 was as follows:

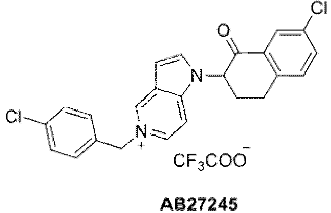

### Step 1):

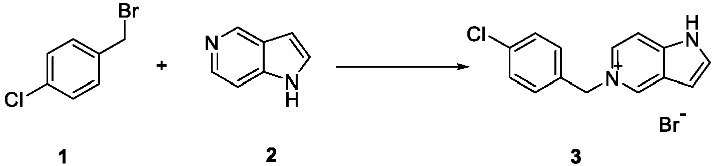

Compound 1 (410 mg, 2 mmol) was dissolved in acetonitrile (10 mL), and compound 2 (118 mg, 1 mmol) was added. The mixture was stirred at 80°C for 2 h, new spot was detected on a plate. The raction mixture was concentrated under reduced pressure to remove the organic solvent, the residue was purified by thin layer chromatography (methanol: dichloromethane = 0-10 %) to afford compound 3 (120 mg).

### Step 2):

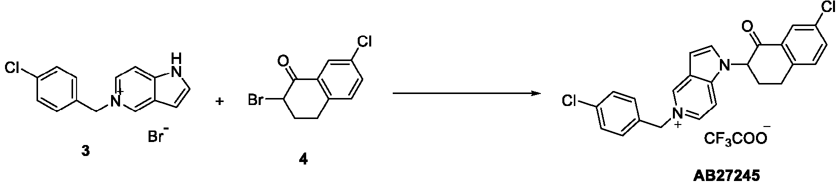

Compound 3 (120 mg, 0.49 mmol) was dissolved in acetonitrile (5 mL), and compound 4 (193 mg, 0.75 mmol) was added. The mixture was replaced with N₂ three times and stirred at 40 °C for overnight, new spot was detected on a plate. The raction mixture was concentrated under reduced pressure to remove the organic solvent, the residue was purified by preparation chromatography (column: Sharpsil-U C18 (250 mm × 30 × 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27245.

MS-ESI and ¹H NMR of the compound AB27245 were as follows:
MS-ESI calculated [M-CF₃COO⁻]⁺: 422.33, Found: 421.10.
¹H NMR (400 MHz, DMSO) δ 9.56 (s, 1H), 8.69 (d, *J =* 6.7 Hz, 1H), 8.18 (d, *J =* 6.8 Hz, 1H), 8.05 (s, 1H), 7.81 (s, 1H), 7.74 (d, *J* = 8.1 Hz, 1H), 7.57 - 7.47 (m, 5H), 7.17 (s, 1H), 6.14 - 6.05 (m, 1H), 5.80 (s, 2H), 3.42 - 3.29 (m, 2H), 3.22 (d, *J =* 15.9 Hz, 1H), 2.99-2.91 (m, 1H).

### Example 99 Synthesis of compound AB27246

The structure of compound AB27246 was as follows:

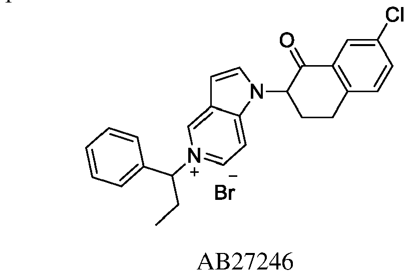

### Step 1):

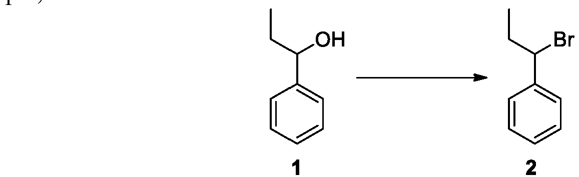

Compound 1 (1 g, 7.35 mmol) was dissolved in dichloromethane (5 mL), and phosphorus tribromide (5.9 g, 22.05 mmol) was slowly added dropwise under ice bath. The mixture was replaced with N₂ three times, naturally heated to room temperature and stirred for 30 min, new spot was detected on a plate. The raction mixture was quenched with saturated aqueous sodium bicarbonate (10 ml) and then extracted with dichloromethane (10 mL × 3), the combined organic phase was washed with saturated saline (100 mL), dried over anhydrous sodium sulfate, filtered and concentrated under reduced pressure to remove the organic solvent to afford compound 2 (1.4 g).

### Step 2):

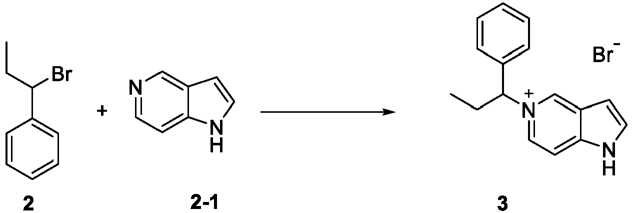

Compound 2-1 (118 mg, 1 mmol) was dissolved in acetonitrile (10 mL), and compound 2 (440 mg, 2 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (dichloromethane: methanol = 10:1) to afford compound 3 (200 mg).

### Step 3):

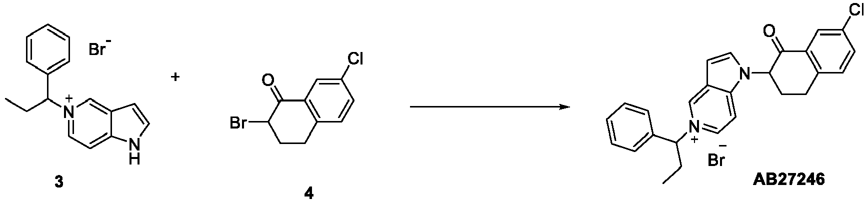

Compound 3 (200 mg, 0.84 mmol) was dissolved in acetonitrile (10 mL), compound 4 (273 mg, 1.27 mmol) was added, and triethylamine (254 mg, 2.52 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography (dichloromethane: methanol = 10:1) to afford compound AB27246.

MS-ESI and ¹H NMR of the compound AB27246 were as follows:
MS-ESI calculated [M-Br]⁺: 415.93, Found: 415.30.
¹H NMR (400 MHz, DMSO) δ 9.66 (s, 1H), 8.77 (s, 1H), 8.18 (d, *J =* 6.1 Hz, 1H), 8.05 (d, *J* = 2.8 Hz, 1H), 7.80 (s, 1H), 7.74 (d, *J =* 8.0 Hz, 1H), 7.56 (dd, *J =* 21.5, 7.6 Hz, 3H), 7.41 (dt, *J=* 13.4, 6.8 Hz, 3H), 7.14 (s, 1H), 6.10 (d, *J =* 10.2 Hz, 1H), 5.89 (t, *J* = 7.4 Hz, 1H), 3.29 (s, 2H), 3.23 (s, 1H), 2.98-2.89 (m, 1H), 2.44 - 2.41 (m, 2H), 0.85 (s, 3H).

### Example 100 Synthesis of compound AB27271

The structure of compound AB27271 was as follows:

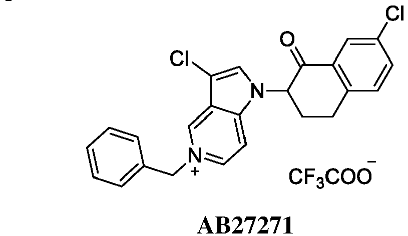

### Step 1):

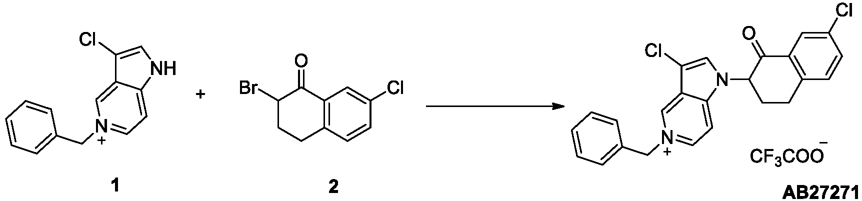

Compound 1 (243 mg, 1 mmol) was dissolved in acetonitrile (5 mL), compound 2 (259 mg, 1 mmol) was added, and triethylamine (303 mg, 3 mmol) was added. The mixture was replaced with N₂ three times and stirred at 40°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27271.

MS-ESI calculated [M-CF₃COO⁻]⁺: 422.33, Found: 422.20.

¹H NMR (400 MHz, CD3OD) δ 9.55 (s, 1H), 8.55 (s, 1H), 8.09 (s, 2H), 7.94 (s, 1H), 7.62 (s, 1H), 7.47 (d, *J* = 7.6 Hz, 6H), 5.93 (d, *J =* 13.2 Hz, 1H), 5.84 (s, 2H), 3.50 - 3.39 (m, 1H), 3.12 (s, 1H), 2.91 (d, *J =* 11.9 Hz, 1H), 2.63 (s, 1H).

### Example 101 Synthesis of compound AB27290

The structure of compound AB27290 was as follows:

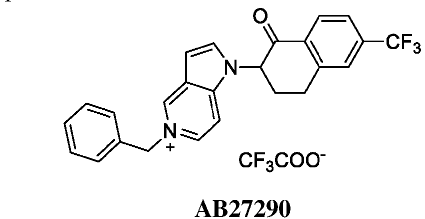

### Step 1):

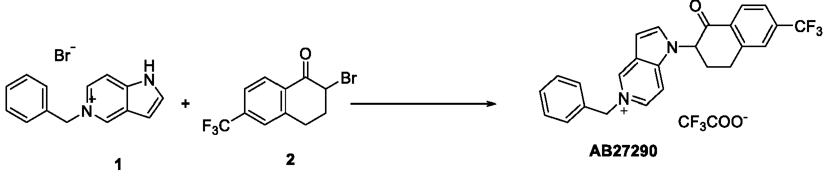

Compound 1 (105 mg, 0.5 mmol) was dissolved in acetonitrile (10 mL), compound 2 (147 mg, 0.5 mmol) and triethylamine (152 mg, 1.5 mmol) were added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm × 30 × 10 um, 100 A); mobile phase: 0.1% aqueous trifluoroacetic acid + acetonitrile; gradient elution: 1-20 min, 5-25% ACN; 20-50 min, 25-35% ACN) to afford compound AB27290.

MS-ESI calculated [M-CF₃COO⁻]⁺: 421.43, Found: 421.00.

¹H NMR (400 MHz, CDsOD) δ 9.39 (s, 1H), 8.51 (d, *J =* 6.9 Hz, 1H), 8.17 (d, *J =* 8.1 Hz, 1H), 8.04 (d, *J =* 6.9 Hz, 1H), 7.93 (d, *J =* 3.2 Hz, 1H), 7.82 (s, 1H), 7.71 (d, *J =* 8.0 Hz, 1H), 7.45 (s, 5H), 7.16 (d, *J =* 3.0 Hz, 1H), 5.99 (dd, *J =* 13.9, 4.3 Hz, 1H), 5.80 (s, 2H), 3.54 (d, *J =* 12.8 Hz, 1H), 3.39 (d, *J =* 17.7 Hz, 1H), 3.07 - 2.89 (m, 1H), 2.66 (d, *J =* 12.3 Hz, 1H).

### Example 102 Synthesis of compound AB27291

The structure of compound AB27291 was as follows:

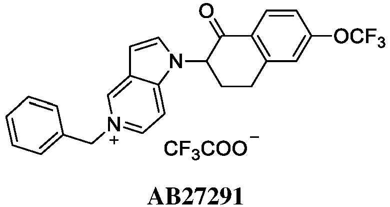

### Step 1:

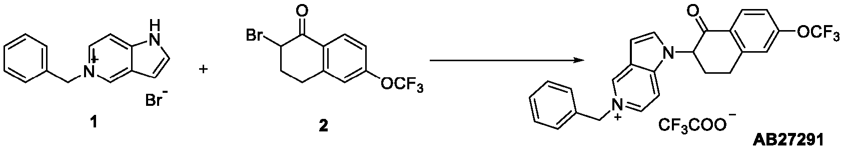

Compound 1 (200 mg, 1 mmol) was dissolved in acetonitrile (10 mL), compound 2 (340 mg, 1.1 mmol) was added, and triethylamine (404 g, 4 mmol) was added. The mixture was stirred at 30°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27291.

MS-ESI and ¹H NMR of the compound AB27291 were as follows:
MS-ESI calculated [M-CF₃COO⁻]⁺: 437.43, Found: 437.15.
¹H NMR (400 MHz, DMSO) δ 9.60 (s, 1H), 8.72 (d, *J =* 6.6 Hz, 1H), 8.18 (d, *J =* 6.8 Hz, 1H), 8.03 (dd, *J* = 17.9, 5.6 Hz, 2H), 7.56 - 7.47 (m, 3H), 7.41 (d, *J* = 7.6 Hz, 4H), 7.17 (d, *J* = 2.3 Hz, 1H), 6.20 - 6.06 (m, 1H), 5.81 (s, 2H), 3.87 - 3.72 (m, 1H), 3.33 (dd, *J =* 45.7, 15.0 Hz, 2H), 2.98 (d, *J =* 10.2 Hz, 1H).

### Example 103 Synthesis of compound AB27313

The structure of compound AB27313 was as follows:

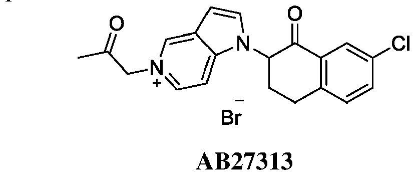

### Step 1):

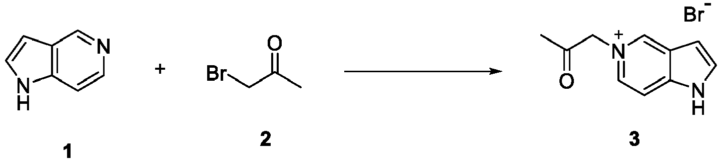

Compound 1 (236 mg, 2 mmol) was dissolved in acetonitrile (10 mL), compound 2 (274 mg, 2 mmol) was added. The mixture was stirred at room temperture for 2 h, new spot was detected on a plate. The raction mixture was filtered and concentrated under reduced pressure to remove the organic solvent to afford compound 3 (172 mg).

### Step 2):

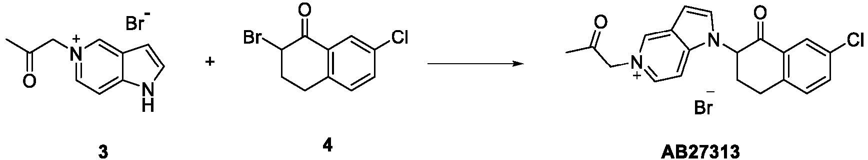

Compound 3 (172 mg, 0.98 mmol) was dissolved in acetonitrile (5 mL), compound 4 (253 mg, 0.98 mmol) was added. The mixture was replaced with N₂ three times and stirred at 40 °C for overnight, new spot was detected on a plate. The raction mixture was concentrated under reduced pressure to remove the organic solvent, the residue was purified by thin layer chromatography (dichloromethane: methanol = 10:1) to afford compound AB27313.

MS-ESI and ¹H NMR of the compound AB27313 were as follows:
MS-ESI calculated [M-Br⁻]⁺: 353.82, Found: 353.20.
¹H NMR (400 MHz, DMSO) δ 9.17 (s, 1H), 8.35 (d, *J =* 6.7 Hz, 1H), 8.18 (d, *J =* 6.6 Hz, 1H), 8.06 (s, 1H), 7.83 (s, 1H), 7.75 (d, *J =* 7.4 Hz, 1H), 7.56 (d, *J =* 8.1 Hz, 1H), 7.21 (s, 1H), 6.12 (d, J = 10.2 Hz, 1H), 5.69 (s, 2H), 3.27 - 3.25 (m, 1H), 3.23 - 3.19 (m, 1H), 2.98 (d, *J =* 11.4 Hz, 1H), 2.51 (s, 1H), 2.30 (s, 3H).

### Example 104 Synthesis of compound AB27316

The structure of compound AB27316 was as follows:

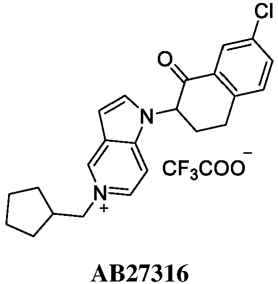

### Step 1):

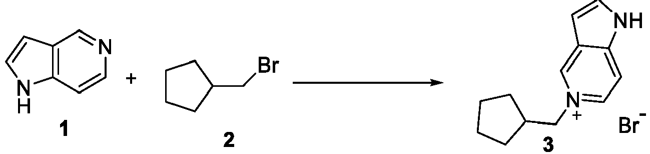

Compound 1 (200 mg, 1.69 mmol) was dissolved in acetonitrile (10 mL), and compound 2 (275 mg, 1.69 mmol) was added at 0 °C. The mixture was replaced with N₂ three times and stirred at 80 °C for overnight, new spot was detected on a plate. The raction mixture was filtered and concentrated under reduced pressure to remove the organic solvent, the residue was purified by thin layer chromatography (dichloromethane: methanol = 10:1) to afford compound 3 (60 mg).

### Step 2):

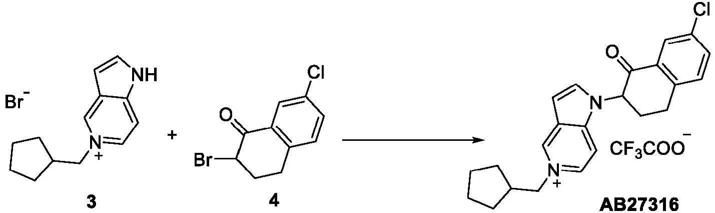

Compound 3 (60 mg, 0.3 mmol) was dissolved in acetonitrile (10 mL), compound 4 (78 mg, 0.3 mmol) was added at 0 °C, and triethylamine (61 mg, 0.6 mmol) was added. The mixture was replaced with N₂ three times and stirred at 80°C for overnight, new spot was detected on a plate. The raction mixture was filtered and concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27316.

MS-ESI and ¹H NMR of the compound AB27316 were as follows:
MS-ESI calculated [M-CF₃COO⁻]⁺: 379.16, Found: 379.30.
¹H NMR (400 MHz, DMSO) δ 9.45 (s, 1H), 8.63 (s, 1H), 8.18 (d, *J =* 4.8 Hz, 1H), 8.04 (s, 1H), 7.82 (s, 1H), 7.74 (d, *J* = 7.5 Hz, 1H), 7.56 (d, *J* = 7.0 Hz, 1H), 7.15 (s, 1H), 6.11 (d, *J* = 13.9 Hz, 1H), 4.52 (d, *J* = 6.1 Hz, 2H), 3.23 (d, *J* = 16.6 Hz, 3H), 2.96 (d, *J* = 11.7 Hz, 1H), 1.67 - 1.49 (m, 6H), 1.25 (d, *J* = 28.9 Hz, 3H).

### Example 105 Synthesis of compound AB27318

The structure of compound AB27318 was as follows:

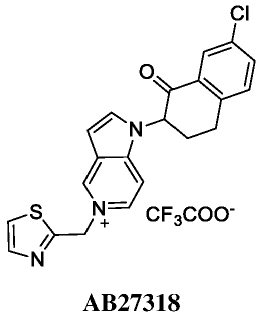

### Step 1:

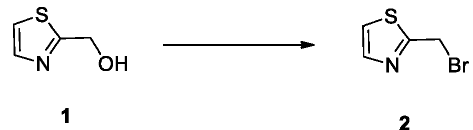

Compound 1 (230.30 mg, 2 mmol) was dissolved in dichloromethane (10 mL), phosphorous oxybromide (1.6 g, 6 mmol) was added at 0 °C. The mixture was stirred at room temperture for 2 h, new spot was detected on a plate. The raction mixture was concentrated under reduced pressure to remove the organic solvent, the residue was purified by thin layer chromatography (dichloromethane: methanol = 10:1) to afford compound 2 (100 mg).

### Step 2):

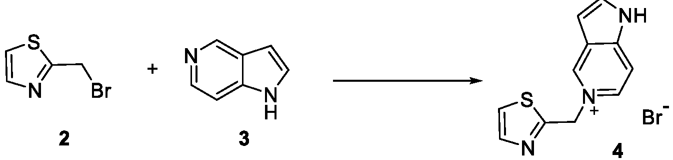

Compound 2 (100 mg, 0.56 mmol) and compound 3 were dissolved in acetonitrile (10 mL). The mixture was replaced with N₂ three times and stirred at room temperture for 2 h, new spot was detected on a plate. The raction mixture was concentrated under reduced pressure to remove the organic solvent, the residue was purified by thin layer chromatography (dichloromethane: methanol = 10:1) to afford compound 4 (70 mg).

### Step 3):

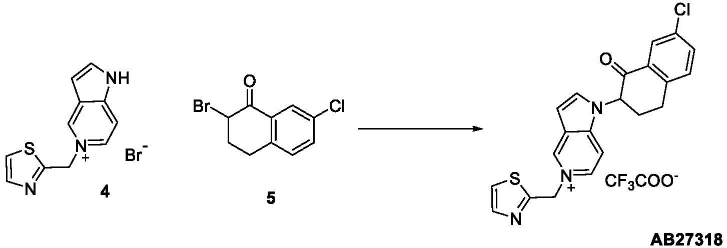

Compound 4 (70 mg, 0.33 mmol) was dissolved in acetonitrile (10 mL), compound 5 (85 mg, 0.33 mmol) and triethylamine (101 mg, 1 mmol) were added. The mixture was stirred at 30°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27318 (5 mg).

MS-ESI and ¹H NMR of the compound AB27318 were as follows:
MS-ESI calculated [M-CF₃COO⁻]⁺: 394.90, Found: 394.05.
¹H NMR (400 MHz,CD₃OD) δ 9.43 (s, 1H), 8.56 (d, *J =* 7.1 Hz, 1H), 8.05 (d, *J =* 7.1 Hz, 1H), 7.95 - 7.90 (m, 2H), 7.83 (d, *J* = 3.2 Hz, 1H), 7.71 (d, *J* = 3.3 Hz, 1H), 7.62 (dd, *J* = 8.3, 2.3 Hz, 1H), 7.46 (d, *J =* 8.3 Hz, 1H), 7.17 (d, *J =* 3.4 Hz, 1H), 6.17 (s, 2H), 5.92 (dd, *J =* 14.0, 4.5 Hz, 1H), 3.45 (td, *J =* 12.8, 6.6 Hz, 1H), 3.26 (d, *J =* 2.7 Hz, 1H), 2.93 (dd, *J =* 13.7, 4.4 Hz, 1H), 2.66-2.58 (m, 1H).

### Example 106 Synthesis of compound AB27345

The structure of compound AB27345 was as follows:

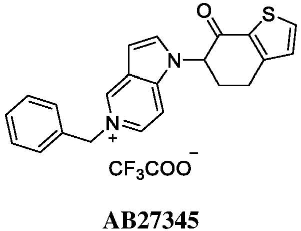

### Step 1):

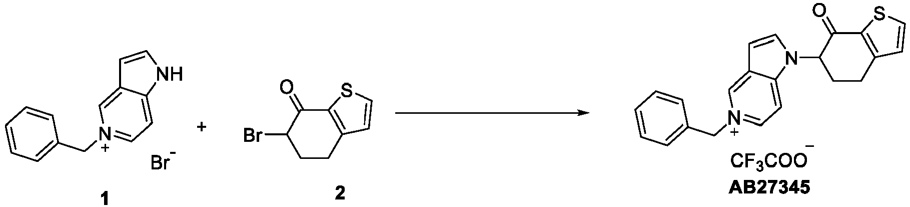

Compound 1 (200 mg, 0.96 mmol) was dissolved in acetonitrile (10 mL), compound 2 (222 mg, 0.96 mmol) was added, and triethylamine (0.4 ml) was added. The mixture was stirred at 40°C for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27345.

MS-ESI calculated [M-CF₃COO⁻]⁺ 359.12, Found: 359.00.

¹H NMR (400 MHz, dmso) δ 9.56 (s, 1H), 8.70 (d, J = 6.2 Hz, 1H), 8.22 - 8.07 (m, 3H), 7.48 (s, 2H), 7.41 (d, J = 7.1 Hz, 3H), 7.25 (d, J = 4.3 Hz, 1H), 7.16 (s, 1H), 6.09 (d, J = 9.6 Hz, 1H), 5.80 (s, 2H), 3.20 (s, 3H), 3.00 (s, 1H).

### Example 107 Synthesis of compound AB27347

The structure of compound AB27347 was as follows:

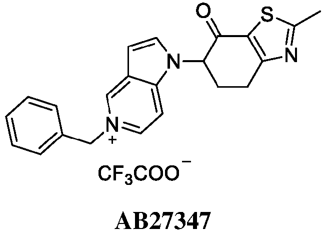

### Step 1:

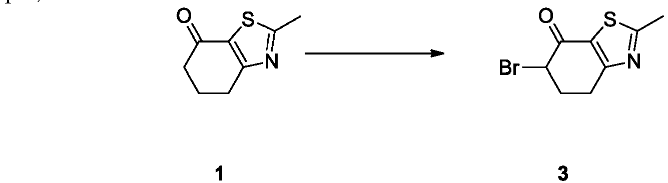

Compound 1 (200 mg, 1.19 mmol) was dissolved in dichloromethane (20 mL), two drops of methanol were added, bis(N,N-dimethylacetamide)hydrogendibromobromate (500 mg, 1.2 mmol) was added. The mixture was stirred at 50 °C for overnight, new spot was detected on a plate. The raction mixture was rotary dried, the residue was purified by thin layer chromatography to afford compound 3 (220 mg).

### Step 2):

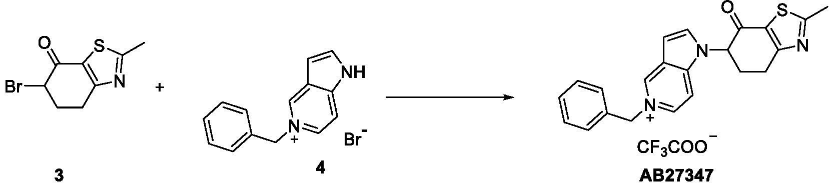

Compound 3 (220 mg, 0.89 mmol) was dissolved in acetonitrile (20 mL), compound 4 (200 mg, 0.89 mmol) was added, and triethylamine (0.4 ml) was added. The mixture was stirred at 40°C for overnight, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27347.

MS-ESI and ¹H NMR of the compound AB27347 were as follows:
MS-ESI calculated [M-CF₃COO⁻] ⁺ 374.48, Found: 374.48.
¹H NMR (400 MHz, dmso) δ 9.58 (s, 1H), 8.72 (d, J = 6.9 Hz, 1H), 8.19 (d, J = 7.0 Hz, 1H), 8.08 (d, J = 3.2 Hz, 1H), 7.50 (d, J = 6.8 Hz, 2H), 7.45 - 7.35 (m, 3H), 7.16 (d, J = 3.0 Hz, 1H), 6.11 (dd, J = 13.5, 4.1 Hz, 1H), 5.80 (s, 2H), 3.27 (d, J = 5.6 Hz, 2H), 3.08 - 2.92 (m, 1H), 2.76 (s, 3H), 2.51 (s, 1H).

### Example 108 Synthesis of compound AB27353

The structure of compound AB27353 was as follows:

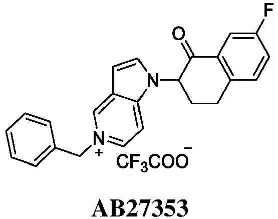

### Step 1):

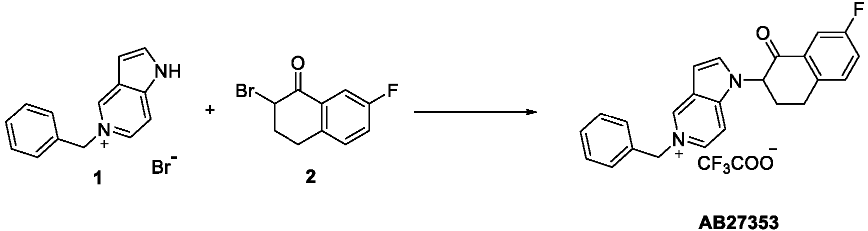

Compound 1 (209 mg, 1 mmol) was dissolved in acetonitrile (10 mL), compound 2 (243 mg, 1 mmol) was added, and triethylamine (303 mg, 3 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27353.

MS-ESI calculated [M-CF₃COO⁻]⁺: 371.43, Found: 371.10.

¹H NMR (399 MHz, dmso) δ 9.56 (s, 1H), 8.69 (d, J = 7.0 Hz, 1H), 8.17 (d, J = 7.0 Hz, 1H), 8.04 (d, J = 3.4 Hz, 1H), 7.55 (d, J = 6.7 Hz, 3H), 7.48 (d, J = 7.2 Hz, 2H), 7.42 - 7.36 (m, 3H), 7.15 (d, J = 3.3 Hz, 1H), 6.08 (dd, J = 13.9, 4.4 Hz, 1H), 5.79 (s, 2H), 3.33 (t, J = 13.0 Hz, 1H), 3.20 (d, J = 15.5 Hz, 1H), 2.94 (dt, J = 12.6, 8.5 Hz, 1H), 2.48 (s, 1H).

### Example 109 Synthesis of compound AB27354

The structure of compound AB27354 was as follows:

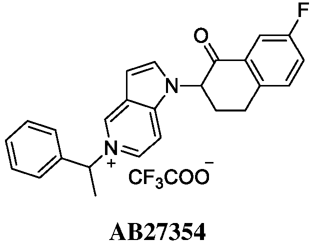

### Step 1):

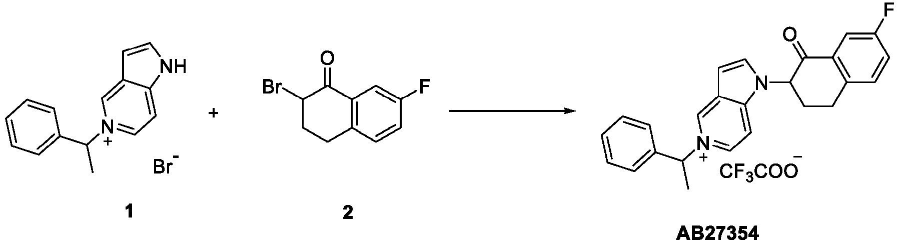

Compound 1 (223 mg, 1 mmol) was dissolved in acetonitrile (10 mL), compound 2 (243 mg, 1 mmol) was added, and triethylamine (303 mg, 3 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27354.

MS-ESI calculated [M-CF₃COO⁻]⁺: 385.45, Found: 385.20.

1H NMR (400 MHz, DMSO) δ 9.63 (s, 1H), 8.72 (d, J = 7.0 Hz, 1H), 8.17 (d, J = 6.9 Hz, 1H), 8.06 (d, J = 3.4 Hz, 1H), 7.56 (d, J = 7.0 Hz, 3H), 7.51 (d, J = 7.3 Hz, 2H), 7.45 - 7.36 (m, 3H), 7.15 (d, J = 3.1 Hz, 1H), 6.17 (d, J = 6.7 Hz, 1H), 6.10 (dd, J = 13.9, 4.2 Hz, 1H), 3.32 (d, J = 13.5 Hz, 1H), 3.21 (d, J = 16.0 Hz, 1H), 2.96 (d, J = 13.5 Hz, 1H), 2.46 - 2.42 (m, 1H), 2.06 (d, J = 6.9 Hz, 3H).

### Example 110 Synthesis of compound AB27459

The structure of compound AB27459 was as follows:

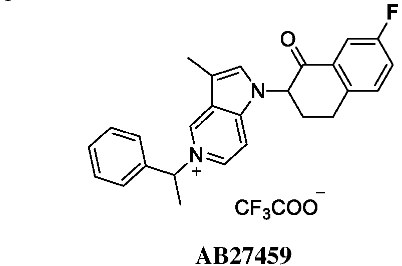

### Step 1):

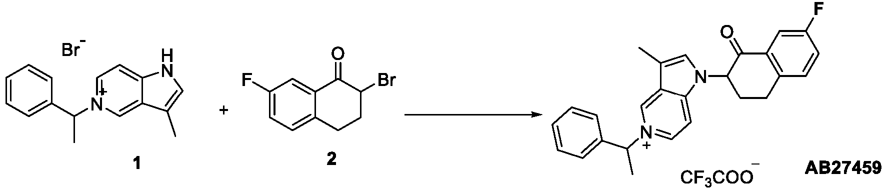

Compound 1 (100 mg, 0.418 mmol) was dissolved in acetonitrile (20 mL), compound 2 (100 mg, 0.418 mol) was added, and N, N-diisopropylethylamine (216 mg, 1.673 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27459.

MS-ESI calculated [M-CF₃COO⁻]⁺: 399.48, Found: 399.35.

¹H NMR (400MHz, DMSO) δ 9.59 (d, *J =* 3.5 Hz, 1H), 8.58 (d, *J =* 7.2 Hz, 1H), 8.04 (d, *J* = 7.2 Hz, 1H), 7.76 (s, 1H), 7.51 (dd, *J* = 7.8, 6.1 Hz, 5H), 7.42 - 7.33 (m, 3H), 6.12 (q, *J =* 7.0 Hz, 1H), 5.99 (dd, *J* = 13.9, 4.5 Hz, 1H), 3.30 (d, *J* = 13.3 Hz, 2H), 3.17 (d, *J* = 16.5 Hz, 1H), 2.85 (dt, 7 = 13.2, 8.7 Hz, 1H), 2.38 (s, 3H), 2.05 (d, 7 = 7.0 Hz, 3H).

### Example 111 Synthesis of compound AB27460

The structure of compound AB27460 was as follows:

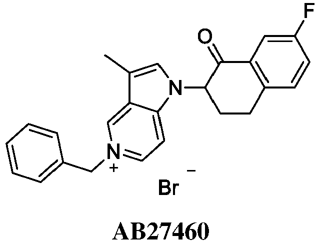

### Step 1):

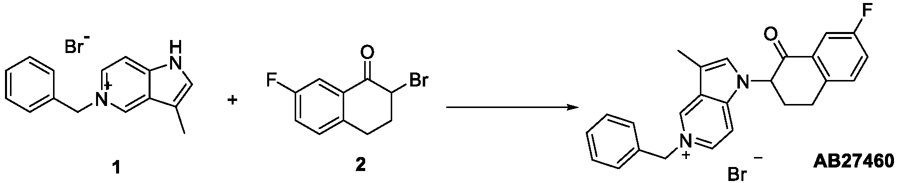

Compound 1 (100 mg, 0.448 mmol) was dissolved in acetonitrile (20 mL), compound 2 (110 mg, 0.448 mol) was added, and N, N-diisopropylethylamine (231 mg, 1.792 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound AB27460.

MS-ESI calculated [M-Br⁻]⁺: 385.45, Found: 385.35.

¹H NMR (400 MHz, DMSO) δ 9.63 (s, 1H), 8.61 (d, *J =* 6.5 Hz, 1H), 8.09 (d, *J =* 7.0 Hz, 1H), 7.79 (s, 1H), 7.54 (d, *J =* 7.7 Hz, 3H), 7.49 (d, *J =* 6.8 Hz, 2H), 7.43 - 7.36 (m, 3H), 6.02 (dd, *J =* 13.7, 4.5 Hz, 1H), 5.77 (s, 2H), 3.22 - 3.14 (m, 2H), 2.87 (d, *J =* 8.9 Hz, 1H), 2.51 (s, 2H), 2.39 (s, 3H)

### Example 112 Synthesis of compound AB27464

The structure of compound AB27464 was as follows:

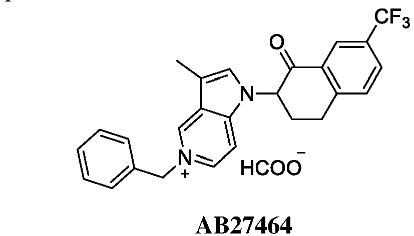

### Step 1):

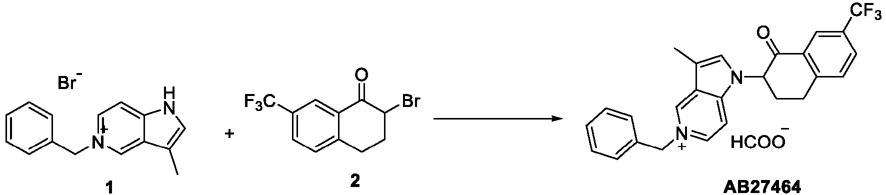

Compound 1 (100 mg, 0.45 mmol) was dissolved in acetonitrile (20 mL), compound 2 (132 mg, 0.45 mol) was added, and N, N-diisopropylethylamine (252 mg, 1.8 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27464.

MS-ESI calculated [M-HCOO⁻]⁺: 435.46, Found: 435.30.

¹H NMR (400 MHz, DMSO) δ 9.67 (s, 1H), 8.63 (d, *J* = 7.1 Hz, 1H), 8.11 (d, *J* = 7.1 Hz, 1H), 8.06 (s, 1H), 8.01 (d, *J =* 8.0 Hz, 1H), 7.79 (s, 1H), 7.73 (d, *J =* 8.2 Hz, 1H), 7.50 (d, *J =* 7.7 Hz, 2H), 7.42 - 7.34 (m, 3H), 6.11 (dd, *J =* 13.9, 4.5 Hz, 1H), 5.78 (s, 2H), 3.68 - 3.38 (m, 2H), 3.10 (d, *J* = 7.3 Hz, 1H), 2.92 (dt, *J =* 12.5, 8.3 Hz, 1H), 2.39 (s, 3H).

### Example 113 Synthesis of compound AB27465

The structure of compound AB27465 was as follows:

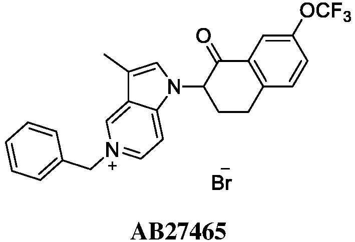

### Step 1):

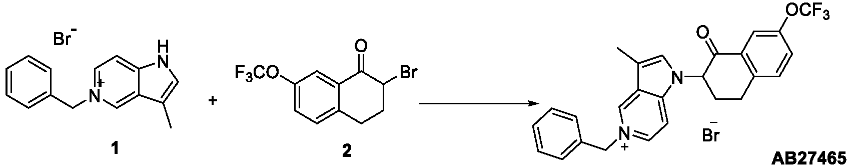

Compound 1 (100 mg, 0. 45 mmol) was dissolved in acetonitrile (20 mL), compound 2 (132 mg, 0.45 mol) was added, and N, N-diisopropylethylamine (252 mg, 1.8 mmol) was added. The mixture was replaced with N₂ three times and stirred at 80°C for overnight, new spot was detected on a plate. The raction mixture was concentrated under reduced pressure to remove the organic solvent, the residue was purified by column chromatographyto afford compound AB27465.

MS-ESI calculated [M-Br⁻]⁺: 451.46, Found: 451.30.

¹H NMR (400 MHz, DMSO) δ 9.63 (s, 1H), 8.62 (d, *J =* 7.2 Hz, 1H), 8.08 (d, *J =* 7.0 Hz, 1H), 7.80 (s, 1H), 7.66 (dd, *J* = 19.5, 7.9 Hz, 3H), 7.49 (d, *J* = 6.7 Hz, 2H), 7.43 - 7.37 (m, 3H), 6.06 - 5.98 (m, 1H), 5.77 (s, 2H), 3.27 (s, 3H), 2.91 (d, *J =* 8.4 Hz, 1H), 2.39 (s, 3H).

### Example 114 Synthesis of compound AB27468

The structure of compound AB27468 was as follows:

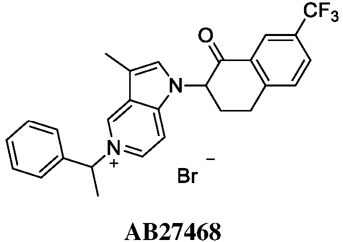

### Step 1):

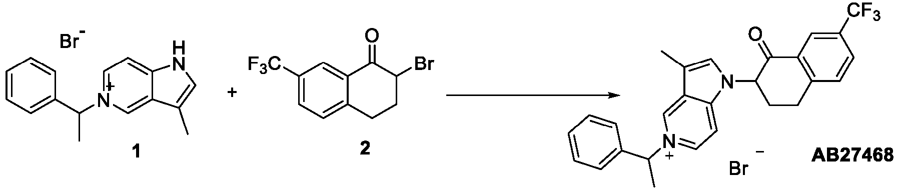

Compound 1 (100 mg, 0.43 mmol) was dissolved in acetonitrile (20 mL), compound 2 (125 mg, 0.43 mol) was added, and N,N-diisopropylethylamine (220 mg, 1.7 mmol) was added. The mixture was replaced with N₂ three times and stirred at 80°C for overnight, new spot was detected on a plate. The raction mixture was concentrated under reduced pressure to remove the organic solvent, the residue was purified by thin layer chromatography to afford compound AB27468.

MS-ESI calculated [M-Br]⁺: 449.49, Found: 449.40.

¹H NMR (400 MHz, DMSO) δ 9.63 (d, *J* = 3.3 Hz, 1H), 8.63 (d, *J* = 7.2 Hz, 1H), 8.10 - 7.99 (m, 3H), 7.81 - 7.71 (m, 2H), 7.52 (d, *J* = 7.9 Hz, 2H), 7.45 - 7.36 (m, 3H), 6.24 - 6.04 (m, 2H), 3.33 - 3.25 (m, 3H), 2.92 (d, *J =* 8.6 Hz, 1H), 2.41 (s, 3H), 2.07 (d, *J =* 6.7 Hz, 3H).

### Example 115 Synthesis of compound AB27469

The structure of compound AB27469 was as follows:

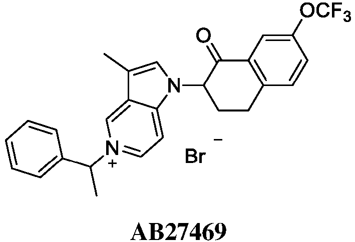

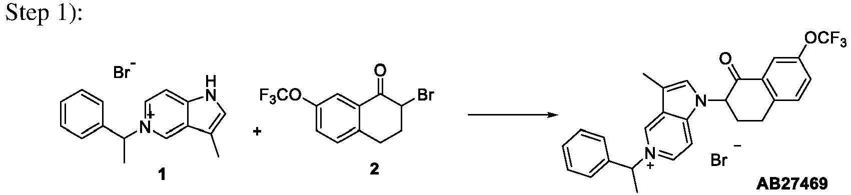

Compound 1 (100 mg, 0.42 mmol) was dissolved in acetonitrile (20 mL), compound 2 (125 mg, 0.43 mol) was added, and N, N-diisopropylethylamine (220 mg, 1.7 mmol) was added. The mixture was replaced with N₂ three times and stirred at 80°C for overnight, new spot was detected on a plate. The raction mixture was concentrated under reduced pressure to remove the organic solvent, the residue was purified by column chromatography to afford compound AB27469.

MS-ESI calculated [M-Br]⁺: 465.49, Found: 465.30.

¹H NMR (400 MHz, DMSO) δ 9.62 (d, *J* = 3.5 Hz, 1H), 8.62 (d, *J* = 6.9 Hz, 1H), 8.06 (d, *J* = 6.9 Hz, 1H), 7.79 (s, 1H), 7.66 (dd, *J =* 19.6, 8.2 Hz, 3H), 7.52 (d, *J =* 6.9 Hz, 2H), 7.43 - 7.34 (m, 3H), 6.13 (d, *J =* 7.0 Hz, 1H), 6.04 (dd, *J =* 14.0, 4.3 Hz, 1H), 3.27 - 3.20 (m, 3H), 2.90 (d, *J =* 9.6 Hz, 1H), 2.40 (s, 3H), 2.07 (d, *J =* 7.0 Hz, 3H).

### Example 116 Synthesis of compound AB27473

The structure of compound AB27473 was as follows:

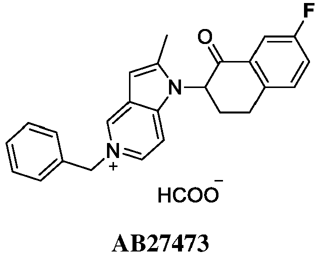

### Step 1):

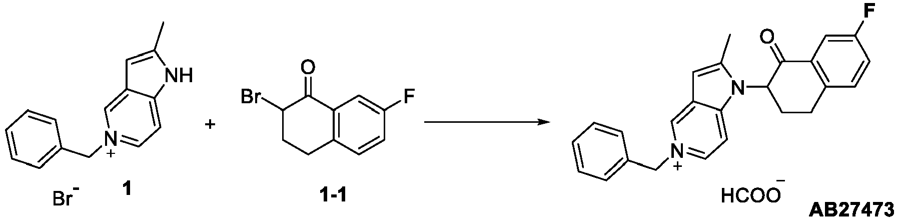

Compound 1 (140 mg, 0.64 mmol) was dissolved in acetonitrile (5 mL) and compound 1-1 (153 mg, 0.63 mmol) was added. The mixture was stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27473.

MS-ESI calculated [M-HCOO⁻]⁺: 385.45, Found: 385.30.

¹H NMR (400 MHz, DMSO-d6) δ 8.37 (s, 1H), 7.71 (s, 1H), 7.51 (s, 1H), 6.89 (d, *J=* 9.1 Hz, 1H), 6.68 (d, *J* = 4.8 Hz, 1H), 6.61 (s, 6H), 6.07 (s, 1H), 4.92 (s, 2H), 3.77 (s, 3H), 2.65 (s, 1H), 2.11 (s, 1H), 1.72-1.68 (m, 3H).

### Example 117 Synthesis of compound AB27485

The structure of compound AB27485 was as follows:

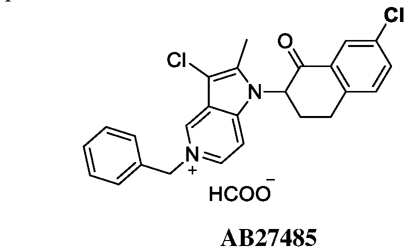

### Step 1):

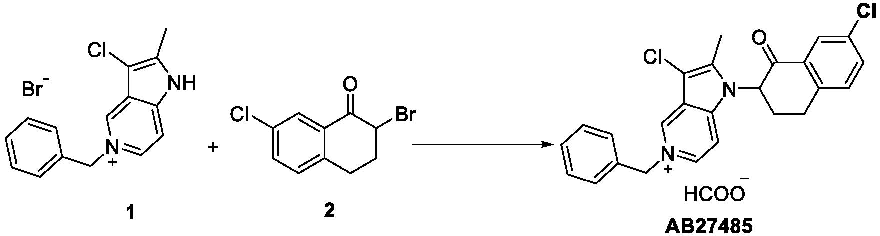

Compound 1 (64 mg, 0.25 mmol) was dissolved in acetonitrile (10 mL), compound 2 (100 mg, 0.37 mmol) was added, and N,N-diisopropylethylamine (128 mg, 0.99 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27485.

MS-ESI calculated [M-HCOO⁻]⁺: 436.35, Found: 435.70.

¹H NMR (400 MHz, CD₃OD) δ 9.35 (s, 1H), 8.52 (s, 1H), 8.40 (s, 1H), 7.98 (s, 1H), 7.63 (d, *J* = 8.3 Hz, 2H), 7.43 (s, 8H), 5.79 (s, 2H), 4.56 (s, 2H), 3.46 (s, 1H), 3.25 - 3.18 (m, 1H), 2.91 (s, 1H), 2.51 (s, 4H).

### Example 118 Synthesis of compound AB27256

The structure of compound AB27256 was as follows:

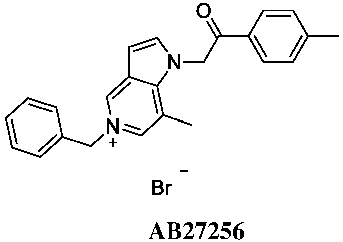

### Step 1):

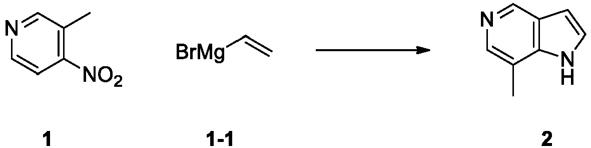

Compound 1 (1.2 g, 9 mmol) was dissolved in tetrahydrofuran solution (20 mL), compound 1-1 (18 mL) was slowly added dropwise at -40 °C. The mixture was stirred at -40°C for 30 min, new spot was detected on a plate. The raction mixture was quenched with H₂O (3 mL) and then extracted with ethyl acetate (20 mL x 3), the combined organic phase was roatary dried under reduced pressure, the residue was purified by thin layer chromatography (dichloromethane:methanol=10:1) to afford compound 2 (370 mg).

### Step 2):

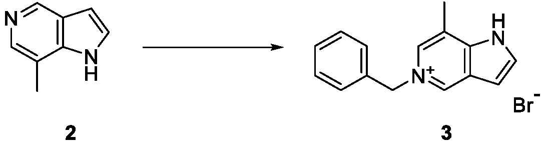

Compound 2 (370 mg, 2.8 mmol) was dissolved in acetonitrile (5 ml), benzyl bromide (615 mg, 3.6 mmol) was added. The mixture was stirred at room temperature for overnight, new spot was detected on a plate. The raction mixture was concentrated under reduced pressure to remove the organic solvent, the residue was purified by thin layer chromatography (dichloromethane:methanol=10:1) to afford compound 3 (340 mg).

### Step 3):

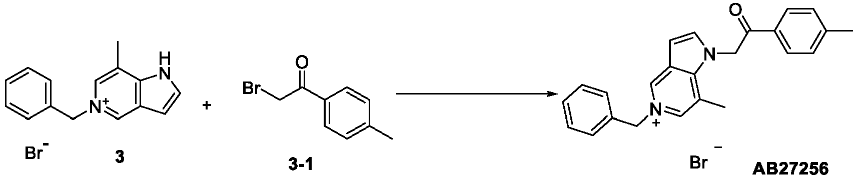

Compound 3 (111 mg, 0.5 mmol) was dissolved in acetonitrile (3 mL), compound 3-1 (213 mg, 1 mmol) was added, and N,N-diisopropylethylamine (200 mg, 1.5 mmol) was added. The mixture was stirred at room temperature for overnight, new spot was detected on a plate. The raction mixture was concentrated under reduced pressure to remove the organic solvent, the residue was purified by thin layer chromatography (dichloromethane:methanol=10:1) to afford compound AB27256.

MS-ESI and ¹H NMR of the compound AB27256 were as follows:
MS-ESI calculated [M-Br]⁺: 355.45, Found: 355.25.
¹H NMR (400 MHz, DMSO) δ 9.46 (s, 1H), 8.46 (s, 1H), 7.96 (d, *J =* 8.1 Hz, 2H), 7.82 (d, *J* = 3.1 Hz, 1H), 7.48 (d, *J =* 6.7 Hz, 2H), 7.41 (dd, *J =* 10.7, 8.0 Hz, 5H), 7.10 (d, *J =* 3.2 Hz, 1H), 6.28 (s, 2H), 5.72 (s, 2H), 2.44 (s, 3H), 2.40 (s, 3H).

### Example 119 Synthesis of compound AB27257

The structure of compound AB27257 was as follows:

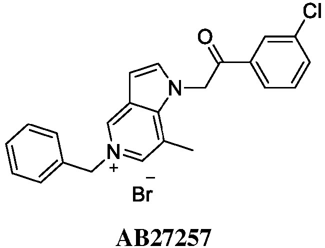

### Step 1):

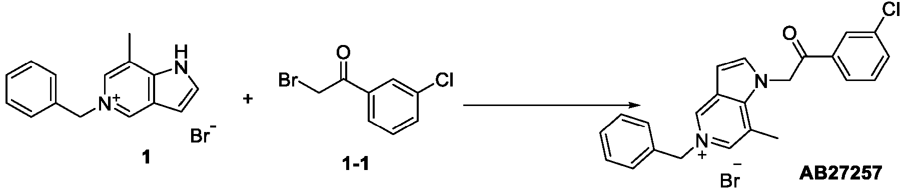

Compound 1 (111 mg , 0.5 mmol) was dissolved in acetonitrile (5 mL), compound 1-1 (233 mg, 1 mmol) was added, and N,N-diisopropylethylamine (200 mg, 1.5 mmol) was added. The mixture was stirred at room temperature for overnight, new spot was detected on a plate. The raction mixture was concentrated under reduced pressure to remove the organic solvent, the residue was purified by thin layer chromatography (dichloromethane:methanol=10:1) to afford compound AB27257.

MS-ESI calculated [M-Br]⁺: 375.87, Found: 375.20.

¹H NMR (399 MHz, DMSO) δ 9.46 (s, 1H), 8.47 (s, 1H), 8.10 (s, 1H), 8.00 (d, *J =* 8.0 Hz, 1H), 7.80 (d, *J* = 13.4 Hz, 2H), 7.67 (d, *J* = 8.0 Hz, 1H), 7.48 (d, *J* = 7.0 Hz, 2H), 7.40 (d, J = 7.3 Hz, 4H), 7.10 (d, *J =* 3.1 Hz, 1H), 6.33 (s, 2H), 5.72 (s, 2H), 2.46 - 2.45 (m, 4H).

### Example 120 Synthesis of compound AB27258

The structure of compound AB27258 was as follows:

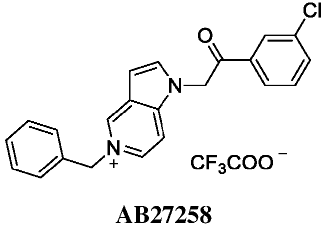

### Step 1):

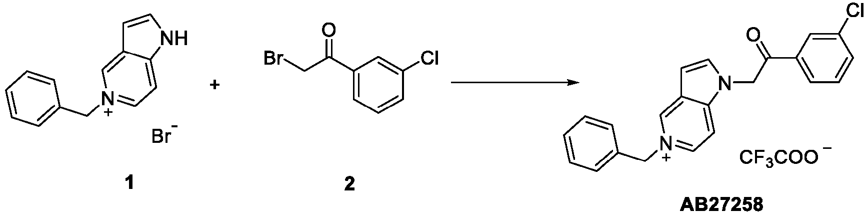

Compound 1 (209 mg, 1 mmol) was dissolved in acetonitrile (10 mL), compound 2 (233 mg, 1 mmol) was added, and triethylamine (303 mg, 3 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27258 (20 mg, yieald: 5.54%).

MS-ESI calculated [M-CF₃COO⁻]⁺: 361.84, Found: 361.30.

¹H NMR (400 MHz, DMSO) δ 9.57 (s, 1H), 8.67 (s, 1H), 8.19 (d, *J* = 4.2 Hz, 1H), 8.08 (s, 1H), 7.98 (s, 1H), 7.89 (s, 1H), 7.81 (s, 1H), 7.65 (s, 1H), 7.48 (s, 2H), 7.39 (s, 3H), 7.12 (s, 1H), 6.18 (s, 2H), 5.79 (s, 2H).

### Example 121 Synthesis of compound AB27259

The structure of compound AB27259 was as follows:

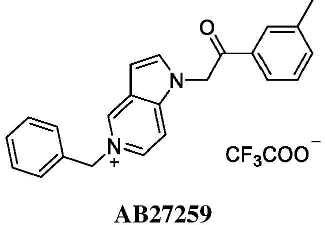

### Step 1):

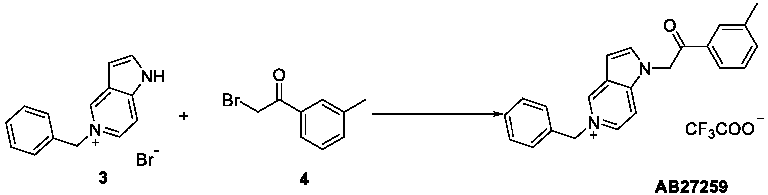

Compound 3 (100 mg, 0.48 mmol) was dissolved in acetonitrile (10 mL), compound 4 (127 mg, 0.58 mmol) was added, and triethylamine (49 mg, 0.48 mmol) was added. The mixture was replaced with N₂ three times and stirred at 40 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27259.

MS-ESI calculated [M-CF₃COO⁻]⁺: 341.43, Found: 341.25.

¹H NMR (400 MHz, CD3OD) δ 9.38 (s, 1H), 8.47 (s, 1H), 7.94 (s, 3H), 7.81 (s, 1H), 7.54 (s, 1H), 7.45 (s, 6H), 7.13 (s, 1H), 6.11 (s, 2H), 5.79 (s, 2H), 2.45 (s, 3H).

### Example 122 Synthesis of compound AB27260

The structure of compound AB27260 was as follows:

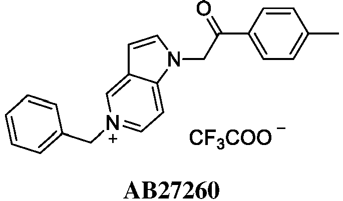

### Step 1:

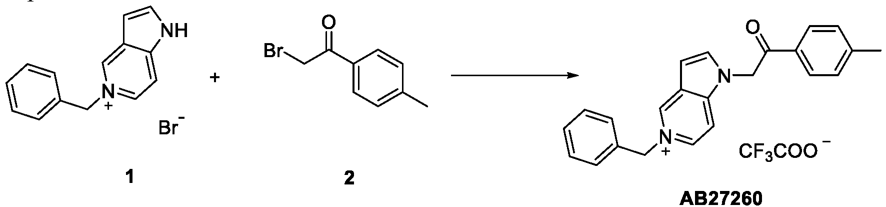

Compound 1 (209 mg, 1 mmol) was dissolved in acetonitrile (10 mL), compound 2 (213 mg, 1 mmol) was added, and triethylamine (303 mg, 3 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27260.

MS-ESI calculated [M-CF₃COO⁻]⁺: 341.43, Found: 341.35.

¹H NMR (400 MHz, DMSO) δ 9.55 (s, 1H), 8.64 (s, 1H), 8.16 (s, 1H), 7.92 (d, *J* = 11.9 Hz, 3H), 7.47 (s, 2H), 7.41 (s, 5H), 7.11 (s, 1H), 6.14 (s, 2H), 5.78 (s, 2H), 2.40 (s, 3H).

### Example 123 Synthesis of compound AB27261

The structure of compound AB27261 was as follows:

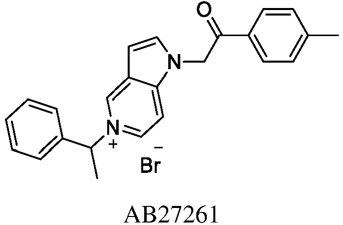

### Step 1):

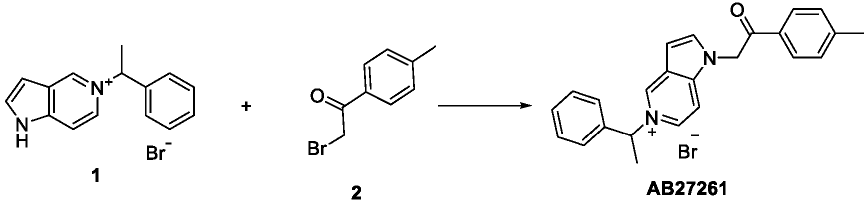

Compound 1 (111 mg, 0.5 mmol) and triethylamine (151.5 mg, 1.5 mmol) were dissolved in acetonitrile (5 mL), and compound 2 (212 mg, 1 mmol) was added. The mixture was replaced with N₂ three times and stirred at 80 °C for overnight, new spot was detected on a plate. The raction mixture was concentrated under reduced pressure to remove the organic solvent, the residue was purified by thin layer chromatography to afford compound AB27261.

MS-ESI calculated [M-Br⁻]⁺ 355.18, Found: 355.35.

¹H NMR (400 MHz, DMSO) δ 9.61 (s, 1H), 8.67 (s, 1H), 8.15 (s, 1H), 7.94 (s, 3H), 7.46 (d, *J* = 32.4 Hz, 7H), 7.09 (s, 1H), 6.15 (s, 3H), 2.40 (s, 3H), 2.05 (s, 3H).

### Example 124 Synthesis of compound AB27262

The structure of compound AB27262 was as follows:

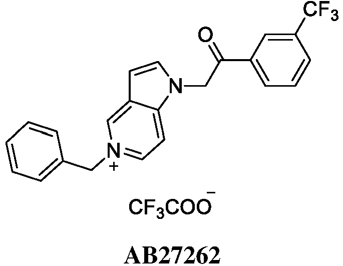

### Step 1):

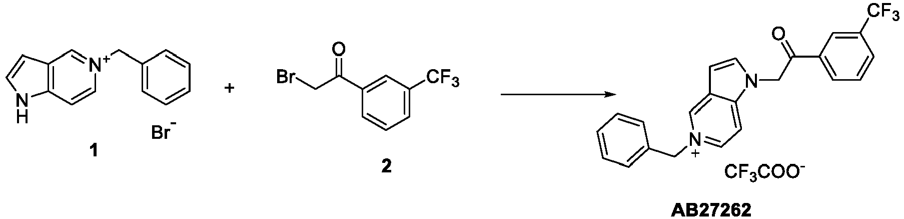

Compound 1 (111 mg, 0.5 mmol) was dissolved in acetonitrile (10 mL), compound 2 (133 mg, 0.5 mmol) was added, and triethylamine (101.19 mg, 1 mmol) was added. The mixture was stirred at 30 °C for overnight, new spot was detected on a plate. The raction mixture was roatary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27262.

MS-ESI calculated [M-CF₃COO⁻]⁺: 395.40, Found: 395.10.

¹H NMR (400 MHz, CD3OD) δ 9.38 (s, 1H), 8.49 (d, *J =* 7.0 Hz, 1H), 8.37 (s, 2H), 8.02 (dd, *J* = 14.8, 7.5 Hz, 2H), 7.86 - 7.79 (m, 2H), 7.45 (s, 5H), 7.14 (d, *J* = 3.1 Hz, 1H), 6.18 (s, 2H), 5.80 (s, 2H).

### Example 125 Synthesis of compound AB27263

The structure of compound AB27263 was as follows:

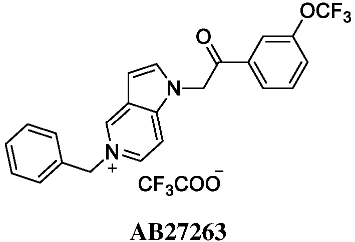

### Step 1):

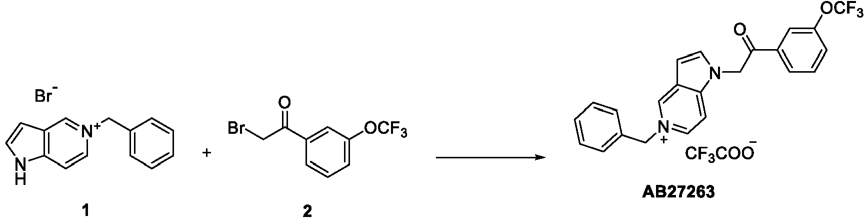

Compound 1 (111 mg, 0.5 mmol) was dissolved in acetonitrile (5 mL), compound 2 (142 mg, 0.5 mmol) was added, and triethylamine (151 mg, 1.5 mmol) was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27263.

MS-ESI calculated [M-CF₃COO⁻]⁺411.40, Found: 411.05.

¹H NMR (400 MHz, CD3OD) δ 9.39 (s, 1H), 8.48 (s, 1H), 8.15 (d, *J* = 5.8 Hz, 1H), 7.97 (s, 2H), 7.81 (s, 1H), 7.74 (s, 1H), 7.67 (s, 1H), 7.45 (s, 5H), 7.13 (s, 1H), 6.14 (s, 2H), 5.80 (s, 2H).

### Example 126 Synthesis of compound AB27264

The structure of compound AB27264 was as follows:

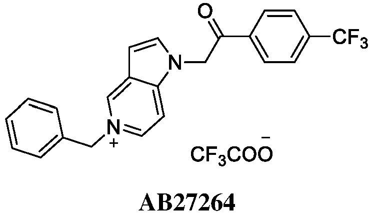

### Step 1):

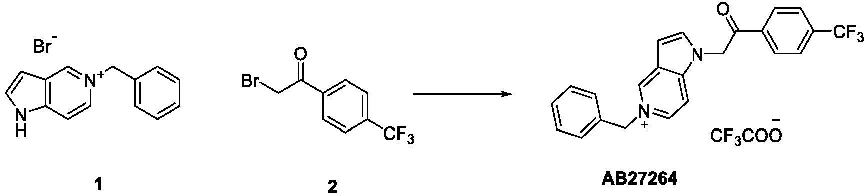

Compound 1 (100 mg, 0.48 mmol) was dissolved in acetonitrile (10 mL), compound 2 (142 mg, 0.53 mmol) was added, and triethylamine (49 mg, 0.48 mmol) was added. The mixture was stirred at 30 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27264.

MS-ESI calculated [M-CF₃COO⁻] ⁺395.40, Found: 395.10.

¹H NMR (400 MHz, CD3OD) δ 9.39 (s, 1H), 8.49 (s, 1H), 8.29 (d, *J =* 6.6 Hz, 2H), 8.01 (d, *J* = 4.9 Hz, 1H), 7.92 (d, *J =* 6.6 Hz, 2H), 7.82 (s, 1H), 7.45 (s, 5H), 7.14 (s, 1H), 6.17 (s, 2H), 5.80 (s, 2H).

### Example 127 Synthesis of compound AB27265

The structure of compound AB27265 was as follows:

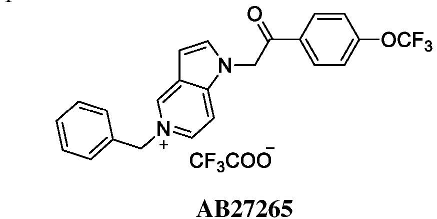

### Step 1):

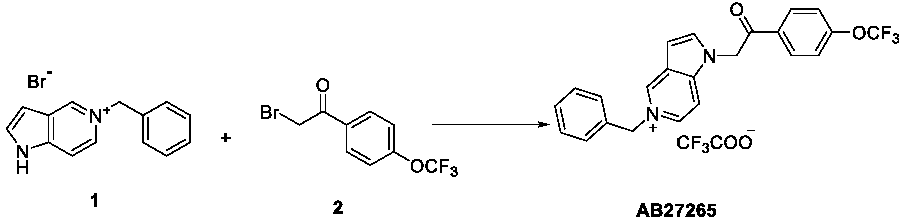

Compound 1 (111 g, 0.5 mmol) was dissolved in acetonitrile (5 mL), compound 2 (142 mg, 0.5 mmol) was added, and triethylamine (151 mg, 1.5 mmol) was added. The mixture was replaced with N₂ three times and stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27265.

MS-ESI calculated [M-CF₃COO⁻]⁺411.40, Found: 411.05.

¹H NMR (400 MHz, DMSO) δ 9.56 (s, 1H), 8.66 (s, 1H), 8.19 (s, 3H), 7.90 (s, 1H), 7.62 (s, 2H), 7.45 (d, *J =* 29.3 Hz, 5H), 7.14 (s, 1H), 6.19 (s, 2H), 5.80 (s, 2H).

### Example 128 Synthesis of compound AB27266

The structure of compound AB27266 was as follows:

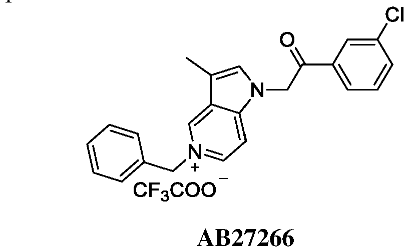

### Step 1):

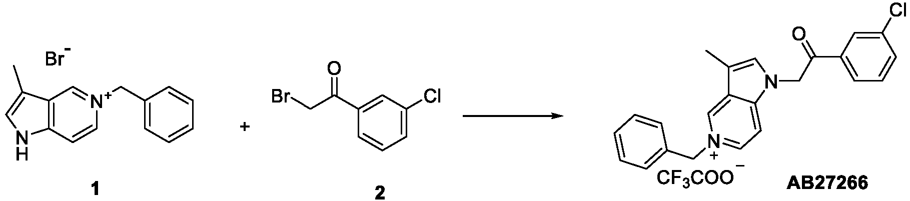

Compound 1 (111 g, 0.5 mmol) was dissolved in acetonitrile (5 mL), and compound 2 (120 mg, 0.55 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27266.

MS-ESI calculated [M-CF₃COO⁻] ⁺375.88, Found: 375.88.

¹H NMR (400 MHz, DMSO) δ 9.66 (s, 1H), 8.63 (d, *J =* 6.8 Hz, 1H), 8.15 - 8.05 (m, 2H), 7.99 (d, *J* = 7.6 Hz, 1H), 7.83 (d, *J =* 7.8 Hz, 1H), 7.70 - 7.62 (m, 2H), 7.51 (d, *J =* 6.9 Hz, 2H), 7.41 (dd, *J =* 15.0, 7.4 Hz, 3H), 6.13 (s, 2H), 5.77 (s, 2H), 2.41 (s, 3H).

### Example 129 Synthesis of compound AB27267

The structure of compound AB27267 was as follows:

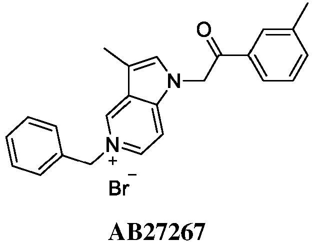

### Step 1):

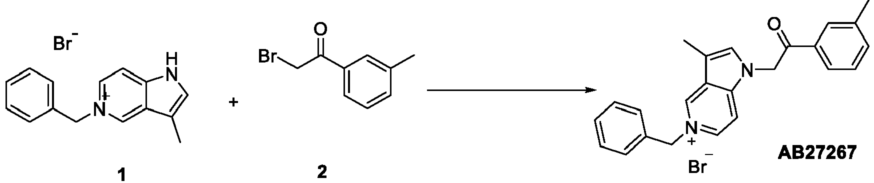

Compound 1 (111 mg, 0.5 mmol) was dissolved in acetonitrile (10 mL), compound 2 (120 mg, 0.5 mmol) was added, and triethylamine (151.5 mg, 1.5 mmol) was added. The mixture was stirred at 30 °C for overnight, new spot was detected on a plate. The raction mixture was rotary dried, the residue was purified by column chromatography to afford compound AB27267.

MS-ESI calculated [M-Br⁻]⁺: 355.46, Found: 355.46.

¹H NMR (400 MHz, DMSO) δ 9.71 (s, 1H), 8.64 (d, *J =* 6.3 Hz, 1H), 8.12 (d, *J =* 6.6 Hz, 1H), 7.86 (d, *J =* 12.5 Hz, 2H), 7.68 (s, 1H), 7.51 (dd, *J =* 18.8, 7.3 Hz, 4H), 7.41 (d, *J =* 7.4 Hz, 3H), 6.14 (s, 2H), 5.80 (s, 2H), 2.41 (s, 6H).

### Example 130 Synthesis of compound AB27268

The structure of compound AB27268 was as follows:

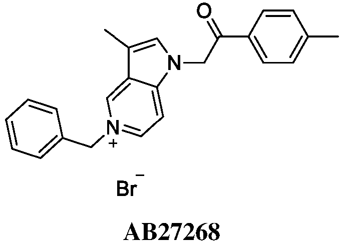

### Step 1):

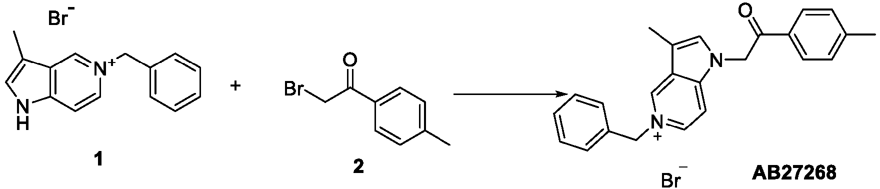

Compound 1 (111 mg, 0.5 mmol) was dissolved in acetonitrile (20 mL), compound 2 (120 mg, 0.55 mmol) was added, and triethylamine (151 mg, 1.5 mmol) was added. The mixture was replaced with N₂ three times and stirred at 40°C for overnight, new spot was detected on a plate. The raction mixture was concentrated under reduced pressure to remove the organic solvent, the residue was purified by thin layer chromatography to afford compound AB27268.

MS-ESI calculated [M-Br⁻] ⁺355.46, Found: 355.25.

¹H NMR (400 MHz, DMSO) δ 9.70 (s, 1H), 8.63 (d, *J* = 6.6 Hz, 1H), 8.11 (d, *J* = 6.8 Hz, 1H), 7.95 (d, *J =* 7.7 Hz, 2H), 7.68 (s, 1H), 7.53 (d, *J =* 6.5 Hz, 2H), 7.46 - 7.35 (m, 5H), 6.11 (s, 2H), 5.79 (s, 2H), 2.41 (s, 6H).

### Example 131 Synthesis of compound AB27269

The structure of compound AB27269 was as follows:

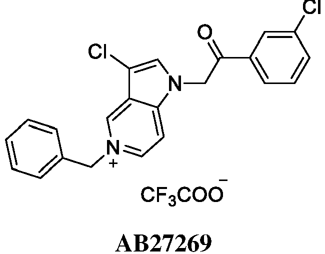

### Step 1):

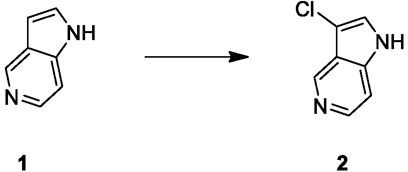

Compound 1 (300 mg, 2.54 mmol) and N-chlorosuccinimide (509 mg, 3.81 mmol) were dissolved in N, N-dimethylformamide (10 mL). The mixture was stirred at room temperature for 16 h, new spot was detected on a plate. The raction mixture was extracted with ethyl acetate and water, the organic phase was rotary dried, the residue was purified by column chromatography to afford compound 2 (200 mg).

### Step 2):

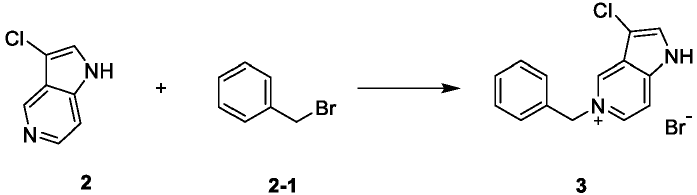

Compound 2 (200 mg, 1.31 mmol) and compound 2-1 (223.68 mg, 1.31 mmol) were dissolved in acetonitrile (10 mL). The mixture was stirred at room temperature for 2 h, new spot was detected on a plate. The raction mixture was filtered, the solid was rotary dried to afford compound 3 (320 mg).

### Step 3):

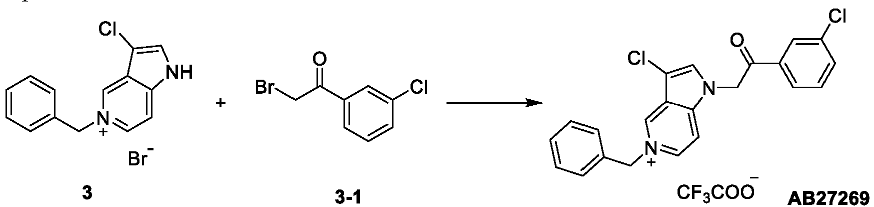

Compound 3 (150 mg, 0.62 mmol), compound 3-1 (216 mg, 0.93 mmol) and triethylamine (186.9 mg, 1.85 mmol) were dissolved in acetonitrile (10 mL). The mixture was stirred at 30 °C for 16 h, new spot was detected on a plate. The raction mixture was rotary dried, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27269.

MS-ESI calculated [M-CF₃COO⁻] ⁺ 396.29, Found: 395.00.

¹H NMR (400 MHz, DMSO) δ 9.76 (s, 1H), 8.73 (s, 1H), 8.26 (s, 1H), 8.09 (d, *J* = 17.0 Hz, 2H), 7.99 (d, *J =* 6.6 Hz, 1H), 7.81 (s, 1H), 7.66 (s, 1H), 7.51 (s, 2H), 7.40 (s, 3H), 6.16 (s, 2H), 5.84 (s, 2H).

### Example 132 Synthesis of compound AB27270

The structure of compound AB27270 was as follows:

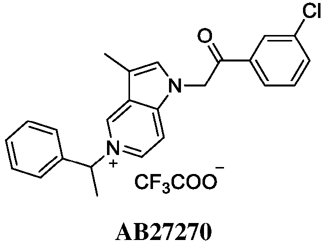

### Step 1):

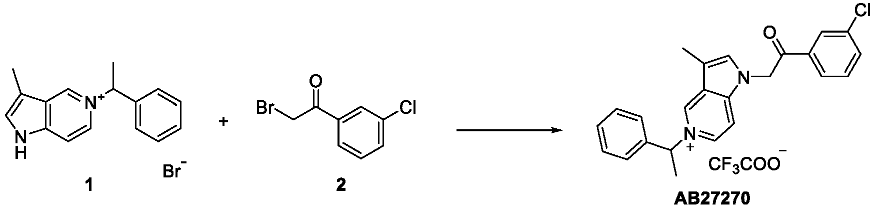

Compound 1 (236 mg, 1 mmol) and triethylamine (303 mg, 3 mmol) were dissolved in acetonitrile (5 mL), and compound 2 (233 mg, 1 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27270.

MS-ESI calculated [M-CF₃COO⁻] ⁺389.90, Found: 389.10.

¹H NMR (400 MHz, DMSO) δ 9.65 (s, 1H), 8.64 (d, *J =* 6.3 Hz, 1H), 8.08 (d, *J =* 6.8 Hz, 2H), 7.99 (d, *J =* 7.4 Hz, 1H), 7.81 (d, *J =* 7.1 Hz, 1H), 7.63 (d, *J =* 7.4 Hz, 2H), 7.54 (d, *J =* 6.5 Hz, 2H), 7.40 (d, *J* = 7.1 Hz, 3H), 6.13 (s, 3H), 2.41 (d, *J* = 7.2 Hz, 3H), 2.07 (d, *J* = 5.9 Hz, 3H).

### Example 133 Synthesis of compound AB27277

The structure of compound AB27277 was as follows:

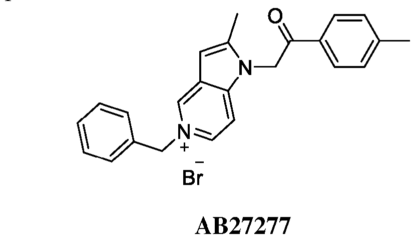

### Step 1):

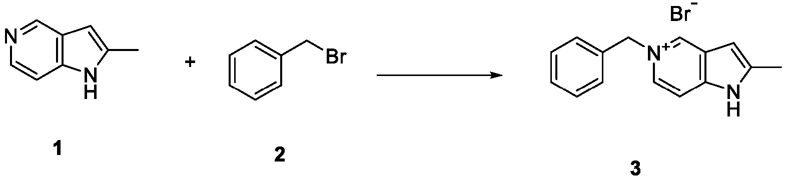

Compound 1 (300 mg, 2.26 mmol) was dissolved in acetonitrile (10 mL), and compound 2 (387 mg, 2.26 mmol) was added. The mixture was stirred at room temperature for 2 h, new spot was detected on a plate. The raction mixture was concentrated under reduced pressure to remove the organic solvent, the residue was purified by thin layer chromatography (methanol: dichloromethane = 0-10 %) to afford compound 3 (400 mg).

### Step 2):

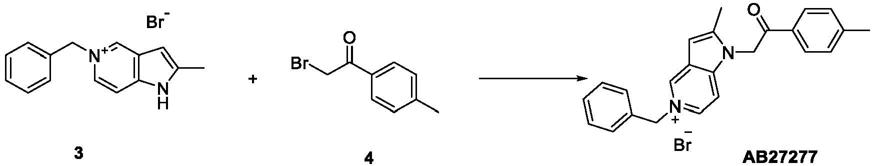

Compound 3 (111 mg, 0.5 mmol) was dissolved in acetonitrile (10 mL), compound 4 (107 mg, 2.26 mmol) was added, and triethylamine (151 mg, 1.5 mmol) was added. The mixture was stirred at 30 °C for overnigh, new spot was detected on a plate. The raction mixture was concentrated under reduced pressure to remove the organic solvent, the residue was purified by thin layer chromatography (methanol: dichloromethane = 0-10 %) to afford compound AB27277.

MS-ESI and ¹H NMR of the compound AB27277 were as follows:
MS-ESI calculated [M-Br]⁺ 355.45, Found: 355.25.
¹H NMR (400 MHz, DMSO) δ 9.40 (s, 1H), 8.60 (d, *J =* 7.0 Hz, 1H), 8.09 (d, *J =* 7.0 Hz, 1H), 7.98 (d, *J =* 8.2 Hz, 2H), 7.47 - 7.35 (m, 7H), 6.89 (s, 1H), 6.12 (s, 2H), 5.75 (s, 2H), 2.40 (s, 3H), 2.36 (s, 3H).

### Example 134 Synthesis of compound AB27357

The structure of compound AB27357 was as follows:

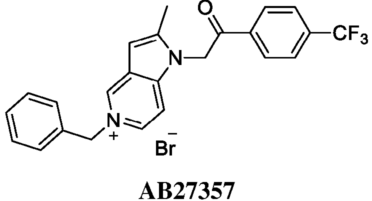

### Step 1):

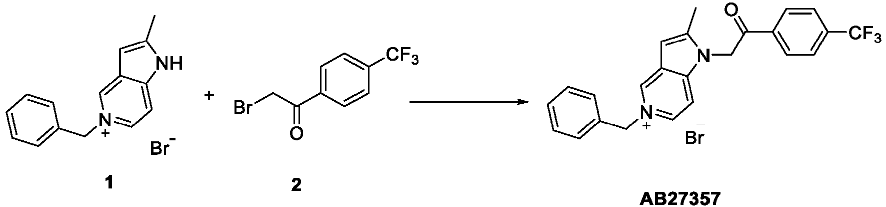

Compound 1 (111 g, 0.5 mmol) was dissolved in acetonitrile (5 mL), compound 2 (133 mg, 0.5 mmol) was added, and triethylamine (151 mg, 1.5 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (dichloromethane: methanol = 10:1) to afford compound AB27357.

MS-ESI calculated [M-Br⁻]⁺ 409.42, Found: 409.10.

¹H NMR (400 MHz, dmso) δ 9.44 (s, 1H), 8.65 (s, 1H), 8.28 (s, 2H), 8.14 (s, 1H), 8.04 (d, J = 5.7 Hz, 2H), 7.44 (d, J = 29.3 Hz, 5H), 6.93 (s, 1H), 6.23 (s, 2H), 5.77 (s, 2H), 2.41 (s, 3H).

### Example 135 Synthesis of compound AB27358

The structure of compound AB27358 was as follows:

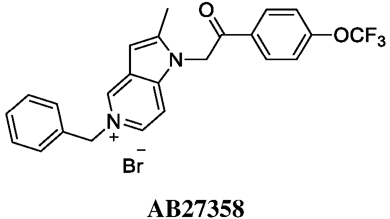

### Step 1):

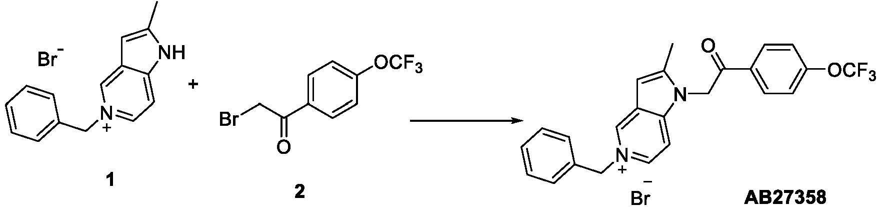

Compound 1 (111 g, 0.5 mmol) was dissolved in acetonitrile (5 mL), compound 2 (142 mg, 0.5 mmol) was added, and triethylamine (151 mg, 1.5 mmol) was added. The mixture was replaced with N₂ three times and stirred at room temperature for overnight, new spot was detected on a plate. The raction mixture was concentrated under reduced pressure to remove the organic solvent, the residue was purified by thin layer chromatography (dichloromethane: methanol = 10:1) to afford compound AB27358.

MS-ESI calculated [M-Br⁻]⁺ 425.42, Found: 425.25.

¹H NMR (399 MHz, dmso) δ 9.41 (s, 1H), 8.62 (d, J = 6.7 Hz, 1H), 8.21 (d, J = 8.4 Hz, 2H), 8.11 (d, J = 6.8 Hz, 1H), 7.63 (d, J = 8.1 Hz, 2H), 7.46 (d, J = 6.6 Hz, 2H), 7.39 (d, J = 7.5 Hz, 3H), 6.90 (s, 1H), 6.17 (s, 2H), 5.75 (s, 2H), 2.38 (s, 3H).

### Example 136 Synthesis of compound AB27455

The structure of compound AB27455 was as follows:

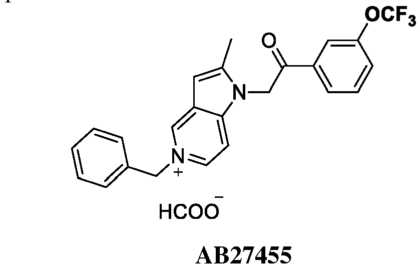

### Step 1):

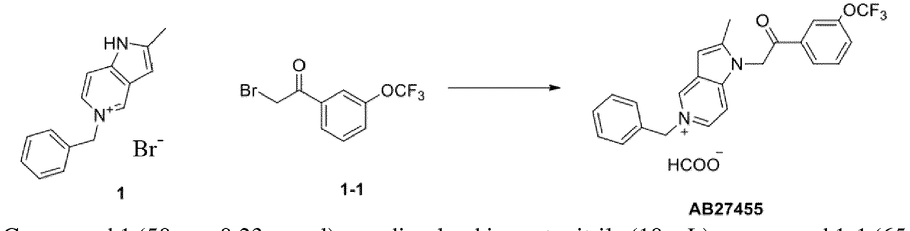

Compound 1 (50 mg, 0.23 mmol) was dissolved in acetonitrile (10 mL), compound 1-1 (65 mg, 0.45 mmol) was added, and N, N-diisopropylethylamine (129 mg, 1 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27455.

MS-ESI calculated [M-HCOO⁻]⁺: 425.42, Found: 425.10.

¹H NMR (399 MHz, dmso) δ 9.41 (s, 1H), 8.62 (d, J = 6.9 Hz, 1H), 8.49 (s, 1H), 8.11 (d, J = 6.8 Hz, 2H), 7.98 (s, 1H), 7.77 (d, J = 4.9 Hz, 2H), 7.45 (d, J = 7.5 Hz, 2H), 7.38 (d, J = 7.4 Hz, 2H), 6.90 (s, 1H), 6.18 (s, 2H), 5.75 (s, 2H), 2.38 (s, 3H).

### Example 137 Synthesis of compound AB27456

The structure of compound AB27456 was as follows:

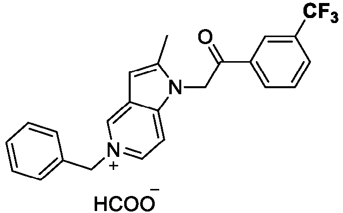

### AB27456

### Step 1):

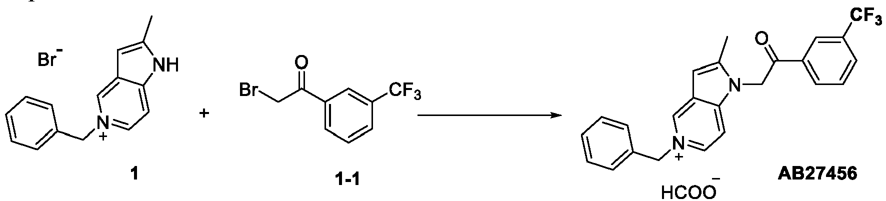

Compound 1 (100 mg, 0.45 mmol) was dissolved in acetonitrile (10 mL), compound 1-1 (120 mg, 0.45 mmol) was added, and N,N-diisopropylethylamine (232 mg, 1.8 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27456.

MS-ESI calculated [M-HCOO⁻]⁺: 409.42, Found: 409.05.

¹H NMR (400 MHz, DMSO) δ 9.42 (s, 1H), 8.62 (d, *J =* 7.3 Hz, 1H), 8.42 (s, 1H), 8.39 - 8.32 (m, 2H), 8.11 (s, 2H), 7.86 (t, *J =* 7.8 Hz, 1H), 7.45 (d, *J =* 6.9 Hz, 2H), 7.39 (d, *J =* 7.5 Hz, 2H), 6.90 (s, 1H), 6.24 (s, 2H), 5.75 (s, 2H), 2.39 (s, 3H).

### Example 138 Synthesis of compound AB271457

The structure of compound AB27457 was as follows:

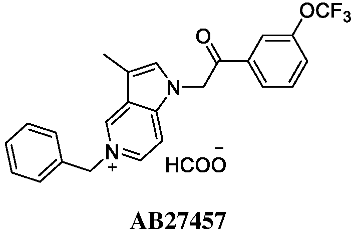

### Step 1):

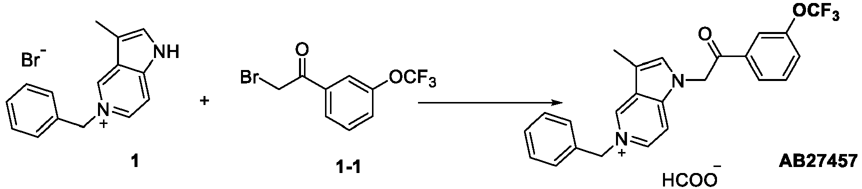

Compound 1 (100 mg, 0.45 mmol) was dissolved in acetonitrile (10 mL), compound 1-1 (141 mg, 0.45 mmol) was added, and N, N-diisopropylethylamine (232 mg, 1.8 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27457.

MS-ESI calculated [M-HCOO⁻]⁺: 425.42, Found: 425.25.

¹H NMR (400 MHz, DMSO) δ 9.69 (s, 1H), 8.63 (d, J = 6.9 Hz, 1H), 8.51 (s, 1H), 8.11 (dd, J = 17.4, 6.2 Hz, 2H), 7.93 (s, 1H), 7.75 (s, 2H), 7.65 (s, 1H), 7.50 (d, J = 7.2 Hz, 2H), 7.44 - 7.28 (m, 4H), 6.15 (s, 2H), 5.78 (s, 2H), 2.39 (s, 3H).

### Example 139 Synthesis of compound AB27458

The structure of compound AB27458 was as follows:

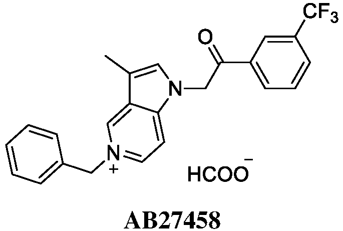

### Step 1):

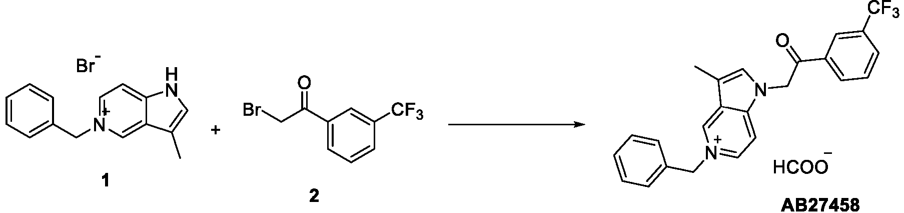

Compound 1 (100 mg, 0.448 mmol) was dissolved in acetonitrile (20 mL), compound 2 (127 mg, 0.448 mol) was added, and N, N-diisopropylethylamine (231 mg, 1.793 mmol) was added. The mixture was replaced with N₂ three times and stirred at 80°C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27458.

MS-ESI calculated [M-HCOO⁻]⁺: 409.42, Found: 409.30.

¹H NMR (400 MHz, DMSO) δ 9.66 (s, 1H), 8.61 (d, *J* = 7.2 Hz, 1H), 8.49 (s, 1H), 8.30 (s, 2H), 8.11 (t, *J* = 6.6 Hz, 2H), 7.85 (t, *J* = 8.2 Hz, 1H), 7.63 (s, 1H), 7.49 (d, *J* = 7.5 Hz, 2H), 7.44 - 7.31 (m, 4H), 6.18 (s, 2H), 5.77 (s, 2H), 2.39 (s, 3H).

### Example 140 Synthesis of compound AB27483

The structure of compound AB27483 was as follows:

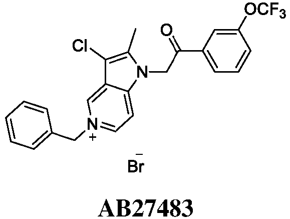

### Step 1):

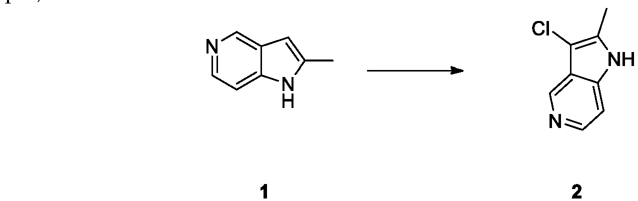

Compound 1 (200 mg, 1.5 mmol) was dissolved in N, N-dimethylacetamide (10 mL), and N-chlorosuccinimide (302 mg, 2.2 mmol) was added. The mixture was stirred at room temperature for overnight, new spot was detected on a plate. The raction mixture was concentrated under reduced pressure to remove the organic solvent, the residue was purified by thin layer chromatography (methanol: dichloromethane = 0-10 %) to afford compound 2 (150 mg).

### Step 2):

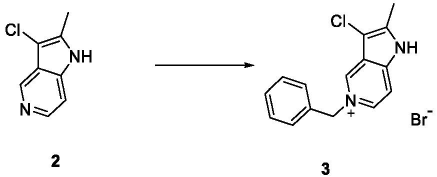

Compound 2 (150 mg, 0.89 mmol) was dissolved in acetonitrile (20 mL), and benzyl bromide (119 mg, 153 mmol) was added. The mixture was stirred at 80 °C for overnigh, new spot was detected on a plate. The raction mixture was concentrated under reduced pressure to remove the organic solvent, the residue was purified by preparation chromatography to afford compound 3 (210 mg).

### Step 3):

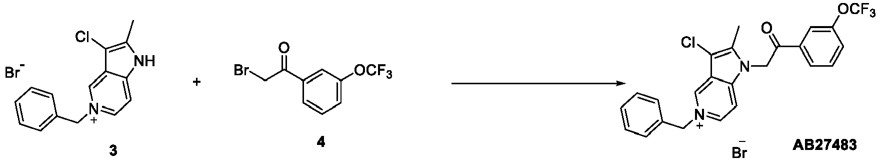

Compound 3 (64 mg, 0.25 mmol) was dissolved in acetonitrile (10 mL), compound 4 (105 mg, 0.37 mmol) was added, and N, N-diisopropylethylamine (128 mg, 0.99 mmol) was added. The mixture was stirred at room temperature for overnight, new spot was detected on a plate. The raction mixture was concentrated under reduced pressure to remove the organic solvent, the residue was purified by thin layer chromatography to afford compound AB27483.

MS-ESI and ¹H NMR of the compound AB27483 were as follows:
MS-ESI calculated [M-Br]⁺: 459.87, Found: 459.00.
¹H NMR (400 MHz, DMSO) δ 9.64 (s, 1H), 8.72 (d, *J =* 7.0 Hz, 1H), 8.24 (d, *J =* 7.0 Hz, 1H), 8.12 (d, *J =* 4.3 Hz, 1H), 7.98 (s, 1H), 7.79 (d, *J =* 4.6 Hz, 2H), 7.51 (d, *J =* 6.5 Hz, 2H), 7.39 (t, *J* = 7.4 Hz, 3H), 6.27 (s, 2H), 5.83 (s, 2H), 2.41 (s, 3H).

### Example 141 Synthesis of compound AB27484

The structure of compound AB27484 was as follows:

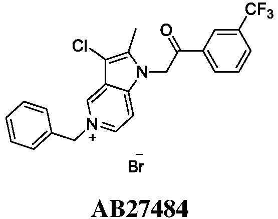

### Step 1):

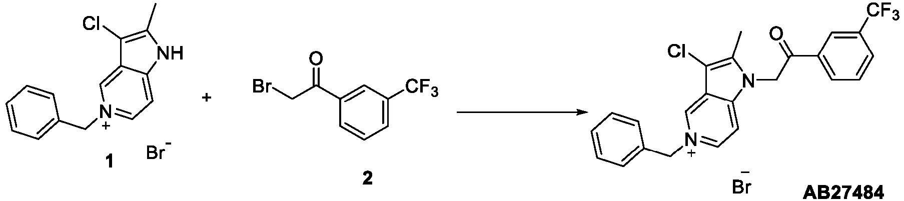

Compound 1 (64 mg, 0.25 mmol) was dissolved in acetonitrile (10 mL), compound 2 (100 mg, 0.37 mmol) was added, and N, N-diisopropylethylamine (128 mg, 0.99 mmol) was added. The mixture was stirred at room temperature for overnight, new spot was detected on a plate. The raction mixture was concentrated under reduced pressure to remove the organic solvent, the residue was purified by thin layer chromatography to afford compound AB27484.

MS-ESI calculated [M-Br]⁺: 443.87, Found: 443.20.

¹H NMR (400 MHz, DMSO) δ 9.64 (s, 1H), 8.71 (d, *J =* 7.1 Hz, 1H), 8.38 - 8.31 (m, 2H), 8.23 (d, *J* = 7.1 Hz, 1H), 8.12 (d, *J* = 7.7 Hz, 1H), 7.87 (s, 1H), 7.50 (d, *J* = 6.6 Hz, 2H), 7.42 - 7.36 (m, 3H), 6.32 (s, 2H), 5.82 (s, 2H), 2.41 (s, 3H).

### Example 142 Synthesis of compound AB27486

The structure of compound AB27486 was as follows:

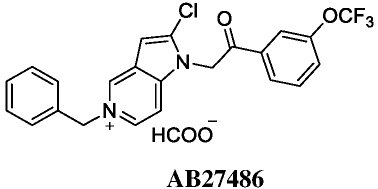

### Step 1:

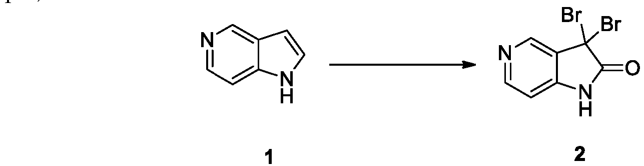

Compound 1 (10 g, 84.7 mmol) was dissolved in tert-butanol (50 mL) and water (50 ml), and liquid bromine (40.6 g, 254 mmol) was added. The mixture was stirred at room temperature for overnight, new spot was detected on a plate. The raction mixture was concentrated under reduced pressure to remove the organic solvent, the residue was purified by preparation chromatography to afford compound 2.

### Step 2):

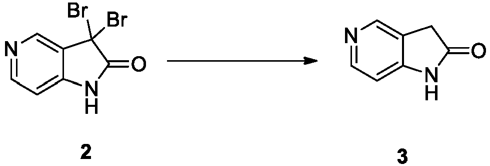

Compound 2 (5 g, 17.18 mmol) was dissolved in tetrahydrofuran (100 ml), and palladium carbon (1 g) was added. The mixture was stirred at room temperature for overnight, new spot was detected on a plate. The raction mixture was concentrated under reduced pressure to remove the organic solvent, the residue was purified by preparation chromatography to afford compound 3.

### Step 3):

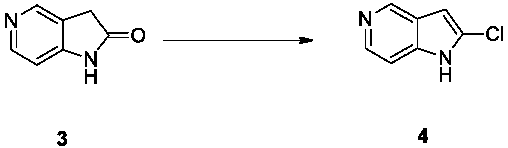

Compound 3 (500 mg, 3.7 mmol) was dissolved in phosphorous trichloride (20 ml). The mixture was stirred at 110°C for 3h, new spot was detected on a plate. The raction mixture was concentrated under reduced pressure to remove the organic solvent, the residue was purified by preparation chromatography to afford compound 4 (64.5 mg).

### Step 4):

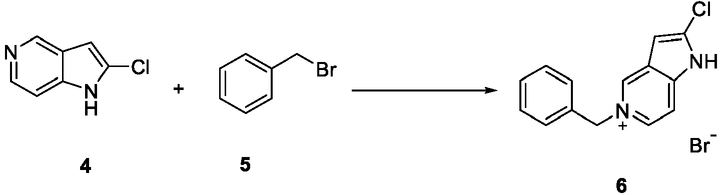

Compound 4 (110 mg, 0.72 mmol) was dissolved in acetonitrile (20 ml), and benzyl bromide (246.12 mg, 0.72 mmol) was added. The mixture was stirred at 80°C for overnight, new spot was detected on a plate. The raction mixture was concentrated under reduced pressure to remove the organic solvent, the residue was purified by preparation chromatography to afford compound 6 (54.8 mg).

### Step 5):

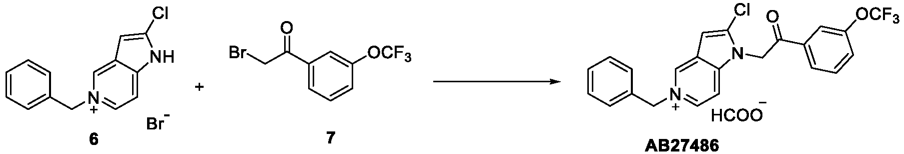

Compound 6 (54.8 mg, 0.23 mmol) was dissolved in acetonitrile (20 ml), compound 7 (100.68 g, 0.339 mmol) and N, N-diisopropylethylamine (116.87 mg, 0.904 mmol) were added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid+ acetonitrile; gradient elution: 1-20 min, 5-25% ACN; 20-50 min, 25-35% ACN) to afford compound AB27486.

MS-ESI and ¹H NMR of the compound AB27486 were as follows:
MS-ESI calculated [M-HCOO⁻]⁺: 445.84, Found: 445.05.
¹H NMR (400 MHz, DMSO) δ 9.57 (s, 1H), 8.77 (s, 1H), 8.48 (s, 1H), 8.28 (s, 4H), 8.15 (s, 1H), 8.02 (s, 1H), 7.78 (s, 2H), 7.43 (d, *J =* 33.7 Hz, 7H), 6.28 (s, 2H), 5.80 (s, 2H).

### Example 143 Synthesis of compound AB27487

The structure of compound AB27487 was as follows:

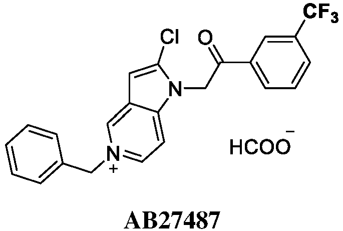

### Step 1:

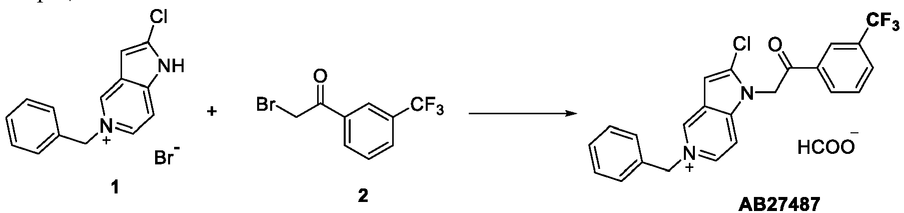

Compound 1 (54.8 mg, 0.23 mmol) was dissolved in acetonitrile (5 ml), and compound 2 (100.68 mg, 0.339 mmol) and N, N-diisopropylethylamine (116.87 mg, 0.904 mmol) were added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid+ acetonitrile; gradient elution: 1-20 min, 5-25% ACN; 20-50 min, 25-35% ACN) to afford compound AB27487.

MS-ESI calculated [M-HCOO⁻]⁺: 429.84, Found: 429.05.

¹H NMR (400 MHz, DMSO) δ 9.60 (s, 1H), 8.79 (s, 1H), 8.49 (s, 1H), 8.39 (d, *J =* 8.9 Hz, 2H), 8.30 (s, 1H), 8.12 (s, 1H), 7.87 (s, 1H), 7.49 (d, *J* = 6.5 Hz, 2H), 7.42 - 7.37 (m, 3H), 6.36 (s, 2H), 5.82 (s, 2H).

### Example 144 Synthesis of compound AB27489

The structure of compound AB27489 was as follows:

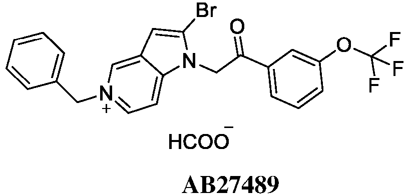

### Step 1:

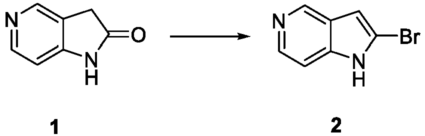

Compound 1 (500 mg, 3.7 mmol) was dissolved in phosphorous oxybromide (20 mL).The mixture was stirred at 60 °C for 3h, new spot was detected on a plate. The raction mixture was concentrated under reduced pressure to remove the organic solvent, the residue was purified by column chromatography to afford compound 2.

### Step 2):

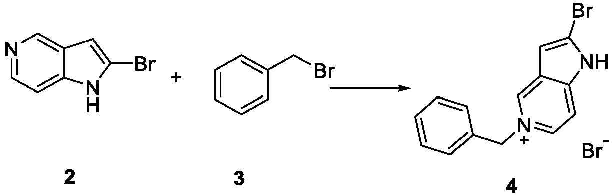

Compound 2 (50 mg, 0.25 mmol) was dissolved in acetonitrile (10 mL), and compound 3 (43 mg, 0.25 mmol) was added. The mixture was stirred at 80 °C for overnigh, new spot was detected on a plate. The raction mixture was concentrated under reduced pressure to remove the organic solvent, the residue was purified by column chromatography to afford compound 4.

### Step 3):

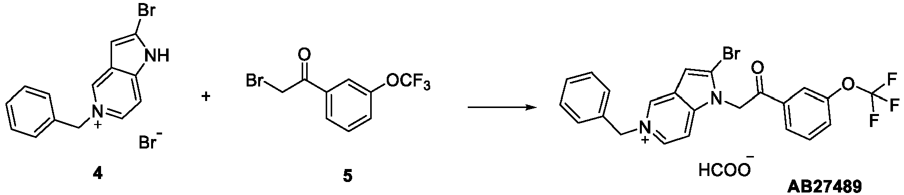

Compound 4 (36.5 mg, 0.19 mmol) was dissolved in acetonitrile (10 mL), compound 5 (62.83 mg, 0.22 mmol) and N,N-diisopropylethylamine (95.64 mg, 0.74 mmol) were added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27489.

MS-ESI and ¹H NMR of the compound AB27489 were as follows:
MS-ESI calculated [M-HCOO⁻]⁺: 490.29, Found: 490.20.
¹H NMR (400 MHz, DMSO) δ 9.56 (s, 1H), 8.75 (d, *J =* 7.6 Hz, 1H), 8.47 (s, 1H), 8.27 (d, *J* = 7.0 Hz, 1H), 8.17 (s, 1H), 8.03 (s, 1H), 7.80 (s, 2H), 7.46 (d, *J =* 9.2 Hz, 3H), 7.40 (d, *J =* 7.1 Hz, 3H), 6.26 (s, 2H), 5.80 (s, 2H).

### Example 145 Synthesis of compound AB27490

The structure of compound AB27490 was as follows:

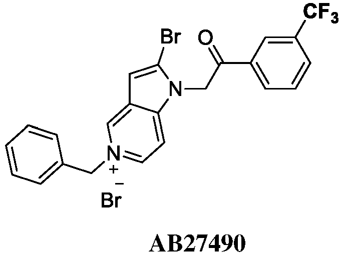

### Step 1):

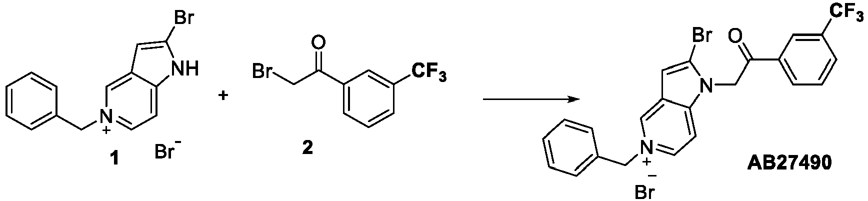

Compound 1 (27 mg, 0.093 mmol) and triethylamine (30.3 mg, 0.3 mmol) were dissolved in acetonitrile (10 ml), and compound 2 (30 mg, 0.11 mmol) was added. The mixture was stirred at 80 °C for 4 h, new spot was detected on a plate. The raction mixture was concentrated under reduced pressure to remove the organic solvent, the residue was purified by thin layer chromatography to afford compound AB27490.

MS-ESI calculated [M-Br]⁺: 474.29, Found: 474.30.

¹H NMR (400 MHz, DMSO) δ 9.56 (s, 1H), 8.75 (d, *J =* 7.6 Hz, 1H), 8.47 (s, 1H), 8.27 (d, *J =* 7.0 Hz, 1H), 8.17 (s, 1H), 8.03 (s, 1H), 7.80 (s, 2H), 7.46 (d, *J =* 9.2 Hz, 3H), 7.40 (d, *J =* 7.1 Hz, 3H), 6.26 (s, 2H), 5.80 (s, 2H).

### Example 146 Synthesis of compound AB27492

The structure of compound AB27492 was as follows:

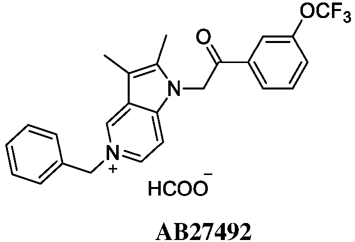

### Step 1):

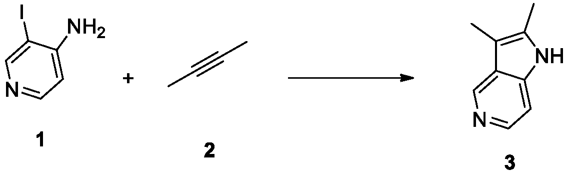

Compound 1 (2.2 g, 10 mmol) was dissolved in N,N-dimethylacetamide (20 mL), compound 2 (1.6 g, 30 mmol), tris(dibenzylideneacetone)dipalladium (365 mg, 0.5 mmol), lithium chloride (420 mg, 10 mmol) and sodium carbonate (2.12 mg, 20 mmol) were added. The mixture was stirred at 120 °C for overnight in a sealed tube, new spot was detected on a plate. The raction mixture was concentrated under reduced pressure to remove the organic solvent, the residue was purified by thin layer chromatography (methanol: dichloromethane = 0-10%) to afford compound 3 (1.1 g).

### Step 2):

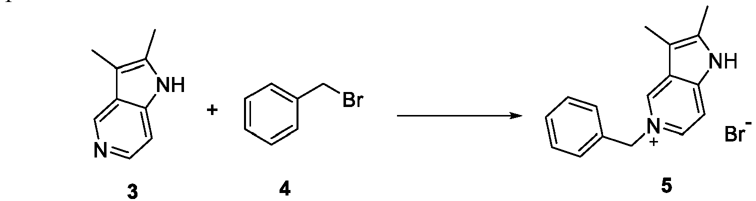

Compound 3 (150 mg, 1 mmol) was dissolved in acetonitrile (20 mL), and compound 4 (190 mg, 1.1 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane = 0-10%) to afford compound 5 (180 mg).

### Step 3):

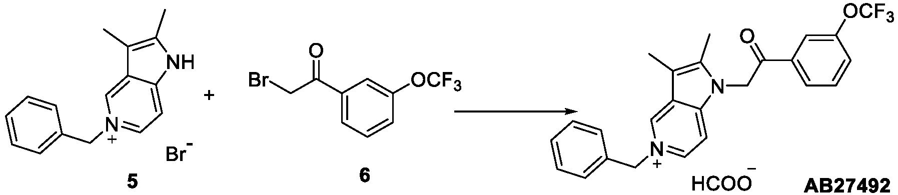

Compound 5 (80 mg, 0.33 mmol) and triethylamine (101 mg, 1 mmol) were dissolved in acetonitrile (20 mL), and compound 6 (144 mg, 0.5 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27492.

MS-ESI and ¹H NMR of the compound AB27492 were as follows:
MS-ESI calculated [M-HCOO⁻]⁺: 439.45, Found: 439.00.
¹H NMR (400 MHz, DMSO) δ 9.52 (s, 1H), 8.56 (d, *J =* 6.9 Hz, 1H), 8.48 (s, 1H), 8.12 (s, 2H), 8.06 (d, *J =* 6.8 Hz, 1H), 7.98 (s, 1H), 7.77 (d, *J =* 4.2 Hz, 2H), 7.47 (d, *J* = 7.3 Hz, 2H), 7.42 - 7.35 (m, 3H), 6.16 (s, 2H), 5.74 (s, 2H), 3.13 (s, 1H), 2.33 (s, 7H), 2.30 (s, 3H).

### Example 147 Synthesis of compound AB27493

The structure of compound AB27493 was as follows:

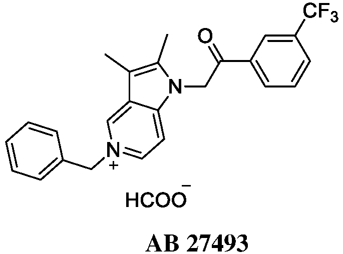

### Step 1):

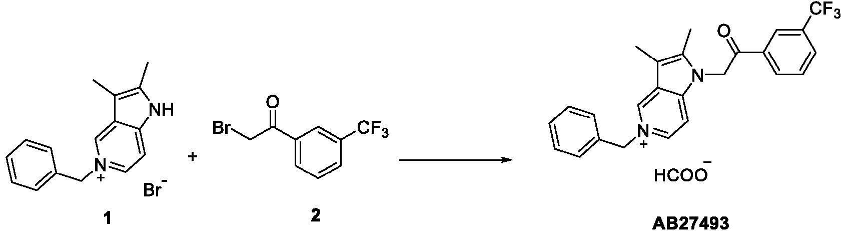

Compound 1 (80 mg, 0.33 mmol) and triethylamine (101 mg, 1 mmol) were dissolved in acetonitrile (20 mL), and compound 2 (133 mg, 0.5 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27493.

MS-ESI calculated [M-HCOO⁻]⁺: 423.45, Found: 423.85.

¹H NMR (400 MHz,DMSO) δ 9.53 (s, 1H), 8.57 (d, *J* = 7.1 Hz, 1H), 8.46 (s, 1H), 8.39 - 8.31 (m, 2H), 8.09 (dd, *J* = 19.8, 7.5 Hz, 2H), 7.87 (d, *J* = 7.8 Hz, 1H), 7.47 (d, *J* = 7.0 Hz, 2H), 7.42 - 7.35 (m, 3H), 6.23 (s, 2H), 5.75 (s, 2H), 2.34 (s, 7H), 2.30 (s, 3H).

### Example 148 Synthesis of compound AB27461

The structure of compound AB27461 was as follows:

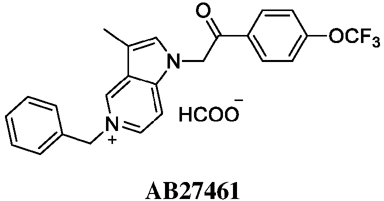

### Step 1):

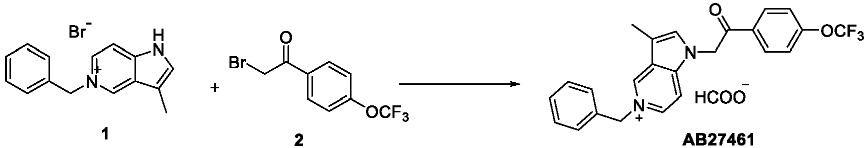

Compound 1 (110 mg, 0.49 mmol) was dissolved in acetonitrile (20 mL), then compound 2 (140 mg, 0.49 mol) was added, and N, N-diisopropylethylamine (252 mg, 2 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27461.

MS-ESI calculated [M-HCOO⁻]⁺: 425.42, Found: 425.25.

¹H NMR (400 MHz, DMSO) δ 9.67 (s, 1H), 8.62 (d, *J =* 7.0 Hz, 1H), 8.51 (d, *J =* 2.8 Hz, 1H), 8.22 - 8.16 (m, 2H), 8.12 (d, *J =* 7.0 Hz, 1H), 7.67 - 7.59 (m, 3H), 7.51 (d, *J =* 6.8 Hz, 2H), 7.40 (td, *J =* 8.5, 2.3 Hz, 3H), 6.13 (s, 2H), 5.78 (s, 2H), 2.41 (s, 3H).

### Example 149 Synthesis of compound AB27463

The structure of compound AB27463 was as follows:

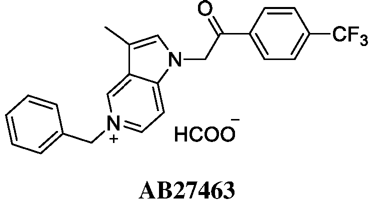

### Step 1:

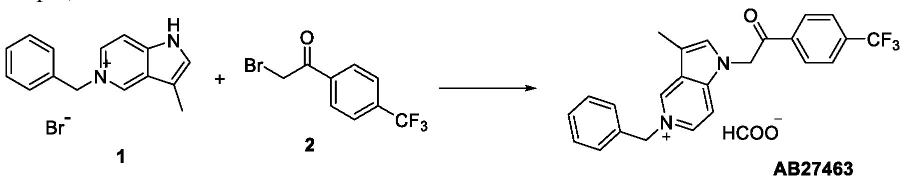

Compound 1 (80 mg, 0.36 mmol) was dissolved in acetonitrile (20 mL), compound 2 (95.8 mg, 0.36 mol) was added, and N, N-diisopropylethylamine (185 mg, 1.43 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid+ acetonitrile; gradient elution: 1-20 min, 5-25% ACN; 20-50 min, 25-35% ACN) to afford compound AB27463.

MS-ESI calculated [M-HCOO⁻]⁺: 409.42, Found: 409.30.

¹H NMR (400 MHz, DMSO) δ 9.63 (s, 1H), 8.60 (d, *J =* 7.0 Hz, 1H), 8.48 (s, 1H), 8.22 (d, *J* = 8.2 Hz, 2H), 8.11 (d, *J =* 7.0 Hz, 1H), 7.99 (d, *J =* 8.0 Hz, 2H), 7.64 (s, 1H), 7.48 (d, *J =* 7.5 Hz, 2H), 7.39 (d, *J =* 7.5 Hz, 2H), 6.15 (s, 2H), 5.76 (s, 2H), 2.39 (s, 3H).

### Example 150 Synthesis of compound AB27478

The structure of compound AB27478 was as follows:

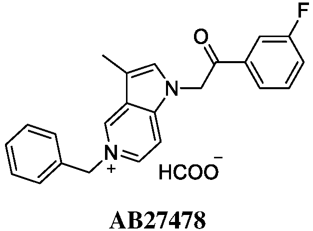

### Step 1):

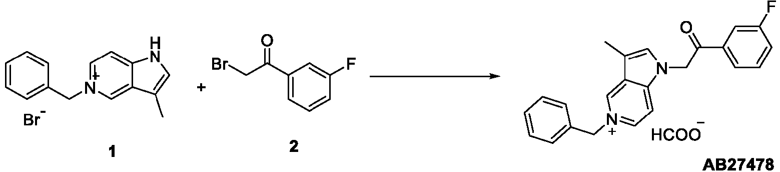

Compound 1 (100 mg, 0.448 mmol) was dissolved in acetonitrile (20 mL), compound 2 (97.4 mg, 0.448 mol) was added, and N, N-diisopropylethylamine (232 mg, 1.792 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27478.

MS-ESI calculated [M-HCOO⁻]⁺: 359.42, Found: 359.30.

¹H NMR (400 MHz, DMSO-d6) δ 9.70 (s, 1H), 8.63 (d, *J =* 6.9 Hz, 1H), 8.51 (s, 1H), 8.12 (d, *J* = 7.0 Hz, 1H), 7.89 (d, *J* = 7.4 Hz, 1H), 7.83 (d, *J* = 9.4 Hz, 1H), 7.69 - 7.56 (m, 3H), 7.51 (d, *J* = 6.9 Hz, 2H), 7.42 - 7.35 (m, 3H), 6.14 (s, 2H), 5.78 (s, 2H), 2.39 (s, 3H).

### Example 151 Synthesis of compound AB27479

The structure of compound AB27479 was as follows:

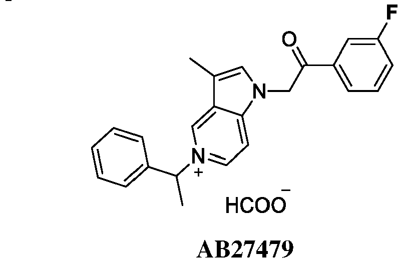

### Step 1):

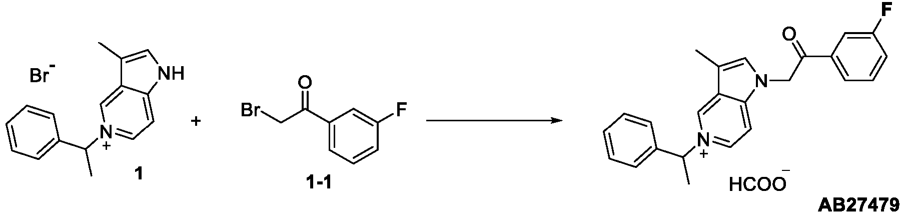

Compound 1 (100 mg, 0.45 mmol) was dissolved in acetonitrile (10 mL), compound 1-1 (120 mg, 0.45 mmol) was added, and N, N-diisopropylethylamine (232 mg, 1.8 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB27479.

MS-ESI calculated [M-HCOO⁻]⁺: 373.44, Found: 373.05.

¹H NMR (400 MHz, DMSO-d6) δ 9.63 (s, 1H), 8.60 (d, *J* = 7.1 Hz, 1H), 8.48 (s, 1H), 8.08 (d, *J =* 7.1 Hz, 1H), 7.88 (d, *J =* 7.7 Hz, 1H), 7.82 (d, *J =* 9.5 Hz, 1H), 7.69 - 7.64 (m, 1H), 7.63 (s, 1H), 7.51 (d, *J =* 7.6 Hz, 2H), 7.42 - 7.35 (m, 3H), 6.17 - 6.07 (m, 3H), 2.40 (s, 3H), 2.06 (d, *J =* 7.0 Hz, 3H).

### Example 152 Synthesis of compound AB27498

The structure of compound AB27498 was as follows:

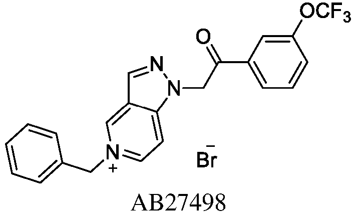

### Step 1):

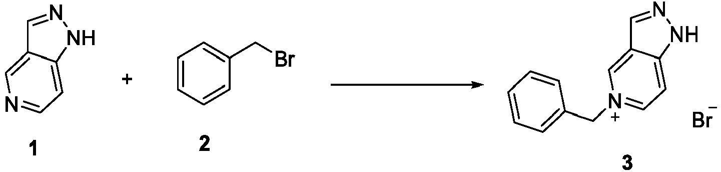

Compound 1 (170 mg, 1 mmol) was dissolved in acetonitrile (20 mL), and compound 2 (119 mg, 1 mmol) was added. The mixture was stirred at 80 °C for overnigh, new spot was detected on a plate. The raction mixture was concentrated under reduced pressure to remove the organic solvent, the residue was purified by preparation chromatography to afford compound 3 (174 mg).

### Step 2):

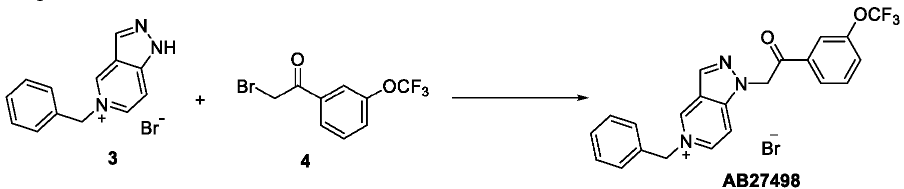

Compound 3 (50 mg, 0.23 mmol) was dissolved in acetonitrile (20 mL), compound 4 (101 mg, 0.35 mmol) was added, and N,N-diisopropylethylamine (120 mg, 0.92 mmol) was added. The mixture was stirred at room temperature for overnight, new spot was detected on a plate. The raction mixture was concentrated under reduced pressure to remove the organic solvent, the residue was purified by thin layer chromatography to afford compound AB27498.

MS-ESI and ¹H NMR of the compound AB27498 were as follows:
MS-ESI calculated [M-Br]⁺: 412.38, Found: 412.25.
¹H NMR (400 MHz, DMSO) δ 9.94 (s, 1H), 8.94 (s, 1H), 8.79 (s, 1H), 8.34 (d, *J* = 7.0 Hz, 1H), 8.26 (d, *J* = 8.3 Hz, 2H), 8.01 (d, *J* = 7.7 Hz, 2H), 7.51 (d, *J* = 6.6 Hz, 3H), 7.42 (d, *J* = 7.5 Hz, 4H), 6.51 (s, 2H), 5.85 (s, 2H).

### Example 153 Synthesis of compound AB27499

The structure of compound AB27499 was as follows:

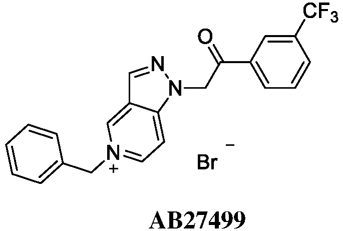

### Step 1):

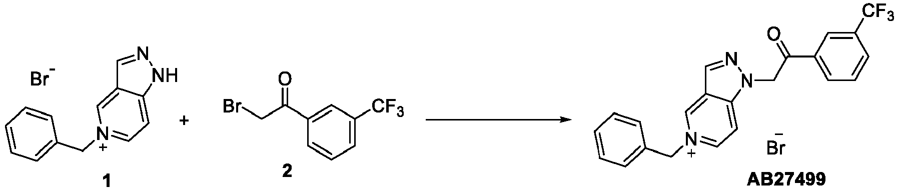

Compound 1 (100 mg, 0.476 mmol) was dissolved in acetonitrile (20 mL), compound 2 (152 mg, 0.57 mmol) was added, and N, N-diisopropylethylamine (246 mg, 1.9 mmol) was added. The mixture was stirred at room temperature for overnight, new spot was detected on a plate. The raction mixture was concentrated under reduced pressure to remove the organic solvent, the residue was purified by column chromatography to afford compound AB27499.

MS-ESI calculated [M-Br]⁺: 396.38, Found: 396.00.

¹H NMR (400 MHz, DMSO) δ 9.95 (s, 1H), 8.94 (s, 1H), 8.80 (d, J = 6.7 Hz, 1H), 8.39 - 8.30 (m, 3H), 8.13 (d, J = 7.8 Hz, 1H), 7.87 (t, J = 8.0 Hz, 1H), 7.52 (d, J = 6.3 Hz, 2H), 7.42 (d, J = 7.6 Hz, 3H), 6.54 (s, 2H), 5.86 (s, 2H).

### Example 154 Synthesis of compound AB31705

The structure of compound AB31705 was as follows:

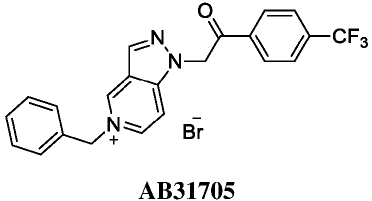

### Step 1):

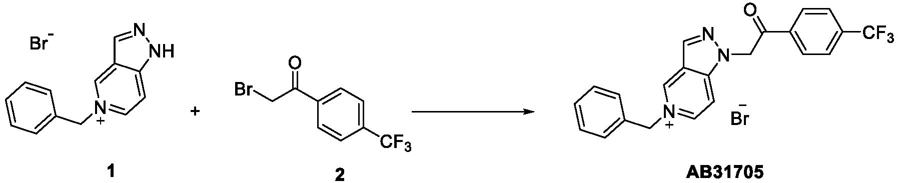

Compound 1 (50 mg, 0.23 mmol) was dissolved in acetonitrile (20 mL), compound 2 (101 mg, 0.35 mmol) was added, and N, N-diisopropylethylamine (120 mg, 0.92 mmol) was added. The mixture was stirred at room temperature for overnight, new spot was detected on a plate. The raction mixture was concentrated under reduced pressure to remove the organic solvent, the residue was purified by thin layer chromatography to afford compound AB31705.

MS-ESI calculated [M-Br]⁺: 396.38, Found: 396.10.

¹H NMR (400 MHz, DMSO) δ 9.94 (s, 1H), 8.94 (s, 1H), 8.80 (d, *J =* 7.1 Hz, 1H), 8.34 (d, *J =* 6.9 Hz, 1H), 8.26 (d, *J* = 8.3 Hz, 2H), 8.01 (d, *J* = 8.5 Hz, 2H), 7.50 (s, 2H), 7.42 (d, *J* = 7.3 Hz, 3H), 6.50 (s, 2H), 5.85 (s, 2H).

### Example 155 Synthesis of compound AB27500

The structure of compound AB27500 was as follows:

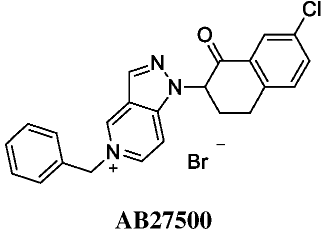

### Step 1):

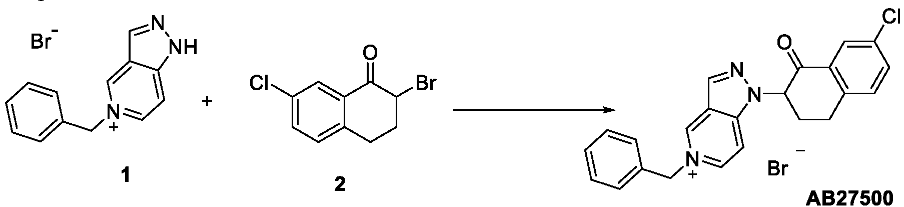

Compound 1 (104 mg, 0.76 mmol) was dissolved in acetonitrile (20 mL), compound 2 (258 mg, 0.9 mmol) was added, and N, N-diisopropylethylamine (393 mg, 3.0 mmol) was added. The mixture was stirred at room temperature for overnight, new spot was detected on a plate. The raction mixture was concentrated under reduced pressure to remove the organic solvent, the residue was purified by thin layer chromatography to afford compound AB27500.

MS-ESI calculated [M-Br]⁺: 388.87, Found: 388.05.

¹H NMR (400 MHz, DMSO) δ 9.93 (s, 1H), 8.93 (s, 1H), 8.80 (d, *J =* 6.9 Hz, 1H), 8.29 (d, *J* = 7.1 Hz, 1H), 7.75 (d, *J =* 21.5 Hz, 2H), 7.50 (s, 3H), 7.42 (d, *J =* 7.5 Hz, 3H), 6.28 (s, 1H), 5.86 (s, 2H), 2.93 - 2.86 (m, 2H), 2.64 (s, 2H).

### Example 156 Synthesis of compound AB31773

The structure of compound AB31773 was as follows:

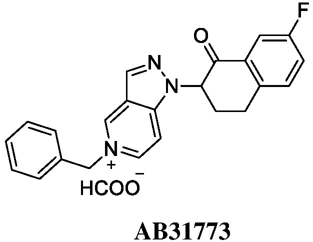

### Step 1):

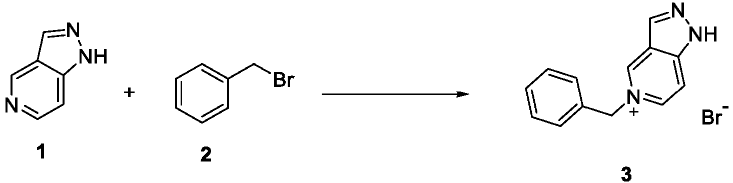

Compound 1 (450 mg, 3.75mmol) was dissolved in acetonitrile (10 mL), compound 2 (641 mg, 3.75 mmol) was added. The mixture was stirred at 80 °C for overnight, new spot was detected on a plate. The raction mixture was concentrated under reduced pressure to remove the organic solvent, the residue was purified by preparation chromatography to afford compound 3 (400 mg).

### Step 2):

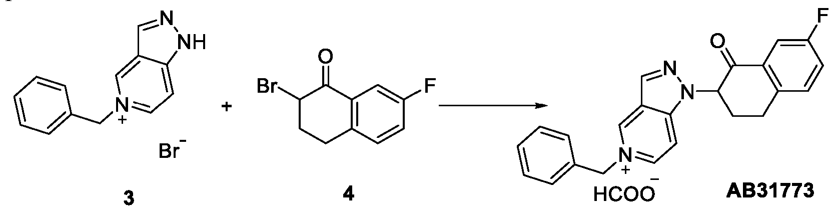

Compound 3 (100 mg, 0.48 mmol) was dissolved in acetonitrile (10 mL), compound 4 (140 mg, 0.57 mmol) was added, and N, N-diisopropylethylamine (248 mg, 1.92 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB31773.

MS-ESI and ¹H NMR of the compound AB31773 were as follows:
MS-ESI calculated [M-HCOO⁻]⁺: 372.41, Found: 372.20.
¹H NMR (400 MHz, DMSO) δ 9.96 (s, 1H), 8.94 (s, 1H), 8.81 (s, 1H), 8.47 (s, 1H), 8.30 (s, 1H), 8.06 (s, 1H), 8.05 - 8.01 (m, 1H), 7.76 (s, 1H), 7.51 (s, 2H), 7.42 (d, *J =* 7.7 Hz, 3H), 6.32 (s, 1H), 5.86 (s, 2H), 3.21 - 3.19 (m, 1H), 3.01 - 2.89 (m, 2H), 2.68 - 2.64 (m, 1H).

### Example 157 Synthesis of compound AB31774

The structure of compound AB31774 was as follows:

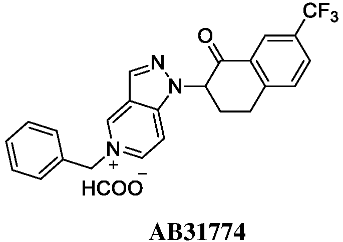

### Step 1):

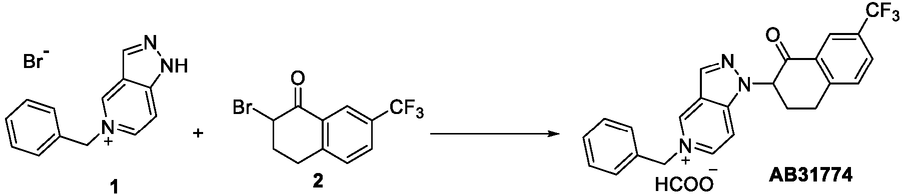

Compound 1 (100 mg, 0.48 mmol) was dissolved in acetonitrile (10 mL), compound 2 (167 mg, 0.57 mmol) was added, and N, N-diisopropylethylamine (248 mg, 1.92 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB21774.

MS-ESI calculated [M-HCOO⁻]⁺: 422.42, Found: 422.20.

¹H NMR (400 MHz, DMSO) δ 9.96 (s, 1H), 8.94 (s, 1H), 8.81 (s, 1H), 8.47 (s, 1H), 8.30 (s, 1H), 8.06 (s, 1H), 8.05 - 8.01 (m, 1H), 7.76 (s, 1H), 7.51 (s, 2H), 7.42 (d, *J =* 7.7 Hz, 3H), 6.32 (s, 1H), 5.86 (s, 2H), 3.21 - 3.19 (m, 1H), 3.01 - 2.89 (m, 2H), 2.68 - 2.64 (m, 1H).

### Example 158 Synthesis of compound AB31775

The structure of compound AB31775 was as follows:

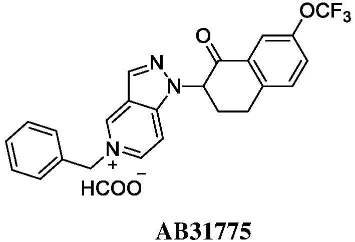

### Step 1):

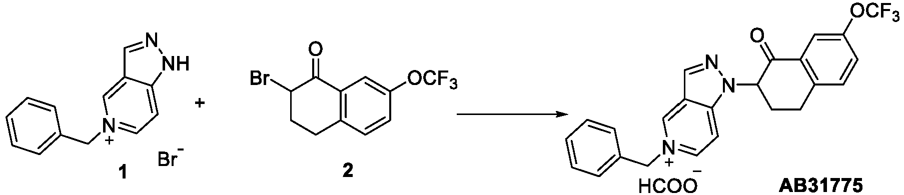

Compound 1 (100 mg, 0.48 mmol) was dissolved in acetonitrile (10 mL), compound 2 (167 mg, 0.57 mmol) was added, and N, N-diisopropylethylamine (248 mg, 1.92 mmol) was added. The mixture was stirred at room temperature for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous formic acid + acetonitrile; gradient elution: 1-20 min, 5-25% ACN; 20-50 min, 25-35% ACN) to afford compound AB31775.

MS-ESI calculated [M-HCOO⁻]⁺: 438.42, Found: 438.25.

¹H NMR (400 MHz, DMSO) δ 9.96 (s, 1H), 8.94 (s, 1H), 8.81 (s, 1H), 8.47 (s, 1H), 8.30 (s, 1H), 8.06 (s, 1H), 8.05 - 8.01 (m, 1H), 7.76 (s, 1H), 7.51 (s, 2H), 7.42 (d, *J* = 7.7 Hz, 3H), 6.32 (s, 1H), 5.86 (s, 2H), 3.21 - 3.19 (m, 1H), 3.01 - 2.90 (m, 2H), 2.68 - 2.63 (m, 1H).

### Example 159 Synthesis of compound AB31776

The structure of compound AB31776 was as follows:

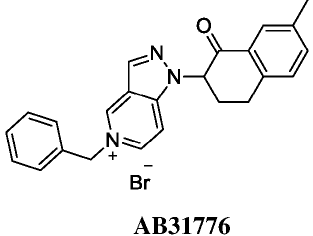

### Step 1):

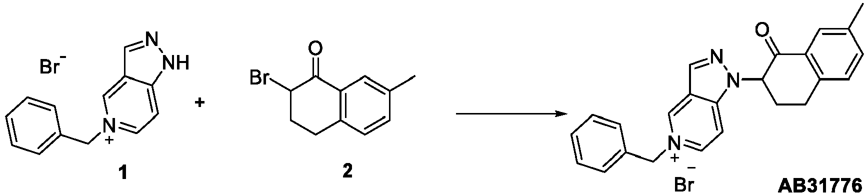

Compound 1 (100 mg, 0.48 mmol) was dissolved in acetonitrile (10 mL), compound 2 (167 mg, 0.57 mmol) was added, and N, N-diisopropylethylamine (248 mg, 1.92 mmol) was added. The mixture was stirred at room temperature for overnight, new spot was detected on a plate. The raction mixture was concentrated under reduced pressure to remove the organic solvent, the residue was purified by column chromatography to afford compound AB31776.

MS-ESI calculated [M-Br]⁺: 368.45, Found: 368.10.

¹H NMR (400 MHz, DMSO) δ 9.96 (s, 1H), 8.92 (s, 1H), 8.82 (d, *J =* 7.2 Hz, 1H), 8.32 (d, *J* = 7.0 Hz, 1H), 7.65 (s, 1H), 7.52 (d, *J =* 6.3 Hz, 1H), 7.48 (d, *J =* 7.5 Hz, 1H), 7.41 (t, *J =* 6.8 Hz, 3H), 7.37 (d, *J =* 7.7 Hz, 1H), 6.25 (dd, *J =* 13.5, 4.5 Hz, 1H), 5.87 (s, 2H), 3.20 (s, 2H), 3.14 (s, 2H), 2.95 - 2.53 (m, 3H).

### Example 160 Synthesis of compound AB31777

The structure of compound AB31777 was as follows:

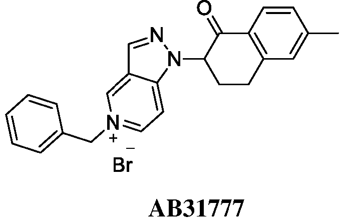

### Step 1):

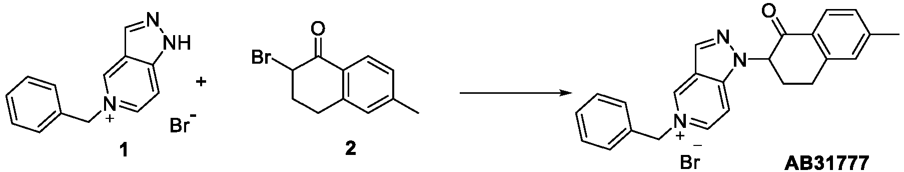

Compound 1 (100 mg, 0.48 mmol) was dissolved in acetonitrile (10 mL), compound 2 (167 mg, 0.57 mmol) was added, and N, N-diisopropylethylamine (248 mg, 1.92 mmol) was added. The mixture was stirred at room temperature for overnight, new spot was detected on a plate. The raction mixture was concentrated under reduced pressure to remove the organic solvent, the residue was purified by column chromatography to afford compound AB31777.

MS-ESI calculated [M-Br]⁺: 368.45, Found: 368.25.

¹H NMR (400 MHz, DMSO) δ 9.94 (s, 1H), 8.92 (s, 1H), 8.81 (d, *J* = 6.9 Hz, 1H), 8.32 (d, *J* = 6.9 Hz, 1H), 7.75 (d, *J =* 8.0 Hz, 1H), 7.52 (d, *J =* 6.8 Hz, 2H), 7.42 (d, *J =* 7.5 Hz, 3H), 7.29 (s, 1H), 7.22 (d, *J =* 7.6 Hz, 1H), 6.23 (d, *J=* 13.6 Hz, 1H), 5.86 (s, 2H), 3.16 (d, *J =* 18.7 Hz, 2H), 2.88 (d, *J =* 9.0 Hz, 1H), 2.61 (s, 2H), 2.37 (s, 3H).

### Example 161 Synthesis of compound AB31806

The structure of compound AB31806 was as follows:

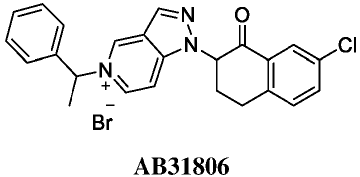

### Step 1):

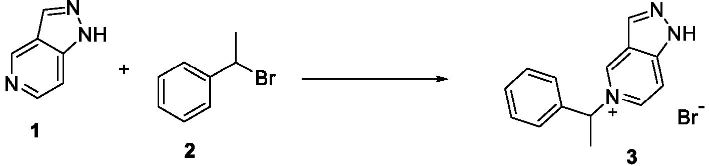

Compound 1 (286 mg, 2.4 mmol) was dissolved in acetonitrile (10 mL), and compound 2 (444 mg, 2.4 mmol) was added. The mixture was stirred at room temperature for overnight, new spot was detected on a plate. The raction mixture was concentrated under reduced pressure to remove the organic solvent, the residue was purified by preparation chromatography to afford compound 3 (400 mg).

### Step 2):

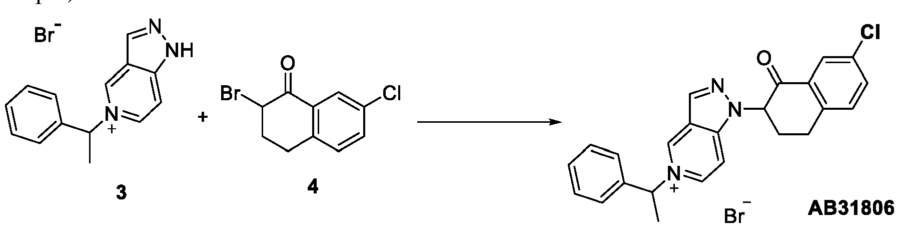

Compound 3 (100 mg, 0.44 mmol) was dissolved in acetonitrile (10 mL), compound 4 (139 mg, 0.53 mmol) was added, and N, N-diisopropylethylamine (173 mg, 1.3 mmol) was added. The mixture was stirred at room temperature for overnight, new spot was detected on a plate. The raction mixture was concentrated under reduced pressure to remove the organic solvent, the residue was purified by column chromatography to afford compound AB31806.

MS-ESI calculated [M-Br]⁺: 402.90, Found: 402.15.

¹H NMR (400 MHz, CD3OD) δ 9.79 (s, 1H), 8.75 (s, 1H), 8.64 (d, *J =* 7.3 Hz, 1H), 8.14 (d, *J* = 7.4 Hz, 1H), 7.89 (s, 1H), 7.61 (s, 1H), 7.50 (s, 2H), 7.46 (d, *J* = 7.9 Hz, 4H), 6.21 (d, *J* = 6.1 Hz, 1H), 4.56 (s, 1H), 3.54 - 3.36 (m, 1H), 3.20 - 3.02 (m, 2H), 2.72 (s, 1H), 2.14 (d, *J* = 7.1 Hz, 3H).

### Example 162 Synthesis of compound AB31702

The structure of compound AB31702 was as follows:

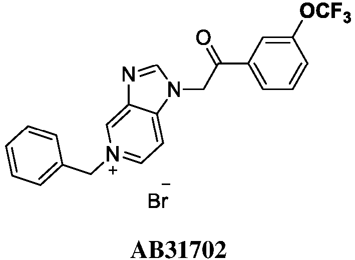

### Step 1):

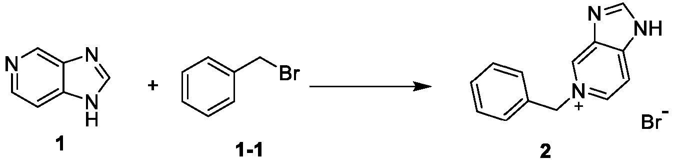

Compound 1 (1 g, 8.4 mmol) was dissolved in acetonitrile (30 mL), and compound 1-1 (1.58 g, 9.24 mmol) was added. The mixture was stirred at 80 °C for overnight, new spot was detected on a plate. The raction mixture was concentrated under reduced pressure to remove the organic solvent, the residue was purified by filtered to afford compound 2 (1.4 g).

### Step 2):

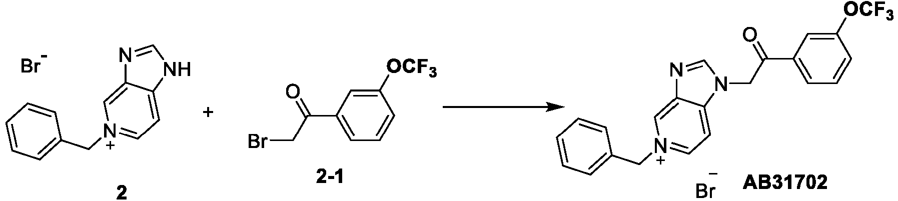

Compound 2 (105 mg, 0.5 mmol) was dissolved in acetonitrile (20 mL), compound 2-1 (212 mg, 0.75 mmol) was added, and N, N-diisopropylethylamine (260 mg, 2 mmol) was added. The mixture was stirred at room temperature for overnight, new spot was detected on a plate. The raction mixture was concentrated under reduced pressure to remove the organic solvent, the residue was purified by thin layer chromatography (methanol: dichloromethane = 0-10 %) to afford compound AB31702.

MS-ESI and ¹H NMR of the compound AB31702 were as follows:
MS-ESI calculated [M-Br]⁺: 412.38, Found: 412.05.
¹H NMR (400 MHz, DMSO) δ 9.93 (s, 1H), 8.89 (d, *J =* 6.5 Hz, 1H), 8.82 (s, 1H), 8.36 (d, *J* = 7.1 Hz, 1H), 8.11 (s, 1H), 7.95 (s, 1H), 7.78 (s, 2H), 7.54 (d, *J =* 7.0 Hz, 2H), 7.41 (d, *J =* 7.5 Hz, 3H), 6.25 (s, 2H), 5.85 (s, 2H).

### Example 163 Synthesis of compound AB31703

The structure of compound AB31703 was as follows:

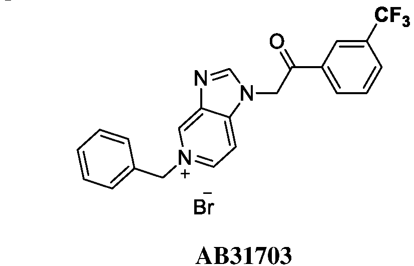

### Step 1):

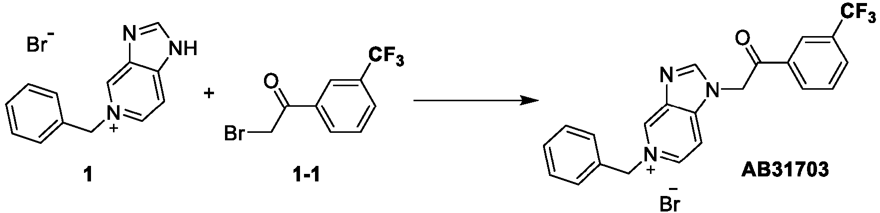

Compound 1 (105 mg, 0.5 mmol) was dissolved in acetonitrile (20 mL), compound 1-1 (200 mg, 0.75 mmol) was added, and N,N-diisopropylethylamine (260 mg, 2 mmol) was added. The mixture was stirred at room temperature for overnight, new spot was detected on a plate. The raction mixture was concentrated under reduced pressure to remove the organic solvent, the residue was purified by thin layer chromatography (methanol: dichloromethane = 0-10 %) to afford compound AB31703.

MS-ESI calculated [M-Br]⁺: 396.38, Found: 396.15.

¹H NMR (400 MHz, DMSO) δ 9.99 (s, 1H), 8.96 - 8.83 (m, 2H), 8.37 (dd, *J* = 20.2, 7.5 Hz, 3H), 8.14 (d, *J =* 7.8 Hz, 1H), 7.90 (d, *J =* 7.8 Hz, 1H), 7.56 (d, *J =* 6.4 Hz, 2H), 7.44 - 7.38 (m, 3H), 6.35 (s, 2H), 5.88 (s, 2H).

### Example 164 Synthesis of compound AB31704

The structure of compound AB31704 was as follows:

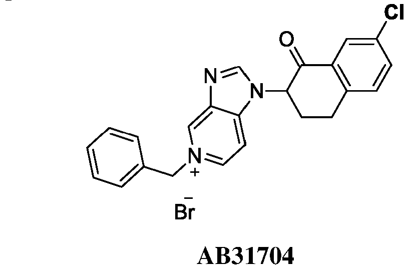

### Step 1):

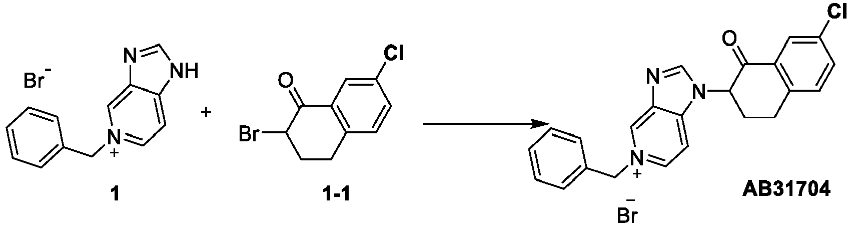

Compound 1 (100 mg, 0.5 mmol) was dissolved in acetonitrile (20 mL), compound 1-1 (324.9 mg, 1.14 mmol) was added, and N, N-diisopropylethylamine (490.2 mg, 3.8 mmol) was added. The mixture was stirred at 80 °C for overnight, new spot was detected on a plate. The raction mixture was concentrated under reduced pressure to remove the organic solvent, the residue was purified by thin layer chromatography (methanol: dichloromethane = 0-10 %) to afford compound AB31704.

MS-ESI calculated [M-Br]⁺: 388.87, Found: 388.10.

¹H NMR (400 MHz, DMSO) δ 9.67 (s, 1H), 9.05-8.85 (m, 1H), 8.73 (s, 1H), 8.27-8.17 (m, 1H), 7.95 (s, 1H), 7.65 (s, 1H), 7.49-7.44 (m, 6H), 5.89 (s, 2H), 3.46 (m, 1H), 3.29 (m, 2H), 2.99 (m, 1H), 2.72 (m, 1H).

### Example 165 Synthesis of compound AB27196

The structure of compound AB27196 was as follows:

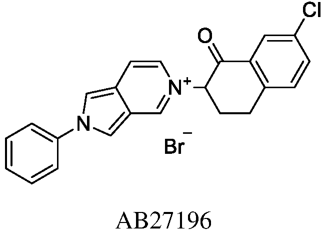

### Step 1):

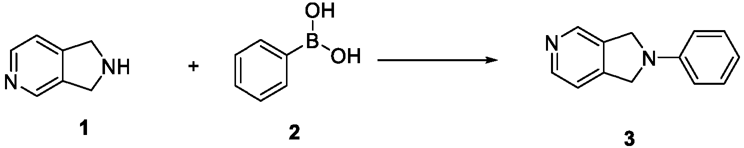

Compound 1 (363 mg, 3 mmol) was dissolved in dichloromethane (50 mL), compound 2 (1.7 g, 9 mmol), copper acetate (543 mg, 3 mmol) and triethylamine (1.7 mL, 12 mmol) were added. The mixture was replaced with N₂ three times and stirred at room temperature for overnight, new spot was detected on a plate. The raction mixture was quenched with water and extracted with dichloromethane (30 mL x 3), the combined organic phases were washed with saturated saline (50 mL), dried over anhydrous sodium sulfate, filtered and concentrated under reduced pressure to remove the organic solvent. The residue was purified by thin layer chromatography (methanol: dichloromethane = 0-10%) to obtain the compound 3 (122 mg)

### Step 2):

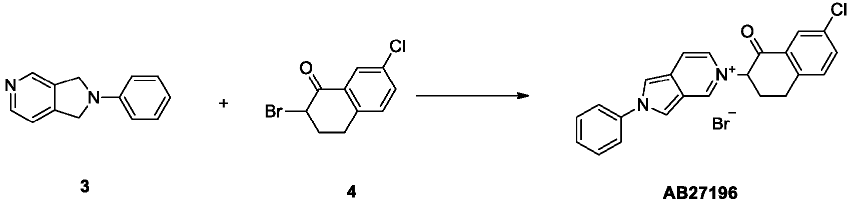

Compound 3 (122 mg, 0.62 mmol) was dissolved in acetonitrile (10 mL), and compound 4 (160.6 mg, 0.62 mmol) was added. The mixture was replaced with N₂ three times and stirred at 80 °C for overnight, new spot was detected on a plate. The raction mixture was concentrated under reduced pressure to remove the organic solvent, the residue was purified by column chromatography (methanol: dichloromthane = 0-30 %) to afford compound AB27196.

MS-ESI and ¹H NMR of the compound AB27196 were as follows:
MS-ESI calculated [M-Br⁻]⁺ 373.85, Found: 373.25.
¹H NMR (400 MHz, cd3od) δ 9.59 (s, 1H), 8.75 (s, 1H), 8.20 (s, 1H), 8.02 (d, J = 19.2 Hz, 2H), 7.85 (s, 3H), 7.66 (s, 4H), 7.49 (s, 1H), 4.77 - 4.69 (m, 1H), 3.45 (d, J = 10.6 Hz, 1H), 3.35 (s, 1H), 3.02 (s, 1H), 2.78 (d, J = 10.3 Hz, 1H).

### Example 166 Synthesis of compound AB27197

The structure of compound AB27197 was as follows:

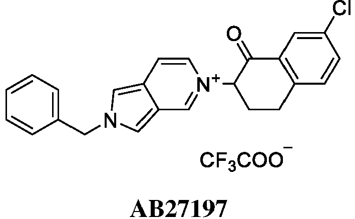

### Step 1):

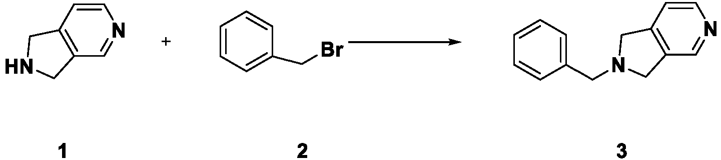

Compound 1 (150 mg, 0.77 mmol) was dissolved in N-N-dimethylformamide (10 mL), sodium hydride (154 mg, 3.85 mmol) was added under ice bath. The mixture was replaced with N₂ three times and stirred for 30 min under ice bath, then compound 2 (160 mg, 0.93 mmol) was injected slowly under ice bath. The mixture was stirred at room temperature for 60 min, new spot was detected on a plate. The raction mixture was quenched with aqueous ammonium chloride and then extracted with ethyl acetate, the combined organic phase was washed with saturated saline, dried over anhydrous sodium sulfate, filtered and concentrated under reduced pressure to remove the organic solvent. The residue was purified by thin layer chromatography (methanol: dichloromethane = 0-10%) to afford compound 3 (100 mg).

### Step 2):

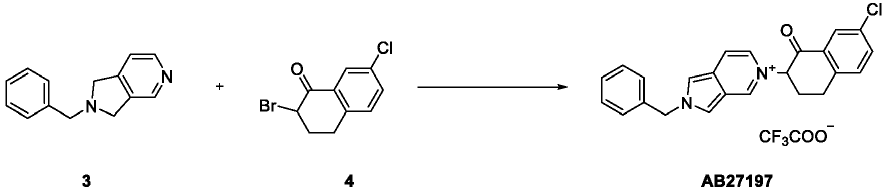

Compound 3 (100 mg, 0.48 mmol) was dissolved in acetonitrile (10 mL), and compound 4 (123 mg, 0.48 mmol) was added. The mixture was replaced with N₂ three times and stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27197.

MS-ESI and ¹H NMR of the compound AB27197 were as follows:
MS-ESI calculated [M-CF₃COO⁻]⁺ 387.89, Found: 387.00.
¹H NMR (400 MHz, dmso-d6) δ 9.58 (s, 1H), 8.63 (s, 1H), 7.99 (d, *J=* 6.3 Hz, 1H), 7.88 (d, *J* = 23.2 Hz, 3H), 7.75 (d, *J=* 7.9 Hz, 1H), 7.54 (d, *J=* 7.8 Hz, 1H), 7.40 (s, 5H), 6.01 (d, *J=* 12.5 Hz, 1H), 5.71 (s, 2H), 3.26 (s, 2H), 2.96 (d, *J=* 9.6 Hz, 1H), 2.64 (s, 1H).

### Example 167 Synthesis of compound AB27287

The structure of compound AB27287 was as follows:

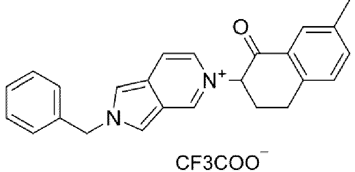

### Step 1):

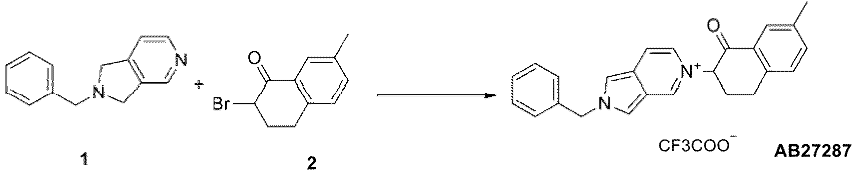

Compound 1 (70 mg, 0.33 mmol) was dissolved in acetonitrile (10 mL), and compound 2 (96 mg, 0.4 mmol) was added. The mixture was replaced with N₂ three times and stirred at 70 °C for overnight, new spot was detected on a plate. The raction mixture was filtered and concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27287.

MS-ESI calculated [M-CF₃COO⁻]⁺: 367.47, Found: 367.10.

¹H NMR (399 MHz, CDsOD) δ 9.41 (s, 1H), 8.36 (s, 1H), 7.89 (d, *J* = 7.2 Hz, 1H), 7.82 (s, 1H), 7.76 - 7.72 (m, 2H), 7.46 (d, *J =* 7.9 Hz, 1H), 7.38 (d, *J =* 4.3 Hz, 5H), 7.32 (d, *J =* 8.0 Hz, 1H), 5.79 (dd, *J* = 13.9, 4.4 Hz, 1H), 5.68 (s, 2H), 3.46 - 3.35 (m, 1H), 3.24 (d, *J =* 17.2 Hz, 1H), 2.92 (dd, *J =* 13.5, 4.4 Hz, 1H), 2.74 - 2.67 (m, 1H), 2.35 (s, 3H).

### Example 168 Synthesis of compound AB27288

The structure of compound AB27288 was as follows:

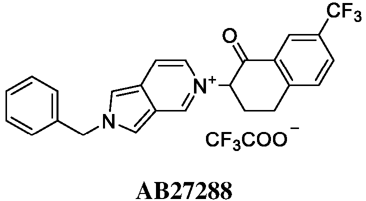

### Step 1):

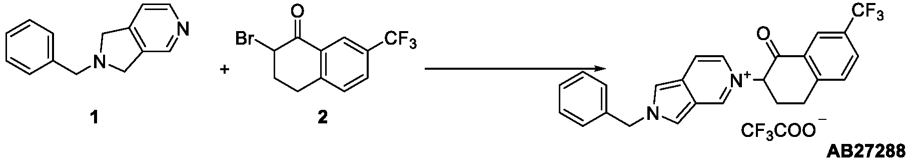

Compound 1 (60 mg, 0.28 mmol) was dissolved in acetonitrile (10 mL), compound 2 (83 mg, 0.28 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27288.

MS-ESI calculated [M-CF₃COO⁻] ⁺ 421.15, Found: 421.20.

¹H NMR (400 MHz, DMSO) δ 9.60 (s, 1H), 8.65 (s, 1H), 8.14 (s, 1H), 8.06 (d, *J =* 8.1 Hz, 1H), 8.01 (d, *J =* 7.0 Hz, 1H), 7.93 (s, 1H), 7.88 (d, *J =* 7.3 Hz, 1H), 7.76 (d, *J =* 8.1 Hz, 1H), 7.43 - 7.33 (m, 5H), 6.06 (dd, *J =* 13.7, 4.1 Hz, 1H), 5.73 (d, *J =* 7.8 Hz, 2H), 3.38 - 3.36 (m, 2H), 3.10 - 3.04 (m, 1H), 2.67 (d, *J =* 10.1 Hz, 1H).

### Example 169 Synthesis of compound AB27289

The structure of compound AB27289 was as follows:

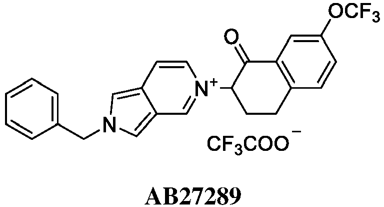

### Step 1):

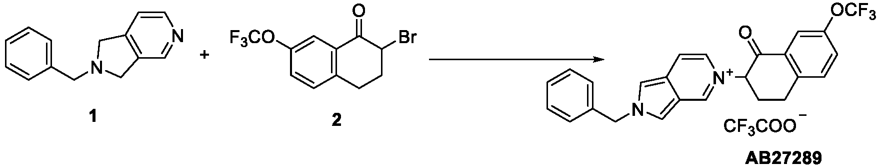

Compound 1 (60 mg, 0.28 mmol) was dissolved in acetonitrile (10 mL), and compound 2 (88 mg, 0.28 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27289.

MS-ESI calculated [M-CF₃COO⁻] ⁺ 437.15, Found: 437.05.

¹H NMR (400 MHz, DMSO) δ 9.59 (s, 1H), 8.64 (s, 1H), 8.00 (d, *J =* 7.1 Hz, 1H), 7.92 (s, 1H), 7.88 (d, *J =* 7.1 Hz, 1H), 7.78 (s, 1H), 7.73 (d, *J =* 8.5 Hz, 1H), 7.66 (d, *J =* 8.4 Hz, 1H), 7.37 (dd, *J* = 16.5, 9.0 Hz, 5H), 6.03 (dd, *J* = 13.9, 4.3 Hz, 1H), 5.72 (s, 2H), 3.31 (s, 2H), 3.00 (d, *J =* 7.5 Hz, 1H), 2.65 (s, 1H).

### Example 170 Synthesis of compound AB27294

The structure of compound AB27294 was as follows:

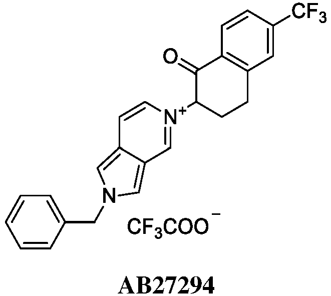

### Step 1:

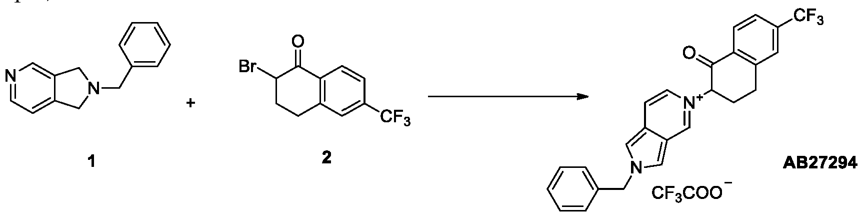

Compound 1 (70 mg, 0.33 mmol) was dissolved in acetonitrile (10 mL), and compound 2 (105 mg, 0.33 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27294.

MS-ESI calculated [M-CF₃COO⁻] ⁺ 421.44, Found: 421.20.

¹H NMR (400 MHz, DMSO) δ 9.61 (s, 1H), 8.66 (s, 1H), 8.12 (d, *J* = 8.1 Hz, 1H), 8.01 (d, *J* = 7.0 Hz, 1H), 7.93 (s, 2H), 7.89 (d, *J* = 7.1 Hz, 1H), 7.80 (d, *J* = 8.1 Hz, 1H), 7.38 (dt, *J* = 11.6, 7.0 Hz, 5H), 6.08 (dd, *J =* 13.8, 3.9 Hz, 1H), 5.73 (s, 2H), 3.39 (s, 2H), 3.09 - 2.95 (m, 1H), 2.73 - 2.62 (m, 1H).

### Example 171 Synthesis of compound AB27295

The structure of compound AB27295 was as follows:

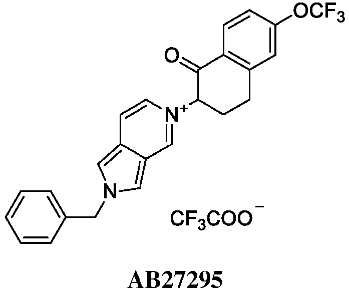

### Step 1):

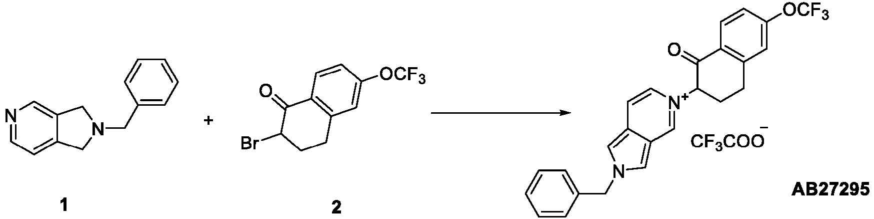

Compound 1 (70 mg, 0.33 mmol) was dissolved in acetonitrile (10 mL), compound 2 (105 mg, 0.33 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27295.

MS-ESI calculated [M-CF₃COO⁻] ⁺ 437.43, Found: 437.20.

¹H NMR (400 MHz, DMSO) δ 9.59 (s, 1H), 8.64 (s, 1H), 8.06 (d, *J =* 8.6 Hz, 1H), 8.00 (d, *J =* 7.1 Hz, 1H), 7.92 (s, 1H), 7.87 (d, *J =* 7.1 Hz, 1H), 7.53 (s, 1H), 7.46 - 7.34 (m, 6H), 6.01 (dd, *J =* 13.8, 3.9 Hz, 1H), 5.72 (s, 2H), 3.33 (s, 2H), 3.00 (d, *J =* 8.5 Hz, 1H), 2.65 (s, 1H).

### Example 172 Synthesis of compound AB27296

The structure of compound AB27296 was as follows:

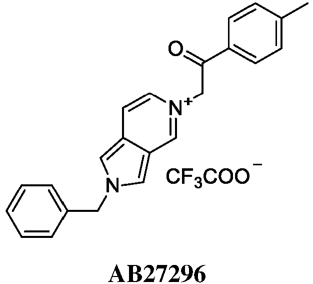

### Step 1):

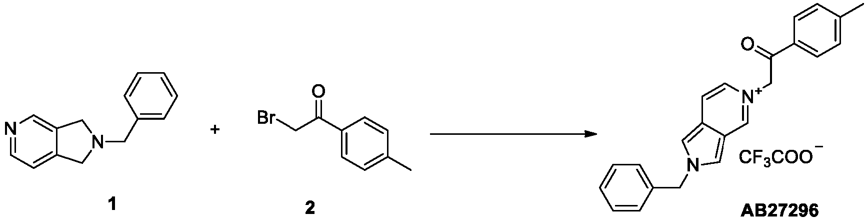

Compound 1 (70 mg, 0.33 mmol) was dissolved in acetonitrile (10 mL), and compound 2 (105 mg, 0.33 mmol) was added. The mixture was stirred at 80 °C for overnight, new spot was detected on a plate. The raction mixture was concentrated under reduced pressure to remove the organic solvent, the residue was purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27296.

MS-ESI calculated [M-CF₃COO⁻] ⁺ 341.43, Found: 341.10.

¹H NMR (400 MHz, DMSO) δ 9.48 (s, 1H), 8.63 (s, 1H), 8.00 - 7.90 (m, 4H), 7.79 (d, *J =* 7.0 Hz, 1H), 7.48 - 7.33 (m, 7H), 6.23 (s, 2H), 5.71 (s, 2H), 2.42 (s, 3H).

### Example 173 Synthesis of compound AB27477

The structure of compound AB27477 was as follows:

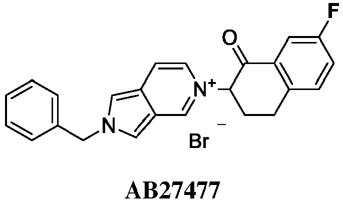

### Step 1:

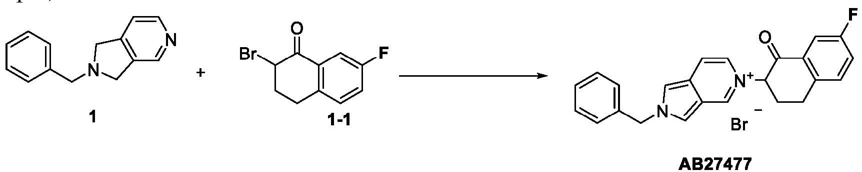

Compound 1 (105 mg, 0.5 mmol) was dissolved in acetonitrile (20 mL), and compound 1-1 (122 mg, 0.5 mol) was added. The mixture was replaced with N₂ three times and stirred at 80 °C for overnight, new spot was detected on a plate, and purified by thin layer chromatography (dichloromethane: methanol = 10:1) to afford compound AB27477.

MS-ESI calculated [M-Br⁻]⁺: 371.43, Found: 371.05.

¹H NMR (400 MHz, DMSO-d6) δ 9.58 (s, 1H), 8.62 (s, 1H), 7.97 (d, *J =* 7.1 Hz, 1H), 7.91 - 7.84 (m, 2H), 7.60 (d, *J* = 8.4 Hz, 1H), 7.55 (d, *J* = 6.6 Hz, 2H), 7.42 - 7.33 (m, 5H), 6.02 (d, *J =* 11.2 Hz, 1H), 5.70 (s, 2H), 3.25 - 3.23 (m, 1H), 2.95 (dt, *J =* 13.0, 8.4 Hz, 2H), 2.62 (d, *J =* 12.1 Hz, 1H).

### Example 174 Synthesis of compound AB27480

The structure of compound AB27480 was as follows:

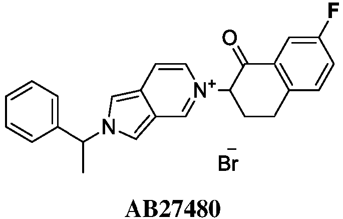

### Step 1):

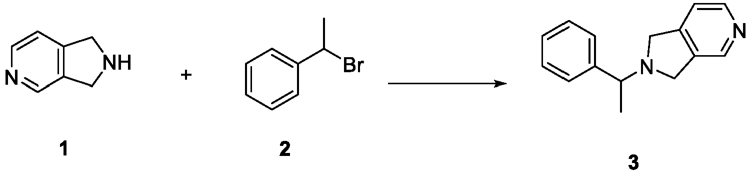

Compound 1 (200 mg, 1 mmol) was dissolved in N, N-dimethylformamide (20 mL). The mixture was replaced with N₂ three times, sodium hydrogen (214.8 mg, 8.95 mmol) was added at 0 °C, and compound 2 (364.5 mg, 1.96 mol) was added after 0.5 h. The mixture was stirred at room temperature for 2 h, new spot was detected on a plate, The raction mixture was concentrated under reduced pressure to remove the organic solvent, the residue was purified by thin layer chromatography (dichloromethane: methanol = 10:1) to afford compound 3 (60 mg).

### Step 2):

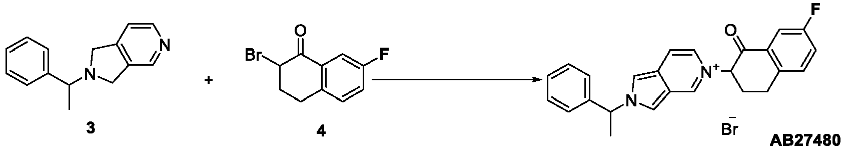

Compound 3 (60 mg, 0.27 mmol) was dissolved in acetonitrile (20 mL), and compound 4 (122 mg, 0.5 mol) was added. The mixture was replaced with N₂ three times and stirred at 80 °C for overnight, new spot was detected on a plate. The raction mixture was purified by thin layer chromatography (dichloromethane: methanol=10:1) to afford compound AB27480.

MS-ESI and ¹H NMR of the compound AB27480 were as follows:
MS-ESI calculated [M-Br⁻]⁺: 385.45, Found: 385.25.
¹H NMR (400 MHz, DMSO-d6) δ 9.58 (s, 1H), 8.70 (s, 1H), 7.98 (d, *J=* 6.3 Hz, 2H), 7.88 (d, *J =* 7.0 Hz, 1H), 7.62 (d, *J =* 8.7 Hz, 1H), 7.59 - 7.55 (m, 2H), 7.43 - 7.32 (m, 5H), 6.11 - 6.00 (m, 2H), 3.27 (s, 2H), 3.05 - 2.91 (m, 1H), 2.63 (d, *J =* 10.1 Hz, 1H), 2.00 (d, *J=* 7.0 Hz, 3H).

### Example 175 Synthesis of compound AB27481

The structure of compound AB27481 was as follows:

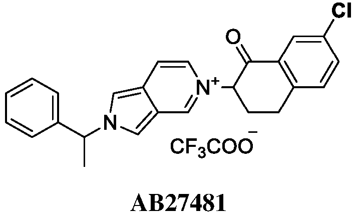

### Step 1):

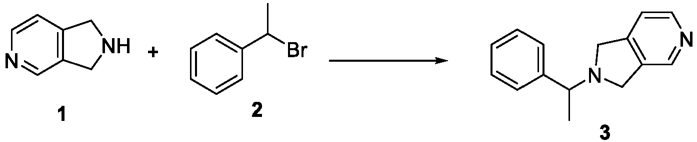

Compound 1 (345 mg, 1.79 mmol) was dissolved in N, N-dimethylformamide (20 mL). The mixture was replaced with N₂ three times, sodium hydrogen (214.8 mg, 8.95 mmol) was added at 0 °C, and compound 2 (364.5 mg, 1.96 mol) was added after 0.5 h. The mixture was stirred at room temperature for 2 h, new spot was detected on a plate, The raction mixture was concentrated under reduced pressure to remove the organic solvent, the residue was purified by thin layer chromatography (dichloromethane: methanol = 20:1) to afford compound 3 (106 mg).

### Step 2):

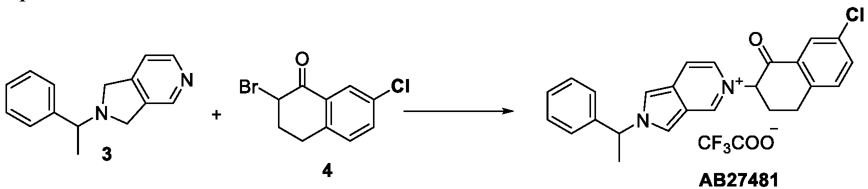

Compound 3 (106 mg, 0.5 mmol) was dissolved in acetonitrile (20 mL), compound 4 (140 mg, 0.5 mol) was added. The mixture was replaced with N₂ three times and stirred at 80 °C for overnight, new spot was detected on a plate, and purified by preparation chromatography (column: Sharpsil-U C18 (250 mm x 30 x 10 um, 100 Å); mobile phase: 0.1% aqueous trifluoroacetic acid + acetonitrile; gradient elution: 1-20 min, 5-25% ACN; 20-50 min, 25-35% ACN) to afford compound AB27481.

MS-ESI and ¹H NMR of the compound AB27481 were as follows:
MS-ESI calculated [M-CF₃COO⁻]⁺: 401.91, Found: 401.25.
¹H NMR (400 MHz, DMSO-d6) δ 9.53 (s, 1H), 8.65 (s, 1H), 8.00 - 7.91 (m, 2H), 7.88 - 7.79 (m, 2H), 7.72 (dd, *J =* 8.2, 2.4 Hz, 1H), 7.52 (d, *J =* 8.2 Hz, 1H), 7.40-7.30 (m, 5H), 6.09 - 5.89 (m, 2H), 3.22 (d, *J =* 15.6 Hz, 2H), 2.98-2.89 (m, 1H), 2.61 (d, *J =* 8.6 Hz, 1H), 1.98 (d, *J =* 7.0 Hz, 3H).

### Example 176 Synthesis of compound AB36527

The structure of compound AB36527 was as follows:

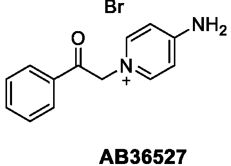

### Step 1):

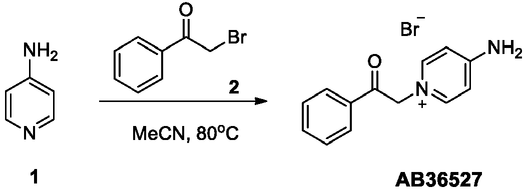

Compound 1 (100 mg, 1.06 mmol, 1.0 eq) was dissolved in acetonitrile (10 mL), compound 2 (211 mg, 1.06 mmol, 1.0 eq) was added. The mixture was reacted at 80°C for 3 h, the reaction mixture was rotary dried, the crude product was purified by fast chromatography (dichloromethane/methanol = 0-10%) to afford compound AB36527.

MS-ESI: calculated [M]⁺: 213.10; Found: 213.10.

¹H NMR (400 MHz, DMSO-d6) δ 8.29 (s, 2H), 8.10 (d, *J=* 8.0 Hz, 2H), 8.03 (d, *J=* 8.0 Hz, 2H), 7.77-7.74 (m, 1H), 7.65-7.61 (m, 2H), 6.91 (d, *J=* 8.0 Hz, 2H), 5.98 (s, 2H).

### Example 177 Synthesis of compound AB36528

The structure of compound AB36528 was as follows:

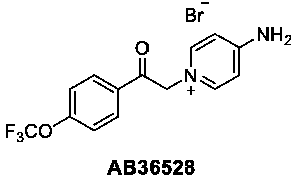

### Step 1):

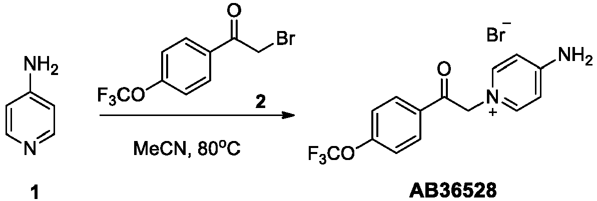

Compound 1 (100 mg, 1.06 mmol, 1.0 eq) was dissolved in acetonitrile (10 mL), and compound 2 (300 mg, 1.06 mmol, 1.0 eq) was added. The mixture was reacted at 80°C for 3 h, the reaction mixture was rotary dried, the crude product was purified by fast chromatography (dichloromethane/methanol = 0-10%) to afford compound AB36528.

MS-ESI: calculated [M]⁺: 297.08; Found: 296.95.

¹H NMR (400 MHz, DMSO-d6) δ 8.28 (s, 2H), 8.17 (d, *J=* 12.0 Hz, 2H), 8.07 (d, *J=* 8.0 Hz, 2H), 7.64 (d, *J=* 8.0 Hz, 2H), 6.91 (d, *J=* 4.0 Hz, 2H), 5.97 (s, 2H).

### Example 178 The inhibitory effect of compounds prepared in the Examples on NCI-H82 cell was determined

The role of expression level of NNMT in the sensitivity of NCI-H82 cell to the compounds prepared in the Examples of the present invention was determined

### Experiment method and result:

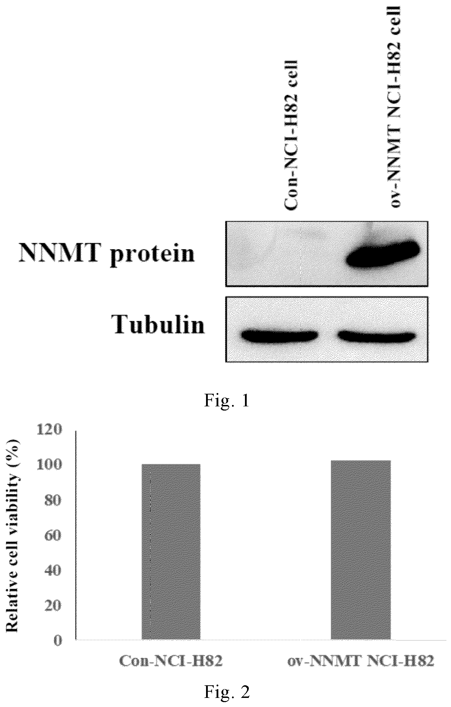

The expression of NNMT protein in NCI-H82 cell was overexpressed by inserting the NNMT gene into NCI-H82 cell using a viral vector, and the NNMT protein-overexpressing NCI-H82 cell (ov-NNMT NCI-H82 cell) was obtained. The normal and untreated NCI-H82 cell (Con-NCI-H82 cell) was used as control. The expressing content of NNMT protein in the ov-NNMT NCI-H82 cell and Con-NCI-H82 cell was detected using Western Blot assay (as shown in Fig.1), it could be seen from Fig.1 that the NNMT protein was overexpressed in the ov-NNMT NCI-H82 cell compared with the expression level of NNMT protein in the Con-NCI-H82 cell.

The cell viability of Con-NCI-H82 cell and ov-NNMT NCI-H82 cell was detected using the Promega CellTiter-Glo kit which reflected cell viability by measuring intracellular ATP content (as shown in Fig.2), it could be seen from Fig.2 that the cell viability of Con-NCI-H82 cell and ov-NNMT NCI-H82 cell was almost the same, and the difference in cell viability was not statistically significant.

The cell viability was detected using the Promega CellTiter-Glo kit which reflected cell viability by measuring intracellular ATP content. The Con-NCI-H82 cell and ov-NNMT NCI-H82 cell was cultured in 10% fetal bovine serum-containing RPMI1640 medium (+P/S). The 50% inhibiting concentration (IC₅₀) of the compounds prepared in the Examples of the present invention on Con-NCI-H82 cell and ov-NNMT NCI-H82 cell was detected using Promega CellTiter-Glo kit, the result was shown in Table 3 below:

**Table 3 the inhibitory effect of the compounds prepared in the Examples of the present invention on Con-NCI-H82 cell and ov-NNMT NCI-H82 cell(IC₅₀, µM)**

| Compound | Con-NCI-H82 cell (IC₅₀, µM) | ov-NNMT NCI-H82 cell (IC₅₀, µM) |
|---|---|---|
| compound AB24995 | 0.920 | 4.152 |
| compound AB24994 | 0.810 | 2.986 |
| compound AB27101 | 0.750 | 2.312 |
| compound AB27356 | 0.130 | 1.741 |
| compound AB27175 | 0.226 | 2.860 |
| compound AB27250 | 0.051 | 0.684 |
| compound AB27171 | 0.063 | 1.052 |
| compound AB27219 | 0.085 | 1.325 |
| compound AB27470 | 0.112 | 2.434 |
| compound AB27177 | 0.131 | 1.859 |
| compound AB27173 | 0.135 | 2.033 |
| compound AB27172 | 0.203 | 2.045 |
| compound AB27249 | 0.222 | 1.523 |
| compound AB27150 | 0.632 | 3.168 |
| compound AB27247 | 0.403 | 1.648 |
| compound AB27248 | 0.431 | 1.743 |
| compound AB24997 | 0.498 | 3.125 |
| compound AB27251 | 0.482 | 2.685 |
| compound AB27200 | 1.123 | 5.684 |
| compound AB27157 | 1.532 | 6.324 |
| compound AB27253 | 1.682 | 5.485 |
| compound AB27252 | 0.968 | 3.688 |
| compound AB27151 | 1.040 | 2.984 |
| compound AB27215 | 1.609 | 10.827 |
| compound AB27214 | 1.743 | 6.698 |
| compound AB27206 | 2.542 | 7.625 |
| compound AB27355 | 0.121 | 1.025 |
| compound AB27140 | 0.182 | 1.582 |
| compound AB27185 | 0.360 | 1.589 |
| compound AB27338 | 0.391 | 2.356 |
| compound AB27339 | 0.341 | 2.895 |
| compound AB27476 | 0.394 | 2.689 |
| compound AB27343 | 2.039 | 5.866 |
| compound AB27121 | 2.256 | 6.454 |
| compound AB27186 | 1.986 | 5.686 |
| compound AB27475 | 2.280 | 8.348 |
| compound AB27482 | 3.424 | 9.568 |
| compound AB27341 | 3.588 | 10.455 |
| compound AB27204 | 3.450 | 13.433 |
| compound AB27254 | 3.590 | 12.665 |
| compound AB27105 | 0.874 | 5.431 |
| compound AB27132 | 0.917 | 4.869 |
| compound AB27103 | 1.482 | 10.982 |
| compound AB27198 | 0.700 | 3.546 |
| compound AB27102 | 1.159 | 4.586 |
| compound AB27147 | 1.389 | 5.483 |
| compound AB27146 | 1.713 | 6.786 |
| compound AB27134 | 3.353 | 12.366 |
| compound AB27137 | 4.362 | 11.984 |
| compound AB27136 | 5.612 | 14.585 |
| compound AB27237 | 0.039 | 0.525 |
| compound AB27325 | 0.022 | 0.268 |
| compound AB27278 | 0.021 | 0.284 |
| compound AB27236 | 0.050 | 0.822 |
| compound AB27225 | 0.083 | 1.083 |
| compound AB27222 | 0.056 | 0.857 |
| compound AB27275 | 0.063 | 0.886 |
| compound AB27327 | 0.067 | 1.044 |
| compound AB27276 | 0.092 | 1.131 |
| compound AB27230 | 0.126 | 1.259 |
| compound AB27238 | 0.151 | 1.154 |
| compound AB27235 | 0.178 | 1.984 |
| compound AB27273 | 0.187 | 1.543 |
| compound AB27232 | 0.290 | 3.048 |
| compound AB27274 | 0.664 | 4.528 |
| compound AB27306 | 0.737 | 5.460 |
| compound AB27272 | 0.776 | 5.481 |
| compound AB27309 | 0.837 | 4.858 |
| compound AB27110 | 0.996 | 5.036 |
| compound AB27234 | 1.274 | 6.958 |
| compound AB27328 | 2.795 | 8.468 |
| compound AB27307 | 2.815 | 6.889 |
| compound AB27330 | 5.108 | 13.458 |
| compound AB27138 | 1.102 | 9.824 |
| compound AB27160 | 0.744 | 5.488 |
| compound AB27241 | 0.014 | 0.184 |
| compound AB27353 | 0.019 | 0.332 |
| compound AB27243 | 0.015 | 0.184 |
| compound AB27190 | 0.013 | 0.182 |
| compound AB27260 | 0.013 | 0.243 |
| compound AB27242 | 0.015 | 0.204 |
| compound AB27354 | 0.013 | 0.222 |
| compound AB27258 | 0.014 | 0.276 |
| compound AB27182 | 0.014 | 0.208 |
| compound AB27240 | 0.015 | 0.285 |
| compound AB27180 | 0.016 | 0.222 |
| compound AB27181 | 0.017 | 0.245 |
| compound AB27259 | 0.018 | 0.235 |
| compound AB27246 | 0.018 | 0.308 |
| compound AB27490 | 0.019 | 0.319 |
| compound AB27455 | 0.022 | 0.265 |
| compound AB27456 | 0.022 | 0.383 |
| compound AB27489 | 0.023 | 0.287 |
| compound AB27256 | 0.022 | 0.253 |
| compound AB27261 | 0.023 | 0.381 |
| compound AB27478 | 0.023 | 0.283 |
| compound AB27178 | 0.024 | 0.344 |
| compound AB27244 | 0.024 | 0.342 |
| compound AB27487 | 0.031 | 0.496 |
| compound AB27263 | 0.032 | 0.322 |
| compound AB27460 | 0.034 | 0.438 |
| compound AB27486 | 0.034 | 0.489 |
| compound AB27271 | 0.034 | 0.567 |
| compound AB27262 | 0.057 | 1.001 |
| compound AB27357 | 0.074 | 1.223 |
| compound AB27257 | 0.075 | 0.968 |
| compound AB27345 | 0.076 | 0.904 |
| compound AB27291 | 0.079 | 1.035 |
| compound AB27473 | 0.081 | 1.210 |
| compound AB27358 | 0.083 | 1.088 |
| compound AB27459 | 0.086 | 1.239 |
| compound AB27245 | 0.088 | 0.868 |
| compound AB27266 | 0.089 | 0.754 |
| compound AB27290 | 0.091 | 0.944 |
| compound AB27268 | 0.104 | 1.325 |
| compound AB27267 | 0.113 | 0.903 |
| compound AB27265 | 0.123 | 0.864 |
| compound AB27465 | 0.160 | 1.032 |
| compound AB27316 | 0.167 | 1.134 |
| compound AB27492 | 0.174 | 1.486 |
| compound AB27464 | 0.175 | 1.352 |
| compound AB27269 | 0.233 | 2.183 |
| compound AB27347 | 0.248 | 3.124 |
| compound AB27479 | 0.258 | 2.046 |
| compound AB27264 | 0.267 | 2.223 |
| compound AB27493 | 0.281 | 3.454 |
| compound AB27270 | 0.401 | 3.689 |
| compound AB27457 | 0.417 | 6.611 |
| compound AB27469 | 0.427 | 5.433 |
| compound AB27318 | 0.445 | 4.986 |
| compound AB27199 | 0.446 | 4.832 |
| compound AB27485 | 0.504 | 7.104 |
| compound AB27468 | 0.571 | 6.033 |
| compound AB27458 | 0.748 | 7.718 |
| compound AB27483 | 1.155 | 7.240 |
| compound AB27484 | 1.188 | 8.034 |
| compound AB27203 | 1.936 | 6.433 |
| compound AB27461 | 1.979 | 9.348 |
| compound AB27463 | 2.062 | 10.586 |
| compound AB27205 | 2.789 | 9.488 |
| compound AB27314 | 3.040 | 5.584 |
| compound AB27313 | 4.370 | 7.358 |
| compound AB27187 | 0.014 | 0.252 |
| compound AB27188 | 0.014 | 0.214 |
| compound AB27191 | 0.010 | 0.184 |
| compound AB27277 | 0.015 | 0.245 |
| compound AB27192 | 0.012 | 0.214 |
| compound AB27193 | 0.009 | 0.154 |
| compound AB27500 | 0.122 | 0.904 |
| compound AB31773 | 0.110 | 1.808 |
| compound AB31777 | 0.128 | 1.986 |
| compound AB31776 | 0.146 | 2.548 |
| compound AB31775 | 0.124 | 2.088 |
| compound AB31806 | 0.257 | 2.964 |
| compound AB31704 | 0.264 | 3.185 |
| compound AB27499 | 0.921 | 6.842 |
| compound AB31774 | 0.970 | 7.922 |
| compound AB31703 | 2.145 | 12.343 |
| compound AB27498 | 2.176 | 10.990 |
| compound AB31702 | 5.465 | 17.483 |
| compound AB31705 | 5.366 | 19.048 |
| compound AB27197 | 0.174 | 1.699 |
| compound AB27477 | 0.164 | 1.984 |
| compound AB27480 | 0.181 | 2.335 |
| compound AB27287 | 0.198 | 2.540 |
| compound AB27288 | 0.344 | 2.489 |
| compound AB27296 | 0.396 | 3.584 |
| compound AB27289 | 0.285 | 2.046 |
| compound AB27481 | 0.228 | 2.727 |
| compound AB27196 | 0.577 | 5.507 |
| compound AB27295 | 0.717 | 8.484 |
| compound AB27294 | 1.053 | 6.533 |
| compound AB27283 | 0.874 | 3.382 |
| compound AB27285 | 0.364 | 1.348 |
| compound AB27299 | 0.674 | 1.838 |
| compound AB36527 | 39.731 | 36.508 |
| compound AB36528 | 19.482 | 18.965 |

Note: IC₅₀ refered to 50% inhibiting concentration, ie, the concentration of the compound when 50% inhibitory effect was achieved. Con-NCI-H82 refered to normal and untreated NCI-H82 cell, which was used as control; ov-NNMT NCI-H82 refered to the NCI-H82 cell transfected with virus vector carrying NNMT gene, the NNMT protein was overexpressed in the ov-NNMT NCI-H82 cell.

As was shown in Table 3, the NNMT protein in NCI-H82 cell was overexpressed in the Example, the results further confirmed that the compounds prepared in the Examples of the present invention had significant inhibitory effect on tumor cell with low or no expression of NNMT gene while the compounds prepared in the Examples of the present invention had weak inhibitory effect on tumor cells with high expression of NNMT gene. Decreasing the expression of NNMT gene in tumor could significantly improve the inhibitory effect of the compounds prepared in the Examples of the present invention on tumor, the expression level of NNMT gene in tumor cell was significantly negative correlation with the sensitivity of tumor cell to the compounds prepared in the Examples of the present invention. Therefore, the compounds prepared in the Examples of the present invention had more significant inhibitory effect on tumor cell with low or no expression of NNMT gene, the tumor cell with low or no expression of NNMT gene were highly sensitive to the compounds prepared in the Examples of the present invention. Therefore, the compounds prepared in the Examples of the present invention has excellent precision treatment on the tumor cell with low expression of NNMT gene.

**Example 179** the sensitivity of different tumor cell lines to the compounds prepared in the Examples of the present invention was studied

The inhibitory effect of the compounds prepared in the Examples of the present invention on various tumor cell lines was detected using cell viability assay reagent.

### Experimental background:

Cell viability was detected using the Promega CellTiter-Glo kit, cell viability was determined by directly measuring intracellular ATP content. In the experiment, the IC₅₀ value of the compounds prepared in the Examples of the present invention on various tumor cell lines was detected.

### Experimental method and result:

Each tumor cell was cultured in relevant medium. After cell passage, the gradient diluted compounds prepared in the Examples of the present invention was added. After 3 days of culture, the relevant IC₅₀ (50% inhibiting concentration) was measured. The name, source and culture conditions of each tumor cell line were as follows:
Cell line NCI-H82 (ATCC, No. HTB-175) was cultured in 10% fetal bovine serum-containing RPMI1640 medium (+P/S).
Cell line G-401 (ATCC, No. CRL-1441) was cultured in 10% fetal bovine serum-containing McCoy's 5a medium (+P/S).
Cell line MDA-MB-453 (ATCC, No. HTB-131) was cultured in 10% fetal bovine serum-containing Leibovitz's L-15 medium (+P/S).
Cell line SW48 (ATCC, No. CCL-231) was cultured in 10% fetal bovine serum-containing Leibovitz's L-15 medium (+P/S).
Cell line CFPAC-1 (ATCC, No. CRL-1918) was cultured in 10% fetal bovine serum-containing IMDM medium (+P/S).
Cell line 786-O (ATCC, No. CRL-1932) was cultured in 10% fetal bovine serum-containing RPMI1640 medium (+P/S).
Cell line GB-1 (JCRB, No. IFO50489) was cultured in 10% fetal bovine serum-containing DMEM medium (+P/S).
Cell line SF-126 (JCRB, No. IFO50286) was cultured in 10% fetal bovine serum-containing EMEM medium (+P/S).

The experimental result showed the sensitivity of different tumor cells to the compounds prepared in the Examples of the present invention, NCI-H82 (human small cell lung cancer cell), G-401 (human renal carcinoma Wilms cell), MDA-MB-453 (breast cancer cell) and SW48 (human colon adenocarcinoma cell) were sensitive to the compounds prepared in the Examples of the present invention with low IC₅₀ value, while 786-O (clear cell renal cell adenocarcinoma cell), CFPAC-1 (human pancreatic cancer cell), GB-1 (human brain glioblastoma cell) and SF126 (human glioblastoma multiforme cell) were not sensitive to the compounds prepared in the Examples of the present invention with high IC₅₀ value.

### Example 180

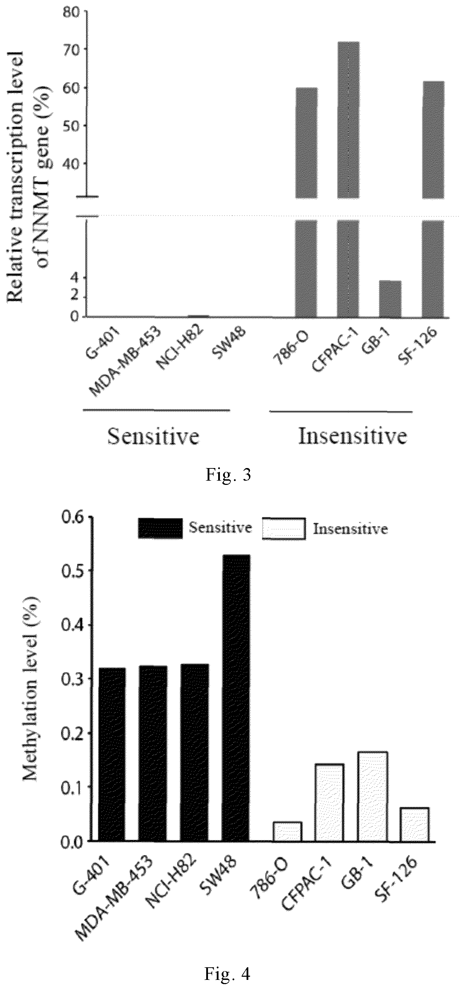

The mRNA transcription level of NNMT gene in four tumor cell lines sensitive to the compounds prepared in the Examples of the present invention and four tumor cell lines insensitive to the compounds prepared in the Examples of the present invention was measured using RT-qPCR gene expression analysis test, and the expression of NNMT gene in the tumor cell lines was measured respectively. The results were shown in Fig.3.

As shown in Fig.3, the mRNA transcription level of NNMT gene in four tumor cell lines (NCI-H82, G-401, MDA-MB-453 and SW48) sensitive to the compounds prepared in the Examples of the present invention and four tumor cell lines (786-O, CFPAC-1, GB-1, and SF126) insensitive to the compounds prepared in the Examples of the present invention was measured using RT-qPCR gene expression analysis test, the results showed the expression of NNMT gene was low in sensitive cell lines (NCI-H82, G-401, MDA-MB-453 and SW48) , and the expression of NNMT gene was high in insensitive cell lines (786-O, CFPAC-1, GB-1 and SF126).

Therefore, the Fig.3 showed that compared with tumor cell lines with high expression of NNMT gene, the inhibitory effect of the compounds prepared in the Examples of the present invention on tumor cell lines with low expression of NNMT gene was significantly enhanced, i.e, the expression of NNMT gene in tumor cell was negatively correlated with the sensitivity of tumor cell to the compounds prepared in the Examples of the present invention. Therefor, the tumor with low expression of NNMT gene was highly sensitive to the compounds prepared in the Examples of the present invention, the compounds prepared in the Examples of the present invention has excellent precision treatment on the tumor with low expression of NNMT gene.

### Example 181

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in four tumor cell lines (NCI-H82, G-401, MDA-MB-453 and SW48) sensitive to the compounds prepared in the Examples of the present invention and four tumor cell lines (786-O, CFPAC-1, GB-1 and SF126) insensitive to the compounds prepared in the Examples of the present invention. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.4, Fig.5, and Fig.6.

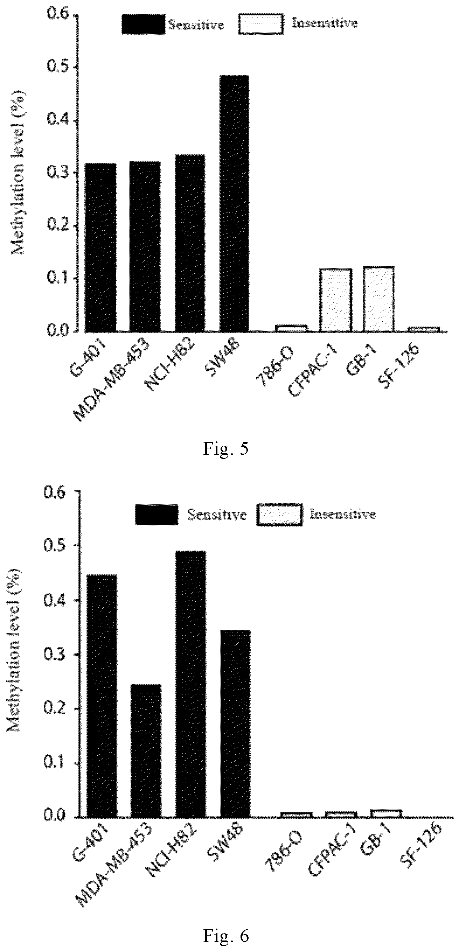

As shown in Fig.4 (the promoter region of NNMT gene), Fig.5 (the region from 1050 bp before the transcription start site to 499 bp after the transcription start site in NNMT gene) and Fig.6 (the region from 1050 bp to 193 bp before the transcription start site in NNMT gene), the compounds prepared in the Examples of the present invention had significantly stronger inhibitory effect on tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, while the compounds prepared in the Examples of the present invention had significantly weaker inhibitory effect on tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499bp after the transcription start site in NNMT gene and the region from 1050 bp to 193 bp before the transcription start site in NNMT gene was positively correlated with the sensitivity of tumor cell to the compounds prepared in the Examples of the present invention.

### Example 182

The methylation of specific DNA CpG sites from 840 bp (i.e., site 114165695 on human chromosome 11) to 469 bp (i.e., site 114166066 on human chromosome 11) before the transcription start site in NNMT gene in three tumor cell lines (NCI-H82, G-401 and SW48) sensitive to the compounds prepared in the Examples of the present invention and three tumor cell lines (786-O, CFPAC-1 and SF126) insensitive to the compounds prepared in the Examples of the present invention was studied.

Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, then PCR amplification and sequencing analysis were performed on the region using corresponding primers to measure the methylation level of CpG site in the DNA region.

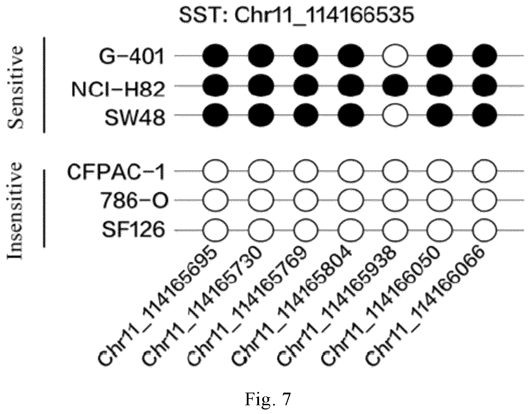

The study showed that almost all of the seven CpG sites (site 114165695, site 114165730, site 114165769, site 114165804, site 114165938, site 114166050 and site 114166066 on the human chromosome 11) were methylated in cell lines (NCI-H82, G-401 and SW48) sensitive to the compounds prepared in the Examples of the present invention, while none of the above seven CpG sites were methylated in cell lines ((786-O, CFPAC-1 and SF126) insensitive to the compounds prepared in the Examples of the present invention, the methylation of related sites was shown in Fig.7.

The sites of the nucleotide sequence in SEQ ID NO: 1 corresponding to the site 114165695, site 114165730, site 114165769, site 114165804, site 114165938, site 114166050 and site 114166066 on the human chromosome 11 were as follows:

| The site on the human chromosome 11 | Corresponding to the sites of the nucleotide sequence in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### Example 183

The methylation level of DNA in cell was maintained by DNA methylation enzymes DNMT3a, DNMT3b and DNMT1. The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumor was determined in the Example.

### Experiment method and result:

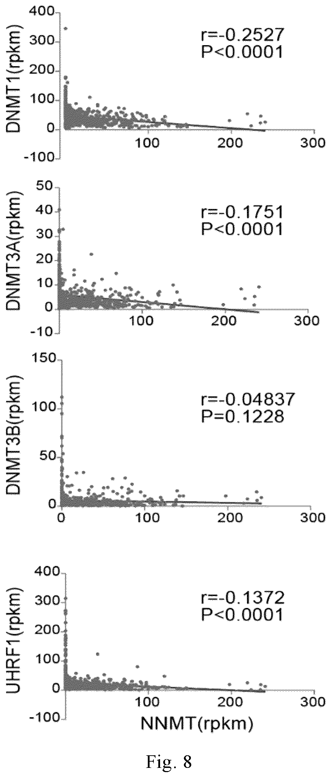

The expression of NNMT gene, DNMT1, UHRF1, DNMT3a and DNMT3b in various cells were obtained from a public database (Cancer Cell Line Encyclopedia, CCLE, 1019 cells in total). Then, the correlation between expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.8.

### The Fig.8 showed the expression of NNMT was negatively correlated with the expression of DNA methylase and UHRF1 in each cell.

### Example 184

**The activity of mitochondria permeability transition pore in related cell** was **investigated.**

### Experimental background:

The mitochondria permeability transition pore (mPTP) was a non-specific channel on the inner membrane of mitochondria, which could allow small molecules with molecular less than 1.5KD to pass freely, and its activity was affected by peroxides (such as H₂O₂), pH and calcium ions in mitochondria. Some cells had active mitochondria permeability transition pore, while some cells had inactive mitochondria permeability transition pore. For cells with active mitochondria permeability transition pore, adding peroxide (such as H₂O₂) could increase the activity of mitochondria permeability transition pore, resulting in a decrease in mitochondria membrane potential. For cells with inactive mitochondria permeability transition pore, adding peroxide (such as H₂O₂) had no significant effect on the activity of the mitochondria permeability transition pore, and the mitochondria membrane potential had no significant change. Based on this principle, whether the mPTP was active or inactive in specific cell could be determined by measuring the change of potential difference of the mitochondria membrane under peroxide stimulation

### Experimental method and result:

Daoy cell (human medulloblastoma cell, ATCC no. HTB-186) were cultured in 10% fetal bovine serum-containing DMEM (+p/s), and then 1.5 µM Cyclosporin A (CSA, CSA could effectively inhibit the activity of mitochondria permeability transition pore) was added to the medium, and the cell cultured in medium without the addition of CsA were used as blank control. The mitochondria membrane potential difference was detected by Tetramethylrhodamine (TMRM), the high fluorescence intensity of TMRM represented the high membrane potential difference, the result was shown in Table 4.

**Table 4 Relative TMRM signal intensity (%) in Daoy cell after different treatments**

| **Cell** | **Daoy cell** | | | |
|---|---|---|---|---|
| CsA | - | - | + | + |
| H₂O₂ | - | + | - | + |
| mean value | 100 | 82 | 125 | 121 |
| Standard deviation | 7 | 11 | 14 | 11 |
| Repeat times | 3 | 3 | 3 | 3 |
| P value | | <0.05 | <0.01 | |

| | | | | |
|---|---|---|---|---|
| Remarks: "+" represented existence, "-" represented none. | | | | |

It could be seen from Table 4 that as for normal Daoy cell cultured in medium without the addition of CSA, the membrane potential was significantly down-regulated under the action of H₂O₂, indicating that the mitochondria permeability transition pore was active in Daoy cell. However, as for normal Daoy cell cultured in medium with the addition of CSA (CsA could inhibit the activity of mitochondria permeability transition pore (mPTP)), the membrane potential was not down-regulated under the action of H₂O₂, indicating that active mPTP of Daoy cell was inhibited by CsA and the mPTP of Daoy cell become inactive, H₂O₂ did not cause a decrease in membrane potential in this case.

Therefore, Table 4 showed that the mitochondria permeability transition pore (mPTP) was active in Daoy cell.

### Example 185

The inhibitory effect of the compounds prepared in the Examples of the present invention on the mPTP-active Daoy cell, and the mPTP-inactive Daoy cell which was constructed by transfecting the Daoy cell with shRNA that specificly induce the degradation of PPIF mRNA.

The peptidyl-prolyl cis-trans isomerase F was abbreviated as PPIF, the protein identification number was UniProtKB/Swiss Prot: P30405, and the gene identification number was NCBI Entrez Gene: 10105

### 1. Construction of Daoy cell having inactive mitochondria permeability transition pore (mPTP)

### 1.1 Experimental background:

The activity of mitochondria permeability transition pore (mPTP) was regulated by the PPIF protein. When PPIF protein was inhibited, mPTP activity was significantly decreased. PPIF protein activity was restricted by the expression level of PPIF protein in cell. The PPIF protein expression level in Daoy cell can be specifically decreased by transfecting the Daoy cell with shRNA that could specificly induce the degradation of PPIF mRNA, thereby constructing Daoy cell having inactive mitochondria permeability transition pore (mPTP) (abbreviated as mPTP-inactive Daoy cell).

### 1.2 Experiment method and result:

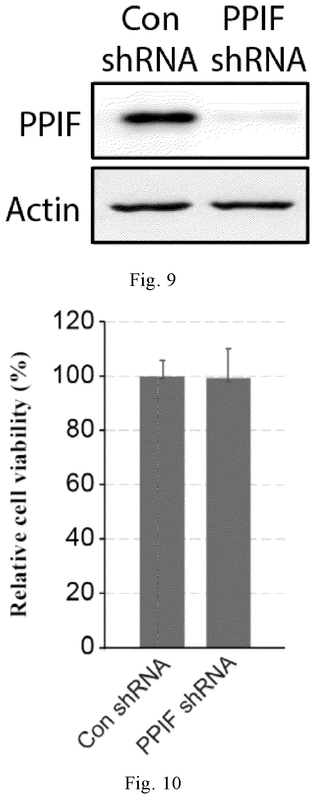

A viral vector carrying shRNA that could specifically induces the degradation of PPIF mRNA was obtained using cloning technology, the shRNA (the nucleotide sequence was GTTCTTCATCTGCACCATAAA (SEQ ID NO: 2)) carried by viral vector could specifically induce the degradation of PPIF mRNA, the Daoy cell was tranfected with the viral vector carring shRNA that could specifically induce the degradation of PPIF mRNA, and the Daoy cell transfected with empty virus vector without carrying shRNA that can specificly induce the degradation of PPIF mRNA was used as control. The expression level of PPIF protein in Daoy cell was detected using Western blot technique, and the result was shown in Fig. 9. The Fig.9 showed the PPIF in the Daoy cell tranfected with the shRNA that could specifically induce the degradation of PPIF (mPTP regulated protein) mRNA was not expressed (i.e. PPIF shRNA in Fig.9), while the PPIF in the Daoy cell not tranfected with the shRNA that could specifically induce the degradation of PPIF mRN was normally expressed (i.e. con shRNA in Fig.9, as the control). The activity of mitochondria permeability transition pore was detected in the Daoy cell transfected with empty virus vector without carrying shRNA that could specificly induce the degradation of PPIF mRNA and the Daoy cell transfected with virus vector carrying shRNA that could specificly induce the degradation of PPIF mRNA was detected, and the mitochondria membrane potential difference was detected by Tetramethylrhodamine (TMRM) according to the method described in Example 184 above, the high fluorescence intensity of TMRM represented the high membrane potential difference, the results were shown in Table 5 and Table 6.

**Table 5 Relative TMRM signal intensity (%) in the Daoy cell transfected with empty virus vector without carrying shRNA that could specificly induce the degradation of PPIF mRNA after different treatments**

| **Cell** | The Daoy cell transfected with empty virus vector without carrying shRNA that could specificly induce the degradation of PPIF mRNA | | | |
|---|---|---|---|---|
| CsA | - | - | + | + |
| H₂O₂ | - | + | - | + |
| mean value | 100 | 79 | 131 | 126 |
| Standard deviation | 6 | 7 | 10 | 8 |
| Repeat times | 3 | 3 | 3 | 3 |

**Table 6 Relative TMRM signal intensity (%) in the Daoy cell transfected with virus vector carrying shRNA that could specificly induce the degradation of PPIF mRNA after different treatments**

| **Cell** | The Daoy cell transfected with virus vector carrying shRNA that could specificly induce the degradation of PPIF mRNA | | | |
|---|---|---|---|---|
| CsA | - | - | + | + |
| H₂O₂ | - | + | - | + |
| mean value | 100 | 95 | 105 | 101 |
| Standard deviation | 5 | 6 | 8 | 11 |
| Repeat times | 3 | 3 | 3 | 3 |

The Table 5 and Table 6 showed the mitochondria permeability transition pore (mPTP) is active in the Daoy cell transfected with empty virus vector without carrying shRNA that could specificly induce the degradation of PPIF mRNA, the mitochondria permeability transition pore (mPTP) is inactive in the Daoy cell transfected with virus vector carrying shRNA that could specificly induce the degradation of PPIF mRNA, therefore, the Daoy cell having inactive mitochondria permeability transition pore (mPTP) was successfully constructed by transfecting the Daoy cell with shRNA that could specificly induce the degradation of PPIF (mPTP regulated protein) mRNA.

The cell viability of the mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNAwas detected using the Promega CellTiter-Glo kit which reflected cell viability by measuring intracellular ATP content, the results was shown in Fig.10, it could be seen from Fig.10 that cell viability of mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNA was almost the same, and the difference in cell viability was not statistically significant.

### 2. The correlation between the inhibitory effect of the compound sprepared in the Examples of the present invention on tumor cell and the activity level of mPTP was investigated

### 2.1 Experimental background:

The inhibitory effect (IC₅₀ value) of the compounds prepared in the Examples of the present invention on the mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA was detected using Promega CellTiter-Glo kit which refleced cell viability by directly measured intracellular ATP content.

### 2.2 Experiment method and result:

The mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA were cultured in 10% fetal bovine serum-containing DMEM (+p/s), the 50% inhibiting concentration (IC₅₀) of the compounds prepared in the Examples of present invention on the two types of cells was detected, and the experimental results were shown in Table 7:

**Table 7 the inhibitory effect of the compounds prepared in the Examples of present invention on the mPTP-active Daoy cell and mPTP-inactive Daoy cell (IC₅₀, µM)**

| **Compound** | **mPTP-active Daoy cell (IC₅₀, µM)** | **mPTP-inactive Daoy cell (IC₅₀, µM)** |
|---|---|---|
| compound AB24995 | 6.32 | 1.98 |
| compound AB27101 | 5.68 | 1.93 |
| compound AB27356 | 5.54 | 0.94 |
| compound AB27175 | 3.86 | 1.23 |
| compound AB27171 | 1.94 | 0.69 |
| compound AB27219 | 5.09 | 2.12 |
| compound AB27470 | 3.23 | 0.93 |
| compound AB27173 | 3.43 | 1.02 |
| compound AB27172 | 3.94 | 1.38 |
| compound AB24997 | 8.56 | 2.34 |
| compound AB27251 | 7.89 | 1.96 |
| compound AB27200 | 6.03 | 2.15 |
| compound AB27355 | 5.92 | 1.17 |
| compound AB27140 | 3.66 | 1.48 |
| compound AB27185 | 4.49 | 1.85 |
| compound AB27338 | 6.62 | 2.09 |
| compound AB27132 | 7.95 | 3.27 |
| compound AB27103 | 8.98 | 4.09 |
| compound AB27198 | 7.55 | 1.89 |
| compound AB27325 | 2.01 | 0.38 |
| compound AB27225 | 5.33 | 1.16 |
| compound AB27222 | 1.75 | 0.66 |
| compound AB27230 | 2.92 | 0.92 |
| compound AB27306 | 9.91 | 3.23 |
| compound AB27309 | 8.58 | 3.37 |
| compound AB27138 | 8.82 | 3.62 |
| compound AB27190 | 0.13 | 0.04 |
| compound AB27182 | 0.16 | 0.05 |
| compound AB27181 | 0.19 | 0.07 |
| compound AB27455 | 0.36 | 0.11 |
| compound AB27456 | 0.38 | 0.13 |
| compound AB27489 | 0.37 | 0.17 |
| compound AB27256 | 0.33 | 0.12 |
| compound AB27261 | 0.38 | 0.18 |
| compound AB27178 | 0.38 | 0.19 |
| compound AB27487 | 0.57 | 0.22 |
| compound AB27486 | 0.58 | 0.28 |
| compound AB27257 | 2.16 | 0.88 |
| compound AB27345 | 6.69 | 2.04 |
| compound AB27459 | 2.43 | 0.84 |
| compound AB27316 | 5.89 | 1.17 |
| compound AB27492 | 3.75 | 1.21 |
| compound AB27347 | 7.35 | 2.89 |
| compound AB27457 | 8.61 | 2.16 |
| compound AB27318 | 8.02 | 3.34 |
| compound AB27458 | 10.28 | 3.01 |
| compound AB27483 | 8.24 | 2.08 |
| compound AB27463 | 10.63 | 4.76 |
| compound AB27187 | 0.21 | 0.05 |
| compound AB27188 | 0.23 | 0.04 |
| compound AB27191 | 0.25 | 0.04 |
| compound AB27277 | 0.16 | 0.03 |
| compound AB27192 | 0.19 | 0.03 |
| compound AB27193 | 0.17 | 0.03 |
| compound AB27500 | 3.18 | 0.62 |
| compound AB31773 | 2.98 | 0.49 |
| compound AB31777 | 6.99 | 1.23 |
| compound AB31775 | 5.38 | 1.39 |
| compound AB31704 | 3.69 | 0.77 |
| compound AB31774 | 9.92 | 1.53 |
| compound AB27498 | 9.89 | 1.96 |
| compound AB27197 | 3.57 | 1.02 |
| compound AB27481 | 2.72 | 0.65 |
| compound AB27196 | 6.57 | 2.08 |
| compound AB27283 | 10.59 | 3.24 |
| compound AB36527 | 39.08 | 40.86 |
| compound AB36528 | 15.72 | 17.89 |

Noted: IC₅₀ refered to 50% inhibiting concentration, ie, the concentration of the inhibitory compound when 50% inhibitory effect was achieved. The mPTP-active Daoy cell refered to the Daoy cell transfected with empty virus vector without carrying shRNA that can specificly induce the degradation of PPIF mRNA; the mPTP-inactive Daoy cell refered to the Daoy cell transfected with virus vector carrying shRNA that can specificly induce the degradation of PPIF mRNA.

As was shown in Table 7, the compounds prepared in the Examples of the present invention had significant inhibitory effect on mPTP-inactive Daoy cell, while the compounds prepared in the Examples of the present invention had weaker inhibitory effect on mPTP-active Daoy cell. Decreasing the mPTP activity in Daoy cell could significantly improve the inhibitory effect of the compounds prepared in the Examples of the present invention on tumor, therefore, the compounds prepared in the Examples of the present invention had more significant inhibitory effect on mPTP-inactive Daoy cell, the mPTP-inactive Daoy cell was highly sensitive to the compounds prepared in the Examples of the present invention. Therefore, the compounds prepared in the Examples of the present invention had excellent precision treatment effect on mPTP-inactive Daoy cell. As mentioned above, when PPIF protein was inhibited, mPTP activity was significantly decreased, inhibiting the expression level of PPIF protein could decrease the mPTP activity in Daoy cell. Therefore, the compounds prepared in the Examples of the present invention had more significant inhibitory effect on Daoy cell with low expression of PPIF protein, while the compounds prepared in the Examples of the present invention had weaker inhibitory effect on Daoy cell with normal expression of PPIF protein, indicating that decreasing the expression level of PPIF protein could significantly improve the inhibitory effect of the compounds prepared in the Examples of the present invention on tumnor. Therefore, the compounds prepared in the Examples of the present invention had more significant inhibitory effect on Daoy cell with low expression of PPIF protein, i.e, the Daoy cell with low expression of PPIF protein was more sensitive to the compounds prepared in the Examples of the present invention compared with the Daoy cell with normal expression of PPIF protein. The compounds prepared in the Example of the present invention had excellent precision treatment on Daoy cell with low expression of PPIF protein.

All documents mentioned in the present invention are incorporated herein by reference, as if each document is individually cited for reference. It should be understood that those skilled in the art will be able to make various changes or modifications to the present invention after reading the teachings of the present invention, which also fall within the scope of the claims appended hereto.

## Claims

1. A compound of formula I, or an optical isomer thereof, or a racemate thereof, or a solvate thereof, or a pharmaceutically acceptable salt thereof, or a deuterated compound thereof 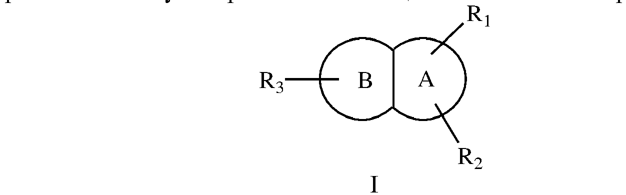 wherein,
ring A is substituted or unsubstituted C6-C14 aromatic ring, substituted or unsubstituted C3-C14 cycloalkane ring, substituted or unsubstituted 3-14 membered heterocycloalkane ring, or substituted or unsubstituted 5-14 membered heteroaromatic ring;
ring B is absent, substituted or unsubstituted C6-C14 aromatic ring, substituted or unsubstituted C3-C14 cycloalkane ring, substituted or unsubstituted 3-14 membered heterocycloalkane ring, or substituted or unsubstituted 5-14 membered heteroaromatic ring;
R₁ is 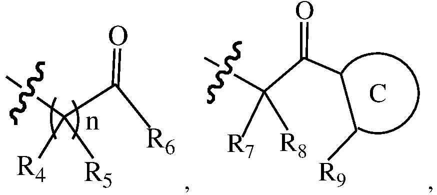
substituted or unsubstituted C6-C16 aryl-substituted or unsubstituted C1-C6 alkyl-;
ring C is substituted or unsubstituted C6-C16 aromatic ring, substituted or unsubstituted C3-C16 cycloalkane ring, substituted or unsubstituted 3-16 membered heterocycloalkane ring, or substituted or unsubstituted 5-16 membered heteroaromatic ring;
R₂ is hydrogen, 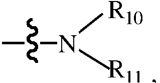 substituted or unsubstituted C6-C12 aryl, substituted or unsubstituted 5-12 membered heteroaryl, substituted or unsubstituted C6-C12 aryl-substituted or unsubstituted C1-C8 alkyl-, substituted or unsubstituted 5-12 membered heteroaryl-substituted or unsubstituted C1-C8 alkyl-;
R₃ is absent, hydrogen, halogen, substituted or unsubstituted C1-C10 alkyl, substituted or unsubstituted C3-C12 cycloalkyl, substituted or unsubstituted C3-C12 cycloalkyl-substituted or unsubstituted C1-C8 alkyl-, substituted or unsubstituted C1-C8 alkoxyl, substituted or unsubstituted C1-C8 alkylthio, substituted or unsubstituted C2-C8 acyl, substituted or unsubstituted C2-C8 acyl-substituted or unsubstituted C1-C8 alkyl-, substituted or unsubstituted C6-C14 aryl, substituted or unsubstituted 5-12 membered heteroaryl, substituted or unsubstituted C6-C14 aryl-sulfuryl-, substituted or unsubstituted C6-C14 aryl-C(O)-, substituted or unsubstituted C6-C14 aryl-C(O)-substituted or unsubstituted C1-C8 alkyl-, substituted or unsubstituted C6-C14 aryl-substituted or unsubstituted C1-C8 alkyl-, substituted or unsubstituted 5-12 membered heteroaryl-substituted or unsubstituted C1-C8 alkyl-;
R₄ and R₅ are each independently hydrogen, substituted or unsubstituted C1-C10 alkyl, substituted or unsubstituted C3-C12 cycloalkyl;
R₆ is substituted or unsubstituted C6-C14 aryl, substituted or unsubstituted 5-12 membered heteroaryl, substituted or unsubstituted C3-C14 cycloalkyl, substituted or unsubstituted 3-14 membered heterocycloalkyl;
R₇ is hydrogen, substituted or unsubstituted C1-C8 alkyl, substituted or unsubstituted C3-C10 cycloalkyl;
R₈ and R₉ are connected to form substituted or unsubstituted C3-C14 cycloalkane ring, substituted or unsubstituted 3-14 membered heterocycloalkane ring;
R₁₀ and R₁₁ are each independently hydrogen, substituted or unsubstituted C1-C10 alkyl, substituted or unsubstituted C2-C10 acyl, substituted or unsubstituted C6-C14 aryl, substituted or unsubstituted 5-12 membered heteroaryl, substituted or unsubstituted C6-C14 aryl-substituted or unsubstituted C1-C6 alkyl-, substituted or unsubstituted C6-C14 aryl-C(O)-substituted or unsubstituted C1-C6 alkyl-, substituted or unsubstituted 5-12 membered heteroaryl-substituted or unsubstituted C1-C6 alkyl-; or R₁₀ and R₁₁ are connected to form substituted or unsubstituted C3-C12 cycloalkane ring, substituted or unsubstituted 3-12 membered heterocycloalkane ring, substituted or unsubstituted 5-12 membered heteroaromatic ring;
n is 0, 1, 2, 3, 4 or 5;
each "substituted" means that one or more (preferably 1, 2, 3, 4, 5, 6, 7, or 8) hydrogen atoms on the ring or group are independently substituted by substituent selected from the group consisting of C1-C8 alkyl, C3-C8 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl;
the heterocyclic ring of the heterocycloalkyl, heteroaryl, heterocycloalkane ring and heteroaromatic ring has 1-4 (preferably 1, 2, 3 or 4) heteroatoms independently selected from the group consisting of N, O and S.

2. The compound of formula I, or an optical isomer thereof, or a racemate thereof, or a solvate thereof, or a pharmaceutically acceptable salt thereof, or a deuterated compound thereof;

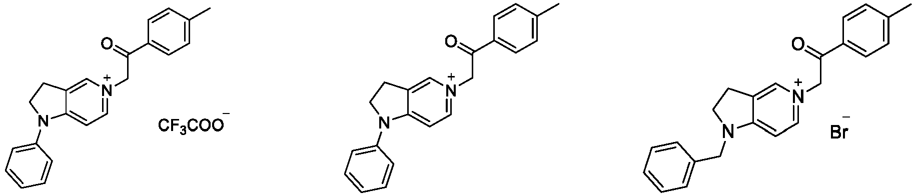

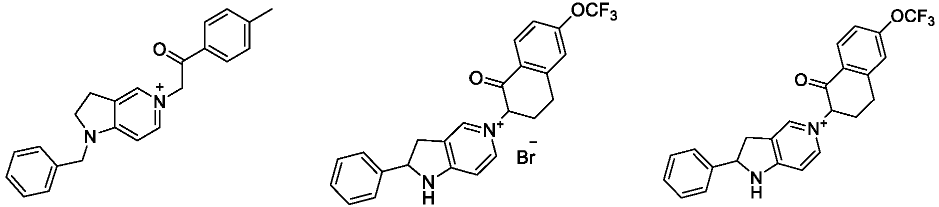

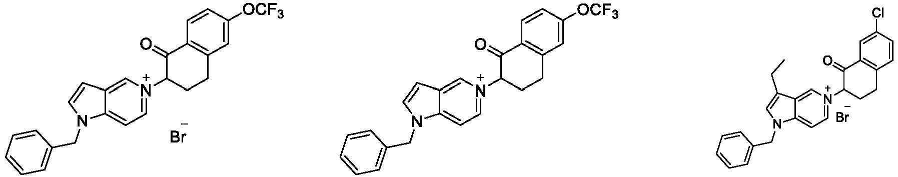

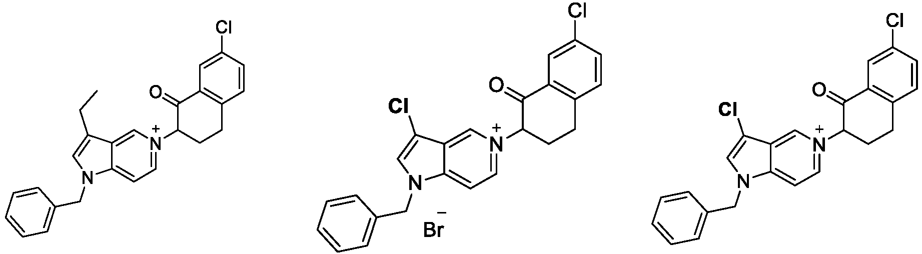

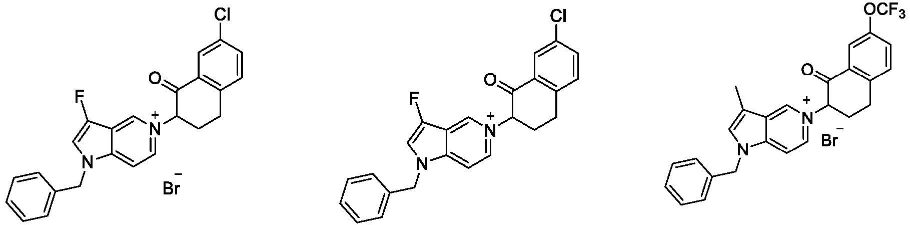

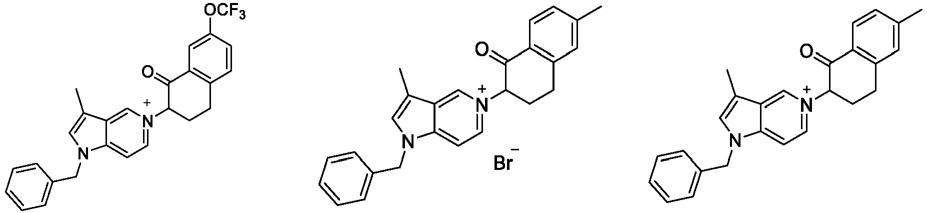

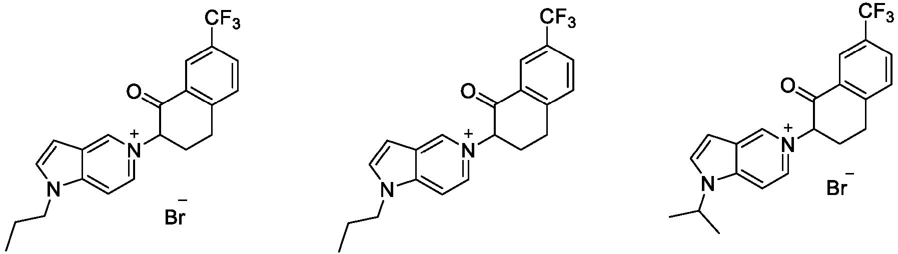

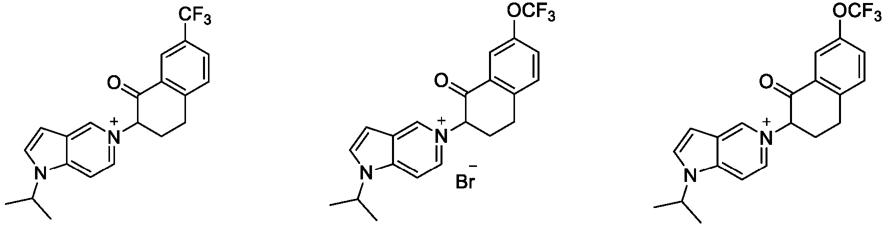

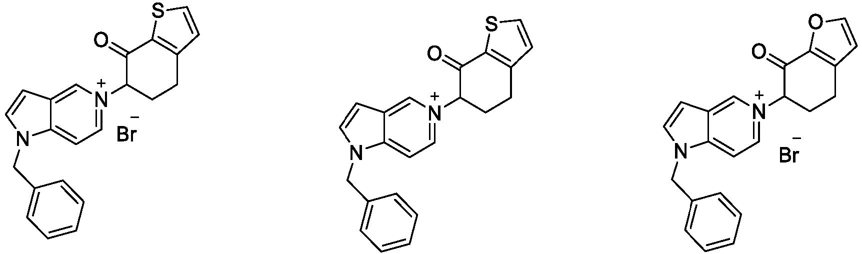

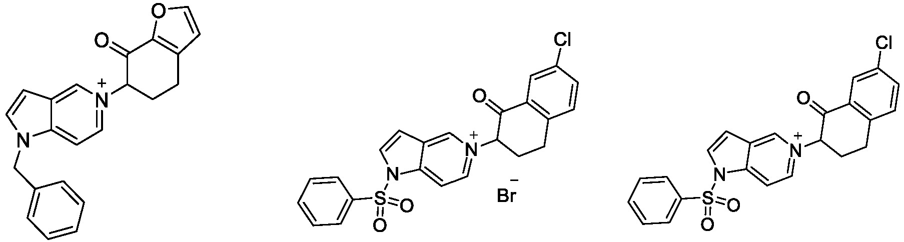

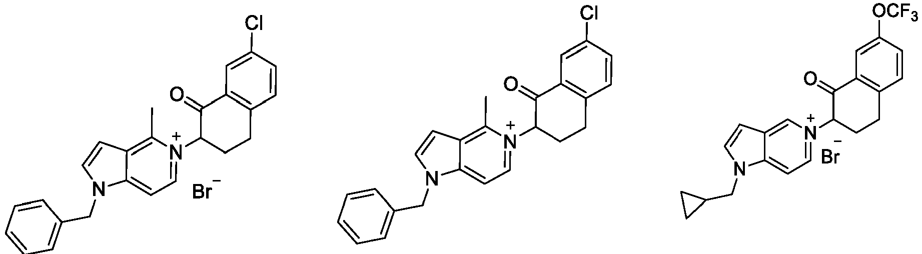

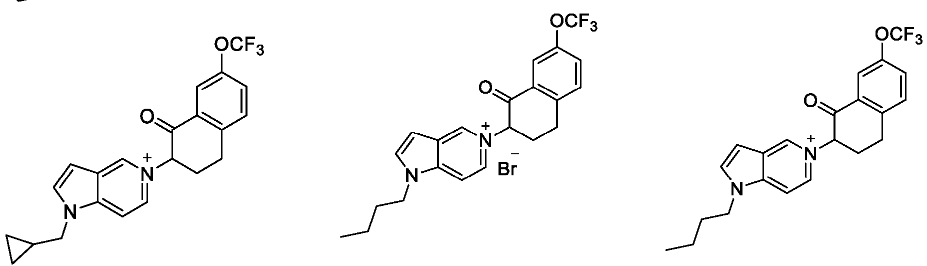

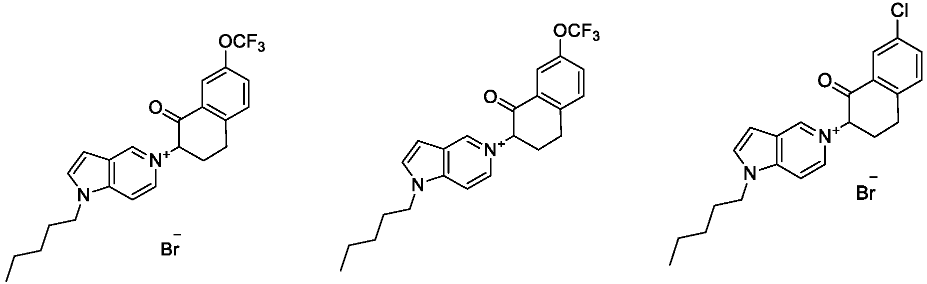

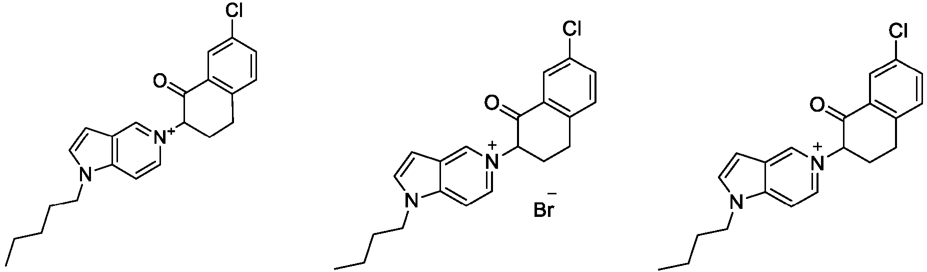

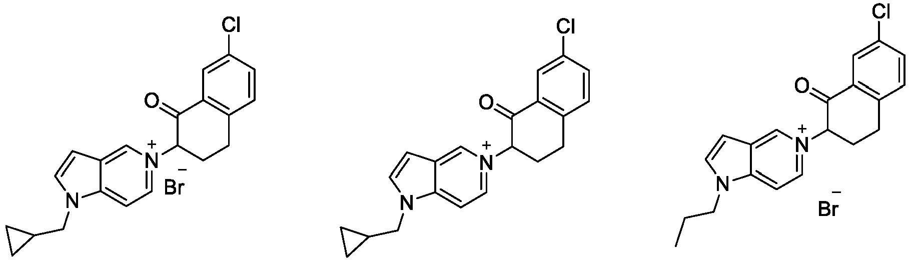

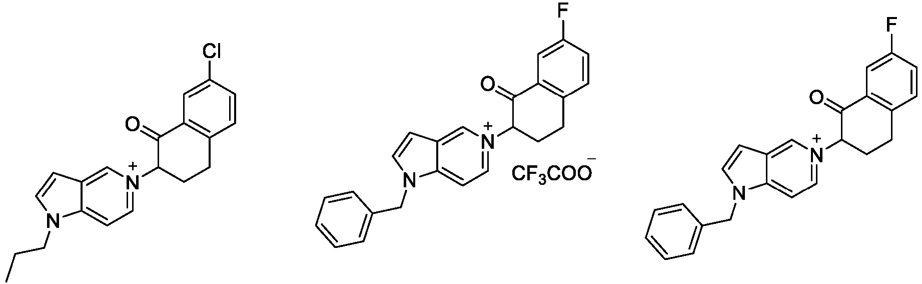

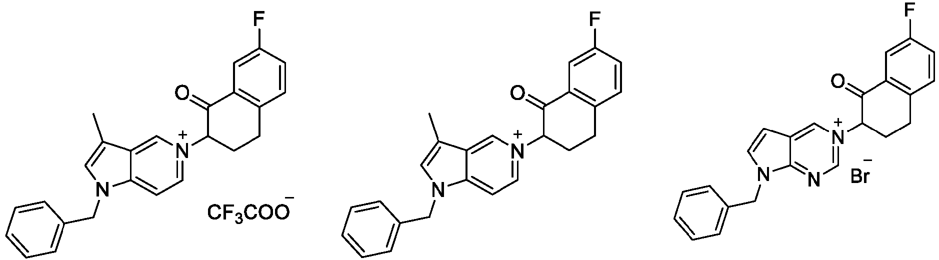

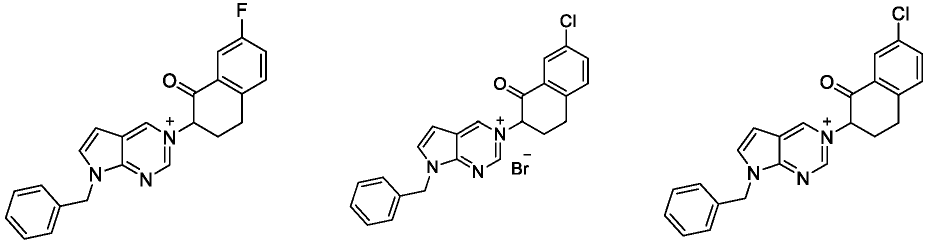

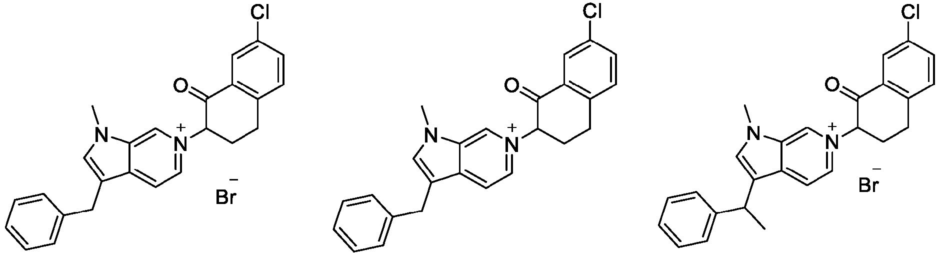

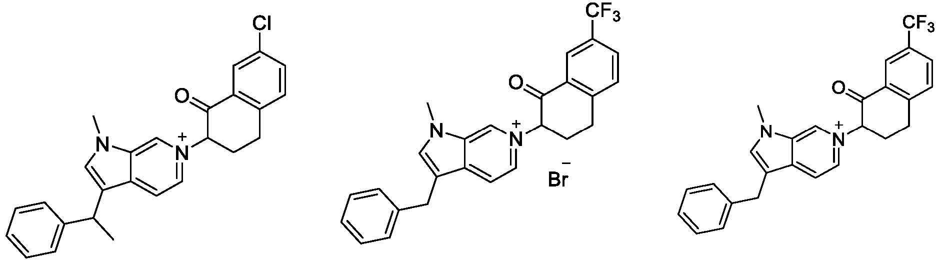

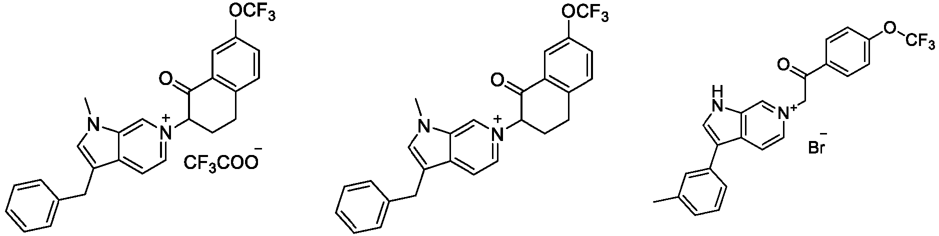

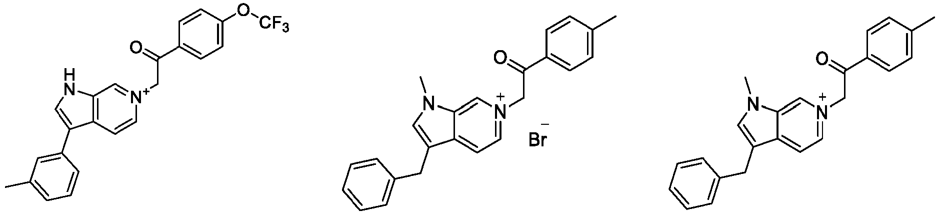

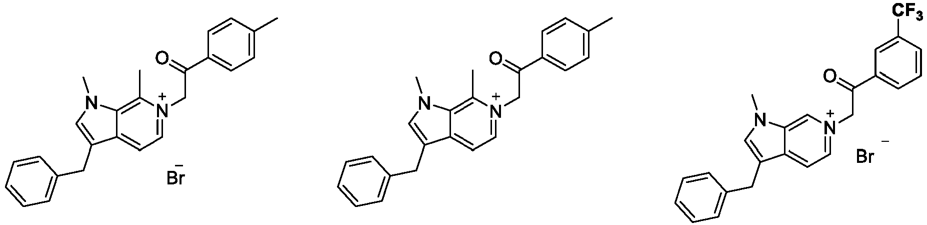

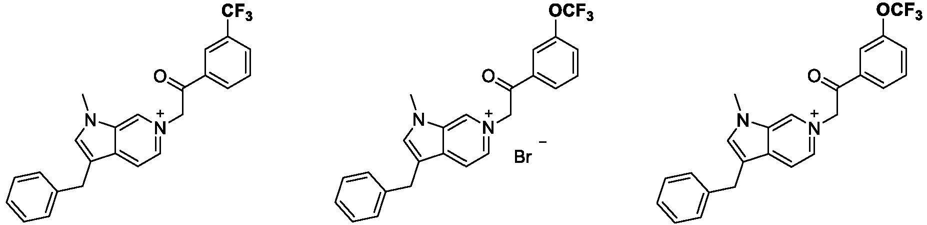

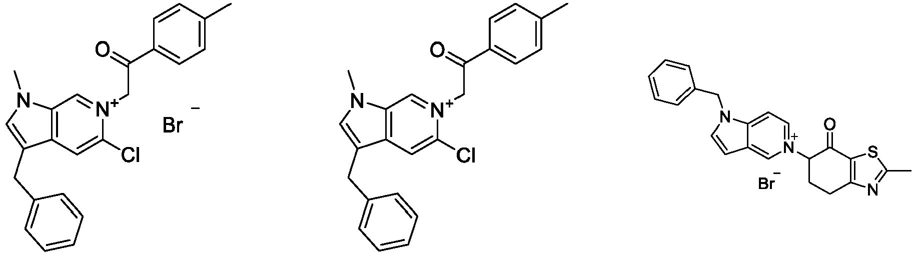

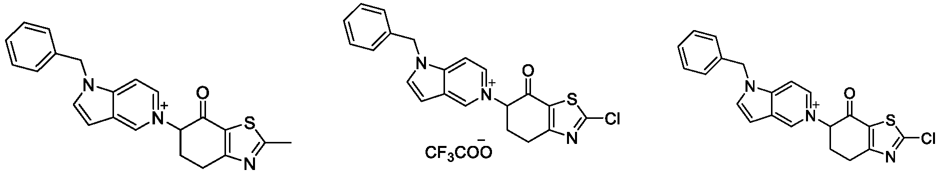

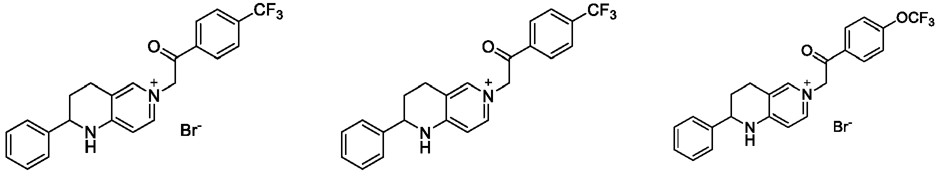

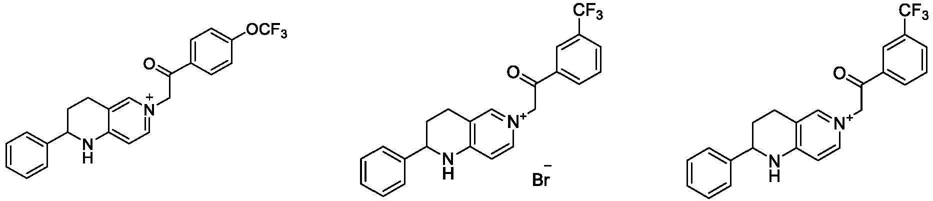

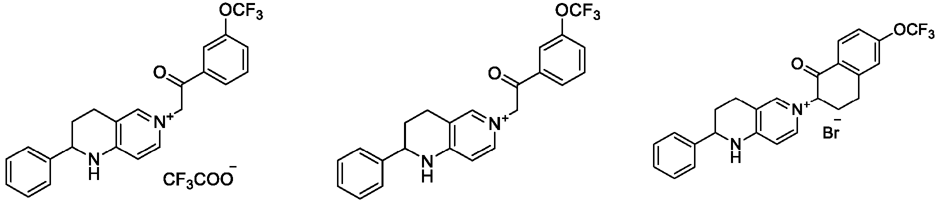

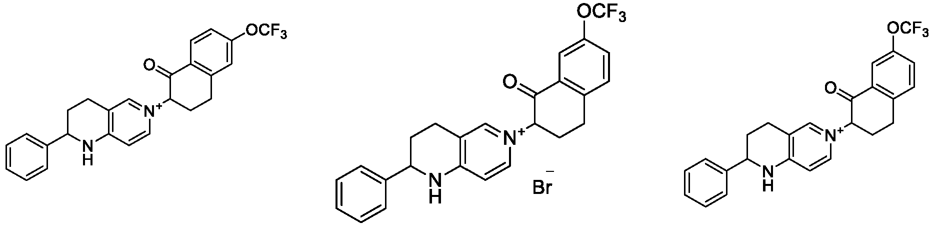

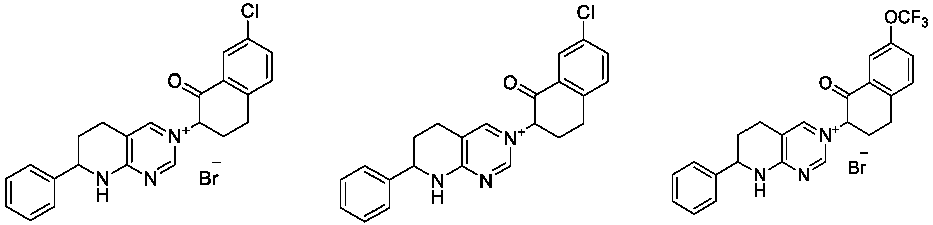

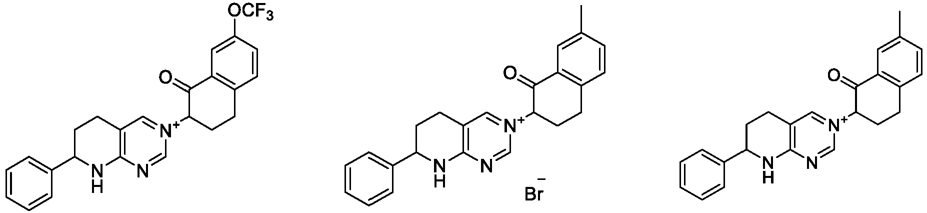

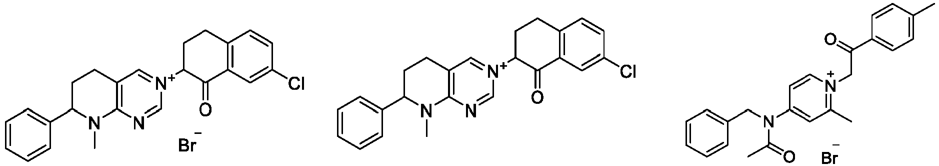

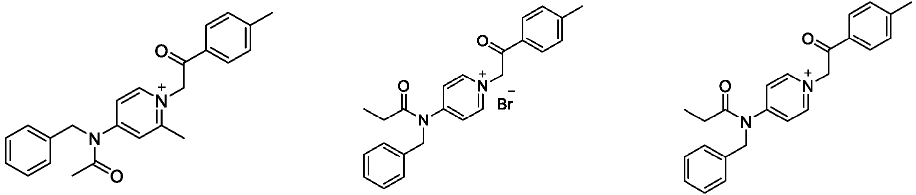

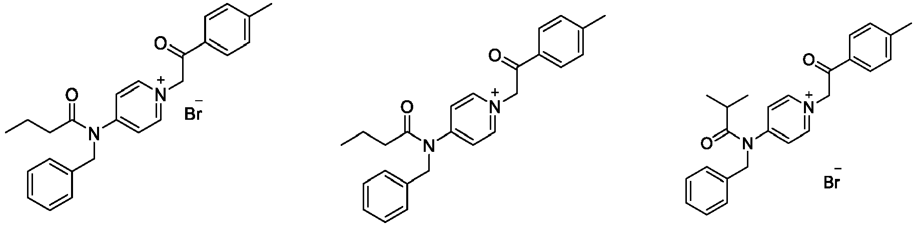

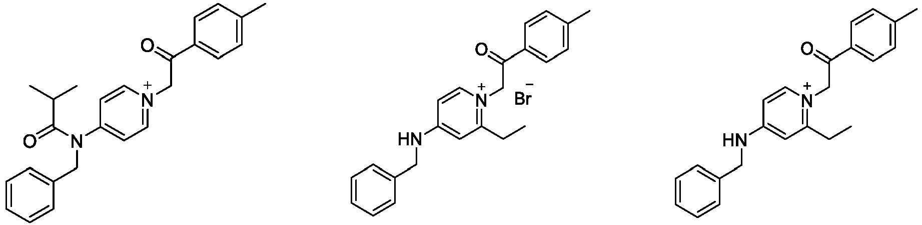

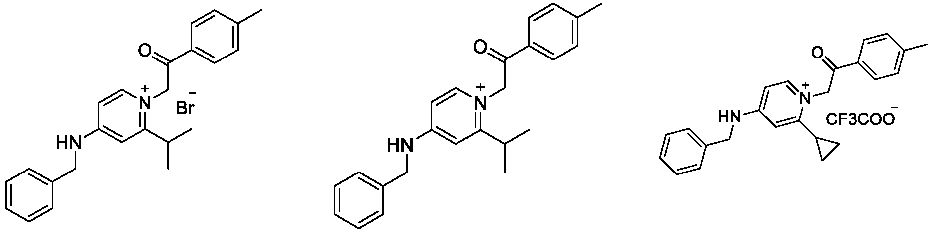

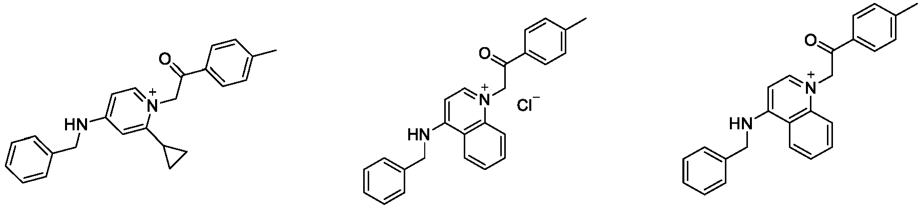

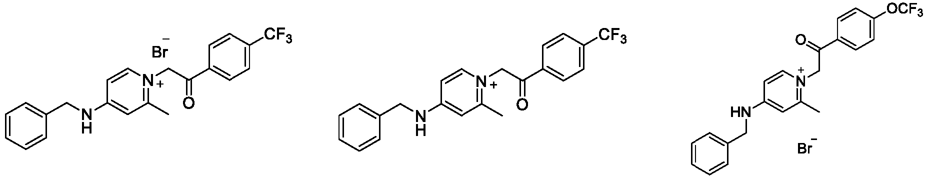

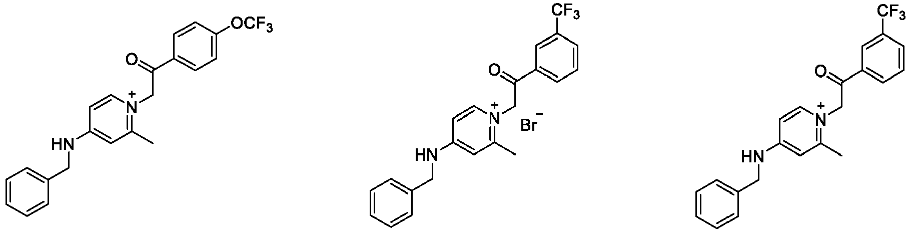

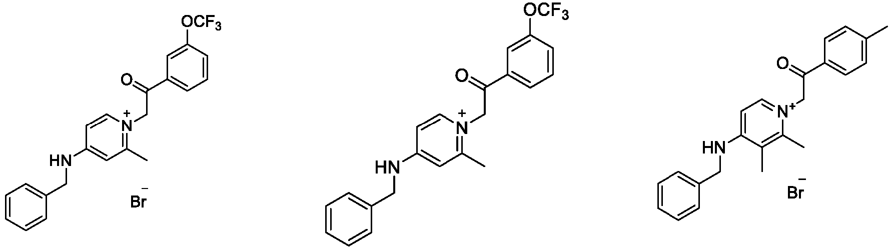

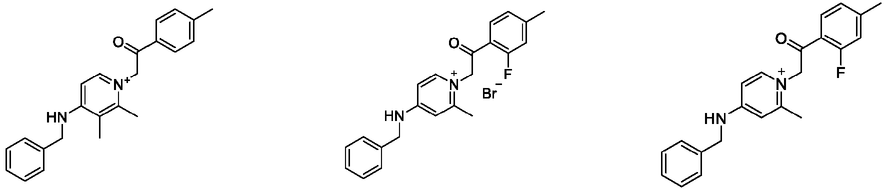

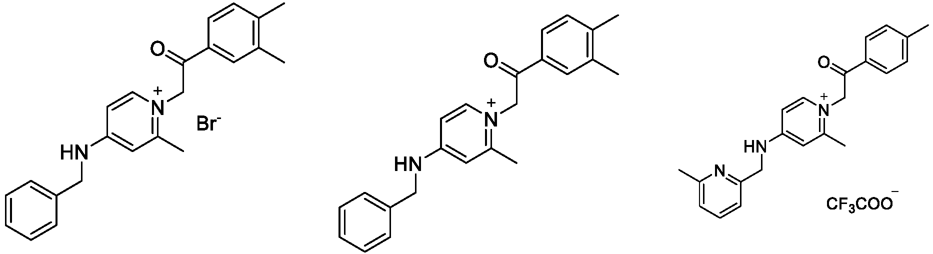

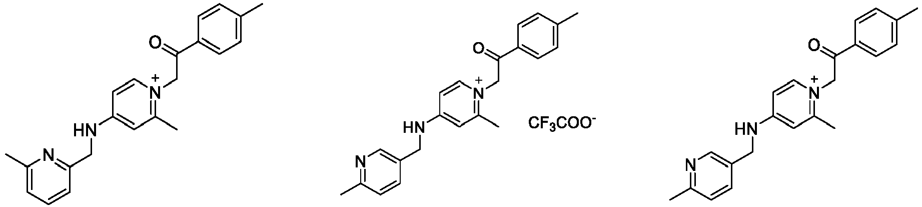

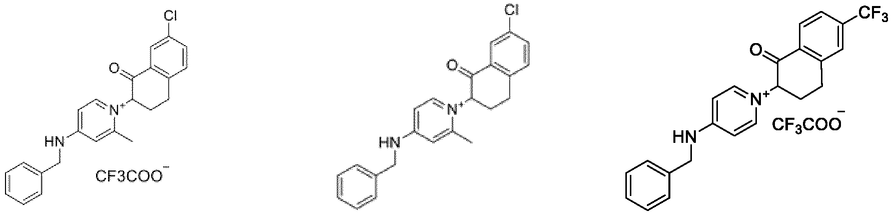

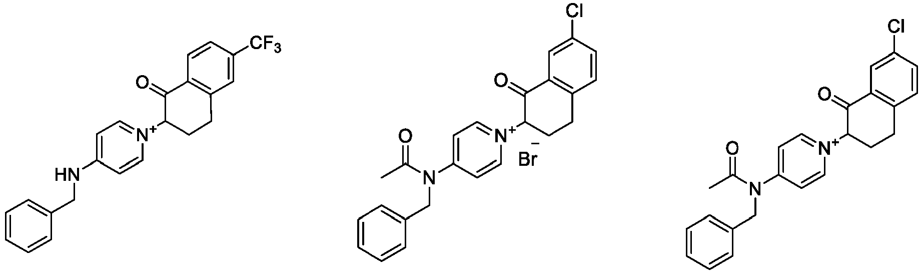

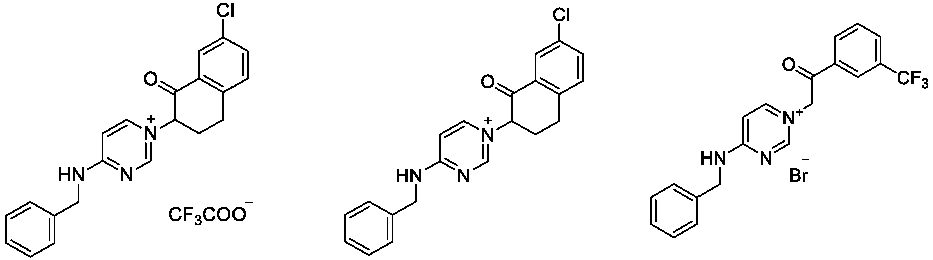

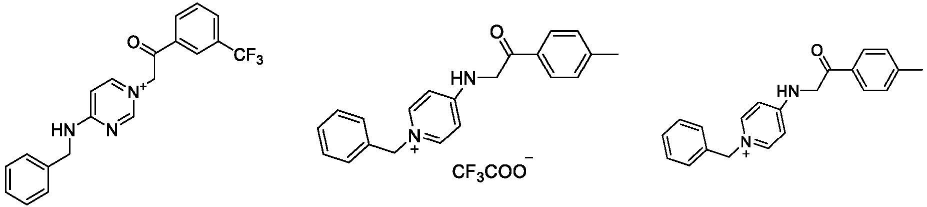

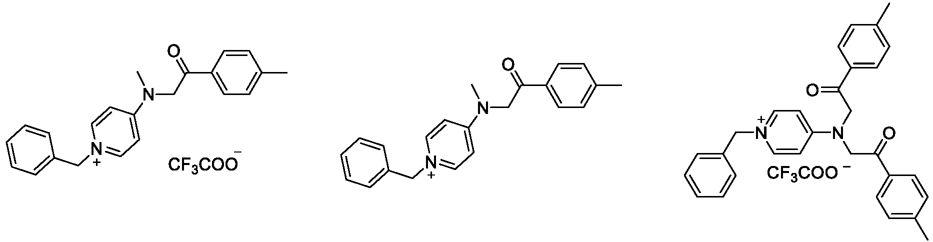

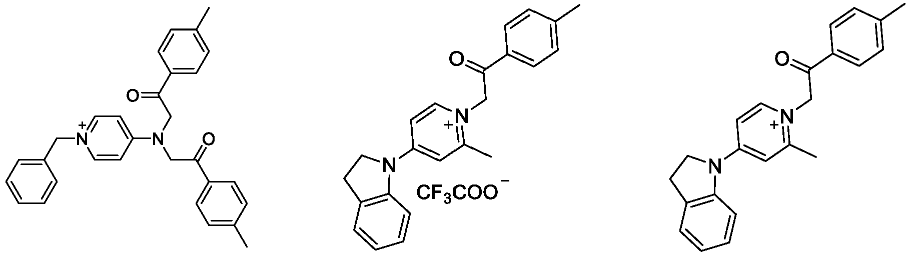

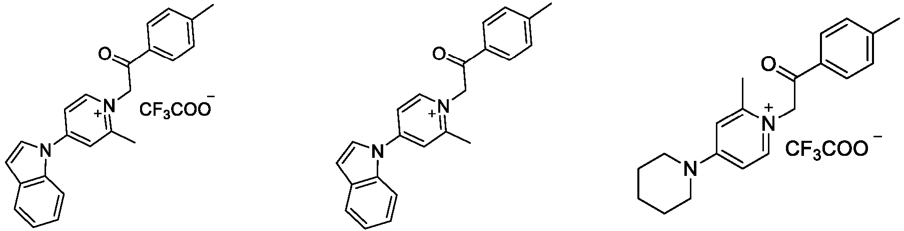

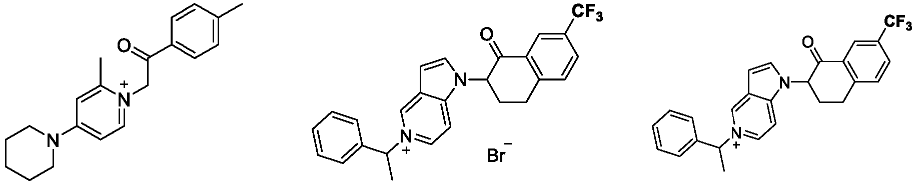

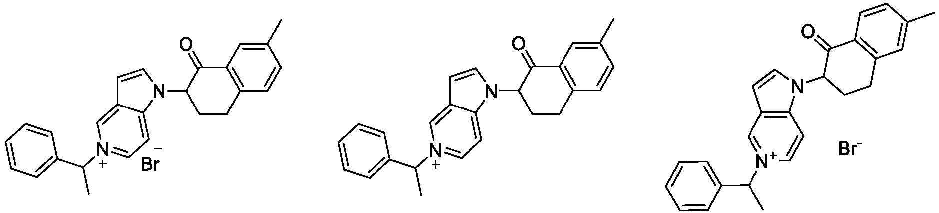

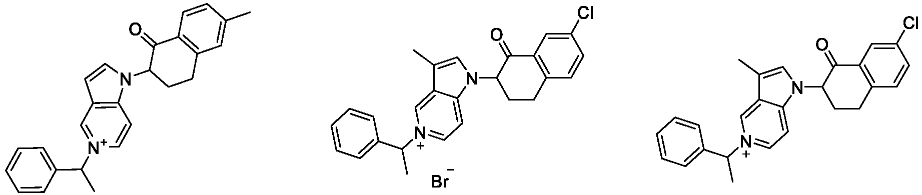

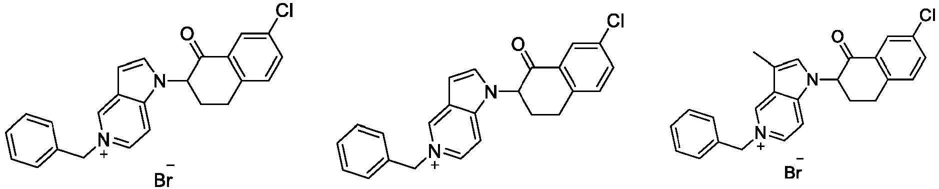

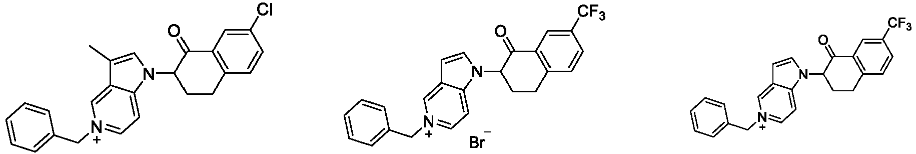

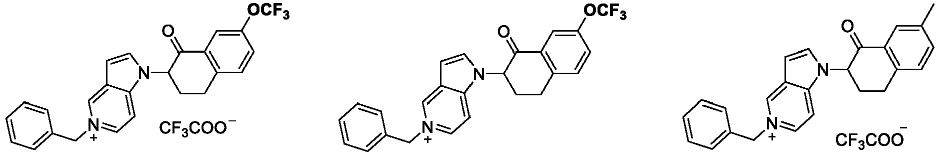

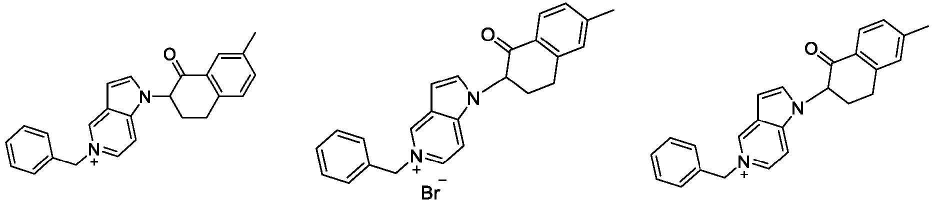

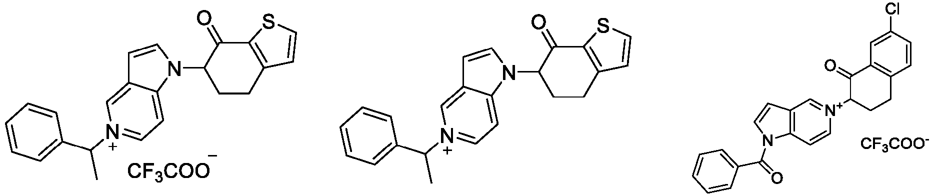

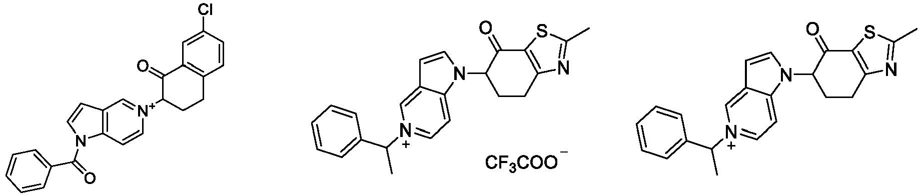

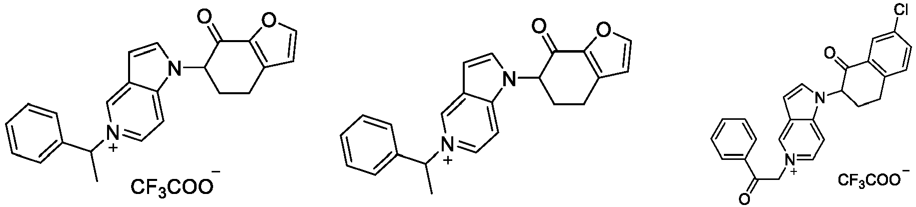

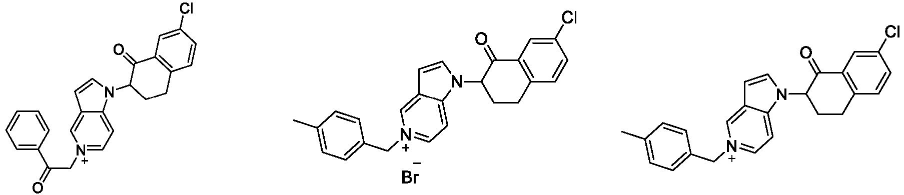

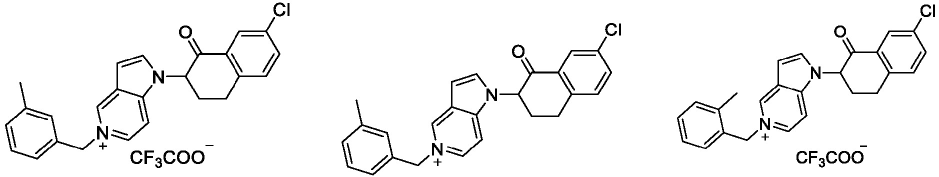

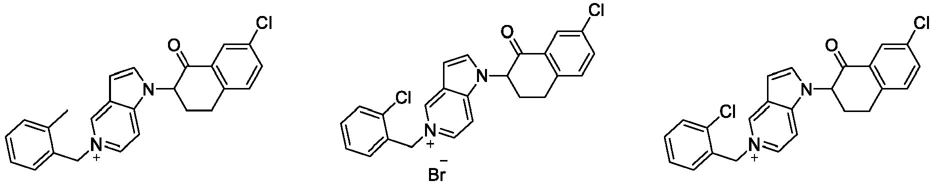

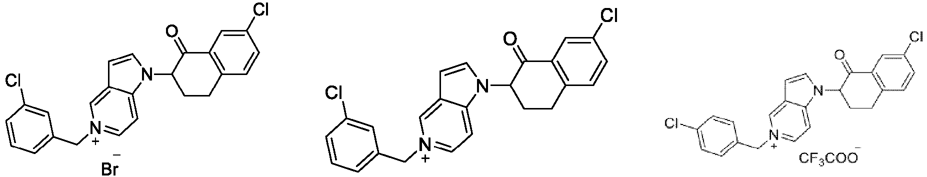

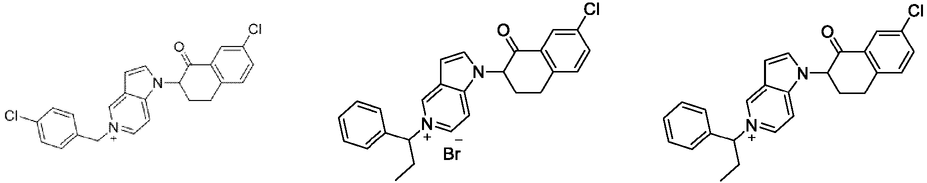

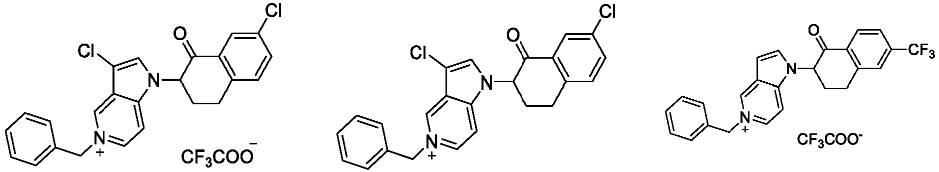

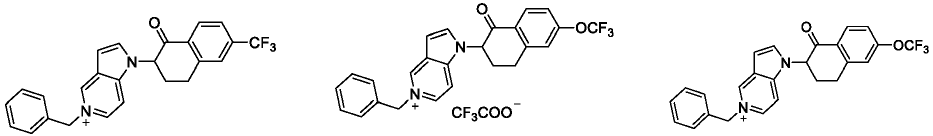

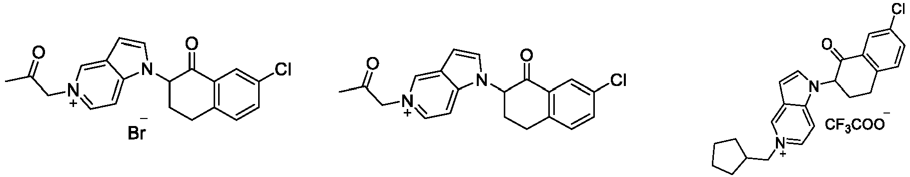

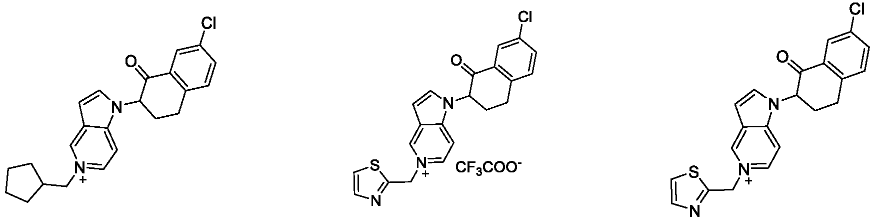

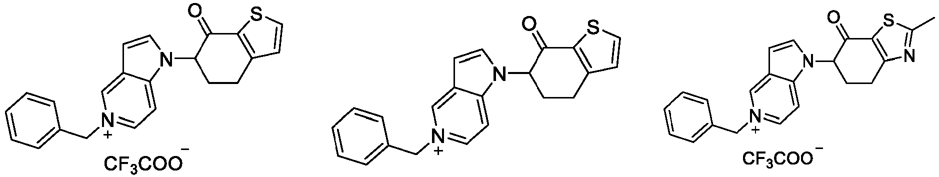

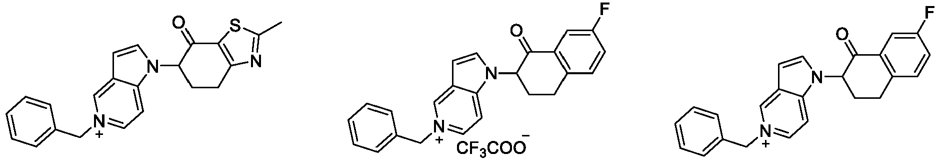

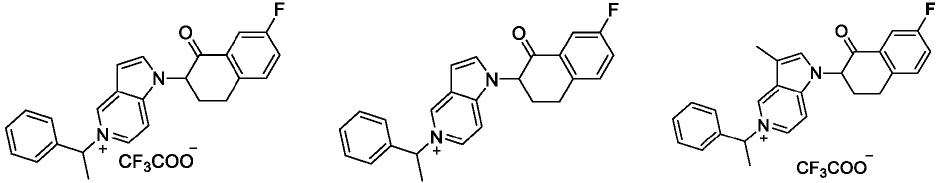

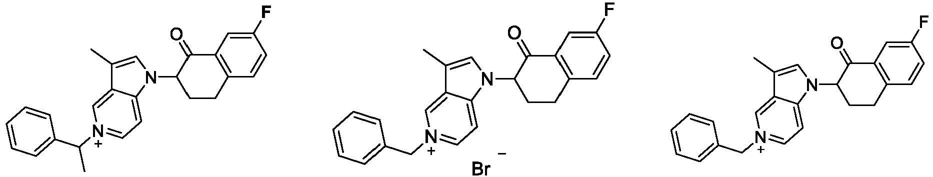

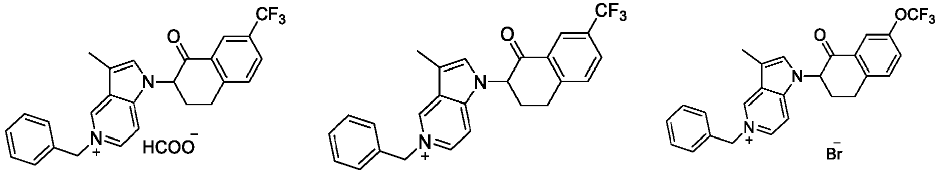

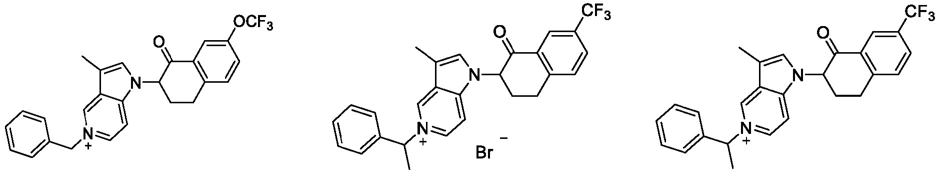

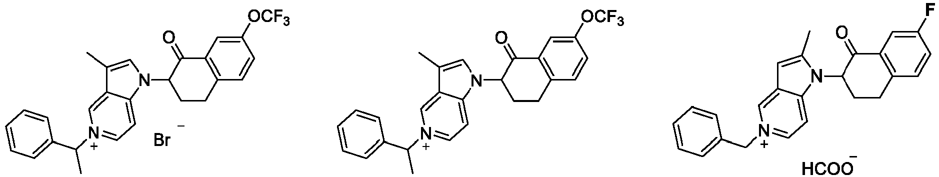

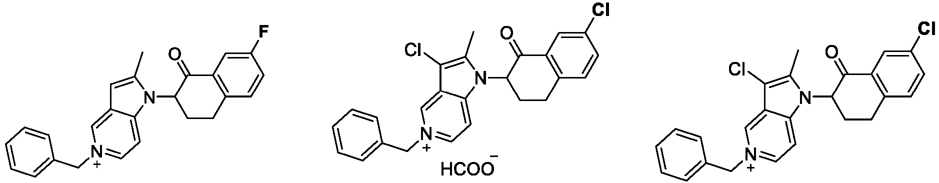

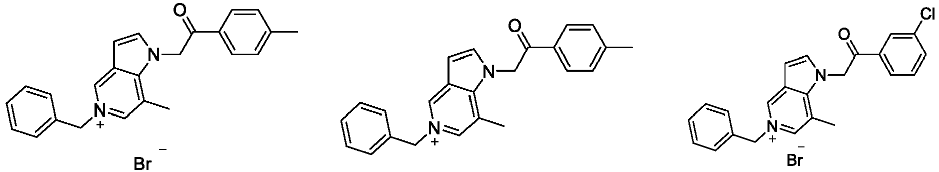

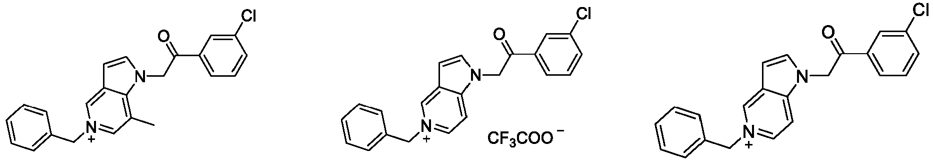

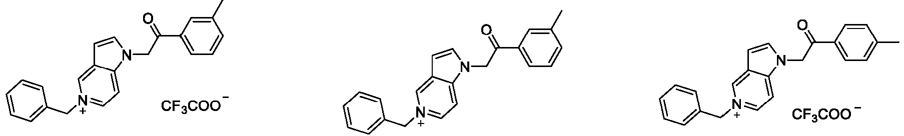

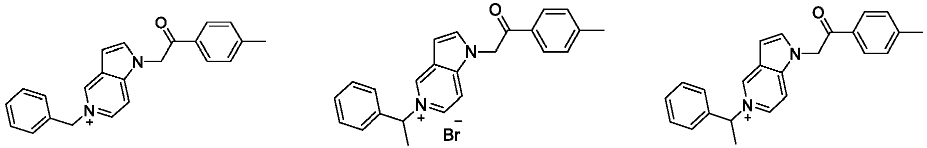

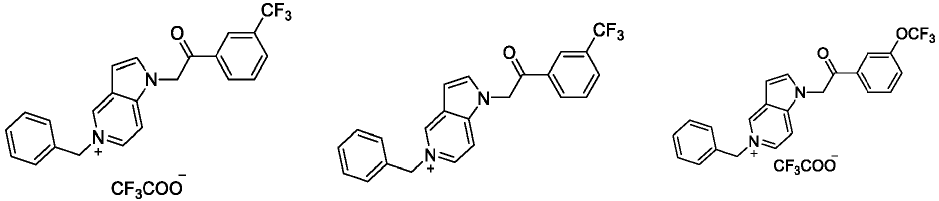

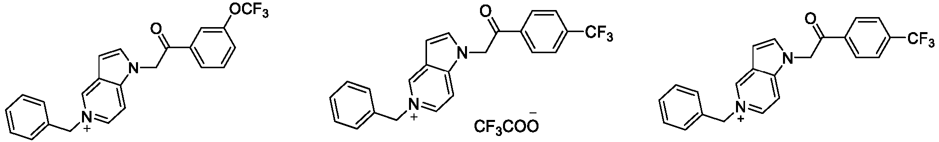

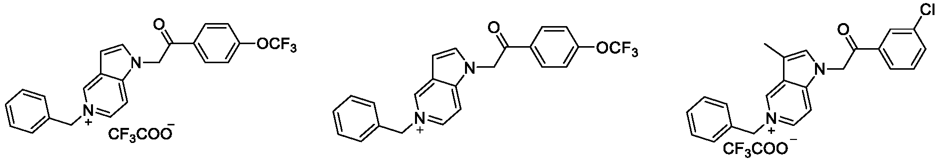

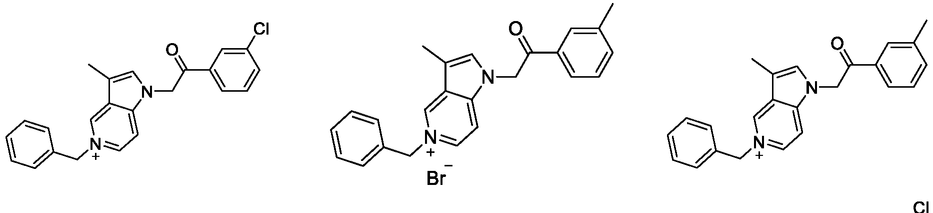

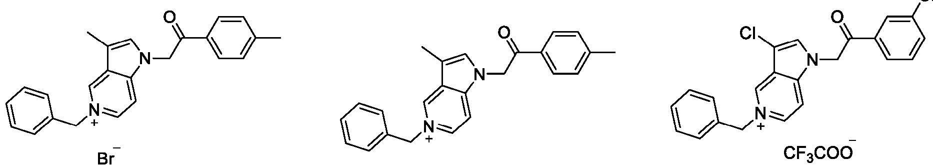

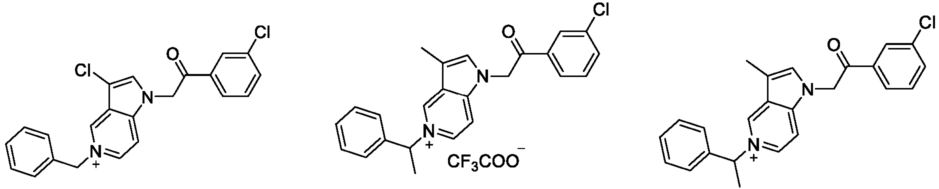

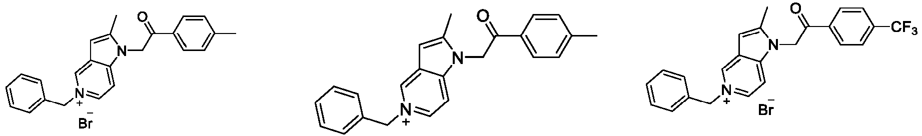

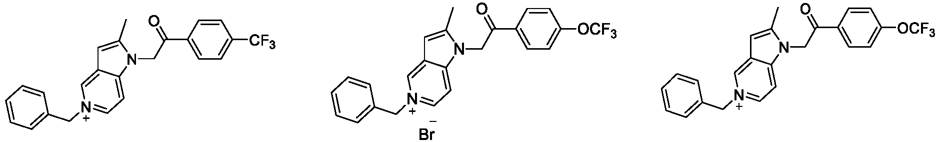

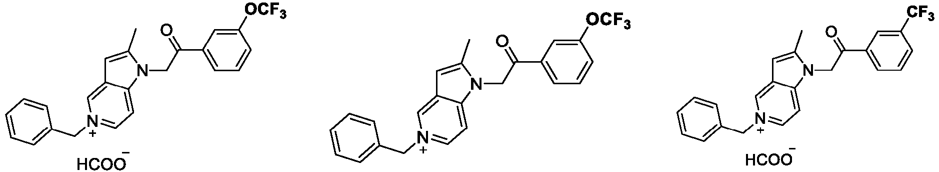

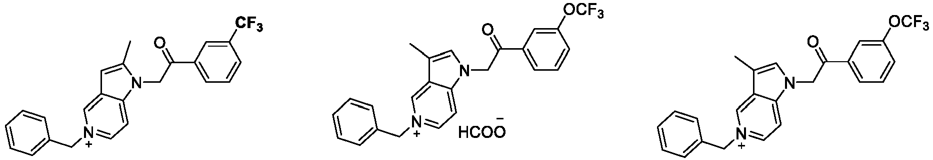

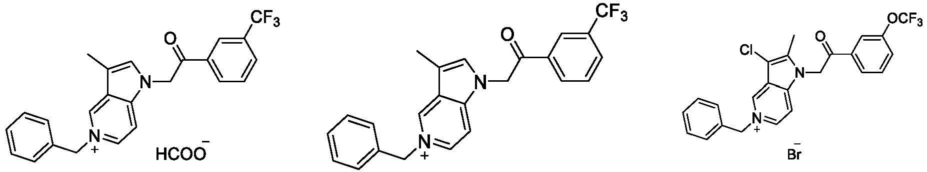

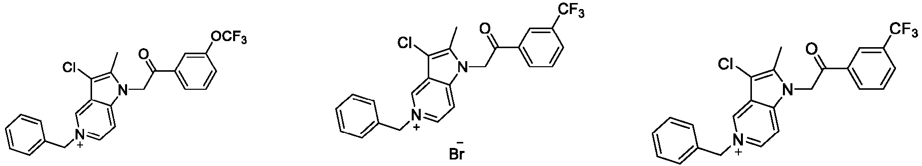

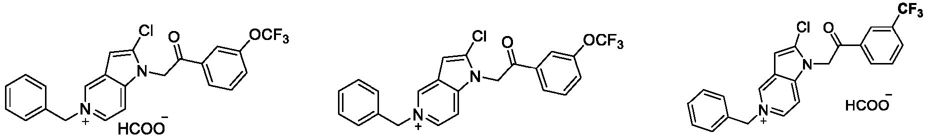

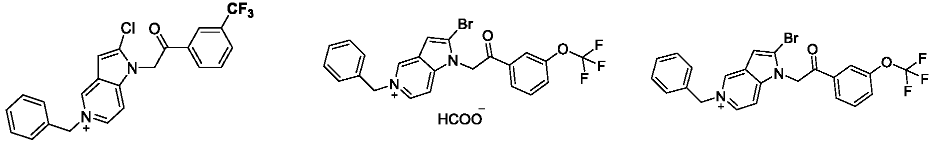

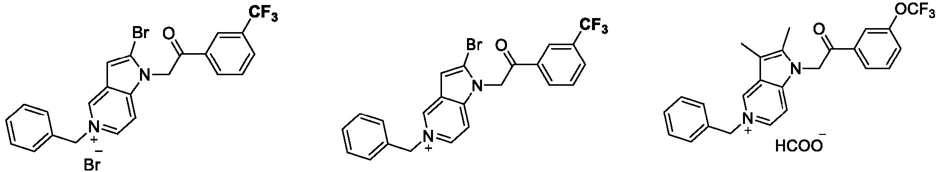

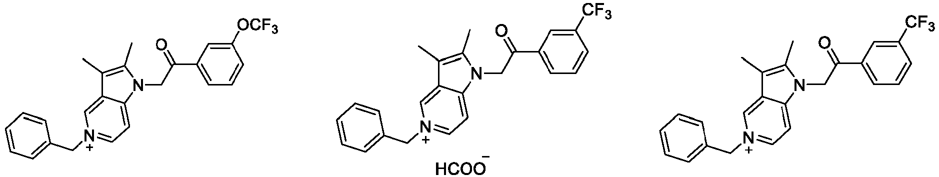

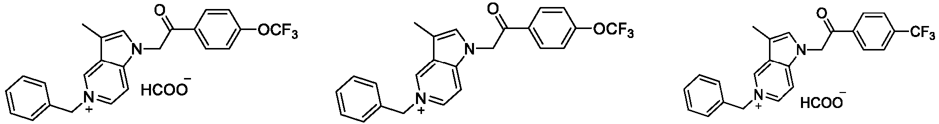

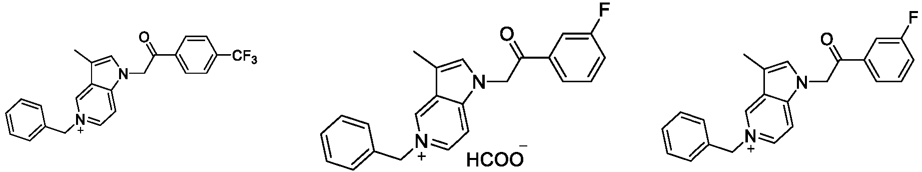

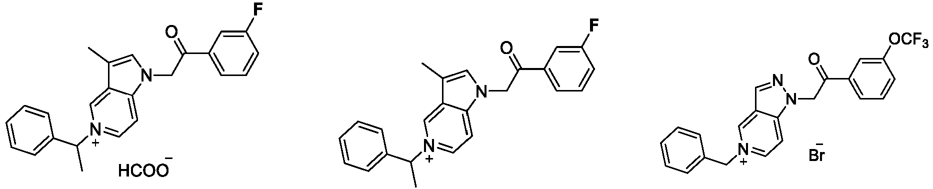

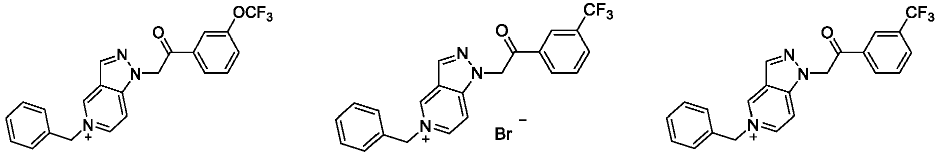

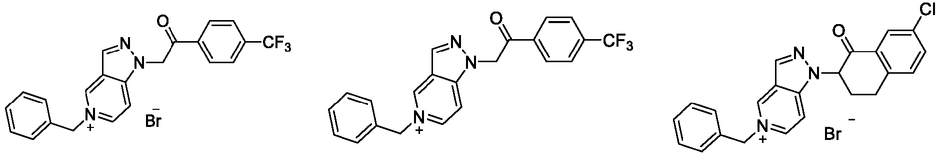

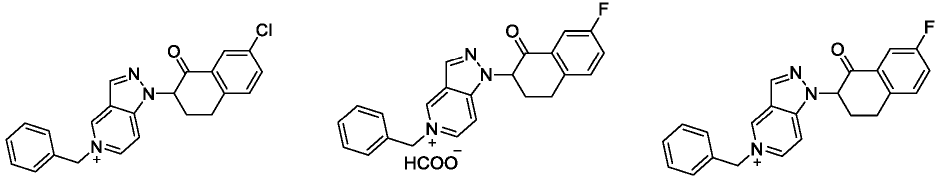

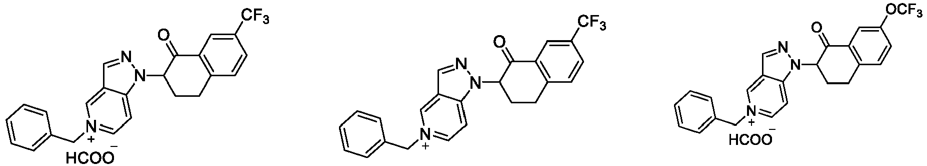

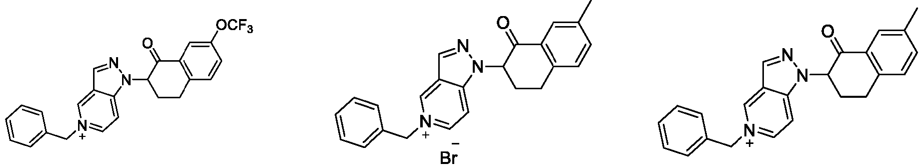

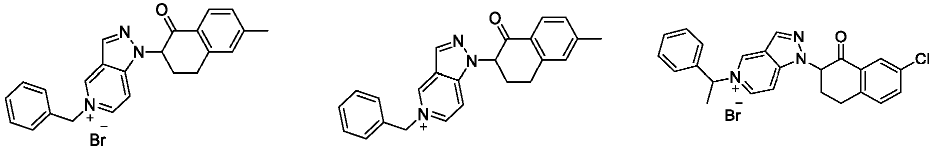

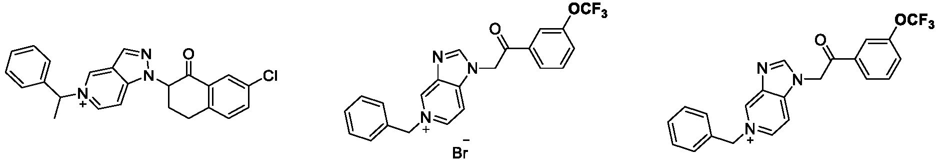

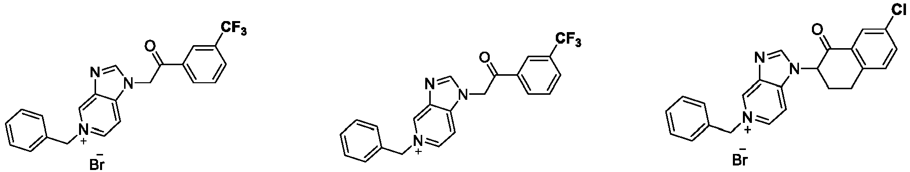

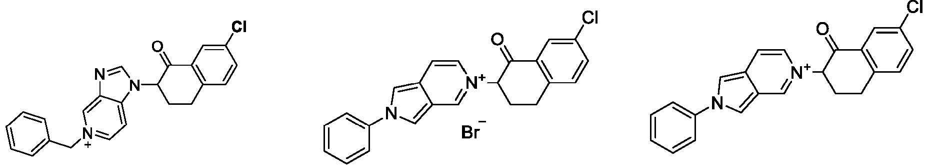

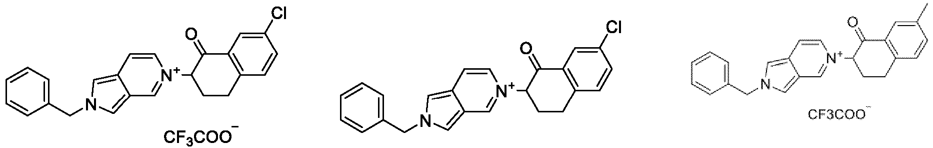

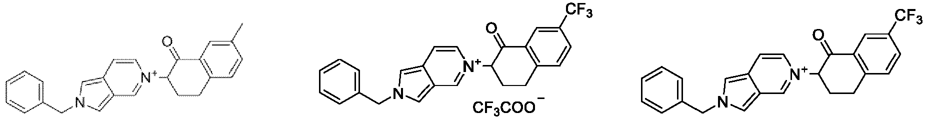

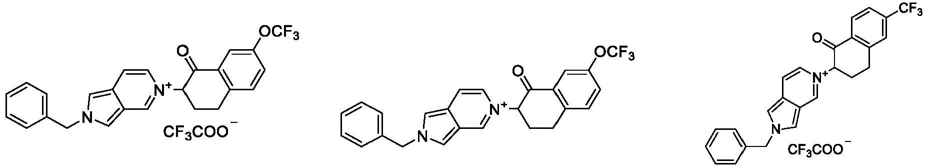

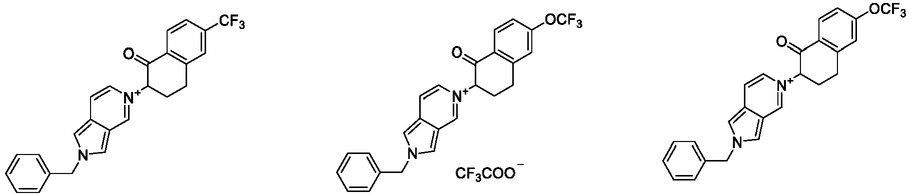

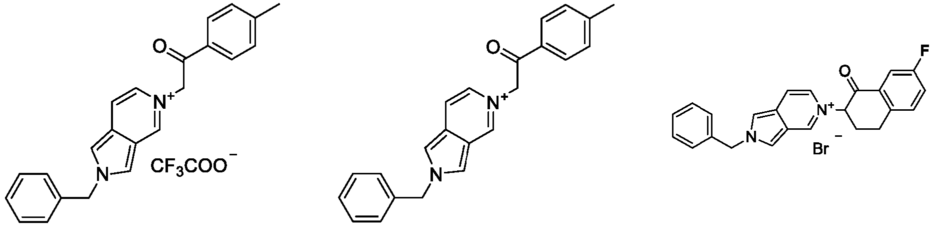

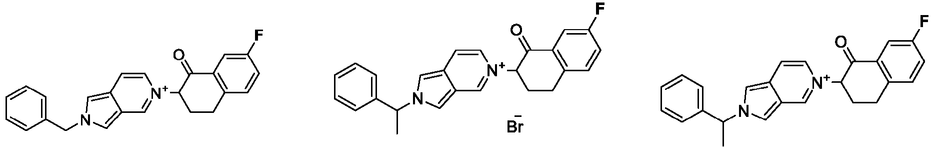

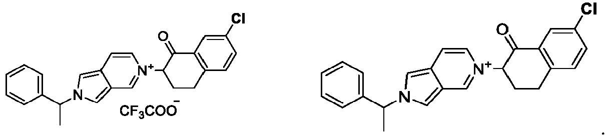

3. A composition, wherein the composition comprises (a) the compound of formula I, or an optical isomer thereof, or a racemate thereof, or a solvate thereof, or a pharmaceutically acceptable salt thereof, or a deuterated compound thereof according to the first aspect of the present invention.

4. A use of the compound of formula I, or an optical isomer thereof, or a racemate thereof, or a solvate thereof, or a pharmaceutically acceptable salt thereof, or a deuterated compound thereof of claim 1 in the preparation of a composition or a preparation for preventing and/or treating tumor.

5. The use of claim 4, wherein the tumor comprises tumor with low expression, no expression, low activity or no activity of mitochondria permeability transition pore;
the tumor comprises tumor with low expression, no expression, low activity or no activity of peptidyl-prolyl cis-trans isomerase F;
the tumor comprises tumor with low or no expression of NNMT gene;
the tumor comprises tumor with high expression of DNA methylase;
the tumor comprises tumor with high expression of UHRFl;
the tumor comprises tumor with high methylation level of nucleotide site of NNMT gene; and/or
the tumor comprises tumor with high methylation level of DNA CpG site of NNMT gene.

6. The use of claim 5, wherein the low expression or low activity of mitochondria permeability transition pore means the ratio (H1/H0) of the expression level or activity level Hlof mitochondria permeability transition pore in a cell ( e.g., tumor cell) to the expression level or activity level H0 of mitochondria permeability transition pore in the same type of cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low expression or low activity of peptidyl-prolyl cis-trans isomerase F means the ratio (G1/G0) of the expression level or activity level Glof peptidyl-prolyl cis-trans isomerase F in a cell ( e.g., tumor cell) to the expression level or activity level G0 of peptidyl-prolyl cis-trans isomerase F in the same type of cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low or no expression of NNMT gene means the ratio (E1/E0) of the expression E1 of NNMT gene in a cell ( e.g., tumor cell ) to the expression E0 of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the tumor with high expression of DNA methylase means the ratio (A1/A0) of the expression level A1 of DNA methylase in the tumor cell to the expression level A0 of DNA methylase in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of UHRF1 means the ratio (F1/F0) of the expression level F1 of UHRF1 in the tumor cell to the expression level F0 of UHRF1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the high methylation level of nucleotide site of NNMT gene means the ratio (L1/L0) of the methylation level L1 of nucleotide site of NNMT gene in a cell ( e.g., tumor cell) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50; and/or
the high methylation level of DNA CpG site of NNMT gene means the ratio (W1/W0) of the methylation level W1 of DNA CpG site of NNMT gene in a cell (e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

7. The use of claim 6, wherein the same type of cell comprises the cell (e.g., the same type of tumor cell) with normal expression, high expression, normal activity or high activity of mitochondria permeability transition pore;
the same type of cell comprises the cell (e.g., the same type of tumor cell) with normal expression, high expression, normal activity or high activity of peptidyl-prolyl cis-trans isomerase F;
the same type of cell comprises the cell (e.g., the same type of tumor cell) with normal or high expression of NNMT gene;
the same type of cell comprises the cell (e.g., the same type of tumor cell) with normal or low expression of DNA methylase;
the same type of cell comprises the cell (e.g., the same type of tumor cell) with normal or low expression of UHRF1;
the same type of cell comprises the cell (e.g., the same type of tumor cell) with normal or low methylation level of nucleotide site of NNMT gene; and/or
the same type of cell comprises the cell (e.g., the same type of tumor cell) with normal or low methylation level of DNA CpG site of NNMT gene.

8. The use of claim 4, wherein the tumor is selected from the group consisting of lung cancer, renal carcinoma, breast cancer, colon cancer, lymphoma, leukemia, pancreatic cancer, brain tumor, liver cancer, prostate cancer, and combinations thereof.

9. A marker for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

10. A use of a detection kit in the preparation of concomitant diagnose kit for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor;
the detection kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

11. The use of claim 10, wherein the concomitant diagnose kit further comprises instruction or label, the instruction or label records as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT genel; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

12. A medicine kit, wherein the medicine kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof of claim 1.

13. A use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug;
the anti-tumor drug is the compound of formula I, or an optical isomer thereof, or a racemate thereof, or a solvate thereof, or a pharmaceutically acceptable salt thereof, or a deuterated compound thereof of claim 1.

14. The use of claim 13, wherein the tumor is the tumor of any one of claims 5-8.

15. A composition, wherein the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.
